# EUROPEAN PATENT APPLICATION

(11) **EP 4 799 696 A2**
(43) Date of publication of application: **02.09.2026**
(21) Application number: 26193065.5
(22) Date of filing: 15.03.2023
(51) Int. Cl.: A61P 27/02

(54) **EXTENDED, HIGH DOSE VEGF ANTAGONIST REGIMENS FOR TREATMENT OF ANGIOGENIC EYE DISORDERS**

(30) Priority: 15.03.2022 US 202263319865 P; 07.09.2022 US 202263404511 P; 08.09.2022 US 202263404889 P; 29.09.2022 US 202263411589 P; 30.09.2022 US 202263412158 P; 01.11.2022 US 202263421296 P; 22.12.2022 US 202263434918 P; 09.02.2023 US 202363444470 P; 22.02.2023 US 202363447577 P; 22.02.2023 CA 3190726
(62) Document number(s) of the earlier application(s) in accordance with Art. 76 EPC: 23162091.5 / 4 245 313
(71) Applicant: Regeneron Pharmaceuticals, Inc., Tarrytown, NY 10591-6707 (US); Bayer Healthcare LLC, Whippany, NJ 07981 (US)
(72) Inventor: VITTI, Robert L, New Jersey, 07675 (US); BERLINER, Alyson J, New York, 10025 (US); CHU, Karen, New York, 10605 (US); ASMUS, Friedrich, Berlin, 14129 (US); DA SILVA LEAL, Sérgio Casimiro, 4153 Reinach (CH); EIßING, Thomas, 40764 Langenfeld (DE); RITTENHOUSE, Kay D, New Jersey, 07869 (US)
(74) Representative: J A Kemp LLP

(57) **Abstract**

The present invention relates to regimens for the treatment of angiogenic eye disorders such as DR and DME characterized by high doses of aflibercept and lengthening intervals between doses.

## Description

This application is related to U.S. provisional patent application no. 63/319,865, filed on March 15, 2022; U.S. provisional patent application no. 63/404,511, filed on September 7, 2022; U.S. provisional patent application no. 63/404,889, filed on September 8, 2022; U.S. provisional patent application no. 63/411,589, filed on September 29, 2022; U.S. provisional patent application no. 63/412,158, filed on September 30, 2022; U.S. provisional patent application no. 63/421,296, filed on November 1, 2022; U.S. provisional patent application no. 63/434,918, filed on December 22, 2022; U.S. provisional patent application no. 63/444,470, filed on February 9, 2023; US provisional patent application no. 63/447,577, filed February 22, 2023; and Canadian Patent application No. 3,190,726, filed February 22, 2023; each of which is herein incorporated by reference in its entirety.

### REFERENCE TO A SEQUENCE LISTING

This application incorporates by reference a computer readable Sequence Listing in ST.26 XML format, titled 11192WO01_Sequence, created on March 13, 2023, and containing 5,464 bytes.

### FIELD OF THE INVENTION

The field of the present invention relates to methods for treating or preventing angiogenic eye disorders by administering a VEGF antagonist.

### BACKGROUND OF THE INVENTION

Diabetic retinopathy (DR) is the most common microvascular complication of diabetes. Diabetic macular edema (DME), a manifestation of diabetic retinopathy, is the primary cause of vision loss and blindness in subjects with diabetes and the most frequent cause of blindness in young and middle-aged adults. If left untreated, approximately half of subjects with DME will lose 2 or more lines of visual acuity (VA) within 2 years. Among subjects with diabetes, the prevalence of clinically significant macular edema (CSME) ranges from 2.77% to 7.6%.

Intravitreally (IVT) administered anti-vascular endothelial growth factor (VEGF) therapies like EYLEA^{®} inhibit neovascular vessel growth and leakage in the retina, and they are currently the standard-of-care for subjects with DME. They not only maintain visual function but also provide clinically meaningful visual gains. Treatment of DME is chronic and life-long in most subjects to suppress retinal edema and recurrences of choroidal neovascularization (CNV). Although the currently approved IVT anti-VEGF therapies are efficacious and well-tolerated, the need for IVT injections every 4 to 8 weeks, specifically in the initial phase and during maintenance of treatment, represents a significant burden to physicians, subjects, and caregivers. While the procedure is straightforward and relatively easy to perform, capacity issues for ensuring an appropriate injection frequency in order to achieve subject outcomes similar to those seen in the pivotal studies represent an increasing challenge to individual practices and the healthcare system, overall. Moreover, high frequency dosing leads to increased burdens on subjects, e.g., to find transportation and miss work. A secondary effect of this burden is a lower probability of non-compliance with the prescribed treatment regimen.

While the efficacy and safety of currently approved VEGF antagonist therapies have been established for the treatment of DME, there remains an unmet medical need for the development of therapies with the potential to reduce treatment burden while providing at least similar or even improved visual outcomes over currently available standard-of-care.

Increasing the molar concentration of VEGF antagonist therapeutic protein in the dosing formulation is a potential way to bring further benefits to subjects with chorioretinal vascular diseases, including DME. A higher dose of aflibercept administered IVT has the potential to prolong the drug's therapeutic effects. The resulting extension of treatment intervals early after the initiation of treatment to every 12 weeks or 16 weeks would reduce the number of injections in the first treatment year. A potential decrease in injection-related treatment burden and safety events with fewer injections could be a significant contribution to subject care and healthcare services.

EYLEA (2 mg dose, administered at a concentration of 40 mg/mL, also called intravitreal aflibercept injection [IAI]) is currently approved in the United States (US) for the treatment of nAMD, and is also approved for the treatment of macular edema following retinal vein occlusion (RVO), diabetic macular edema (DME), and diabetic retinopathy (DR).

### SUMMARY OF THE INVENTION

The present invention provides methods for treating or preventing diabetic retinopathy and/or diabetic macular edema comprising administering one or more doses (*e.g*., of ≥8 mg) of aflibercept such that the clearance of free aflibercept from the ocular compartment is about 0.367-0.458 mL/day (*e.g*., 0.41 mL/day) after an intravitreal injection of aflibercept and the time for the amount for free aflibercept to reach the lower limit of quantitation (LLOQ) in the ocular compartment of a subject after said intravitreal injection of aflibercept is about 15 weeks; and the time for free aflibercept to reach the lower limit of quantitation (LLOQ) in the plasma (*e.g*., about 0.0156 mg/L) of a subject after said intravitreal injection of aflibercept is about 3.5 weeks; for example, wherein the aflibercept is administered in an aqueous pharmaceutical formulation wherein the aflibercept has less than about 3.5% high molecular weight species immediately after manufacture and purification and/or less than or equal to about 6% high molecular weight species after storage for about 24 months at about 2-8°C. In an embodiment of the invention, the aqueous pharmaceutical formulation comprises an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C. In an embodiment of the invention, the method comprises administering a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 (preferably 4) weeks after the immediately preceding dose; and wherein each tertiary dose is administered about 12-20 (preferably 12, 16 or 20) weeks after the immediately preceding dose.

The present invention provides a method for slowing the clearance of free aflibercept from the ocular compartment after an intravitreal injection relative to the rate of clearance of aflibercept from the ocular compartment after an intravitreal injection of ≤ 4 mg aflibercept comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose. In an embodiment of the invention, the clearance of free aflibercept from the ocular compartment is about 34% slower than that from the ocular compartment after an intravitreal injection of ≤ 4 mg aflibercept, *e.g*., wherein the clearance of free aflibercept from the ocular compartment is about 0.367-0.458 mL/day or 0.41 mL/day after an intravitreal injection of ≥ 8 mg aflibercept.

The present invention also provides a method for increasing the time for the amount of free aflibercept to reach the lower limit of quantitation (LLOQ) in the ocular compartment of a subject after an intravitreal injection of aflibercept relative to the time to reach LLOQ of the amount of free aflibercept in the ocular compartment of a subject after an intravitreal injection of about 2 mg aflibercept, *e.g*., increasing by greater than 1.3 weeks, for example, by about 6 weeks-to more than 10 weeks, for example, to about 15 weeks, comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose.

The present invention also provides a method for increasing the time for free aflibercept to reach the lower limit of quantitation (LLOQ) in the plasma (*e.g*., about 0.0156 mg/L) of a subject after an intravitreal injection of aflibercept relative to the time to reach LLOQ of free aflibercept in the plasma of a subject after an intravitreal injection of about 2 mg aflibercept, *e.g*., increased by more than 1.5 weeks, for example by about 2 weeks-to about 3.5 weeks, comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose. In an embodiment of the invention, the ≥8 mg aflibercept is administered in an aqueous pharmaceutical formulation including aflibercept which includes one or more of histidine-based buffer, arginine (*e.g.*, L-arginine, for example, L-arginine HCl), a sugar or polyol such as sucrose and having a pH of about 5.8. In an embodiment of the invention, the aflibercept has less than about 3.5% high molecular weight species immediately after manufacture and purification and/or less than or equal to about 6% high molecular weight species after storage for about 24 months at about 2-8°C; for example, wherein the ≥8 mg aflibercept is in an aqueous pharmaceutical formulation comprising an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

The present invention provides a method for treating or preventing diabetic retinopathy (DR) and/or diabetic macular edema (DME), in a subject in need thereof, for improving best corrected visual acuity (BCVA) in a subject in need thereof with DR and/or DME; or for promoting retinal drying in a subject with DR and/or DME in need thereof; comprising administering to an eye of the subject, one or more doses of about 8 mg or more of VEGF receptor fusion protein, preferably aflibercept, once every 12, 13, 14, 15, 16, 17, 18, 19 or 20 or 12-20 or 12-16 or 16-20 weeks. In an embodiment of the invention, the method comprises
administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12-20 weeks after the immediately preceding dose.

The present invention includes a method for treating or preventing diabetic retinopathy (DR) and/or diabetic macular edema (DME), in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 weeks after the immediately preceding dose.

The present invention also includes a method for treating or preventing diabetic retinopathy (DR) and/or diabetic macular edema (DME), in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 16 weeks after the immediately preceding dose.

The present provides a method for treating or preventing diabetic retinopathy (DR) and/or diabetic macular edema (DME), in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 20 weeks after the immediately preceding dose.

In an embodiment of the invention, the method for treating or preventing DR and/or DME in a subject comprises comprising administering, to a subject in need thereof, 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days). In an embodiment of the invention, the method comprises administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days). In an embodiment of the invention, the method comprises administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days). In an embodiment of the invention, the method comprises administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (+/- 7 days).

The present invention also provides a method for treating or preventing diabetic retinopathy and/or diabetic macular edema, in a subject in need thereof: (1) wherein the subject has received an initial 2 mg dose of VEGF receptor fusion protein, then the method comprises, after 1 month, administering to the subject the initial 8 mg dose of VEGF receptor fusion protein and, 1 month thereafter, the 1^{st} 8 mg secondary dose of VEGF receptor fusion protein; and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (2) wherein the subject has received an initial 2 mg dose of VEGF receptor fusion protein, then the method comprises, after 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (3) wherein the subject has received an initial 2 mg dose of VEGF receptor fusion protein, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (4) wherein the subject has received an initial 2 mg dose of VEGF receptor fusion protein, then the method comprises, after 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; (5) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (6) wherein the subject has received an initial 2 mg dose of VEGF receptor fusion protein and a 1^{st} 2 mg secondary dose of VEGF receptor fusion protein after 1 month, then the method comprises, after another 1 month, administering to the subject a first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (7) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (8) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; (9) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (10) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (11) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (12) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen; (13) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the initial 8 mg dose of VEGF receptor fusion protein and 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (14) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the first 8 mg secondary dose of VEGF receptor fusion protein and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (15) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (16) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 2 months, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; (17) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; thereafter, then the method comprises, after 2 months, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (18) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; thereafter, then the method comprises, after 2 months, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (19) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month; thereafter, then the method comprises, after 2 months, administering to the subject the 2^{nd} 8 mg secondary dose of VEGF receptor fusion protein and, 12 or 16 or 20 weeks thereafter, one or more 12 or 16 weekly 8 mg maintenance doses of VEGF receptor fusion protein according to the HDq12 or HDq16 or HDq20 dosing regimen; (20) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month, thereafter, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen; (21) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (22) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (23) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 2^{nd} 8 mg secondary dose of VEGF receptor fusion protein and, 12 or 16 or 20 weeks thereafter, one or more 12 or 16 or 20 weekly 8 mg maintenance doses of VEGF receptor fusion protein according to the HDq12 or HDq16 or HDq20 dosing regimen; or (24) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen; wherein, (i) said HDq12 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 weeks after the immediately preceding dose; (ii) said HDq16 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 16 weeks after the immediately preceding dose; and (iii) said HDq20 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 20 weeks after the immediately preceding dose-preferably, wherein the VEGF receptor fusion protein is aflibercept.

The present invention also provides a method for treating or preventing diabetic retinopathy and/or diabetic macular edema, in a subject in need thereof who has been on a dosing regimen for treating or preventing said disorder wherein: (a) the subject has received an initial 8 mg dose of VEGF receptor fusion protein then the method comprises, after 1 month, administering to the subject the first 8 mg secondary dose of VEGF receptor fusion protein and 1 month thereafter, administering the 2^{nd} 8 mg secondary dose of VEGF receptor fusion protein; and then, every 12 or 16 or 20 weeks thereafter, administering one or more 8 mg maintenance doses of VEGF receptor fusion protein according to the HDq12 or HDq16 or HDq20 dosing regimen; or (b) the subject has received an initial 8 mg dose of VEGF receptor fusion protein & 1^{st} 8 mg secondary dose of VEGF receptor fusion protein after 1 month, then the method comprises, after another 1 month, administering to the subject the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; or (c) the subject has received an initial 8 mg dose of VEGF receptor fusion protein & 1^{st} 8 mg secondary dose of VEGF receptor fusion protein after 1 month & the 2^{nd} 8 mg secondary dose of VEGF receptor fusion protein after another month, then the method comprises, after 12 or 16 or 20 weeks administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; or (d) the subject has received an initial 8 mg dose of VEGF receptor fusion protein & a 1^{st} 8 mg secondary dose of VEGF receptor fusion protein after 1 month & the 2^{nd} 8 mg secondary dose of VEGF receptor fusion protein after another month, then every 12 or 16 or 20 weeks thereafter, the subject has received one or more 8 mg maintenance doses of VEGF receptor fusion protein; and, then the method comprises, after 12 or 16 or 20 weeks from the last maintenance dose of VEGF receptor fusion protein, administering to the subject one or more 8 mg maintenance doses of VEGF receptor fusion protein and all further 8 mg maintenance doses of VEGF receptor fusion protein every 12 or 16 or 20 weeks according to the HDq12 or HDq16 or HDq20 dosing regimen; wherein, (i) said HDq12 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 weeks after the immediately preceding dose; (ii) said HDq16 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 16 weeks after the immediately preceding dose; and (iii) said HDq20 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 20 weeks after the immediately preceding dose-preferably, wherein the VEGF receptor fusion protein is aflibercept.

The present invention also provides a method for treating or preventing an angiogenic eye disorder (preferably DR and/or DME), in a subject in need thereof who has been on a dosing regimen for treating or preventing the disorder calling for a single initial dose of about 2 mg of VEGF receptor fusion protein, preferably aflibercept, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen, comprising administering to an eye of the subject, an 8 mg dose of VEGF receptor fusion protein, evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with 8 mg VEGF receptor fusion protein; or evaluating the subject in about 8 or 10 or 12 weeks after said administering and, if, in the judgment of the treating physician dosing every 12 weeks is appropriate, then administering another 8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.

In an embodiment of the invention, a subject in a method of the present invention has been on a dosing regimen for treating or preventing diabetic retinopathy and/or diabetic macular edema of a single initial dose of about 2 mg of a VEGF receptor fusion protein, preferably aflibercept, followed by 4 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose.

The present invention also provides a method for treating or preventing diabetic retinopathy or diabetic macular edema, in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 or 16 weeks after the immediately preceding dose; further comprising, after receiving one or more of said tertiary doses about 12 or 16 after the immediately preceding dose, lengthening the tertiary dose interval from 12 weeks to 16 weeks; 12 weeks to 20 weeks; or 16 weeks to 20 weeks, after the immediately preceding dose. For example, in an embodiment of the invention, during said treatment, the subject exhibits (a) <5 letter loss in BCVA; and/or (b) central retinal thickness (CRT) <300 or 320 µm. In an embodiment of the invention, the method further comprises evaluating BVCA and/or CRT in the subject and, if the subject exhibits (a) <5 letter loss in BCVA; and/or (b) CRT <300 or 320 µm, lengthening the tertiary dose interval.

The present invention also provides a method treating or preventing diabetic retinopathy and/or diabetic macular edema, in a subject in need thereof, comprising administering to an eye of the subject, a single initial dose of about ≥8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 or 16 or 20 weeks after the immediately preceding dose; further comprising, after receiving one or more of said tertiary doses about 12 or 16 or 20 weeks after the immediately preceding dose, shortening the tertiary dose interval from 12 weeks to 8 weeks; 16 weeks to 12 weeks; 16 weeks to 8 weeks, 20 weeks to 8 weeks, 20 weeks to 12 weeks, or 20 weeks to 16 weeks. In an embodiment of the invention, during said treatment, the subject exhibits (a) > 10 letter loss in BCVA relative to baseline; and/or (b) > 50 µm increase in CRT relative to baseline. For example, in an embodiment of the invention, the method further comprises evaluating BVCA and/or CRT in the subject and, if the subject exhibits (a) > 10 letter loss in BCVA relative to baseline; and/or (b) > 50 µm increase in CRT relative to baseline, shortening the tertiary dose interval.

In an embodiment of the invention, if (a) greater than 5 letters are lost in BCVA (ETDRS), relative to the BCVA observed at about 12 weeks after treatment initiation; (b) a greater than 25 micrometers increase in CRT is observed relative to the CRT observed at about 12 weeks after treatment initiation; and/or (c) there is a new onset foveal neovascularization or foveal hemorrhage; *e.g*., at week 16 or week 20 after treatment initiation, then, the interval between tertiary doses is decreased from 12 weeks or 16 weeks to 8 weeks; or (a) greater than 5 letters are lost in BCVA (ETDRS), relative to the BCVA observed at about 12 weeks after treatment initiation; (b) a greater than 25 micrometers increase in CRT is observed relative to the CRT observed at about 12 weeks after treatment initiation; and/or (c) there is a new onset foveal neovascularization or foveal hemorrhage; *e.g*., at week 24 after treatment initiation, then, the interval between tertiary doses is decreased from 16 weeks to 12 weeks.

The present invention provides a method for treating or preventing diabetic retinopathy and/or diabetic macular edema, in a subject in need thereof, comprising administering to an eye of the subject 3 doses of about 8 mg VEGF receptor fusion protein, preferably aflibercept, in a formulation that comprises about 114.3 mg/ml VEGF receptor fusion protein at an interval of once every 4 weeks; wherein after said 3 doses, administering one or more doses of the VEGF receptor fusion protein at an interval which is lengthened up to 12, 16 or 20 weeks.

The present invention includes a method for treating or preventing diabetic retinopathy and/or diabetic macular edema, in a subject in need thereof, comprising administering to an eye of the subject, a single initial dose of about 8 mg or more of VEGF receptor fusion protein, preferably aflibercept, followed by 2 secondary doses of about 8 mg or more of the VEGF receptor fusion protein, wherein each secondary dose is administered about 2 to 4 (preferably 4) weeks after the immediately preceding dose; and, after said doses, a) determining if the subject meets at least one criterion for reducing or lengthening one or more intervals by 2 weeks, 3 weeks, 4 weeks or 2-4 weeks between doses of the VEGF receptor fusion protein; and b) if said determination is made, administering further doses of the VEGF receptor fusion protein at said reduced or lengthened intervals between doses wherein criteria for lengthening the interval include: 1. <5 letter loss in BCVA; and/or 2. CRT <300 or 320 micrometers; and, wherein criteria for reducing the interval include: 1. >10 letter loss in BCVA; 2. persistent or worsening DME; and/or 3. >50 micrometers increase in CRT. In an embodiment of the invention, wherein criteria for lengthening the interval include both: 1. <5 letter loss in BCVA from week 12; and 2. CRT <300 or 320 micrometers as measured by SD-O*CT; and*/*or wherein* criteria for reducing the interval include both: 1. >10 letter loss in BCVA, *e.g*., from week 12 in association with persistent or worsening DME; and 2. >50 micrometers increase in CRT, *e.g*., from week 12. In an embodiment of the invention, if said criteria are met, said interval is lengthened to 12, 16 or 20 weeks.

The present invention provides a method for treating or preventing diabetic retinopathy and/or diabetic macular edema, in a subject in need thereof that has been pre-treated with one or more 2 mg doses of VEGF receptor fusion protein, preferably aflibercept, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12-20 weeks after the immediately preceding dose.

The present invention provides a method for treating or preventing an angiogenic eye disorder, preferably DR and/or DME, in a subject in need thereof, comprising administering to an eye of the subject, (1) a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 8 weeks after the immediately preceding dose; or (2) one or more doses of 8 mg or more of VEGF receptor fusion protein about every 4 weeks.

In an embodiment of the invention, subjects having certain exclusion criteria are excluded from treatment or are not excluded from treatment if exclusion criteria are not met. For example, a subject having any one or more of ocular or periocular infection; active intraocular inflammation; and/or hypersensitivity; is excluded from administration of VEGF receptor fusion protein to the eye. In an embodiment of the invention, a method of the present invention further comprises a step of evaluating the subject for: ocular or periocular infection; active intraocular inflammation; and/or hypersensitivity; and excluding the subject from said administration if any one or more if found in the subject.

In an embodiment of the invention, subjects are monitored for adverse events, such as conjunctival hemorrhage, cataract, vitreous detachment, vitreous floaters, corneal epithelium defect and/or increased intraocular pressure. If such AEs are identified, the identified AE may be treated and/or such treatment or prevention may be ceased.

In an embodiment of the invention, a method includes preparation prior to administration of a VEGF receptor fusion protein, preferably aflibercept. For example, wherein the method comprises, prior to each administration, providing or having available- one single-dose glass vial having a protective plastic cap and a stopper containing an aqueous formulation comprising 8 mg VEGF receptor fusion protein in about 70 microliters; a filter needle, *e.g*., one 18-gauge x 1½-inch, 5-micron, filter needle that includes a tip and a bevel; an invention needle, *e.g*., one 30-gauge x ½-inch injection needle; and a syringe, *e.g*., one 1-mL Luer lock syringe having a graduation line marking for 70 microliters of volume; packaged together; then (1) visually inspecting the aqueous formulation in the vial and, if particulates, cloudiness, or discoloration are visible, then using another vial of aqueous formulation containing the VEGF receptor fusion protein; (2) removing the protective plastic cap from the vial; and (3) cleaning the top of the vial with an alcohol wipe; then using aseptic technique: (4) removing the 18-gauge x 1½-inch, 5-micron, filter needle and the 1 mL syringe from their packaging; (5) attaching the filter needle to the syringe by twisting it onto the Luer lock syringe tip; (6) pushing the filter needle into the center of the vial stopper until the needle is completely inserted into the vial and the tip touches the bottom or a bottom edge of the vial; (7) withdrawing all of the VEGF receptor fusion protein vial contents into the syringe, keeping the vial in an upright position, slightly inclined, while ensuring the bevel of the filter needle is submerged into the liquid; (8) continuing to tilt the vial during withdrawal keeping the bevel of the filter needle submerged in the formulation; (9) drawing the plunger rod sufficiently back when emptying the vial in order to completely empty the filter needle; (10) removing the filter needle from the syringe and disposing of the filter needle; (11) removing the 30-gauge x ½-inch injection needle from its packaging and attaching the injection needle to the syringe by firmly twisting the injection needle onto the Luer lock syringe tip; (12) holding the syringe with the needle pointing up, and checking the syringe for bubbles, wherein if there are bubbles, gently tapping the syringe with a finger until the bubbles rise to the top; and (13) slowly depressing the plunger so that the plunger tip aligns with the graduation line that marks 70 microliters on the syringe. In an embodiment of the invention, injection of VEGF receptor fusion protein is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.

In an embodiment of the invention, the subject has been receiving a dosing regimen for treating or preventing diabetic retinopathy and/or diabetic macular edema calling for: a single initial dose of about 2 mg of VEGF receptor fusion protein, followed by 4 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; wherein the subject is at any phase (initial dose, secondary dose or tertiary dose) of the 2 mg VEGF receptor fusion protein dosing regimen.

In an embodiment of the invention, one or more secondary doses is 2 secondary doses; 2 to 4 weeks is about 4 weeks; 12-20 weeks is about 12 weeks; 12-20 weeks is about 16 weeks; 12-20 weeks is about 20 weeks; 12-20 weeks is about 12-16 weeks; 8-16 weeks is about 12 weeks; 8-16 weeks is about 16 weeks; 8-16 weeks is about 12-16 weeks; 2 to 4 weeks is about 4 weeks and one or more secondary doses is 2 secondary doses; 12-20 weeks is about 12 weeks and one or more secondary doses is 2 secondary doses; 12-20 weeks is about 16 weeks and one or more secondary doses is 2 secondary doses; 12-20 weeks is about 20 weeks and one or more secondary doses is 2 secondary doses; 12-20 weeks is about 12-16 weeks and one or more secondary doses is 2 secondary doses; 2 to 4 weeks is about 4 weeks and one or more secondary doses is 2 secondary doses and the VEGF receptor fusion protein is aflibercept; 12-20 weeks is about 12 weeks and one or more secondary doses is 2 secondary doses and the VEGF receptor fusion protein is aflibercept; 12-20 weeks is about 16 weeks and one or more secondary doses is 2 secondary doses and the VEGF receptor fusion protein is aflibercept; 12-20 weeks is about 20 weeks and one or more secondary doses is 2 secondary doses and the VEGF receptor fusion protein is aflibercept; and/or 12-20 weeks is about 12-16 weeks and one or more secondary doses is 2 secondary doses and the VEGF receptor fusion protein is aflibercept.

In an embodiment of the invention, the VEGF receptor fusion protein: comprises amino acids 27-457 of the amino acid sequence set forth in SEQ ID NO: 2; is selected from the group consisting of: aflibercept and conbercept; comprises two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2; comprises two polypeptides that comprise an immunoglobin-like (Ig) domain 2 of VEGFR1, an Ig domain 3 of a VEGFR2, and a multimerizing component; comprises two polypeptides that comprise an immunoglobin-like (Ig) domain 2 of VEGFR1, an Ig domain 3 of VEGFR2, an Ig domain 4 of VEGFR2 and a multimerizing component; or comprises two VEGFR1R2-FcΔC1(a) polypeptides encoded by the nucleic acid sequence of SEQ ID NO: 1. In an embodiment of the invention, the VEGF receptor fusion protein comprises or consists of amino acids 27-457 of the amino acid sequence set forth in SEQ ID NO: 2.

In an embodiment of the invention, the VEGF receptor fusion protein is in an aqueous pharmaceutical formulation selected from the group consisting of A-KKKK. In an embodiment of the invention, the VEGF receptor fusion protein is in an aqueous pharmaceutical formulation comprising about 114.3 mg/ml VEGF receptor fusion protein, preferably aflibercept.

In an embodiment of the invention, the VEGF receptor fusion protein is administered to both eyes of the subject.

In an embodiment of the invention, the VEGF receptor fusion protein, preferably aflibercept, is administered from a syringe or pre-filled syringe, *e.g*., which is glass or plastic, and/or sterile; *e.g*., with a 30 gauge × ½-inch sterile injection needle.

In an embodiment of the invention, a subject has previously received one or more doses of 2 mg VEGF receptor fusion protein, *e.g*., Eylea. One or more further doses than specifically mentioned may be administered to a subject.

In an embodiment of the invention, a subject who has received 2 mg of VEGF receptor fusion protein, preferably aflibercept, has received the protein in an aqueous pharmaceutical formulation comprising 40 mg/ml VEGF receptor fusion protein, 10 mM sodium phosphate, 40 mM NaCl, 0.03% polysorbate 20 and 5% sucrose, with a pH of 6.2.

In an embodiment of the invention, the 8 mg VEGF receptor fusion protein is in an aqueous pharmaceutical formulation including ≥100 mg/ml VEGF receptor fusion protein, histidine-based buffer and arginine (preferably L-arginine); *e.g*., comprising a sugar or polyol (*e.g*., sucrose). In an embodiment of the invention, the formulation has a pH of about 5.8. For example, the formulation may include about 103-126 mg/ml of the VEGF receptor fusion protein, histidine-based buffer and arginine; in an embodiment of the invention, including about 114.3 mg/ml of the VEGF receptor fusion protein, histidine-based buffer and arginine.

In an embodiment of the invention, the 8 mg of VEGF receptor fusion protein is administered in a volume of about 100 µl or less, about 75 µl or less; about 70 µl or less; or about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl; e.g., in a volume of about 70 ± 4 or 5 microliters.

In an embodiment of the invention, the methods herein include the step of administering the VEGF receptor fusion protein, preferably aflibercept, to both eyes of the subject, *e.g*., intravitreally.

In an embodiment of the invention, the subject achieves and/or maintains one or more of, an improvement in Diabetic Retinopathy Severity Scale (DRSS); an improvement in best corrected visual acuity; a dry retina; a gain in best corrected visual acuity; a BCVA of at least 69 letters; a foveal center without fluid; a decrease in central retinal thickness (CRT); no vascular leakage as measured by fluorescein angiography (FA); an improvement from pre-treatment baseline in National Eye Institute Visual Function Questionnaire (NEI-VFQ) total score; a retina without fluid (total fluid, intraretinal fluid [IRF] and/or subretinal fluid [SRF]) at the foveal center and in center subfield; maintenance of a fluid-free retina (total fluid, IRF and/or SRF at foveal center and in the center subfield); a lack of macular edema; a retina free of fluid on spectral domain optical coherence tomography (SD-OCT); Does not deviate from the HDq12 or HDq16 or HDq20 treatment regimen once started; Non-inferior BVCA compared to that of aflibercept which is intravitreally dosed at 2 mg approximately every 4 weeks for the first 3, 4 or 5 injections followed by 2 mg approximately once every 8 weeks or once every 2 months; Increase in BCVA (according to ETDRS letter score) of about 7, 8 or 9 letters by week 60 from start of treatment, wherein the baseline BCVA is about 61, 62 or 63; BCVA (according to ETDRS letter score) of at least about 69 letters by week 48 or 60 from start of treatment; Does not lose 5, 10, 15 or 69 letters or more BCVA after week 12, 24, 36, 48, 60, 72, 84, 90 or 96 from start of treatment; Improvement in BCVA (according to ETDRS letter score) by week 12, 24, 36, 48, 60, 72, 84, 90 or 96 from start of treatment; Improvement in BVCA by week 4, week 8, week 12, week 16, week 20, week 24, week 28, week 32, week 36, week 40, week 44, or week 48 from start of treatment; Between weeks 48 and 60, a BCVA score (according to ETDRS letter score) of about 69, 70, 71, 72 or 73; Between weeks 36 and 48, a change in BCVA score (according to ETDRS letter score) from initiation of treatment of about 7, 8 or 9 wherein the BCVA at any point between week 36 to 48 is about 60 or 70; Between weeks 48 and 60, a change in BCVA score (according to ETDRS letter score) from initiation of treatment of about 7, 8 or 9, wherein the BCVA at any point between week 48 to 60 is about 69, 70, 71, 72 or 73; Increase in BCVA as measured by the Early Treatment Diabetic Retinopathy Study (ETDRS) visual acuity chart or Snellen equivalent by week 4, 8, 12, 16, 20, 24, 28, 32, 36, 40, 44 or 48 weeks from start of treatment by ≥4 letters, ≥5 letters, ≥6 letters, ≥7 letters, ≥8 letters, > 9 letters or > 10 letters; Does not lose 5, 10 or 15 letters by week 48 or 60 from start of treatment (according to ETDRS letter score); Gains at least 5, 10 or 15 letter by week 48 or 60 from start of treatment (according to ETDRS letter score); Improvement in BCVA, by 4 weeks after initiation of treatment, of about 4 or 5 letters (ETDRS or Snellen equivalent) when on HDq12 regimen; or of about 4 or 5 letters (ETDRS or Snellen equivalent) when on HDq16 regimen; Improvement in BCVA, by 8 weeks after initiation of treatment, of about 6 letters (ETDRS or Snellen equivalent) when on HDq12 regimen; or of about 5 or 6 letters (ETDRS or Snellen equivalent) when on HDq16 regimen; Improvement in BCVA, by 12 weeks after initiation of treatment, of about 6 or 7 letters (ETDRS or Snellen equivalent) when on HDq12 regimen; or of about 6 letters (ETDRS or Snellen equivalent) when on HDq16 regimen; Improvement in BCVA, by 16 weeks after initiation of treatment, of about 6 or 7 letters (ETDRS or Snellen equivalent) when on HDq12 regimen; or of 7 letters (ETDRS or Snellen equivalent) when on HDq16 regimen; Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen; Improvement in BCVA, by 24 weeks after initiation of treatment, of about 7 letters (ETDRS or Snellen equivalent) when on HDq12 regimen; or of about 5 or 6 letters (ETDRS or Snellen equivalent) when on HDq16 regimen; Improvement in BCVA, by 28 weeks after initiation of treatment, of about 7 or 8 letters (ETDRS or Snellen equivalent) when on HDq12 regimen; or of about 7 or 8 letters (ETDRS or Snellen equivalent) when on HDq16 regimen; Improvement in BCVA, by 32 weeks after initiation of treatment, of about 7 letters (ETDRS or Snellen equivalent) when on HDq12 regimen; or of about 7 or 8 letters (ETDRS or Snellen equivalent) when on HDq16 regimen; Improvement in BCVA, by 36 weeks after initiation of treatment, of 8 letters (ETDRS or Snellen equivalent) when on HDq12 regimen; or of about 6 or 7 letters (ETDRS or Snellen equivalent) when on HDq16 regimen; Improvement in BCVA, by 40 weeks after initiation of treatment, of about 8 letters (ETDRS or Snellen equivalent) when on HDq12 regimen; or of about 6 or 7 letters (ETDRS or Snellen equivalent) when on HDq16 regimen; Improvement in BCVA, by 44 weeks after initiation of treatment, of about 8 letters (ETDRS or Snellen equivalent) when on HDq12 regimen; or of about 7 or 8 letters (ETDRS or Snellen equivalent) when on HDq16 regimen; Improvement in BCVA, by 48 weeks after initiation of treatment, of about 8 or 9 letters (ETDRS or Snellen equivalent) when on HDq12 regimen; or of about 7 or 8 letters (ETDRS or Snellen equivalent) when on HDq16 regimen; An improvement in BCVA by about week 8 after initiation of treatment which is maintained thereafter during the treatment regimen to at least week 48; A BCVA by 4 weeks after initiation of treatment of about 68 letters (ETDRS or Snellen equivalent) when on the HDq12 regimen; or a BCVA of about 66 letters (ETDRS or Snellen equivalent) when on the HDq16 regimen; A BCVA by 8 weeks after initiation of treatment of about 70 letters (ETDRS or Snellen equivalent) when on the HDq12 regimen; or a BCVA of about 67 letters (ETDRS or Snellen equivalent) when on the HDq16 regimen; A BCVA by 12 weeks after initiation of treatment of about 70 letters (ETDRS or Snellen equivalent) when on the HDq12 regimen; or a BCVA of about 68 letters (ETDRS or Snellen equivalent) when on the HDq16 regimen; A BCVA by 16 weeks after initiation of treatment of about 71 letters (ETDRS or Snellen equivalent) when on the HDq12 regimen; or a BCVA of about 69 letters (ETDRS or Snellen equivalent) when on the HDq16 regimen; A BCVA by 20 weeks after initiation of treatment of about 70 letters (ETDRS or Snellen equivalent) when on the HDq12 regimen; or a BCVA of about 68 letters (ETDRS or Snellen equivalent) when on the HDq16 regimen; A BCVA by 24 weeks after initiation of treatment of about 71 letters (ETDRS or Snellen equivalent) when on the HDq12 regimen; or a BCVA of about 67 letters (ETDRS or Snellen equivalent) when on the HDq16 regimen; A BCVA by 28 weeks after initiation of treatment of about 72 letters (ETDRS or Snellen equivalent) when on the HDq12 regimen; or a BCVA of about 70 letters (ETDRS or Snellen equivalent) when on the HDq16 regimen; A BCVA by 32 weeks after initiation of treatment of about 71 letters (ETDRS or Snellen equivalent) when on the HDq12 regimen; or a BCVA of about 70 letters (ETDRS or Snellen equivalent) when on the HDq16 regimen; A BCVA by 36 weeks after initiation of treatment of about 71 letters (ETDRS or Snellen equivalent) when on the HDq12 regimen; or a BCVA of about 68 letters (ETDRS or Snellen equivalent) when on the HDq16 regimen; A BCVA by 40 weeks after initiation of treatment of about 72 letters (ETDRS or Snellen equivalent) when on the HDq12 regimen; or a BCVA of about 69 letters (ETDRS or Snellen equivalent) when on the HDq16 regimen; A BCVA by 44 weeks after initiation of treatment of about 72 letters (ETDRS or Snellen equivalent) when on the HDq12 regimen; or a BCVA of about 70 letters (ETDRS or Snellen equivalent) when on the HDq16 regimen; A BCVA by 48 weeks after initiation of treatment of about 73 letters (ETDRS or Snellen equivalent) when on the HDq12 regimen; or a BCVA of about 70 letters (ETDRS or Snellen equivalent) when on the HDq16 regimen; A BCVA improvement, by week 48 following treatment initiation, of about 9 or 10 letters (ETDRS or Snellen equivalent) when baseline BCVA is about <73 ETDRS letters when on HDq12 regimen; A BCVA improvement, by week 48 following treatment initiation, of about 5 or 6 letters (ETDRS or Snellen equivalent) when baseline BCVA is about >73 ETDRS letters when on HDq12 regimen; A BCVA improvement, by week 48 following treatment initiation, of about 8 or 9 letters (ETDRS or Snellen equivalent) when baseline BCVA is about <73 ETDRS letters when on HDq16 regimen; A BCVA improvement, by week 48 following treatment initiation, of about 4 or 5 letters (ETDRS or Snellen equivalent) when baseline BCVA is about >73 ETDRS letters when on HDq16 regimen; A BCVA improvement, by week 48 following treatment initiation, of about 7 or 8 letters (ETDRS or Snellen equivalent) when baseline CRT is about < about 400 micrometers when on HDq12 regimen; A BCVA improvement, by week 48 following treatment initiation, of about 9 or 10 letters (ETDRS or Snellen equivalent) when baseline CRT is about >400 micrometers when on HDq12 regimen; A BCVA improvement, by week 48 following treatment initiation, of about 5 or 6 letters (ETDRS or Snellen equivalent) when baseline CRT is about < about 400 micrometers when on HDq16 regimen; A BCVA improvement, by week 48 following treatment initiation, of about 9 or 10 letters (ETDRS or Snellen equivalent) when baseline CRT is about > about 400 micrometers when on HDq16 regimen; Gain of >5, >10 or ≥15 letters BCVA (according to ETDRS letter score) by week 12, 24, 36, 48, 60, 72, 84, 90 or 96 from start of treatment; ≥ 2 or >3 step improvement in Diabetic Retinopathy Severity Scale (DRSS), by week 12, 24, 36, 48, 60, 72, 84, 90 or 96 from start of treatment; ≥ 2 step improvement in diabetic retinopathy severity scale (DRSS) by week 4, week 8, week 12, 16, 20, 24, 28, 32, 36, 40, 44 or 48 weeks from start of treatment; Retina without fluid (total fluid, intraretinal fluid [IRF] and/or subretinal fluid [SRF]) at the foveal center and in center subfield by week 12, 24, 36, 48, 60, 72, 84, 90 or 96 from start of treatment as measured by optical coherence tomography (OCT); No vascular leakage as measured by fluorescein angiography (FA) by week 12, 24, 36, 48, 60, 72, 84, 90 or 96 from start of treatment; Maintenance of a fluid-free retina (total fluid, IRF and/or SRF at foveal center and in the center subfield) by week 12, 24, 36, 48, 60, 72, 84, 90 or 96 from start of treatment; Reduction in total area of fluorescein leakage within ETDRS grid (mm2) at week 48 or 60 by about 12, 13 or 14 mm² or more as measured by fluorescein angiography; Retina free of fluid on spectral domain optical coherence tomography (SD-OCT) by week 12, 24, 36, 48, 60, 72, 84, 90 or 96 from start of treatment; Retina without fluid (total fluid, intraretinal fluid [IRF] and/or subretinal fluid [SRF]) at the foveal center by week 4, 8, 12, 16, 20, 24, 28, 32, 36, 40, 44 or 48 weeks from start of treatment; Dry retina by week 12, 24, 36, 48, 60, 72, 84, 90 or 96 from start of treatment; Foveal center without fluid by week 12, 24, 36, 48, 60, 72, 84, 90 or 96 from start of treatment as measured by optical coherence tomography (OCT); A change in central retinal thickness, by 4 weeks after initiation of treatment of about -118 or -118.3 micrometers when on the HDq12 regimen; or of about -124 or -125 or -124.9 or -125.5 micrometers when on the HDq16 regimen; A change in central retinal thickness, by 8 weeks after initiation of treatment of about -137 or - 137.4 micrometers when on the HDq12 regimen; or of about -139 or -140 or -139.6 or -140.3 micrometers when on the HDq16 regimen; A change in central retinal thickness, by 12 weeks after initiation of treatment of about -150 or -150.1 micrometers when on the HDq12 regimen; or of about -152 or -153 or -152.7 or -153.4 micrometers when on the HDq16 regimen; A change in central retinal thickness, by 16 weeks after initiation of treatment of about -139 or -139.4 micrometers when on the HDq12 regimen; or of about -145 or -146 or -145.5 or -146.4 micrometers when on the HDq16 regimen; A change in central retinal thickness, by 20 weeks after initiation of treatment of about -117 or -117.1 micrometers when on the HDq12 regimen; or of about -112 or -113 or -112.5 or -113.3 micrometers when on the HDq16 regimen; A change in central retinal thickness, by 24 weeks after initiation of treatment of about -158 or -158.1 micrometers when on the HDq12 regimen; or of about -103 or -104 or -103.8 or -104.3 micrometers when on the HDq16 regimen; A change in central retinal thickness, by 28 weeks after initiation of treatment of about -146 or -147 or -146.7 micrometers when on the HDq12 regimen; or of about -162 or -162.3 micrometers when on the HDq16 regimen; A change in central retinal thickness, by 32 weeks after initiation of treatment of about -132 micrometers when on the HDq12 regimen; or of about -145 or -146 or -145.8 micrometers when on the HDq16 regimen; A change in central retinal thickness, by 36 weeks after initiation of treatment of about -168 or -168.1 micrometers when on the HDq12 regimen; or of about -124 or -125 or - 124.7 or -125.2 micrometers when on the HDq16 regimen; A change in central retinal thickness, by 40 weeks after initiation of treatment of about -163 micrometers when on the HDq12 regimen; or of about -122 or -123 or -122.5 or -123.1 micrometers when on the HDq16 regimen; A change in central retinal thickness, by 44 weeks after initiation of treatment of about -147 or - 148 or -147.4 micrometers when on the HDq12 regimen; or of about -164 or -164.1 or -164.3 micrometers when on the HDq16 regimen; A change in central retinal thickness, by 48 weeks after initiation of treatment of about -171 or -172 or -171.7 micrometers when on the HDq12 regimen; or of about -148 or -149 or -148.3 or -149.4 micrometers when on the HDq16 regimen; A change in central retinal thickness, by 60 weeks after initiation of treatment of about -181.95 or -176.24 micrometers when on the HDq12 regimen; or of about -166.26 or -167.18 micrometers when on the HDq16 regimen; A reduction in central retinal thickness by week 4, 5, 6, 7 or 8 after initiation of treatment which is maintained within about ±17, ±18 or ±19 micrometers thereafter during the treatment regimen to at least week 48 from initiation of treatment; Decrease in central retinal thickness by about 100, 125, 150, 175 or 200 micrometers by week 12, 24, 36, 48, 60, 72, 84, 90 or 96 from start of treatment; Reduction in central retinal thickness of about 148-182 micrometers by about week 48 or 60 from start of treatment as measured by optical coherence tomography (OCT)) wherein the baseline CRT is about 449, 450, 455 or 460 micrometers; Decrease in central retinal thickness (CRT) by at least about 100, 125, 130, 135, 140, 145, 149, 150, 155, 160, 165, 170, 171, 172, 173, 174 or 175 micrometers by week 4, 8, 12, 16, 20, 24, 28, 32, 36, 40, 44 or 48 from start of treatment; at about 0.1667 days after the first dose, free aflibercept concentration in plasma of about 0.149 (±0.249) mg/l; wherein, at baseline, free aflibercept concentration in plasma was not detectable wherein the subject has not received intravitreal aflibercept treatment for at least 12 weeks; At about 0.3333 days after the first dose, free aflibercept in plasma of about 0.205 (±0.250) mg/l; wherein, at baseline, free aflibercept in plasma not detectable wherein the subject has not received intravitreal aflibercept treatment for at least 12 weeks; At about 1 days after the first dose, free aflibercept in plasma of about 0.266 (±0.211) mg/l wherein, at baseline, free aflibercept in plasma not detectable wherein the subject has not received intravitreal aflibercept treatment for at least 12 weeks; At about 2 days after the first dose, free aflibercept in plasma of about 0.218 (±0.145) mg/l wherein, at baseline, free aflibercept in plasma not detectable wherein the subject has not received intravitreal aflibercept treatment for at least 12 weeks; At about 4 days after the first dose, free aflibercept in plasma of about 0.140 (±0.0741) mg/l wherein, at baseline, free aflibercept in plasma not detectable wherein the subject has not received intravitreal aflibercept treatment for at least 12 weeks; At about 7 days after the first dose, free aflibercept in plasma of about 0.0767 (±0.0436) mg/l wherein, at baseline, free aflibercept in plasma not detectable, wherein the subject has not received intravitreal aflibercept treatment for at least 12 weeks; At about 14 days after the first dose, free aflibercept in plasma of about 0.0309 (±0.0241) mg/l wherein at baseline free aflibercept in plasma not detectable wherein the subject has not received intravitreal aflibercept treatment for at least 12 weeks; At about 21 days after the first dose, free aflibercept in plasma of about 0.0171 (±0.0171) mg/l wherein, at baseline, free aflibercept in plasma not detectable wherein the subject has not received intravitreal aflibercept treatment for at least 12 weeks; At about 28 days after the first dose, free aflibercept in plasma of about 0.00730 (±0.0113) mg/l wherein, at baseline, free aflibercept in plasma not detectable wherein the subject has not received intravitreal aflibercept treatment for at least 12 weeks; At about 0.1667 days after the first dose, adjusted bound aflibercept in plasma of about 0.00698 (±0.0276) mg/l wherein, at baseline, there is about 0.00583 mg/l (±0.0280) adjusted bound aflibercept wherein the subject has not received intravitreal aflibercept treatment for at least 12 weeks; At about 0.3333 days after the first dose, adjusted bound aflibercept in plasma of about 0.00731 (±0.0279) mg/l wherein, at baseline, there is about 0.00583 mg/l (±0.0280) adjusted bound aflibercept wherein the subject has not received intravitreal aflibercept treatment for at least 12 weeks; At about 1 days after the first dose, adjusted bound aflibercept in plasma of about 0.0678 (±0.0486) mg/l wherein, at baseline, there is about 0.00583 mg/l (±0.0280) adjusted bound aflibercept wherein the subject has not received intravitreal aflibercept treatment for at least 12 weeks; At about 2 days after the first dose, adjusted bound aflibercept in plasma of about 0.138 (±0.0618) mg/l wherein at baseline there is about 0.00583 mg/l (±0.0280) adjusted bound aflibercept wherein the subject has not received intravitreal aflibercept treatment for at least 12 weeks; At about 4 days after the first dose, adjusted bound aflibercept in plasma of about 0.259 (±0.126) mg/l wherein at baseline there is about 0.00583 mg/l (±0.0280) adjusted bound aflibercept wherein the subject has not received intravitreal aflibercept treatment for at least 12 weeks; At about 7 days after the first dose, adjusted bound aflibercept in plasma of about 0.346 (±0.151) mg/l wherein at baseline there is about 0.00583 mg/l (±0.0280) adjusted bound aflibercept wherein the subject has not received intravitreal aflibercept treatment for at least 12 weeks; At about 14 days after the first dose, adjusted bound aflibercept in plasma of about 0.374 (±0.110) mg/l wherein at baseline there is about 0.00583 mg/l (±0.0280) adjusted bound aflibercept wherein the subject has not received intravitreal aflibercept treatment for at least 12 weeks; At about 21 days after the first dose, adjusted bound aflibercept in plasma of about 0.343 (±0.128) mg/l wherein at baseline there is about 0.00583 mg/l (±0.0280) adjusted bound aflibercept wherein the subject has not received intravitreal aflibercept treatment for at least 12 weeks; At about 28 days after the first dose, adjusted bound aflibercept in plasma of about 0.269 (±0.149) mg/l wherein at baseline there is about 0.00583 mg/l (±0.0280) adjusted bound aflibercept wherein the subject has not received intravitreal aflibercept treatment for at least 12 weeks; the maximum concentration of free aflibercept in the plasma is reached about 0.965 days after the first dose; Reaches a maximum concentration of about 0.310 mg/l (±0.263) free aflibercept in the plasma; Free aflibercept in the plasma of from about 0 to about 1.08 mg/L; Free aflibercept maximum concentration in the plasma (mg/l) per dose (mg) of aflibercept of about 0.0388 (±0.0328) mg/l/mg; The maximum concentration of adjusted bound aflibercept in the plasma is reached about 14 days after the first dose; Reaches a maximum concentration of about 0.387 mg/l (±0.135) adjusted bound aflibercept in the plasma; Adjusted bound aflibercept concentration in the plasma of from about 0.137 to about 0.774 mg/L; Adjusted bound aflibercept in the plasma maximum (mg/l) per dose (mg) of aflibercept of about 0.0483 (±0.0168) mg/l/mg; Does not have anti-drug antibodies against aflibercept after 48 or 60 weeks of treatment; Improvement from pre-treatment baseline in National Eye Institute Visual Function Questionnaire (NEI-VFQ) total score; and/or Lack of macular edema. For example, in an embodiment of the invention, a dry retina lacks intraretinal fluid and/or subretinal fluid; or retinal drying is characterized by no intraretinal fluid (IRF) and no subretinal fluid (SRF) in the eye of the subject, after the subject has received three monthly doses of the VEGF receptor fusion protein, preferably aflibercept.

In an embodiment of the invention, reference to 4, 8, 12, 16, 20, 24, 28, 32, 36, 40, 44, 48, 52, 56 or 60 weeks from start of treatment is about 48 weeks or 60 weeks from start of treatment.

In an embodiment of the invention, 1 initial dose, 2 secondary doses and 3 tertiary doses of VEGF receptor fusion protein, *e.g*., aflibercept, are administered to the subject in the first year; 1 initial dose, 2 secondary doses and 2 tertiary doses of VEGF receptor fusion protein, *e.g*., aflibercept, are administered to the subject in the first year; or 1 initial dose, 2 secondary doses and 3 tertiary doses of VEGF receptor fusion protein, *e.g*., aflibercept, are administered to the subject in the first year followed by 2 -4 tertiary doses in the second year.

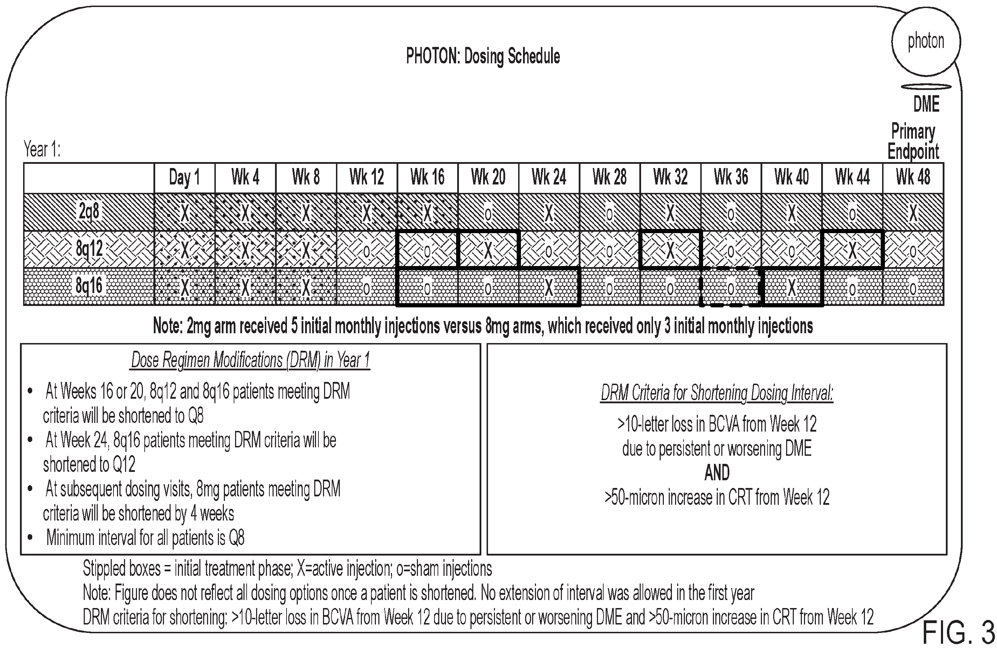

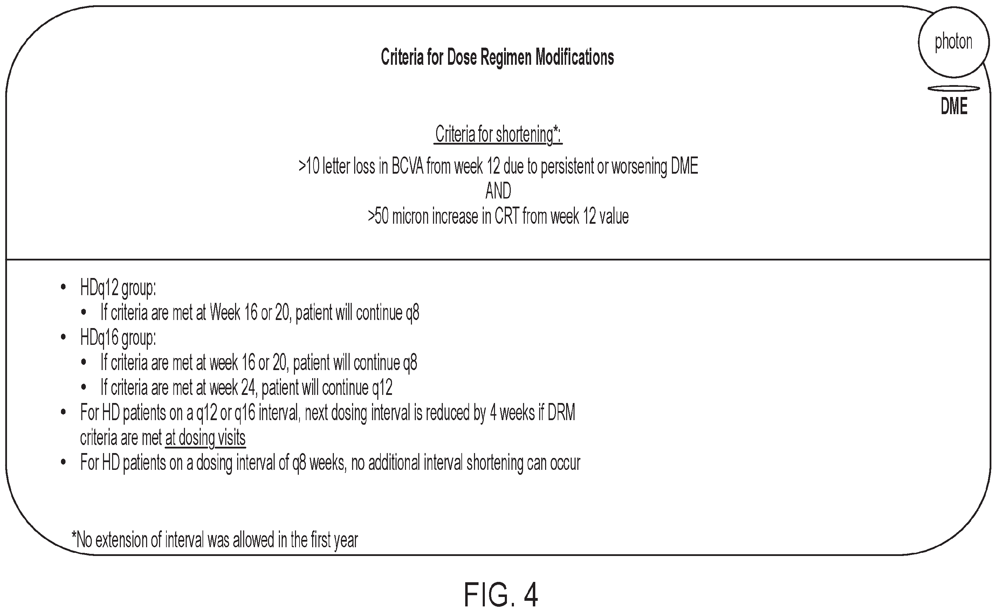

In an embodiment of the invention, the interval between doses are adjusted (increased/maintained/reduced) based on visual and/or anatomic outcomes, *e.g*., according to criteria as set forth in Figure 3 and/or Figure 4.

The present invention also provides a kit comprising a container comprising VEGF receptor fusion protein; and Instruction for use of VEGF receptor fusion protein, wherein the container is a vial or a pre-filled syringe, wherein the container comprises ≥ 100 mg/mL VEGF receptor fusion protein, wherein the container comprises ≥ 114.3 mg/mL VEGF receptor fusion protein, wherein the instruction comprises instruction for the administration of aflibercept to DME/AMD patients, wherein the instruction comprises instruction that aflibercept 8 mg treatment is initiated with 1 injection per month (every 4 weeks) for 3 consecutive doses, wherein the instruction comprises instruction that after the initial 3 consecutive doses the injection interval may be lengthened up to every 16 week or every 20 week, and wherein the instruction comprises instruction that the treatment interval may be adjusted based on the physician's judgement of visual and/or anatomic outcomes.

The present invention provides aflibercept for use in the treatment or prevention of diabetic retinopathy and/or diabetic macular edema in a subject in need thereof comprising administering one or more doses of aflibercept at an interval and quantity whereby the clearance of free aflibercept from the ocular compartment is about 0.367-0.458 mL/day after an intravitreal injection of aflibercept, the time for the amount for free aflibercept to reach the lower limit of quantitation (LLOQ) in the ocular compartment of a subject after said intravitreal injection of aflibercept is about 15 weeks; and the time for free aflibercept to reach the lower limit of quantitation (LLOQ) in the plasma of the subject after said intravitreal injection of aflibercept is about 3.5 weeks.

The present invention provides aflibercept for use in a method for slowing the clearance of free aflibercept from the ocular compartment after an intravitreal injection relative to the rate of clearance of aflibercept from the ocular compartment after an intravitreal injection of < 4 mg aflibercept wherein the method comprises intravitreally injecting into an eye of a subject in need thereof, a single initial dose of about 8 mg or more of aflibercept, followed by one or more secondary doses of about 8 mg or more of the aflibercept, followed by
one or more tertiary doses of about 8 mg or more of the aflibercept;
wherein each secondary dose is administered about 2 to 4 weeks after the immediately preceding dose; and
wherein each tertiary dose is administered about 12-20 weeks after the immediately preceding dose.

The present invention provides aflibercept for use a method for increasing the time for the amount of free aflibercept to reach the lower limit of quantitation (LLOQ) in the ocular compartment of a subject after an intravitreal injection of aflibercept relative to the time to reach LLOQ of the amount of free aflibercept in the ocular compartment of a subject after an intravitreal injection of about 2 mg aflibercept,
wherein the method comprises intravitreally injecting into an eye of a subject in need thereof,
a single initial dose of about 8 mg or more of aflibercept, followed by
one or more secondary doses of about 8 mg or more of the aflibercept, followed by one or more tertiary doses of about 8 mg or more of the aflibercept;
wherein each secondary dose is administered about 2 to 4 weeks after the immediately preceding dose; and
wherein each tertiary dose is administered about 12-20 weeks after the immediately preceding dose.

The present invention provides aflibercept for use in a method for increasing the time for free aflibercept to reach the lower limit of quantitation (LLOQ) in the plasma of a subject after an intravitreal injection of aflibercept relative to the time to reach LLOQ of free aflibercept in the plasma of a subject after an intravitreal injection of about 2 mg aflibercept, wherein the method comprises intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose.

The present invention provides a VEGF receptor fusion protein for use in a method
- for treating or preventing diabetic retinopathy (DR) and/or diabetic macular edema (DME), in a subject in need thereof,
- for improving best corrected visual acuity in a subject in need thereof with DR and/or DME; or
- for promoting retinal drying in a subject with DR and/or DME in need thereof;
wherein the method comprises administering to an eye of the subject, one or more doses of about 8 mg or more of VEGF receptor fusion protein once every 12, 13, 14, 15, 16, 17, 18, 19 or 20 or 12-20 or 12-16 or 16-20 weeks.

The present invention provides aflibercept for use in the treatment or prevention of diabetic retinopathy and/or diabetic macular edema, in a subject in need thereof, wherein the treatment or prevention comprises initiating the treatment with 1 injection of 8 mg aflibercept per month (every 4 weeks) for three consecutive doses followed by one or more injection once every 8 - 16 weeks or 8 - 20 weeks, wherein the concentration of aflibercept of each said dose is 114.3 mg/mL or wherein the application volume of each said dose is 70 µL. In an embodiment of the invention the treatment interval between two subsequent administrations of 8 mg aflibercept is adjusted (increased/maintained/reduced) based on visual and/or anatomic outcomes such as but not limited to letter gain or letter loss in BCVA; increase or reduction in CRT; presence or absence of subretinal fluid; or presence or absence of hemorraghe or persistent or worsening DME. In an embodiment of the invention the treatment interval is reduced by 2-4 weeks, 2 weeks, 3 weeks or by 4 weeks compared to the previous treatment interval in case said subject has been identified as one with meeting at least one of the following criteria for reduction of the treatment interval: > 5 letter or > 10 letter loss in BCVA; CRT of >300 or 320 µm; > 50 µm increase in CRT; or 2. persistent or worsening DME. In an embodiment of the invention the treatment interval is extended by 2-4 weeks, 2 weeks, 3 weeks or by 4 weeks compared to the previous treatment interval in case said subject has been identified as one with meeting at least one of the following criteria for extending the treatment interval: < 5 letter < 10 letter loss in BCVA; CRT <300 or 320 µm; > 50 µm decrease in CRT; absence of subretinal fluid; or absence of hemorraghe.

The present invention provides a VEGF receptor fusion protein for use in the treatment or prevention of diabetic macular edema, in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days) or every 8 - 20 weeks (2 - 5 months, +/- 7 days).

The present invention provides a VEGF receptor fusion protein for use in the treatment or prevention of diabetic retinopathy (DR), in a subject in need thereof , wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days) or every 8 - 20 weeks (2 - 5 months, +/- 7 days).

The present invention provides a VEGF receptor fusion protein for use in the treatment or prevention of diabetic macular edema (DME), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).

The present invention provides a VEGF receptor fusion protein for use in the treatment or prevention of diabetic retinopathy (DR), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).

The present invention provides a VEGF receptor fusion protein for use in the treatment or prevention of diabetic macular edema (DME), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).

The present invention provides a VEGF receptor fusion protein for use in the treatment or prevention of diabetic retinopathy (DR), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).

The present invention provides a VEGF receptor fusion protein for use in the treatment or prevention of diabetic macular edema (DME), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (2 - 4 months, +/- 7 days).

The present invention provides a VEGF receptor fusion protein for use in the treatment or prevention of diabetic retinopathy (DR), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (2 - 4 months, +/- 7 days).

The present invention provides a VEGF receptor fusion protein for use in the treatment or prevention of diabetic retinopathy and/or diabetic macular edema, in a subject in need thereof:
(1) wherein the subject has received an initial 2 mg dose of VEGF receptor fusion protein, then the method comprises, after 1 month, administering to the subject the initial 8 mg dose of VEGF receptor fusion protein and, 1 month thereafter, the 1st 8 mg secondary dose of VEGF receptor fusion protein; and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(2) wherein the subject has received an initial 2 mg dose of VEGF receptor fusion protein, then the method comprises, after 1 month, administering to the subject the first 8 mg secondary dose of VEGF receptor fusion protein and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(3) wherein the subject has received an initial 2 mg dose of VEGF receptor fusion protein, then the method comprises, after 1 month, administering to the subject the 2nd 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
(4) wherein the subject has received an initial 2 mg dose of VEGF receptor fusion protein, then the method comprises, after 1 month, administering to the subject the 1st 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
(5) wherein the subject has received an initial 2 mg dose of VEGF receptor fusion protein and a 1st 2 mg secondary dose of VEGF receptor fusion protein after 1 month, then the method comprises, after another 1 month, administering to the subject the initial 8 mg dose of VEGF receptor fusion protein and, 1 month thereafter, the 1st 8 mg secondary dose of VEGF receptor fusion protein; and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(6) wherein the subject has received an initial 2 mg dose of VEGF receptor fusion protein and a 1st 2 mg secondary dose of VEGF receptor fusion protein after 1 month, then the method comprises, after another 1 month, administering to the subject a first 8 mg secondary dose of VEGF receptor fusion protein and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(7) wherein the subject has received an initial 2 mg dose of VEGF receptor fusion protein and a 1st 2 mg secondary dose of VEGF receptor fusion protein after 1 month, then the method comprises, after another 1 month, administering to the subject the 2nd 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
(8) wherein the subject has received an initial 2 mg dose of VEGF receptor fusion protein and a 1st 2 mg secondary dose of VEGF receptor fusion protein after 1 month, then the method comprises, after another 1 month, administering to the subject the 1st 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
(9) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month, then the method comprises, after another 1 month, administering to the subject the initial 8 mg dose of VEGF receptor fusion protein and, 1 month thereafter, the 1st 8 mg secondary dose of VEGF receptor fusion protein; and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(10) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month, then the method comprises, after another 1 month, administering to the subject the first 8 mg secondary dose of VEGF receptor fusion protein and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(11) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month, then the method comprises, after another 1 month, administering to the subject the 2nd 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
(12) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month, then the method comprises, after 2 months, administering to the subject the 1st 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
(13) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, then the method comprises, after 1 month, administering to the subject the initial 8 mg dose of VEGF receptor fusion protein and 1 month thereafter, the 1st 8 mg secondary dose of VEGF receptor fusion protein; and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(14) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, then the method comprises, after 1 month, administering to the subject the first 8 mg secondary dose of VEGF receptor fusion protein and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(15) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, then the method comprises, after 1 month, administering to the subject the 2nd 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
(16) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, then the method comprises, after 2 months, administering to the subject the 1st 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
(17) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month; and a 4th 2 mg secondary dose of VEGF receptor fusion protein after 1 month; thereafter, then the method comprises, after 2 months, administering to the subject the initial 8 mg dose of VEGF receptor fusion protein and, 1 month thereafter, the 1st 8 mg secondary dose of VEGF receptor fusion protein; and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(18) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month; and a 4th 2 mg secondary dose of VEGF receptor fusion protein after 1 month; thereafter, then the method comprises, after 2 months, administering to the subject the first 8 mg secondary dose of VEGF receptor fusion protein and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(19) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, and a 4th 2 mg secondary dose of VEGF receptor fusion protein after 1 month; thereafter, then the method comprises, after 2 months, administering to the subject the 2nd 8 mg secondary dose of VEGF receptor fusion protein and, 12 or 16 or 20 weeks thereafter, one or more 12 or 16 weekly 8 mg maintenance doses of VEGF receptor fusion protein according to the HDq12 or HDq16 or HDq20 dosing regimen;
(20) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, and a 4th 2 mg secondary dose of VEGF receptor fusion protein after 1 month, thereafter, then the method comprises, after 2 months, administering to the subject the 1st 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
(21) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month, and a 4th 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the initial 8 mg dose of VEGF receptor fusion protein and, 1 month thereafter, the 1st 8 mg secondary dose of VEGF receptor fusion protein; and 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(22) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month; and a 4th 2 mg secondary dose of VEGF receptor fusion protein after 1 month; and one or more 2 mg maintenance doses every 8 weeks thereafter, then the method comprises, 2 months after the last VEGF receptor fusion protein maintenance dose administering to the subject the first 8 mg secondary dose of VEGF receptor fusion protein and, 1 month thereafter, the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
(23) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month; and a 4th 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 2nd 8 mg secondary dose of VEGF receptor fusion protein and, 12 or 16 or 20 weeks thereafter, one or more 12 or 16 or 20 weekly 8 mg maintenance doses of VEGF receptor fusion protein according to the HDq12 or HDq16 or HDq20 dosing regimen;
   or
(24) wherein the subject has received an initial 2 mg dose of VEGF receptor fusion protein and a 1st 2 mg secondary dose of VEGF receptor fusion protein after 1 month and a 2nd 2 mg secondary dose of VEGF receptor fusion protein after another 1 month and a 3rd 2 mg secondary dose of VEGF receptor fusion protein after 1 month; and a 4th 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 1st 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
wherein,
(i) said HDq12 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12 weeks after the immediately preceding dose;
(ii) said HDq16 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 16 weeks after the immediately preceding dose;
   and
(iii) said HDq20 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 20 weeks after the immediately preceding dose.

The present invention provides aflibercept for use in the treatment or prevention of diabetic macular edema or diabetic retinopathy, in a subject in need thereof, comprising administering to an eye of the subject, a single initial dose 8 mg aflibercept, followed by one or more tertiary doses of about 8 mg of aflibercept; wherein each tertiary dose is administered about 8, 12, 16, or 20 weeks after the immediately preceding dose. In an embodiment of the invention, the subject is not a treatment naive subject, or the subject was pre-treated with a VEGF antagonist or preferably the subject was pre-treated with 8 mg aflibercept or with 2 mg aflibercept.

The present invention provides aflibercept for use in the treatment or prevention of diabetic macular edema or diabetic retinopathy, in a subject which was pre-treated with 2 mg aflibercept, comprising administering to an eye of the subject a single initial dose of about 8 mg aflibercept, followed by one or more secondary doses of about 8 mg of aflibercept, followed by one or more tertiary doses of about 8 mg aflibercept, wherein each secondary dose is administered about 4 weeks after the immediately preceding dose and wherein each tertiary dose is administered about 8, 10, 12, 14, 16, 18 or 20 weeks after the immediately preceding dose. In an embodiment of the invention, the administration of one or more doses of 8 mg aflibercept to an eye of the subject is according to HDq12, HDq16, HDq20, or treat and extent dosing regimen.

The present invention provides a VEGF receptor fusion protein for use in the treatment or prevetion of diabetic retinopathy and/or diabetic macular edema, in a subject in need thereof who has been on a dosing regimen for treating or preventing said disorder wherein:
(a) the subject has received an initial 8 mg dose of VEGF receptor fusion protein then the method comprises, after 1 month, administering to the subject the first 8 mg secondary dose of VEGF receptor fusion protein and 1 month thereafter, administering the 2nd 8 mg secondary dose of VEGF receptor fusion protein; and then, every 12 or 16 or 20 weeks thereafter, administering one or more 8 mg maintenance doses of VEGF receptor fusion protein according to the HDq12 or HDq16 or HDq20 dosing regimen;
   or
(b) the subject has received an initial 8 mg dose of VEGF receptor fusion protein & 1st 8 mg secondary dose of VEGF receptor fusion protein after 1 month, then the method comprises, after another 1 month, administering to the subject the 2nd 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   or
(c) the subject has received an initial 8 mg dose of VEGF receptor fusion protein & 1st 8 mg secondary dose of VEGF receptor fusion protein after 1 month & the 2nd 8 mg secondary dose of VEGF receptor fusion protein after another month, then the method comprises, after 12 or 16 or 20 weeks administering to the subject the 1st 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   or
(d) the subject has received an initial 8 mg dose of VEGF receptor fusion protein & a 1st 8 mg secondary dose of VEGF receptor fusion protein after 1 month & the 2nd 8 mg secondary dose of VEGF receptor fusion protein after another month, then every 12 or 16 or 20 weeks thereafter, the subject has received one or more 8 mg maintenance doses of VEGF receptor fusion protein; and, then the method comprises, after 12 or 16 or 20 weeks from the last maintenance dose of VEGF receptor fusion protein, administering to the subject one or more 8 mg maintenance doses of VEGF receptor fusion protein and all further 8 mg maintenance doses of VEGF receptor fusion protein every 12 or 16 or 20 weeks according to the HDq12 or HDq16 or HDq20 dosing regimen;
wherein,
(i) said HDq12 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12 weeks after the immediately preceding dose;
(ii) said HDq16 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 16 weeks after the immediately preceding dose;
   and
(iii) said HDq20 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 20 weeks after the immediately preceding dose.

The present invention provides a VEGF receptor fusion protein for use in the treatment or prevention of angiogenic eye disorder, in a subject in need thereof who has been on a dosing regimen for treating or preventing the disorder calling for a single initial dose of about 2 mg of VEGF receptor fusion protein, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen,comprising administering to an eye of the subject, an 8 mg dose of VEGF receptor fusion protein, evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with 8 mg VEGF receptor fusion protein; or evaluating the subject in about 8 or 10 or 12 weeks after said administering and, if, in the judgment of the treating physician dosing every 12 weeks is appropriate, then administering another 8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.

The present invention provides a VEGF receptor fusion protein for use in the treatment and prevention of diabetic retinopathy or diabetic macular edema, in a subject in need thereof, wherein the treatment or prevention comprises administering to an eye of the subject, a single initial dose of about 8 mg or more of a VEGF receptor fusion protein, followed by one or more secondary doses, preferably 2 dosis, of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 weeks after the immediately preceding dose; further comprising, after receiving one or more of said tertiary doses about 12 or 16 after the immediately preceding dose, lengthening the tertiary dose interval from
- 12 weeks to 16 weeks;
- 12 weeks to 20 weeks; or
- 16 weeks to 20 weeks,
after the immediately preceding dose.

The present invention provides a VEGF receptor fusion protein for use in the treatment and prevention of diabetic retinopathy and/or diabetic macular edema, in a subject in need thereof, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 or 20 weeks after the immediately preceding dose; further comprising, after receiving one or more of said tertiary doses about 12 or 16 or 20 weeks after the immediately preceding dose, shortening the tertiary dose interval from
- 12 weeks to 8 weeks;
- 16 weeks to 12 weeks;
- 16 weeks to 8 weeks,
- 20 weeks to 8 weeks,
- 20 weeks to 12 weeks, or
- 20 weeks to 16 weeks.

The present invention provides a VEGF receptor fusion protein for use in the treatment and prevention of diabetic retinopathy and/or diabetic macular edema, in a subject in need thereof, comprising administering to an eye of the subject 3 doses of about 8 mg VEGF receptor fusion protein in a formulation that comprises about 114.3 mg/ml VEGF receptor fusion protein at an interval of once every 4 weeks; wherein after said 3 doses, administering one or more doses of the VEGF receptor fusion protein at an interval which is lengthened up to 12, 16 or 20 weeks.

The present invention provides a VEGF receptor fusion protein for use in the treatment and prevention of diabetic retinopathy and/or diabetic macular edema, in a subject in need thereof, comprising administering to an eye of the subject, a single initial dose of about 8 mg or more of VEGF receptor fusion protein, followed by 2 secondary doses of about 8 mg or more of the VEGF receptor fusion protein, wherein each secondary dose is administered about 2 to 4 weeks after the immediately preceding dose; and, after said doses,
a) determining if the subject meets at least one criterion for reducing or lengthening one or more intervals by 2 weeks, 3 weeks, 4 weeks or 2-4 weeks between doses of the VEGF receptor fusion protein; and
b) if said determination is made, administering further doses of the VEGF receptor fusion protein at said reduced or lengthened intervals between doses

wherein criteria for lengthening the interval include:
   1. <5 letter loss in BCVA; and/or
   2. CRT <300 or 320 micrometers;
and, wherein criteria for reducing the interval include:
   1. >10 letter loss in BCVA;
   2. persistent or worsening DME; and/or
   3. >50 micrometers increase in CRT.

The present invention provides a VEGF receptor fusion protein for use in the treatment and prevention of diabetic retinopathy and/or diabetic macular edema, in a subject in need thereof that has been pre-treated with one or more 2 mg doses of VEGF receptor fusion protein, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks after the immediately preceding dose.

The present invention provides a VEGF receptor fusion protein for use in the treatment and prevention of an angiogenic eye disorder, in a subject in need thereof, comprising administering to an eye of the subject,
(1) a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 8 weeks after the immediately preceding dose; or
(2) one or more doses of 8 mg or more of VEGF receptor fusion protein about every 4 weeks.

A VEGF receptor fusion protein for use in the treatment and prevention of an angiogenic eye disorder wherein the treatment or prevention comprises, prior to each administration, providing
- one single-dose glass vial having a protective plastic cap and a stopper containing an aqueous formulation comprising 8 mg VEGF receptor fusion protein in about 70 microliters;
- one 18-gauge x 1½-inch, 5-micron, filter needle that includes a tip and a bevel;

packaged together; then
   (1) visually inspecting the aqueous formulation in the vial and, if particulates, cloudiness, or discoloration are visible, then using another vial of aqueous formulation containing the VEGF receptor fusion protein;
   (2) removing the protective plastic cap from the vial; and
   (3) cleaning the top of the vial with an alcohol wipe; then
using aseptic technique:
   (4) removing the 18-gauge x 1½-inch, 5-micron, filter needle and the 1 mL syringe from their packaging;
   (5) attaching the filter needle to the syringe by twisting it onto the Luer lock syringe tip;
   (6) pushing the filter needle into the center of the vial stopper until the needle is completely inserted into the vial and the tip touches the bottom or a bottom edge of the vial;
   (7) withdrawing all of the VEGF receptor fusion protein vial contents into the syringe, keeping the vial in an upright position, slightly inclined, while ensuring the bevel of the filter needle is submerged into the liquid;
   (8) continuing to tilt the vial during withdrawal keeping the bevel of the filter needle submerged in the formulation;
   (9) drawing the plunger rod sufficiently back when emptying the vial in order to completely empty the filter needle;
   (10) removing the filter needle from the syringe and disposing of the filter needle;
   (11) removing the 30-gauge x ½-inch injection needle from its packaging and attaching the injection needle to the syringe by firmly twisting the injection needle onto the Luer lock syringe tip;
   (12) holding the syringe with the needle pointing up, and checking the syringe for bubbles,
      wherein if there are bubbles, gently tapping the syringe with a finger until the bubbles rise to the top; and
   (13) slowly depressing the plunger so that the plunger tip aligns with the graduation line that marks 70 microliters on the syringe.

The present invention provides a VEGF receptor fusion protein for use in the treatment and prevention of diabetic retinopathy and/or diabetic macular edema in a subject in need thereof, wherein the subject has been receiving a dosing regimen for treating or preventing diabetic retinopathy and/or diabetic macular edema calling for: a single initial dose of about 2 mg of VEGF receptor fusion protein, followed by 4 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose;wherein the subject is at any phase (initial dose, secondary dose or tertiary dose) of the 2 mg VEGF receptor fusion protein dosing regimen.

The present invention provides a VEGF receptor fusion protein for use in the treatment and prevention of diabetic retinopathy and/or diabetic macular edema in a subject in need thereof, wherein 8 mg of a VEGF receptor fusion protein is in an aqueous pharmaceutical formulation comprising about 103-126 mg/ml VEGF receptor fusion protein, histidine-based buffer and arginine.

The present invention provides a VEGF receptor fusion protein for use in the treatment and prevention of diabetic retinopathy and/or diabetic macular edema in a subject in need thereof wherein 8 mg of a VEGF receptor fusion protein is an aqueous pharmaceutical formulation comprising about 114.3 mg/ml VEGF receptor fusion protein, histidine-based buffer and arginine.

The present invention provides aflibercept for use in the treatment and prevention of diabetic retinopathy and/or diabetic macular edema in a subject in need thereof wherein the >8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8°C.

The present invention provides a VEGF receptor fusion protein for use in the treatment and prevention of diabetic retinopathy and/or diabetic macular edema in a subject in need thereof wherein the >8 mg VEGF receptor fusion protein is in an aqueous pharmaceutical formulation comprising:
at least about 100 mg/ml of a VEGF receptor fusion protein;
about 10-100 mM L-arginine;
sucrose;
a histidine-based buffer; and
a surfactant;
wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

The present invention provides a VEGF receptor fusion protein for use in the treatment and prevention of diabetic retinopathy and/or diabetic macular edema in a subject in need thereof wherein 8 mg of VEGF receptor fusion protein is in an aqueous pharmaceutical formulation comprising
- >100 mg/ml VEGF receptor fusion protein, histidine-based buffer and L-arginine;
- 140 mg/ml aflibercept; 20 mM histidine-based buffer; 5 % sucrose; 0.03 % polysorbate 20; 10 mM L-arginine; pH 5.8;
- 150 + 15 mg/ml aflibercept, 10 mM phosphate-based buffer, 8 + 0.8% (w/v) sucrose, 0.02-0.04% (w/v) polysorbate 20 and 50 mM L-arginine, pH 5.9-6.5;
- 103-126 mg/ml aflibercept, 10 + 1 mM histidine-based buffer, 5 + 0.5% (w/v) sucrose, 0.02-0.04% (w/v) polysorbate 20, and 50 + 5 mM L-arginine, pH 5.5-6.1;
- 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
- 114.3 mg/ml aflibercept, 10 mM histidine-based buffer, 5% (w/v) sucrose, 0.03% (w/v) polysorbate 20 and 50 mM L-arginine, pH 5.8;
- >100 mg/ml aflibercept, histidine-based buffer and L-arginine;
- >100 mg/ml aflibercept at about pH 5.8, wherein the formulation forms <3% HMW aggregates after incubation at 5°C for 2 months;
- About 114.3 mg/mL aflibercept; 10 mM - 50 mM histidine-based buffer, sugar, nonionic surfactant, L-Arginine, pH 5.8;
- About 114.3 mg/mL aflibercept; 10 mM His/His-HCl-based buffer, 5% sucrose, 0.03% polysorbate-20, 50 mM L-Arginine, pH 5.8; or
- about 114.3 mg/mL aflibercept; arginine monohydrochloride; histidine; histidine hydrochloride, monohydrate; polysorbate 20; sucrose and water for injection.

The present invention provides a VEGF receptor fusion protein for use in the treatment and prevention of diabetic retinopathy and/or diabetic macular edema in a subject in need thereof wherein the subject achieves and/or maintains one or more of,
- an improvement in Diabetic Retinopathy Severity Scale (DRSS), e.g., by at least 2 or 3 steps;
- an improvement in best corrected visual acuity;
- a dry retina;
- a gain in best corrected visual acuity;
- a gain in best corrected visual acuity of at least 5, 10 or 15 letters
- a BCVA of at least 69 letters;
- a decrease in central retinal thickness (CRT);
- no vascular leakage as measured by fluorescein angiography (FA);
- an improvement from pre-treatment baseline in National Eye Institute Visual Function Questionnaire (NEI-VFQ-25) total score;
- a retina without fluid (total fluid, intraretinal fluid [IRF] and/or subretinal fluid [SRF]) at the foveal center and in center subfield;
- maintenance of a fluid-free retina (total fluid, IRF and/or SRF at foveal center and in the center subfield);
- a lack of macular edema;
- a retina free of fluid on spectral domain optical coherence tomography (SD-OCT); and/or
- Does not deviate from the HDq12 or HDq16 treatment regimen once started.

The present invention provides a VEGF receptor fusion protein for use in the treatment and prevention of diabetic retinopathy and/or diabetic macular edema in a subject in need thereof, wherein the subject achieves and/or maintains one or more of:
- Non-inferior BVCA compared to that of aflibercept which is intravitreally dosed at 2 mg approximately every 4 weeks for the first 3, 4 or 5 injections followed by 2 mg approximately once every 8 weeks or once every 2 months;
- Increase in BCVA (according to ETDRS letter score) of about 7, 8 or 9 letters by week 60 from start of treatment, wherein the baseline BCVA is about 61, 62 or 63;
- BCVA (according to ETDRS letter score) of at least about 69 letters by week 48 or 60 from start of treatment;
- Does not lose 5, 10, 15 or 69 letters or more BCVA after week 12, 24, 36, 49, 60, 72, 84 or 90 from start of treatment;
- Improvement in BCVA (according to ETDRS letter score) by week 12, 24, 36, 49, 60, 72, 84 or 90 from start of treatment;
- Improvement in BVCA by week 4, week 8, week 12, week 16, week 20, week 24, week 28, week 32, week 36, week 40, week 44, or week 48 from start of treatment;
- Between weeks 48 and 60, a BCVA score (according to ETDRS letter score) of about 69, 70, 71, 72 or 73;
- Between weeks 36 and 48, a change in BCVA score (according to ETDRS letter score) from initiation of treatment of about 7, 8 or 9 wherein the BCVA at any point between week 36 to 48 is about 60 or 70;
- Between weeks 48 and 60, a change in BCVA score (according to ETDRS letter score) from initiation of treatment of about 7, 8 or 9, wherein the BCVA at any point between week 48 to 60 is about 69, 70, 71, 72 or 73;
- Increase in BCVA as measured by the Early Treatment Diabetic Retinopathy Study (ETDRS) visual acuity chart or Snellen equivalent by week 4, 8, 12, 16, 20, 24, 28, 32, 36, 40, 44 or 48 weeks from start of treatment by ≥4 letters, ≥5 letters, ≥6 letters, ≥7 letters, ≥8 letters, > 9 letters or > 10 letters;
- Does not lose 5, 10 or 15 letters by week 48 or 60 from start of treatment (according to ETDRS letter score);
- Gains at least 5, 10 or 15 letter by week 48 or 60 from start of treatment (according to ETDRS letter score);
- Improvement in BCVA, by 4 weeks after initiation of treatment, of about 4 or 5 letters (ETDRS or Snellen equivalent) when on HDq12 regimen; or of about 4 or 5 letters (ETDRS or Snellen equivalent) when on HDq16 regimen;
- Improvement in BCVA, by 8 weeks after initiation of treatment, of about 6 letters (ETDRS or Snellen equivalent) when on HDq12 regimen; or of about 5 or 6 letters (ETDRS or Snellen equivalent) when on HDq16 regimen;
- Improvement in BCVA, by 12 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 16 weeks after initiation of treatment, of about 6 or 7 letters (ETDRS or Snellen equivalent) when on HDq12 regimen; or of 7 letters (ETDRS or Snellen equivalent) when on HDq16 regimen;
- Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
- Improvement in BCVA, by 24 weeks after initiation of treatment, of about 7 letters (ETDRS or Snellen equivalent) when on HDq12 regimen; or of about 5 or 6 letters (ETDRS or Snellen equivalent) when on HDq16 regimen;
- Improvement in BCVA, by 28 weeks after initiation of treatment, of about 7 or 8 letters (ETDRS or Snellen equivalent) when on HDq12 regimen; or of about 7 or 8 letters (ETDRS or Snellen equivalent) when on HDq16 regimen;
- Improvement in BCVA, by 32 weeks after initiation of treatment, of about 7 letters (ETDRS or Snellen equivalent) when on HDq12 regimen; or of about 7 or 8 letters (ETDRS or Snellen equivalent) when on HDq16 regimen;
- Improvement in BCVA, by 36 weeks after initiation of treatment, of 8 letters (ETDRS or Snellen equivalent) when on HDq12 regimen; or of about 6 or 7 letters (ETDRS or Snellen equivalent) when on HDq16 regimen;
- Improvement in BCVA, by 40 weeks after initiation of treatment, of about 8 letters (ETDRS or Snellen equivalent) when on HDq12 regimen; or of about 6 or 7 letters (ETDRS or Snellen equivalent) when on HDq16 regimen;
- Improvement in BCVA, by 44 weeks after initiation of treatment, of about 8 letters (ETDRS or Snellen equivalent) when on HDq12 regimen; or of about 7 or 8 letters (ETDRS or Snellen equivalent) when on HDq16 regimen;
- Improvement in BCVA, by 48 weeks after initiation of treatment, of about 8 or 9 letters (ETDRS or Snellen equivalent) when on HDq12 regimen; or of about 7 or 8 letters (ETDRS or Snellen equivalent) when on HDq16 regimen;
- An improvement in BCVA by about week 8 after initiation of treatment which is maintained thereafter during the treatment regimen to at least week 48;
- A BCVA by 4 weeks after initiation of treatment of about 68 letters (ETDRS or Snellen equivalent) when on the HDq12 regimen; or a BCVA of about 66 letters (ETDRS or Snellen equivalent) when on the HDq16 regimen;
- A BCVA by 8 weeks after initiation of treatment of about 70 letters (ETDRS or Snellen equivalent) when on the HDq12 regimen; or a BCVA of about 67 letters (ETDRS or Snellen equivalent) when on the HDq16 regimen;
- A BCVA by 12 weeks after initiation of treatment of about 70 letters (ETDRS or Snellen equivalent) when on the HDq12 regimen; or a BCVA of about 68 letters (ETDRS or Snellen equivalent) when on the HDq16 regimen;
- A BCVA by 16 weeks after initiation of treatment of about 71 letters (ETDRS or Snellen equivalent) when on the HDq12 regimen; or a BCVA of about 69 letters (ETDRS or Snellen equivalent) when on the HDq16 regimen;
- A BCVA by 20 weeks after initiation of treatment of about 70 letters (ETDRS or Snellen equivalent) when on the HDq12 regimen; or a BCVA of about 68 letters (ETDRS or Snellen equivalent) when on the HDq16 regimen;
- A BCVA by 24 weeks after initiation of treatment of about 71 letters (ETDRS or Snellen equivalent) when on the HDq12 regimen; or a BCVA of about 67 letters (ETDRS or Snellen equivalent) when on the HDq16 regimen;
- A BCVA by 28 weeks after initiation of treatment of about 72 letters (ETDRS or Snellen equivalent) when on the HDq12 regimen; or a BCVA of about 70 letters (ETDRS or Snellen equivalent) when on the HDq16 regimen;
- A BCVA by 32 weeks after initiation of treatment of about 71 letters (ETDRS or Snellen equivalent) when on the HDq12 regimen; or a BCVA of about 70 letters (ETDRS or Snellen equivalent) when on the HDq16 regimen;
- A BCVA by 36 weeks after initiation of treatment of about 71 letters (ETDRS or Snellen equivalent) when on the HDq12 regimen; or a BCVA of about 68 letters (ETDRS or Snellen equivalent) when on the HDq16 regimen;
- A BCVA by 40 weeks after initiation of treatment of about 72 letters (ETDRS or Snellen equivalent) when on the HDq12 regimen; or a BCVA of about 69 letters (ETDRS or Snellen equivalent) when on the HDq16 regimen;
- A BCVA by 44 weeks after initiation of treatment of about 72 letters (ETDRS or Snellen equivalent) when on the HDq12 regimen; or a BCVA of about 70 letters (ETDRS or Snellen equivalent) when on the HDq16 regimen;
- A BCVA by 48 weeks after initiation of treatment of about 73 letters (ETDRS or Snellen equivalent) when on the HDq12 regimen; or a BCVA of about 70 letters (ETDRS or Snellen equivalent) when on the HDq16 regimen;
   A BCVA improvement, by week 48 following treatment initiation, of about 9 or 10 letters (ETDRS or Snellen equivalent) when baseline BCVA is about <73 ETDRS letters when on HDq12 regimen;
- A BCVA improvement, by week 48 following treatment initiation, of about 5 or 6 letters (ETDRS or Snellen equivalent) when baseline BCVA is about >73 ETDRS letters when on HDq12 regimen;
- A BCVA improvement, by week 48 following treatment initiation, of about 8 or 9 letters (ETDRS or Snellen equivalent) when baseline BCVA is about <73 ETDRS letters when on HDq16 regimen;
- A BCVA improvement, by week 48 following treatment initiation, of about 4 or 5 letters (ETDRS or Snellen equivalent) when baseline BCVA is about >73 ETDRS letters when on HDq16 regimen;
- A BCVA improvement, by week 48 following treatment initiation, of about 7 or 8 letters (ETDRS or Snellen equivalent) when baseline CRT is about < about 400 micrometers when on HDq12 regimen;
- A BCVA improvement, by week 48 following treatment initiation, of about 9 or 10 letters (ETDRS or Snellen equivalent) when baseline CRT is about >400 micrometers when on HDq12 regimen;
- A BCVA improvement, by week 48 following treatment initiation, of about 5 or 6 letters (ETDRS or Snellen equivalent) when baseline CRT is about < about 400 micrometers when on HDq16 regimen;
- A BCVA improvement, by week 48 following treatment initiation, of about 9 or 10 letters (ETDRS or Snellen equivalent) when baseline CRT is about > about 400 micrometers when on HDq16 regimen;
- Gain of >5, >10 or ≥15 letters BCVA (according to ETDRS letter score) by week 12, 24, 36, 49, 60, 72, 84 or 90 from start of treatment;
- ≥ 2 or >3 step improvement in Diabetic Retinopathy Severity Scale (DRSS), by week 12, 24, 36, 49, 60, 72, 84 or 90 from start of treatment;
- ≥ 2 step improvement in diabetic retinopathy severity scale (DRSS) by week 4, week 8, week 12, 16, 20, 24, 28, 32, 36, 40, 44 or 48 weeks from start of treatment;
- Retina without fluid (total fluid, intraretinal fluid [IRF] and/or subretinal fluid [SRF]) at the foveal center and in center subfield by week 12, 24, 36, 49, 60, 72, 84 or 90 from start of treatment as measured by optical coherence tomography (OCT);
- No vascular leakage as measured by fluorescein angiography (FA) by week 12, 24, 36, 49, 60, 72, 84 or 90 from start of treatment;
- Maintenance of a fluid-free retina (total fluid, IRF and/or SRF at foveal center and in the center subfield) by week 12, 24, 36, 49, 60, 72, 84 or 90 from start of treatment;
- Reduction in total area of fluorescein leakage within ETDRS grid (mm2) at week 48 or 60 by about 12, 13 or 14 mm2 or more as measured by fluorescein angiography;
- Retina free of fluid on spectral domain optical coherence tomography (SD-OCT) by week 12, 24, 36, 49, 60, 72, 84 or 90 from start of treatment;
- Retina without fluid (total fluid, intraretinal fluid [IRF] and/or subretinal fluid [SRF]) at the foveal center by week 4, 8, 12, 16, 20, 24, 28, 32, 36, 40, 44 or 48 weeks from start of treatment;
- Dry retina by week 12, 24, 36, 49, 60, 72, 84 or 90 from start of treatment;
- Foveal center without fluid by week 12, 24, 36, 49, 60, 72, 84 or 90 from start of treatment as measured by optical coherence tomography (OCT);
- A change in central retinal thickness, by 4 weeks after initiation of treatment of about - 118 or -118.3 micrometers when on the HDq12 regimen; or of about -124 or -125 or - 124.9 or -125.5 micrometers when on the HDq16 regimen;
- A change in central retinal thickness, by 8 weeks after initiation of treatment of about - 137 or -137.4 micrometers when on the HDq12 regimen; or of about -139 or -140 or - 139.6 or -140.3 micrometers when on the HDq16 regimen;
- A change in central retinal thickness, by 12 weeks after initiation of treatment of about -150 or -150.1 micrometers when on the HDq12 regimen; or of about -152 or -153 or - 152.7 or -153.4 micrometers when on the HDq16 regimen;
- A change in central retinal thickness, by 16 weeks after initiation of treatment of about -139 or -139.4 micrometers when on the HDq12 regimen; or of about -145 or -146 or - 145.5 or -146.4 micrometers when on the HDq16 regimen;
- A change in central retinal thickness, by 20 weeks after initiation of treatment of about -117 or -117.1 micrometers when on the HDq12 regimen; or of about -112 or -113 or - 112.5 or -113.3 micrometers when on the HDq16 regimen;
- A change in central retinal thickness, by 24 weeks after initiation of treatment of about -158 or -158.1 micrometers when on the HDq12 regimen; or of about -103 or -104 or - 103.8 or -104.3 micrometers when on the HDq16 regimen;
- A change in central retinal thickness, by 28 weeks after initiation of treatment of about -146 or -147 or -146.7 micrometers when on the HDq12 regimen; or of about -162 or - 162.3 micrometers when on the HDq16 regimen;
- A change in central retinal thickness, by 32 weeks after initiation of treatment of about -132 micrometers when on the HDq12 regimen; or of about -145 or -146 or -145.8 micrometers when on the HDq16 regimen;
- A change in central retinal thickness, by 36 weeks after initiation of treatment of about -168 or -168.1 micrometers when on the HDq12 regimen; or of about -124 or -125 or - 124.7 or -125.2 micrometers when on the HDq16 regimen;
- A change in central retinal thickness, by 40 weeks after initiation of treatment of about -163 micrometers when on the HDq12 regimen; or of about -122 or -123 or -122.5 or - 123.1 micrometers when on the HDq16 regimen;
- A change in central retinal thickness, by 44 weeks after initiation of treatment of about -147 or -148 or -147.4 micrometers when on the HDq12 regimen; or of about -164 or - 164.1 or -164.3 micrometers when on the HDq16 regimen;
- A change in central retinal thickness, by 48 weeks after initiation of treatment of about -171 or -172 or -171.7 micrometers when on the HDq12 regimen; or of about -148 or - 149 or -148.3 or -149.4 micrometers when on the HDq16 regimen;
- A change in central retinal thickness, by 60 weeks after initiation of treatment of about -181.95 or -176.24 micrometers when on the HDq12 regimen; or of about -166.26 or - 167.18 micrometers when on the HDq16 regimen;
- A change in central retinal thickness of about -118 or -119 or -118.3 micrometers, between initiation of treatment (week 0) and week 4 when on the HDq12 regimen;
- A change in central retinal thickness of about -19, -20 or -19.1 micrometers, between weeks 4 and 8 when on the HDq12 regimen;
- A change in central retinal thickness of about -12, -13 or -12.7 micrometers, between weeks 8 and 12 when on the HDq12 regimen;
- A change in central retinal thickness of about -40, or -41 micrometers, between weeks 20 and 24 when on the HDq12 regimen;
- A change in central retinal thickness of about -36, -37 or -36.1 micrometers, between weeks 32 and 36 when on the HDq12 regimen;
- A change in central retinal thickness of about -24, -25 or -24.3 micrometers, between weeks 44 and 48 when on the HDq12 regimen;
- A change in central retinal thickness of -4, -5 or -4.5 micrometers, between weeks 48 and 60 when on the HDq12 regimen;
- A change in central retinal thickness of about -124, -125 or -124.9 micrometers, between initiation of treatment (week 0) and week 4 when on the HDq16 regimen;
- A change in central retinal thickness of about -14, -15 or -14.7 micrometers, between weeks 4 and 8 when on the HDq16 regimen;
- A change in central retinal thickness of about -13, -14 or -13.1 micrometers, between weeks 8 and 12 when on the HDq16 regimen;
- A change in central retinal thickness of about -58, -59 or -58.5 micrometers, between weeks 24 and 28 when on the HDq16 regimen;
- A change in central retinal thickness of about -41, -42 or -41.6 micrometers, between weeks 40 and 44 when on the HDq16 regimen;
- A reduction in central retinal thickness by week 4, 5, 6, 7 or 8 after initiation of treatment which is maintained within about +17, +18 or +19 micrometers thereafter during the treatment regimen to at least week 48 from initiation of treatment;
- Decrease in central retinal thickness by about 100, 125, 150, 175 or 200 micrometers by week 12, 24, 36, 49, 60, 72, 84 or 90 from start of treatment;
- Reduction in central retinal thickness of about 148-182 micrometers by about week 48 or 60 from start of treatment as measured by optical coherence tomography (OCT)) wherein the baseline CRT is about 449, 450, 455 or 460 micrometers;
- Decrease in central retinal thickness (CRT) by at least about 100, 125, 130, 135, 140, 145, 149, 150, 155, 160, 165, 170, 171, 172, 173, 174 or 175 micrometers by week 4, 8, 12, 16, 20, 24, 28, 32, 36, 40, 44 or 48 from start of treatment;
- At about 0.1667 days after the first dose, free aflibercept in plasma of about 0.149 (±0.249) mg/l; wherein, at baseline, free aflibercept in was plasma not detectable wherein the subject has not received intravitreal aflibercept treatment for at least 12 weeks;
- At about 0.3333 days after the first dose, free aflibercept in plasma of about 0.205 (±0.250) mg/l; wherein, at baseline, free aflibercept in plasma not detectable wherein the subject has not received intravitreal aflibercept treatment for at least 12 weeks;
- At about 1 days after the first dose, free aflibercept in plasma of about 0.266 (±0.211) mg/l wherein, at baseline, free aflibercept in plasma not detectable wherein the subject has not received intravitreal aflibercept treatment for at least 12 weeks;
- At about 2 days after the first dose, free aflibercept in plasma of about 0.218 (±0.145) mg/l wherein, at baseline, free aflibercept in plasma not detectable wherein the subject has not received intravitreal aflibercept treatment for at least 12 weeks;
- At about 4 days after the first dose, free aflibercept in plasma of about 0.140 (±0.0741) mg/l wherein, at baseline, free aflibercept in plasma not detectable wherein the subject has not received intravitreal aflibercept treatment for at least 12 weeks;
- At about 7 days after the first dose, free aflibercept in plasma of about 0.0767 (±0.0436) mg/l wherein, at baseline, free aflibercept in plasma not detectable, wherein the subject has not received intravitreal aflibercept treatment for at least 12 weeks;
- At about 14 days after the first dose, free aflibercept in plasma of about 0.0309 (±0.0241) mg/l wherein at baseline free aflibercept in plasma not detectable wherein the subject has not received intravitreal aflibercept treatment for at least 12 weeks;
- At about 21 days after the first dose, free aflibercept in plasma of about 0.0171 (±0.0171) mg/l wherein, at baseline, free aflibercept in plasma not detectable wherein the subject has not received intravitreal aflibercept treatment for at least 12 weeks;
- At about 28 days after the first dose, free aflibercept in plasma of about 0.00730 (±0.0113) mg/l wherein, at baseline, free aflibercept in plasma not detectable wherein the subject has not received intravitreal aflibercept treatment for at least 12 weeks;
- At about 0.1667 days after the first dose, adjusted bound aflibercept in plasma of about 0.00698 (±0.0276) mg/l wherein, at baseline, there is about 0.00583 mg/l (±0.0280) adjusted bound aflibercept wherein the subject has not received intravitreal aflibercept treatment for at least 12 weeks;
- At about 0.3333 days after the first dose, adjusted bound aflibercept in plasma of about 0.00731 (±0.0279) mg/l wherein, at baseline, there is about 0.00583 mg/l (±0.0280) adjusted bound aflibercept wherein the subject has not received intravitreal aflibercept treatment for at least 12 weeks;
- At about 1 days after the first dose, adjusted bound aflibercept in plasma of about 0.0678 (±0.0486) mg/l wherein, at baseline, there is about 0.00583 mg/l (±0.0280) adjusted bound aflibercept wherein the subject has not received intravitreal aflibercept treatment for at least 12 weeks;
- At about 2 days after the first dose, adjusted bound aflibercept in plasma of about 0.138 (±0.0618) mg/l wherein at baseline there is about 0.00583 mg/l (±0.0280) adjusted bound aflibercept wherein the subject has not received intravitreal aflibercept treatment for at least 12 weeks;
- At about 4 days after the first dose, adjusted bound aflibercept in plasma of about 0.259 (±0.126) mg/l wherein at baseline there is about 0.00583 mg/l (±0.0280) adjusted bound aflibercept wherein the subject has not received intravitreal aflibercept treatment for at least 12 weeks;
- At about 7 days after the first dose, adjusted bound aflibercept in plasma of about 0.346 (±0.151) mg/l wherein at baseline there is about 0.00583 mg/l (±0.0280) adjusted bound aflibercept wherein the subject has not received intravitreal aflibercept treatment for at least 12 weeks;
- At about 14 days after the first dose, adjusted bound aflibercept in plasma of about 0.374 (±0.110) mg/l wherein at baseline there is about 0.00583 mg/l (±0.0280) adjusted bound aflibercept wherein the subject has not received intravitreal aflibercept treatment for at least 12 weeks;
- At about 21 days after the first dose, adjusted bound aflibercept in plasma of about 0.343 (±0.128) mg/l wherein at baseline there is about 0.00583 mg/l (±0.0280) adjusted bound aflibercept wherein the subject has not received intravitreal aflibercept treatment for at least 12 weeks;
- At about 28 days after the first dose, adjusted bound aflibercept in plasma of about 0.269 (±0.149) mg/l wherein at baseline there is about 0.00583 mg/l (±0.0280) adjusted bound aflibercept wherein the subject has not received intravitreal aflibercept treatment for at least 12 weeks;
- The maximum level of free aflibercept in the plasma is reached about 0.965 days after the first dose;
- Reaches a maximum level of about 0.310 mg/l (±0.263) free aflibercept in the plasma;
- Free aflibercept in the plasma of from about 0 to about 1.08 mg/L;
- Free aflibercept in the plasma maximum (mg/l) per dose (mg) of aflibercept of about 0.388 (±0.0328) mg/l/mg;
- The maximum level of adjusted bound aflibercept in the plasma is reached about 14 days after the first dose;
- Reaches a maximum level of about 0.387 mg/l (±0.135) adjusted bound aflibercept in the plasma;
- Adjusted bound aflibercept in the plasma of from about 0.137 to about 0.774 mg/L;
- Adjusted bound aflibercept in the plasma maximum (mg/l) per dose (mg) of aflibercept of about 0.483 (±0.0168) mg/l/mg;
- Does not have anti-drug antibodies against aflibercept after 48 or 60 weeks of treatment;
- Improvement from pre-treatment baseline in National Eye Institute Visual Function Questionnaire (NEI-VFQ) total score; and/or
- Lack of macular edema.

The present invention provides a VEGF receptor fusion protein for use in the treatment and prevention of diabetic retinopathy and/or diabetic macular edema in a subject in need thereof wherein
- 1 initial dose, 2 secondary doses and 3 tertiary doses of said > 8 mg VEGF receptor fusion protein are administered to the subject in the first year;
- wherein 1 initial dose, 2 secondary doses and 2 tertiary doses of said > 8 mg VEGF receptor fusion protein are administered to the subject in the first year; or
- wherein 1 initial dose, 2 secondary doses and 3 tertiary doses of said > 8 mg VEGF receptor fusion protein are administered to the subject in the first year followed by 2 -4 tertiary doses in the second year.

The present invention provides a VEGF receptor fusion protein for use in the treatment and prevention of diabetic retinopathy and/or diabetic macular edema in a subject in need thereof wherein the interval between doses of 8 mg VEGF receptor fusion protein is adjusted (increased/maintained/reduced) based on visual and/or anatomic outcomes.

The present invention provides a VEGF receptor fusion protein for use in the treatment and prevention of diabetic retinopathy and/or diabetic macular edema in a subject in need thereof wherein the doses of 8 mg VEGF receptor fusion protein are administered according to pro re nata (PRN), capped PRN or treat and extend (T&E) dosing regimen.

The present invention also provides a kit comprising i) a container comprising a VEGF receptor fusion protein, preferably aflibercept and ii) instruction for use of the VEGF fusion protein. In an embodiment of the invention, the container is a vial or a pre-filled syringe. The vial a type I glass vial containing a nominal fill volume of abut 0.26 mL solution for intravitreal injection. In an embodiment of the invention the container comprises the VEGF receptor fusion protein at a concentration of more or equal to 100 mg/mL or the container comprises aflibercept at a concentration of about 114.3 mg/mL. In an embodiment of the invention, the instruction for use comprising instruction for use of the VEGF fusion protein or aflibercept for the treatment of DME and /or AMD. In an embodiment of the invention, the instruction for use comprises the information that i) the container comprises 8 mg (114.3 mg/mL) aflibercept solution for intravitreal injection, ii) each single-dose vial provides a usable amount to deliver a single dose of 70 microliters containing 8 mg aflibercept to adult patients, iii) the recommended dose is 8 mg aflibercept (equivalent to 70 microliters solution for injection), iv) 8 mg aflibercept treatment is initiated with 1 injection per month (every 4 weeks) for 3 consecutive doses, v) injection intervals may then be extended up to every 16 weeks or 20 weeks vi) the treatment interval may be adjusted based on the physician's judgement of visual and/or anatomic outcomes and /or vii) that 8 mg aflibercept / 0.07 mL is provided as a sterile, aqueous solution containing arginine monohydrochloride; histidine; histidine hydrochloride, monohydrate; polysorbate 20; sucrose and water for injection.

### BRIEF DESCRIPTION OF THE FIGURES

**Figure 1****:** Summary of PHOTON clinical trial.
**Figure 2****:** Key eligibility criteria (inclusion criteria and exclusion criteria) of PHOTON clinical trial.
**Figure 3****:** Dosing schedule and dose regimen modification (DRM) criteria of PHOTON clinical trial.
**Figure 4****:** Criteria for dose regimen modifications of PHOTON clinical trial.
**Figure 5****:** Patient disposition at week 48 in PHOTON clinical trial.
**Figure 6****:** Baseline demographics of subjects in PHOTON clinical trial.
**Figure 7****:** Baseline characteristics of the study eye of subjects in PHOTON clinical trial.
**Figure 8****:** Mean number of injections through week 48 in PHOTON clinical trial.
**Figure 9*****:** Mean change in Best Corrected Visual Acuity (BCVA) through week 48 in PHOTON clinical trial. Least squares mean change from baseline at week 48 shown.

| **Week** | **2q8** | **HDq12** | **HDq16** |
|---|---|---|---|
| 0 | 0.0 | 0.0 | 0.0 |
| 4 | 5.3 | 4.5 | 4.4 |
| 8 | 6.9 | 6 | 5.9 |
| 12 | 7.3 | 6.7 | 6.2 |
| 16 | 7.5 | 6.8 | 7 |
| 20 | 7.9 | 6.4 | 6.3 |
| 24 | 7.6 | 7.3 | 5.8 |
| 28 | 8.4 | 7.6 | 7.8 |
| 32 | 8.2 | 7.1 | 7.5 |
| 36 | 8.9 | 8 | 6.7 |
| 40 | 8.4 | 8.4 | 6.8 |
| 44 | 8.6 | 8.3 | 7.7 |
| 48 | 9.2 | 8.8 | 7.9 |

| | | | |
|---|---|---|---|
| *the present invention includes methods for achieving approximately the indicated improvement in BVCA by the indicated timepoint when receiving the indicated treatment regimen for treating DR and/or DME. | | | |

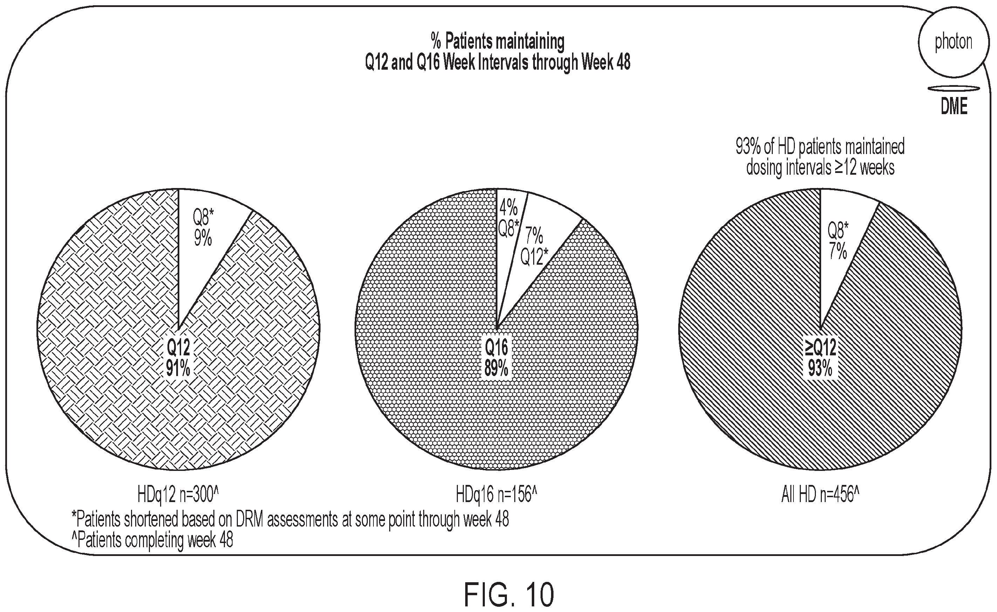

**Figure 10****:** Percentage of subjects maintaining Q12 week and Q16 week intervals through week 48 in PHOTON clinical trial.

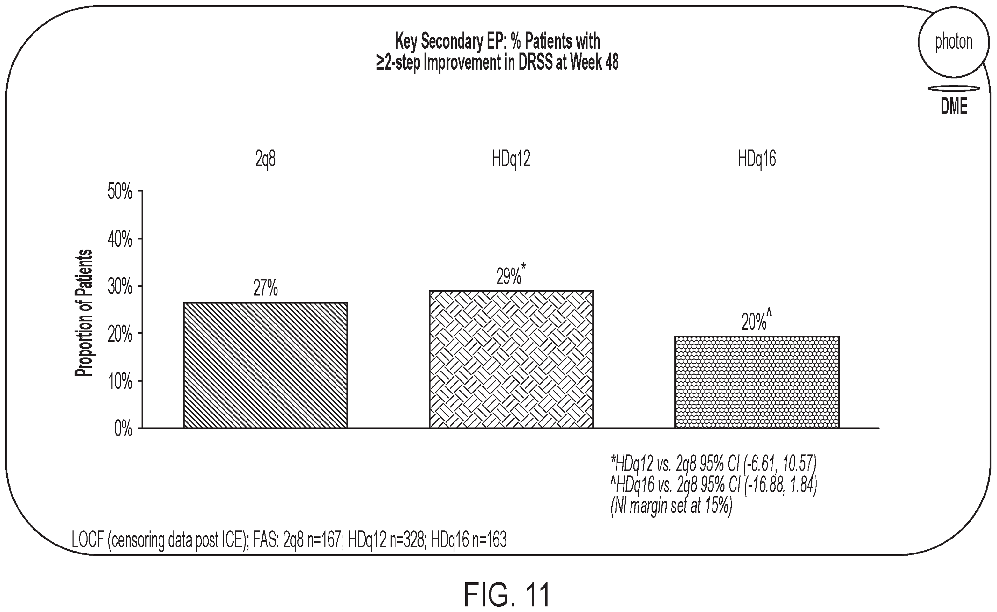

**Figure 11****:** Key secondary endpoint of percentage of subjects with ≥2 step improvement in Diabetic Retinopathy Severity Scale (DRSS) at week 48 in PHOTON clinical trial.

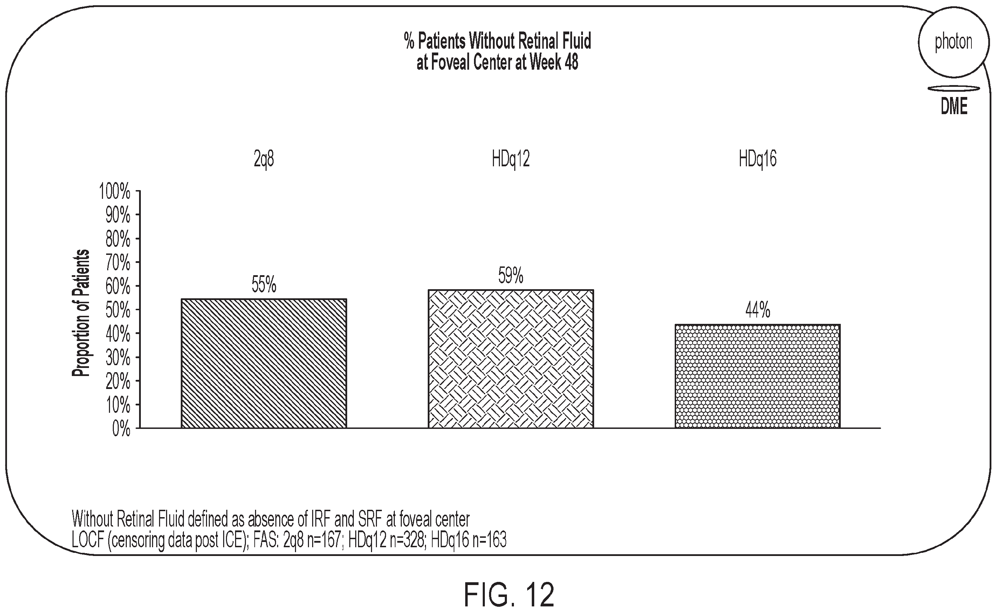

**Figure 12****:** Percentage of subjects without retinal fluid at foveal center at week 48 in PHOTON clinical trial.

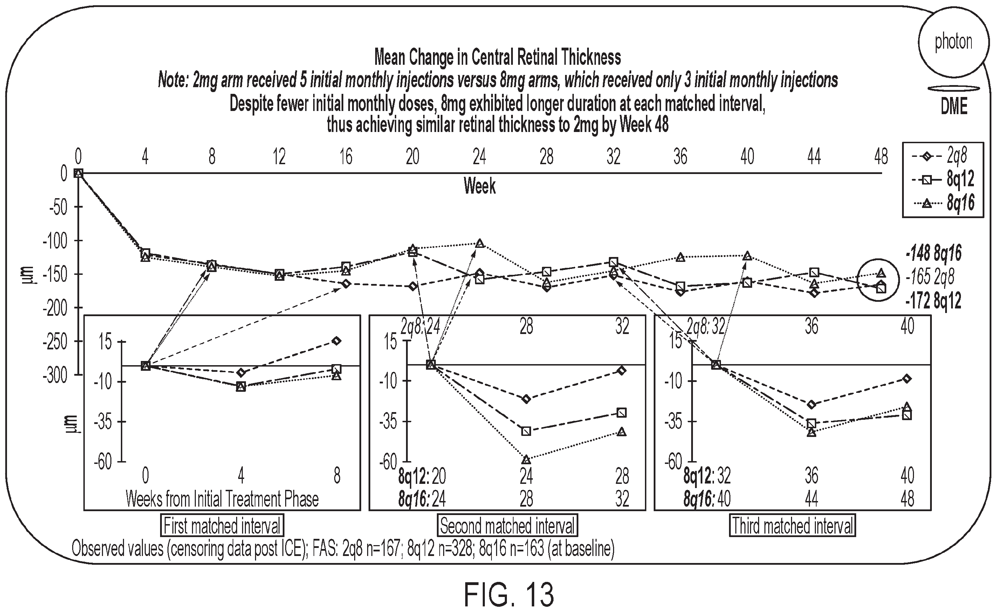

**Figure 13*****:** Mean change from baseline in central retinal thickness through week 48 in PHOTON clinical trial. Various matched intervals are highlighted in three insets.

| **Week** | **2q8** | **HDq12** | **HDq16** |
|---|---|---|---|
| 0 | 0 | 0 | 0.0 |
| 4 | -121.2 | -118.3 | -124.9 |
| 8 | -135.6 | -137.4 | -139.6 |
| 12 | -150.1 | -150.1 | -152.7 |
| 16 | -164.2 | -139.4 | -145.5 |
| 20 | -168.5 | -117.1 | -112.5 |
| 24 | -148.5 | -158.1 | -103.8 |
| 28 | -169.8 | -146.7 | -162.3 |
| 32 | -152.1 | -132 | -145 |
| 36 | -176.7 | -168.1 | -124.7 |
| 40 | -160.6 | -163 | -122.5 |
| 44 | -178.6 | -147.4 | -164.1 |
| 48 | -165.3 | -171.7 | -148.3 |

| | | | |
|---|---|---|---|
| *the present invention includes methods for achieving approximately the indicated reduction in central retinal thickness by the indicated timepoint when receiving the indicated treatment regimen for treating DR and/or DME. | | | |

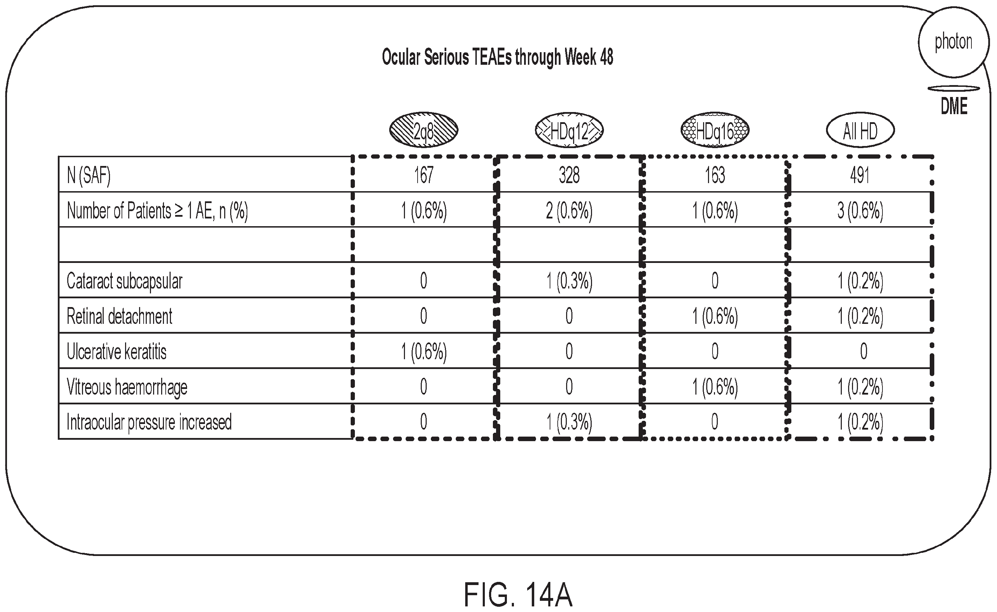

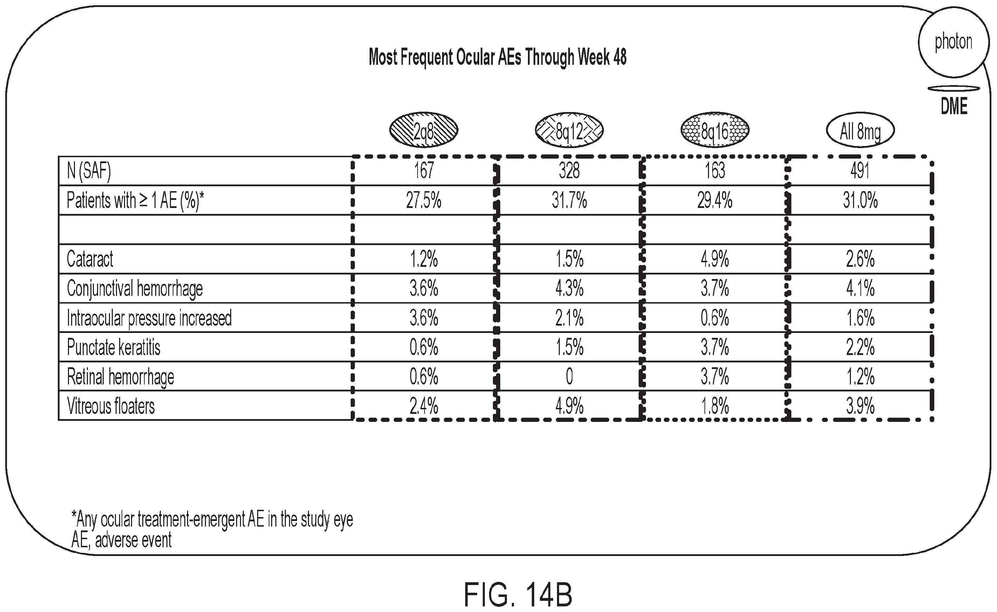

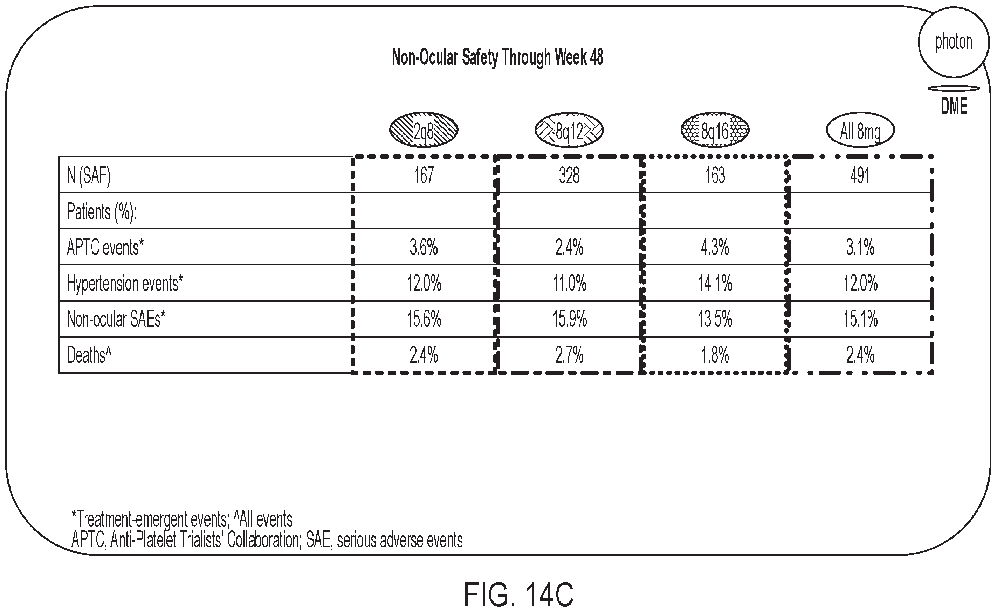

**Figures 14A****,** **14B** **and** **14C****:** Ocular serious Treatment Emergent Adverse Events (TEAEs) through week 48 in PHOTON clinical trial (Fig. 14A); Most Frequent Adverse Events (AEs) through week 48 (Fig. 14B); Non-Ocular Safety through week 48 (Fig. 14C).

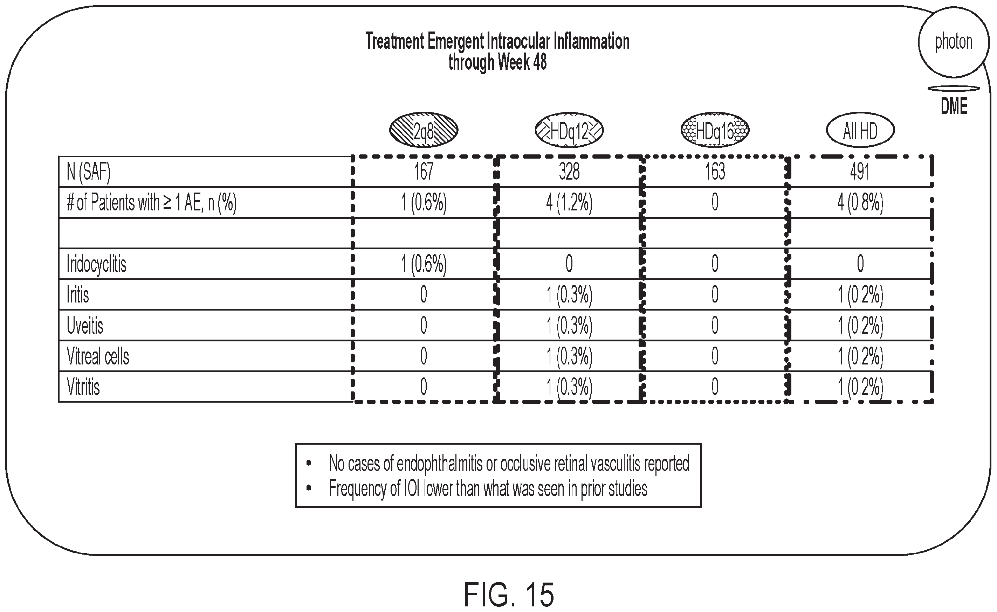

**Figure 15****:** Treatment emergent intraocular inflammation through week 48 in PHOTON clinical trial.

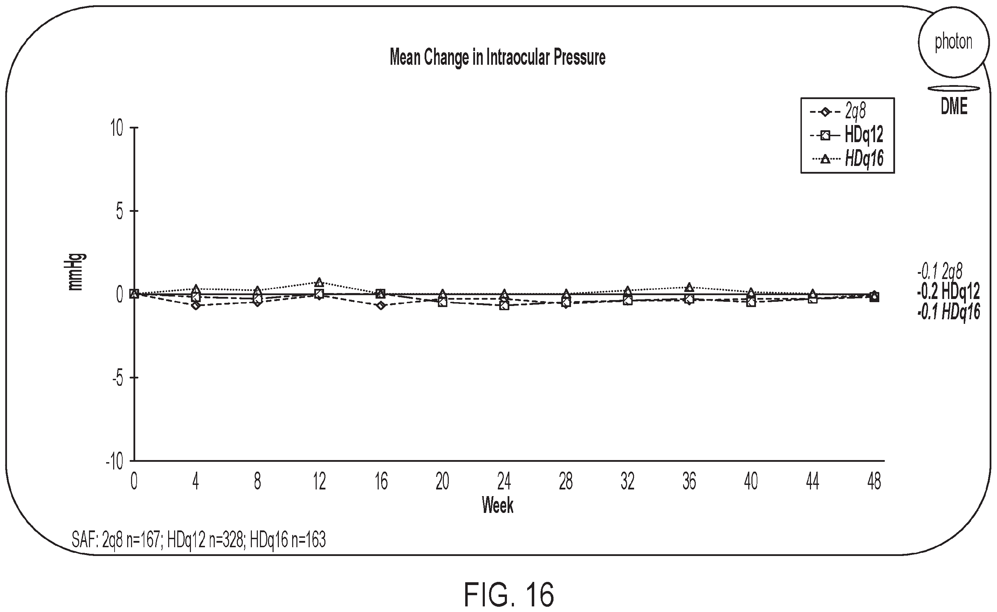

**Figure 16****:** Mean change from baseline in intraocular pressure through week 48 in PHOTON clinical trial.

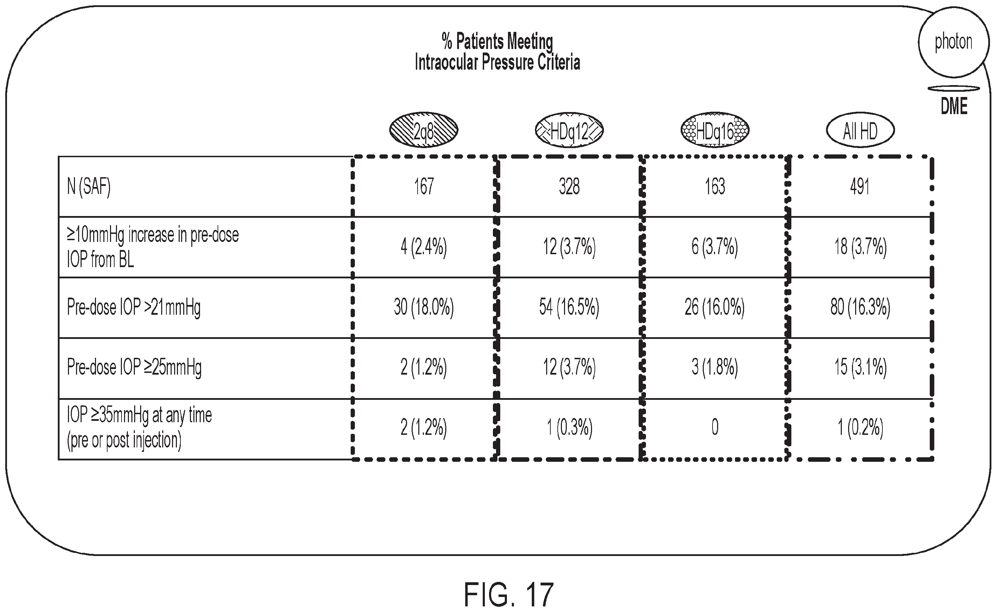

**Figure 17****:** Percentage of subjects meeting intraocular pressure criteria in PHOTON clinical trial.

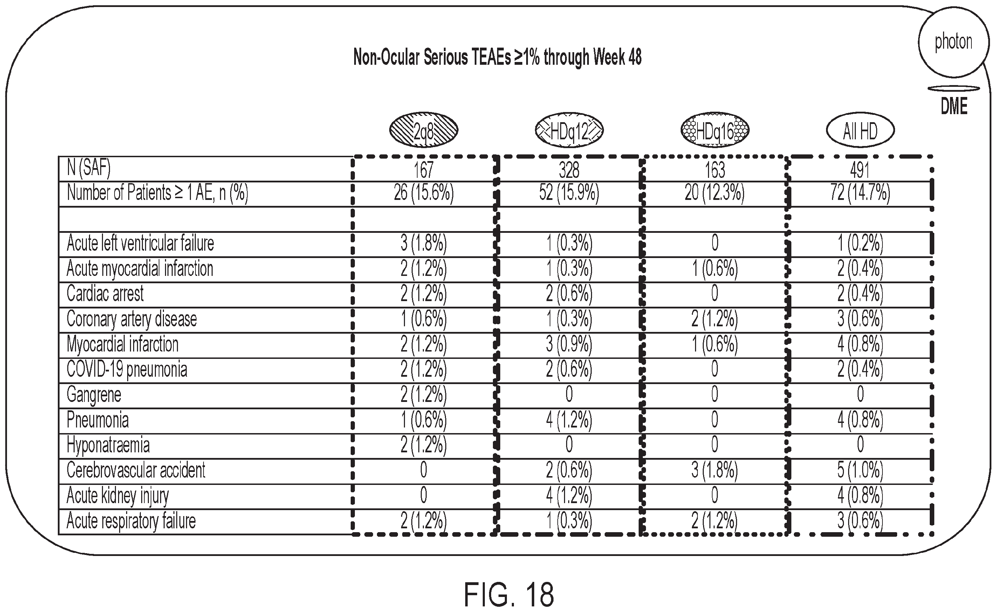

**Figure 18****:** Non-Ocular Serious TEAEs ≥1% through week 48 in PHOTON clinical trial.

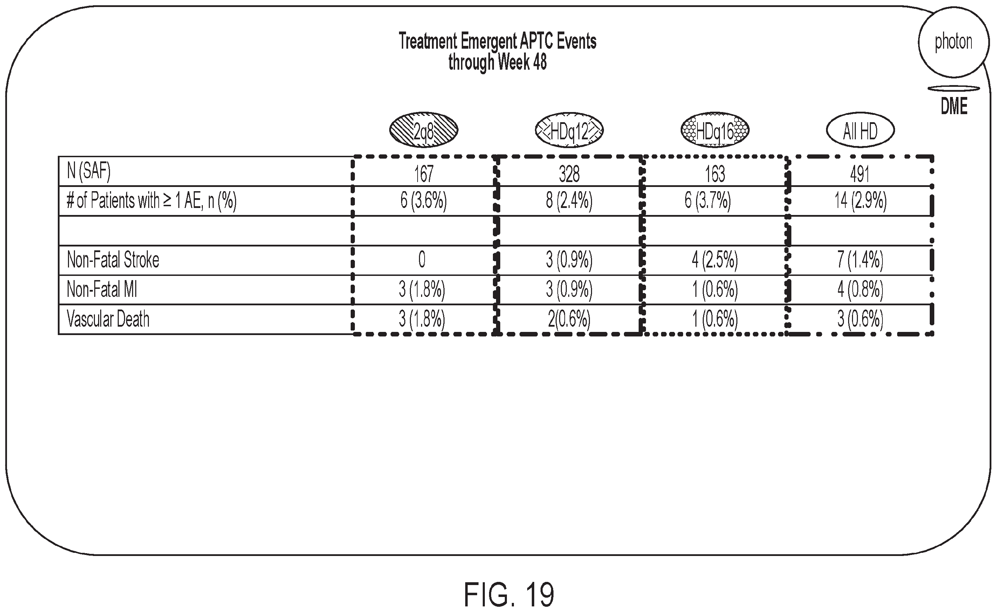

**Figure 19****:** Treatment emergent Anti-Platelet Trialists' Collaboration (APTC) events through week 48 in PHOTON clinical trial.

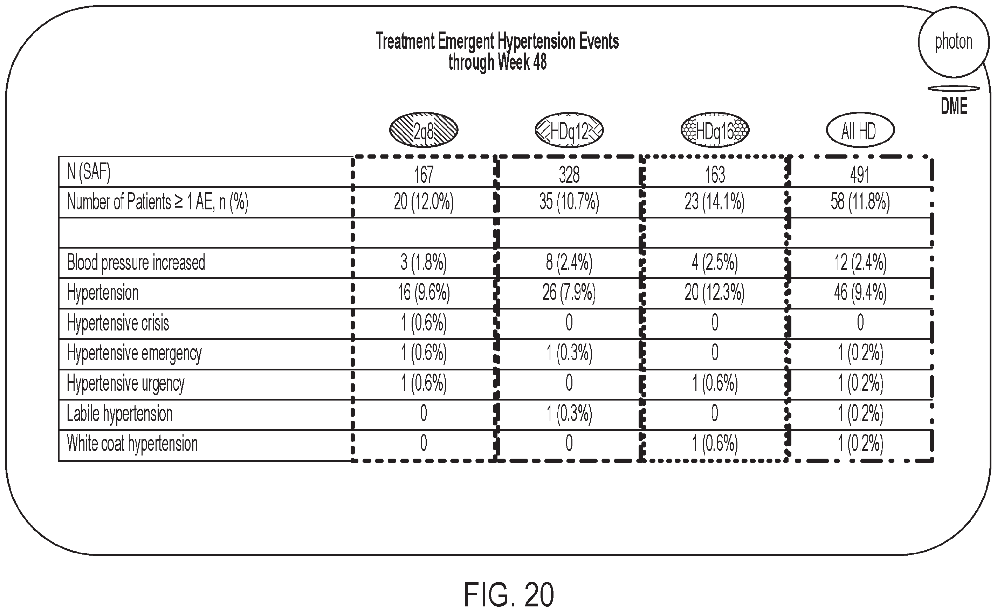

**Figure 20****:** Treatment emergent hypertension events though week 48 in PHOTON clinical trial.

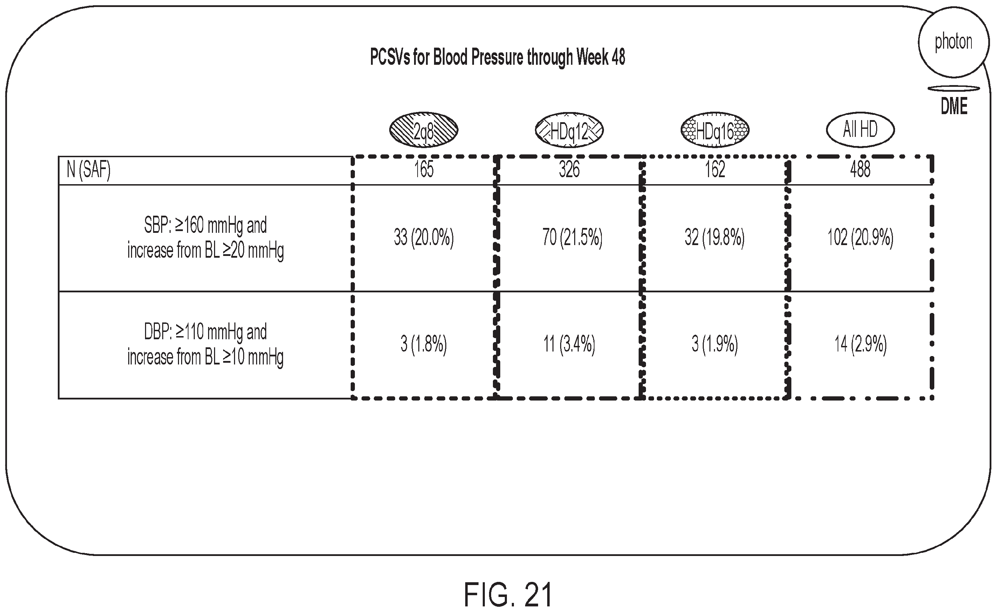

**Figure 21****:** Potentially Clinically Significant Values (PCSVs) for blood pressure through week 48 in PHOTON clinical trial.

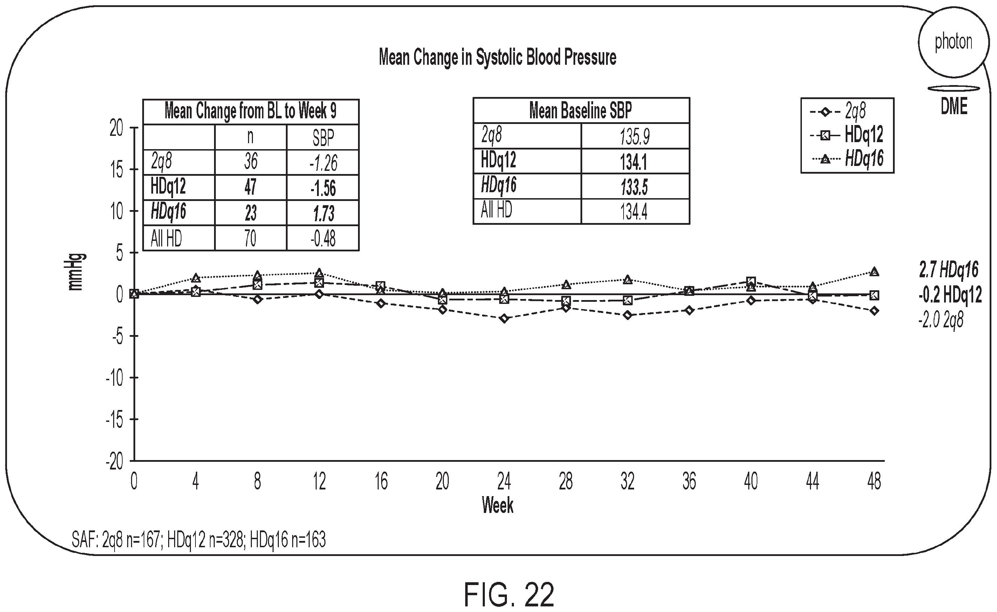

**Figure 22****:** Mean change from baseline in systolic blood pressure through week 48 in PHOTON clinical trial. Baseline to week 9 and mean baseline shown in insets.

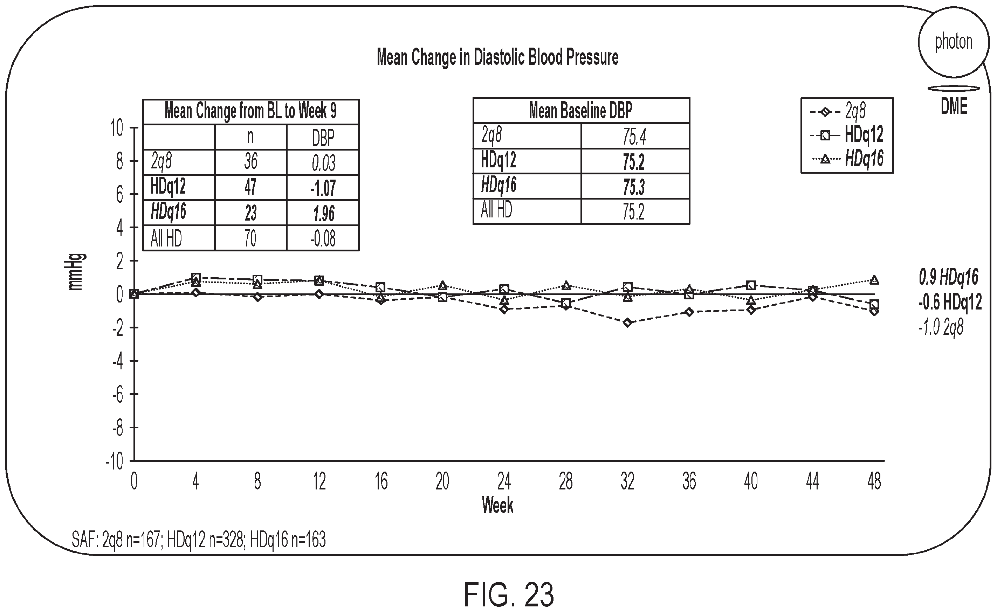

**Figure 23****:** Mean change from baseline in diastolic blood pressure through week 48 in PHOTON clinical trial. Baseline to week 9 and mean baseline shown in insets.

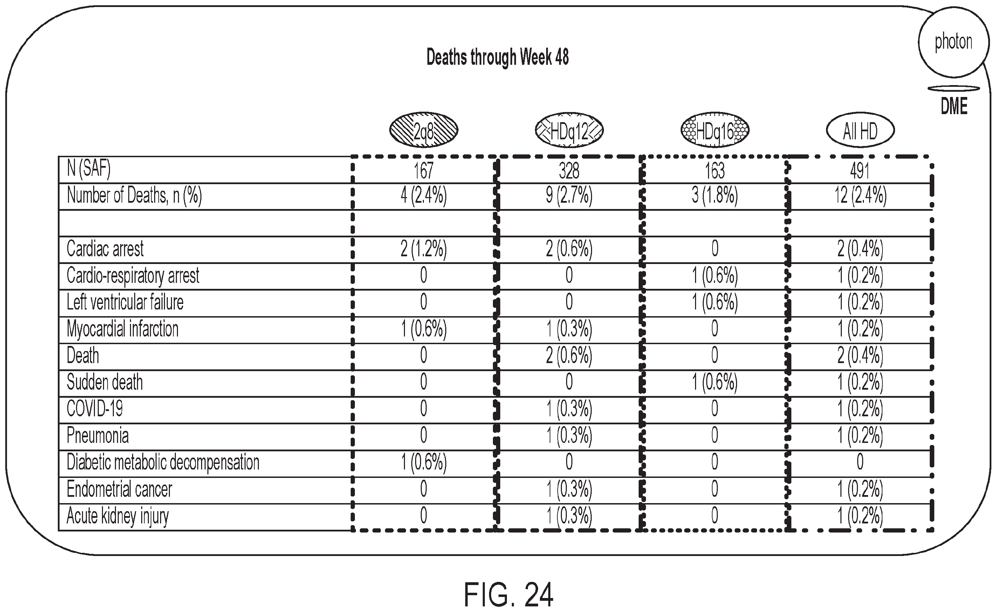

**Figure 24****:** Deaths through week 48 in PHOTON clinical trial.

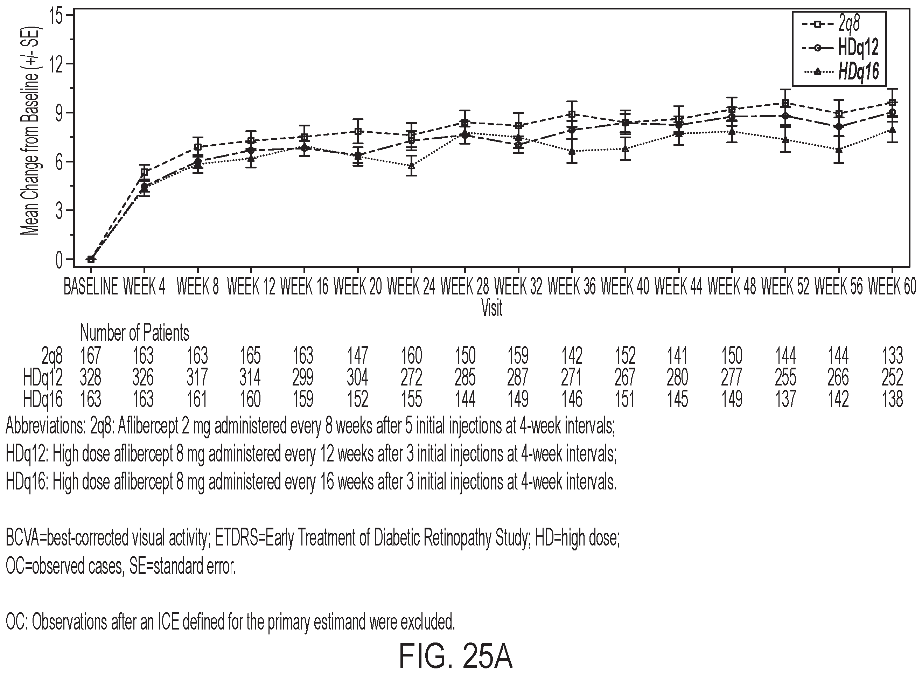

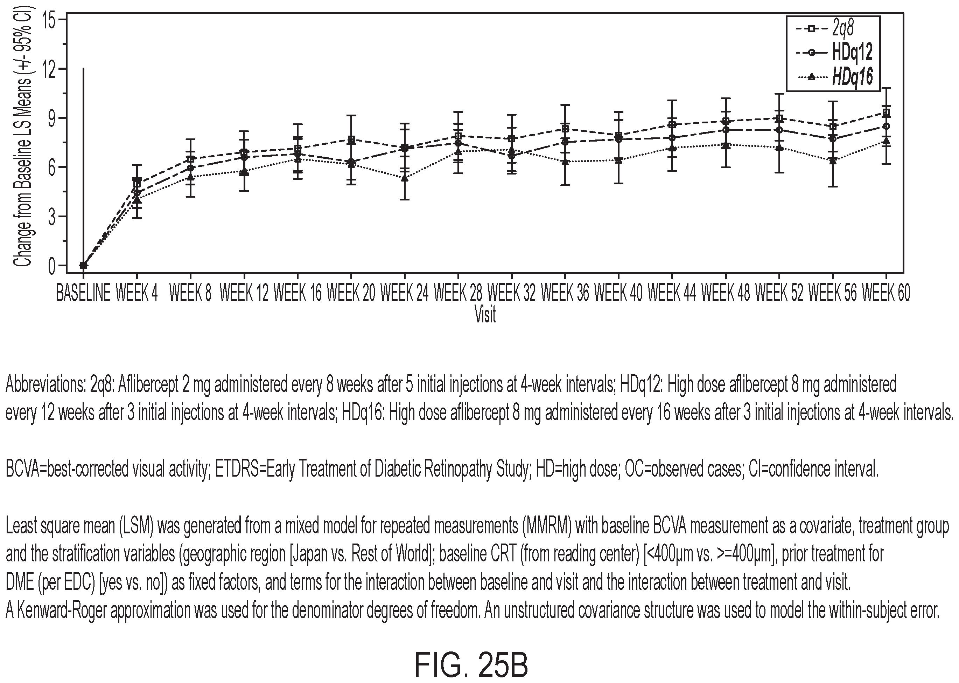

**Figures 25A** **and** **25B****:** (A)Mean Change from Baseline in BCVA Score (ETDRS Letters) in Study Eye through Week 60, OC (Full Analysis Set); (B) Least Square Mean Change from Baseline in BCVA Score (ETDRS Letters) in Study Eye through Week 60 (Full Analysis Set). Abbreviations: 2q8: Aflibercept 2 mg administered every 8 weeks after 5 initial injections at 4-week intervals; HDq12: High dose aflibercept 8 mg administered every 12 weeks after 3 initial injections at 4-week intervals; HDq16: High dose aflibercept 8 mg administered every 16 weeks after 3 initial injections at 4-week intervals. BCVA=best-corrected visual activity; ETDRS=Early Treatment of Diabetic Retinopathy Study; HD=high dose; OC=observed cases, SE=standard error. OC: Observations after an ICE defined for the primary estimand were excluded.

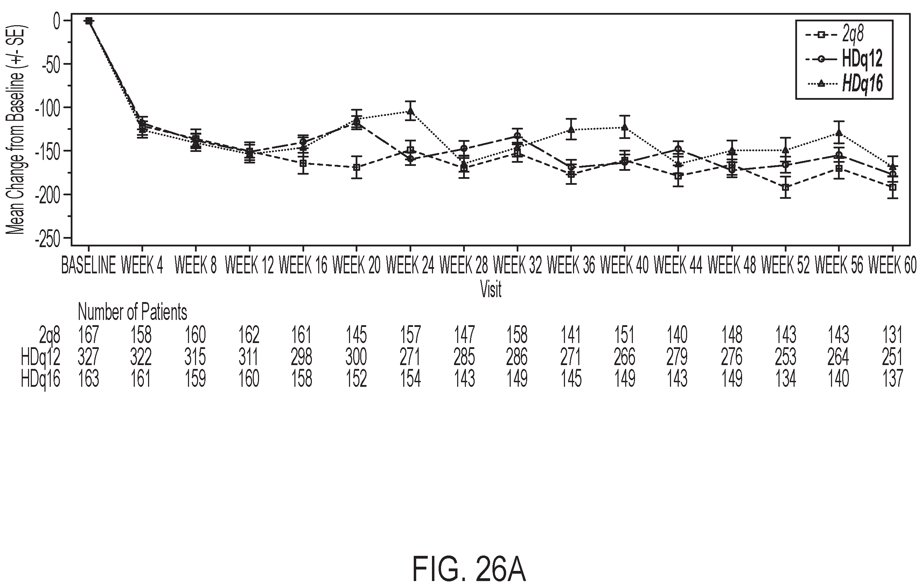

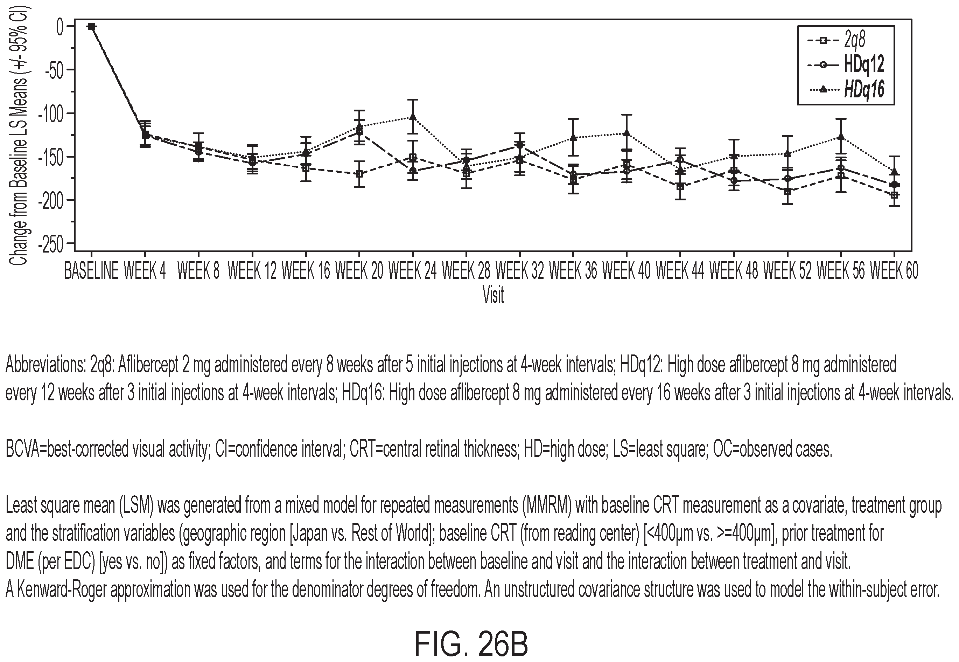

**Figures 26A** **and** **26B****:** (A) Mean Change from Baseline in Central Retinal Thickness (microns) by Visit through Week 60, OC (Full Analysis Set) (B) Least Square Mean Change from Baseline in Central Retinal Thickness (microns) by Visit through Week 60, OC (Full Analysis Set). Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. ICE=intercurrent events; OC=observed case; SE=standard error. OC= observations after an ICE defined for the primary estimand were excluded.

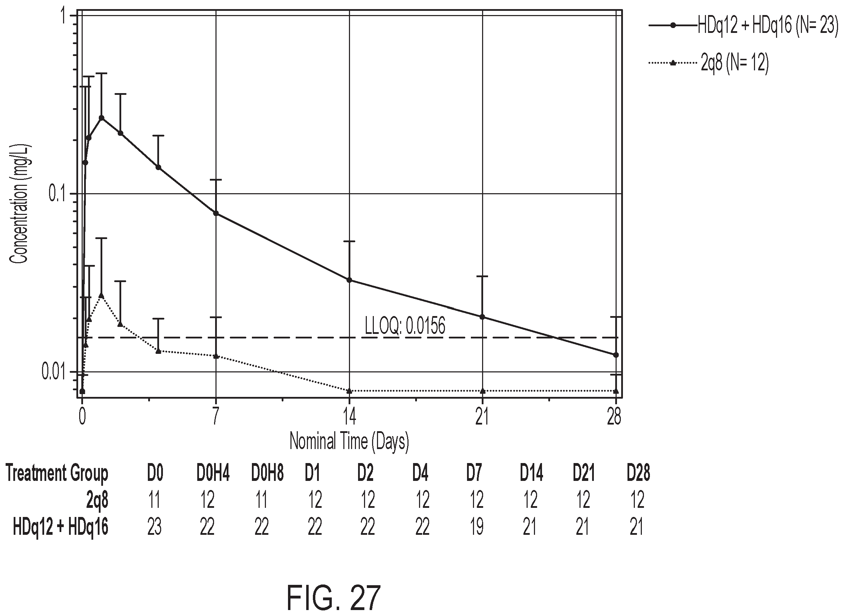

**Figure 27****:** Mean (+SD) Concentrations (mg/l) of Free Aflibercept in Plasma by Nominal Time and Treatment in Participants with DME with Unilateral Treatment in the Dense PK Sampling sub-study (Study VGFTe-HD-DME-1934, Log-Scaled, [DPKS]). N=Number of subjects; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses; LLOQ=Lower limit of quantitation; D=Day; H=Hour Note: Table under the figure presents the number of subjects contributing to the statistics for the corresponding visits/timepoints and treatments. Concentrations below the LLOQ were set to LLOQ/2. HDq12+HDq16 = combined data from treatment groups HDq12 and HDq16. Patients in the dense PK sub-study only received aflibercept injections unilaterally.

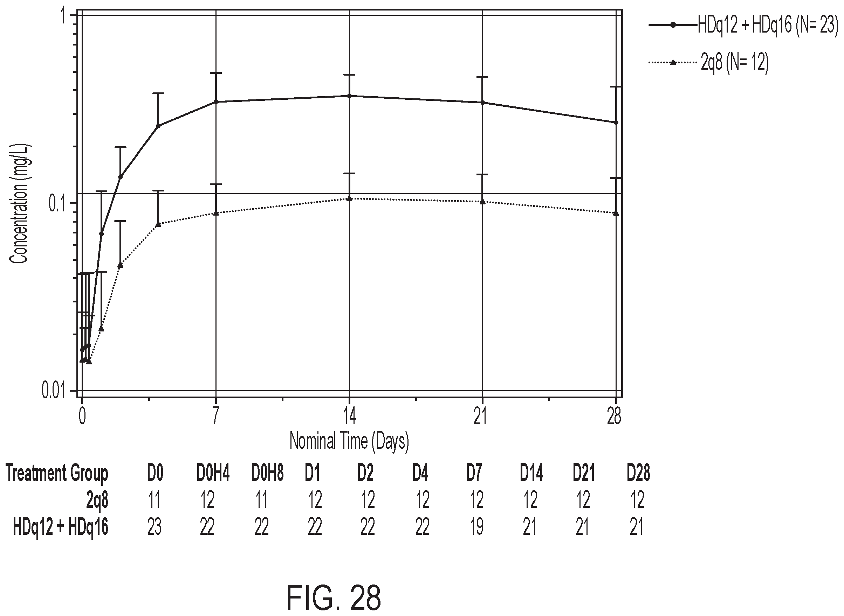

**Figure 28****:** Mean (+SD) Concentrations (mg/l) of Adjusted Bound Aflibercept in Plasma by Nominal Time and Treatment in Participants with DME with Unilateral Treatment in the Dense PK Sampling sub-study (Study VGFTe-HD-DME-1934, Log-Scaled, [DPKS]). N=Number of subjects; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses; LLOQ=Lower limit of quantitation; D=Day; H=Hour. Note: Table under the figure presents the number of subjects contributing to the statistics for the corresponding visits/timepoints and treatments. Concentrations below the LLOQ were set to LLOQ/2. Adjusted bound aflibercept = 0.717*bound aflibercept. HDq12+HDq16 = combined data from treatment groups HDq12 and HDq16. Patients in the dense PK sub-study only received aflibercept injections unilaterally.

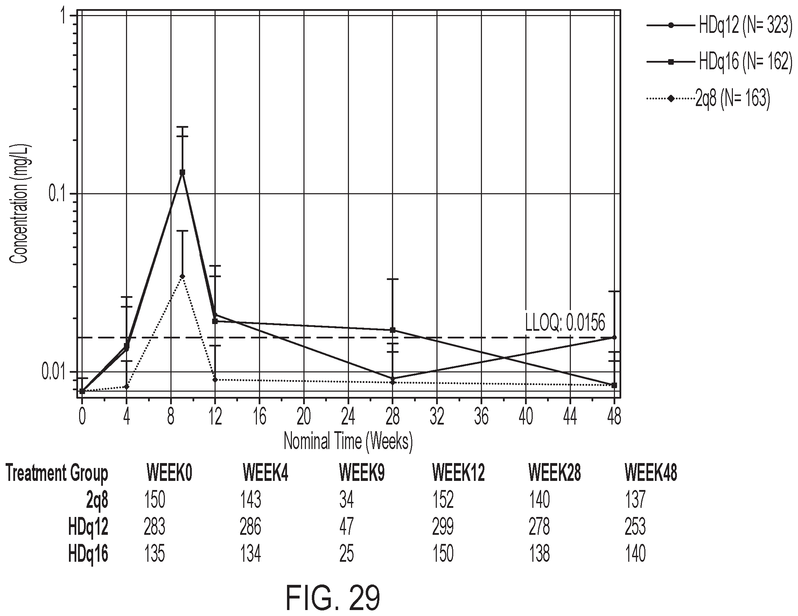

**Figure 29****:** Mean (+SD) Concentrations (mg/l) of Free Aflibercept in Plasma by Nominal Time and Treatment Group in Participants with DME in the Sparse PK Sampling Study (Study VGFTe-HD-DME-1934, Log-Scaled, [PKAS]). N=Number of subjects; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses; LLOQ=Lower limit of quantitation; PKAS=Pharmacokinetics analysis set. Note: Table under the figure presents the number of subjects contributing to the statistics for the corresponding visits/timepoints and treatment groups. Concentrations below the LLOQ were set to LLOQ/2. Post-dose samples and samples collected during the dense PK sub-study (from post dose on Day 0 through Day 21) were excluded.

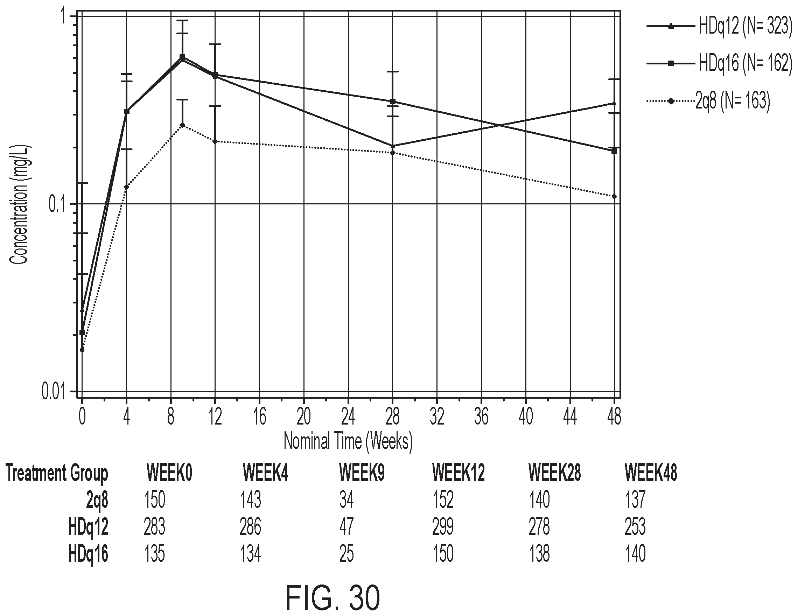

**Figure 30****:** Mean (+SD) Concentrations (mg/l) of Adjusted Bound Aflibercept in Plasma by Nominal Time and Treatment Group in Participants with DME in the Sparse PK Sampling Study (Study VGFTe-HD-DME-1934, Log-Scaled, [PKAS]); N=Number of subjects; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses; LLOQ =¾Lower limit of quantitation. Note: Table under the figure presents the number of subjects contributing to the statistics for the corresponding visits/timepoints and treatment groups. Concentrations below the LLOQ were set to LLOQ/2. Adjusted bound aflibercept = 0.717*bound aflibercept. Post-dose samples and samples collected during the dense PK sub-study (from post dose on Day 0 through Day 21) were excluded.

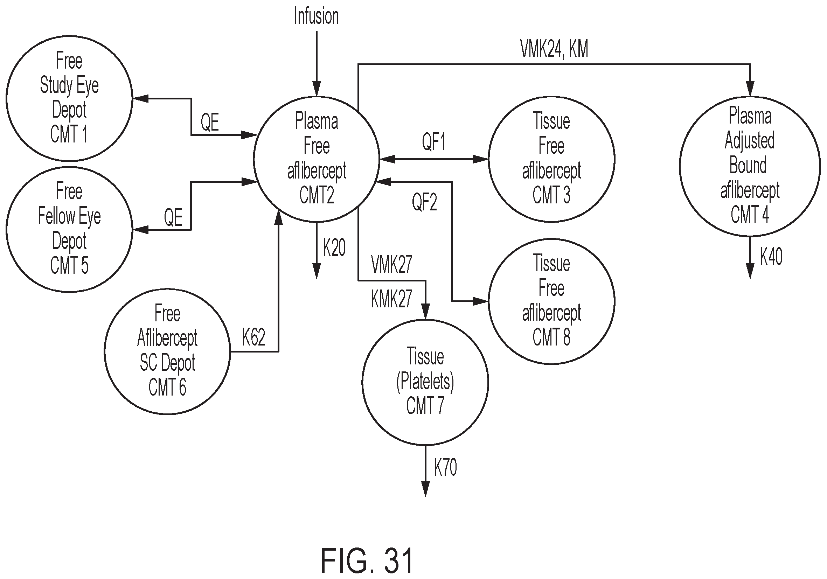

**Figure 31****:** Structural Representation of a Population Pharmacokinetic Model Following IV, SC, and IVT Administration of Aflibercept. CMT = compartment, IV = intravenous, IVT = intravitreal, K20 = elimination rate constant for free aflibercept, K40 = elimination rate constant for adjusted bound aflibercept, K62 = rate of absorption from subcutaneous injection depot compartment, K70 = elimination rate constant from tissue (platelet) compartment; QE = inter-compartmental clearance between ocular compartment and central compartment of free aflibercept, QF1 and QF2 = inter-compartmental clearances of free aflibercept, VMK24, KM = saturable Michaelis-Menten type binding of free aflibercept with VEGF; VMK27, KMK27 = saturable elimination from plasma compartment to tissue compartment (platelets) CMT 2 and CMT 4 are both representative of the plasma compartment and volumes are assumed to be equal.

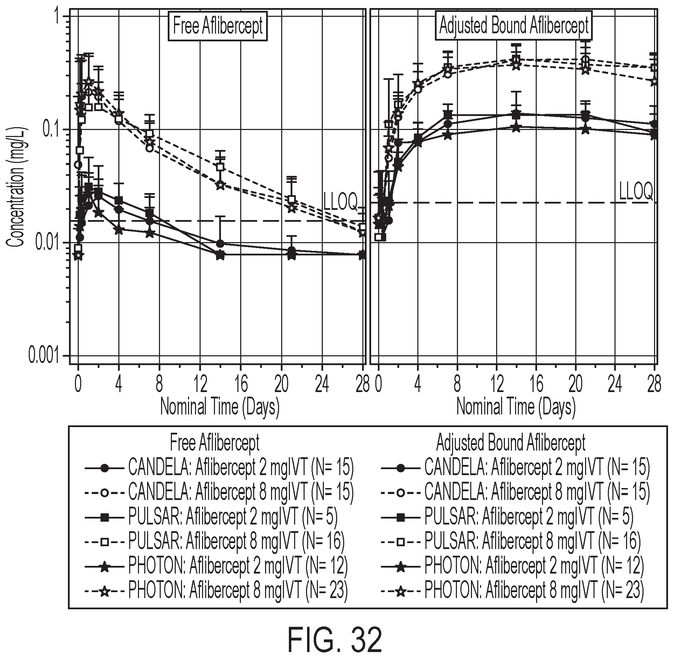

**Figure 32****:** Mean (+SD) Concentrations (mg/l) of Free and Adjusted Bound Aflibercept Over 28 Days for Single 2 mg and 8 mg IVT Doses of Aflibercept in nAMD or DME in the Dense PK Sub-studies (DPKS, Log-Scaled). LQ = below limit of quantification, DME = Diabetic Macular Edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, SD = standard deviation Adjusted Bound Aflibercept = 0.717*Bound Aflibercept Note: Concentrations below the LLOQ (0.0156 mg/L for Free and 0.0224 mg/L for Adjusted Bound Aflibercept) were set to LLOQ/2. Note: 8 mg HD aflibercept data for the first 28 days (obtained from PULSAR or PHOTON) is a combination of data from participants who received HDq12 or HDq16. One participant in PULSAR with an outlier free aflibercept concentration at day 28 that is greater than 10-fold of the mean concentration is excluded. Records after fellow-eye treatment are excluded. Data Source: drug concentration data from the week 48 database lock for PULSAR and PHOTON and final lock for CANDELA.

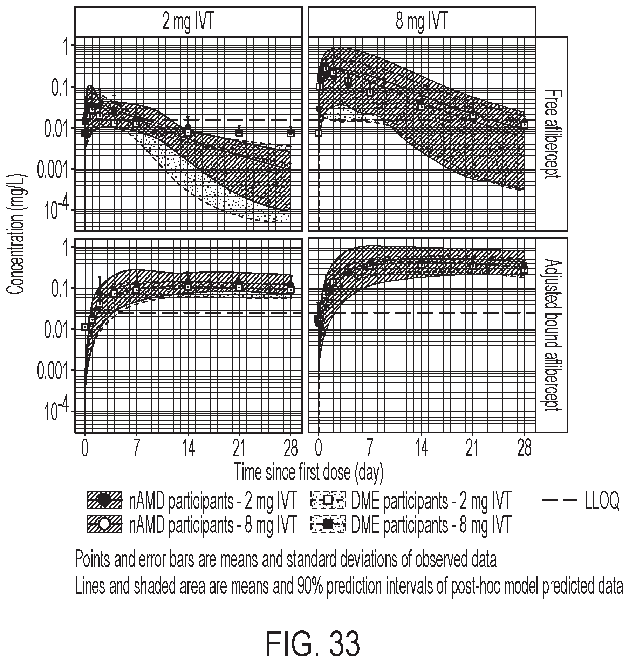

**Figure 33****:** Observed and Model-Predicted Concentrations (mg/l) of Free and Adjusted Bound Aflibercept in Plasma Over 28 days After a Single IVT Injection for Participants with nAMD or DME in the Dense PK Sub-studies (DPKS), Stratified by Dose and Population. DME = diabetic macular edema, IVT = intravitreally, LLOQ = lower limit of quantitation, nAMD = neovascular age-related macular degeneration, PK = pharmacokinetic Observed concentrations below the lower limit of quantitation (LLOQ; 0.0156 mg/L for free and 0.0224 mg/L for adjusted bound aflibercept) were set to LLOQ/2. Data source: Drug concentration data from dense PK sub-study in PHOTON, PULSAR, and CANDELA.

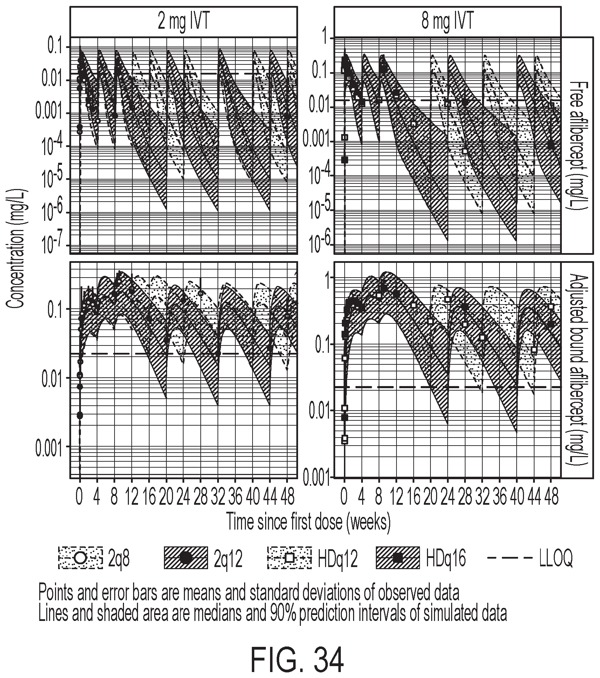

**Figure 34****:** Overlay of Observed and Model-Predicted Concentrations (mg/l) of Free and Adjusted Bound Aflibercept in Plasma for Combined nAMD and DME Populations. 2q8 = aflibercept 2 mg administered every 8 weeks, after 3 initial injections at 4-week intervals, 2q12 = aflibercept 2 mg administered every 12 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, nAMD = neovascular age-related macular degeneration Observed concentrations below the lower limit of quantitation (LLOQ; 0.0156 mg/L for free and 0.0224 mg/L for adjusted bound aflibercept) were set to LLOQ/2. Data source: Drug concentration data from CANDELA, PHOTON, and PULSAR.

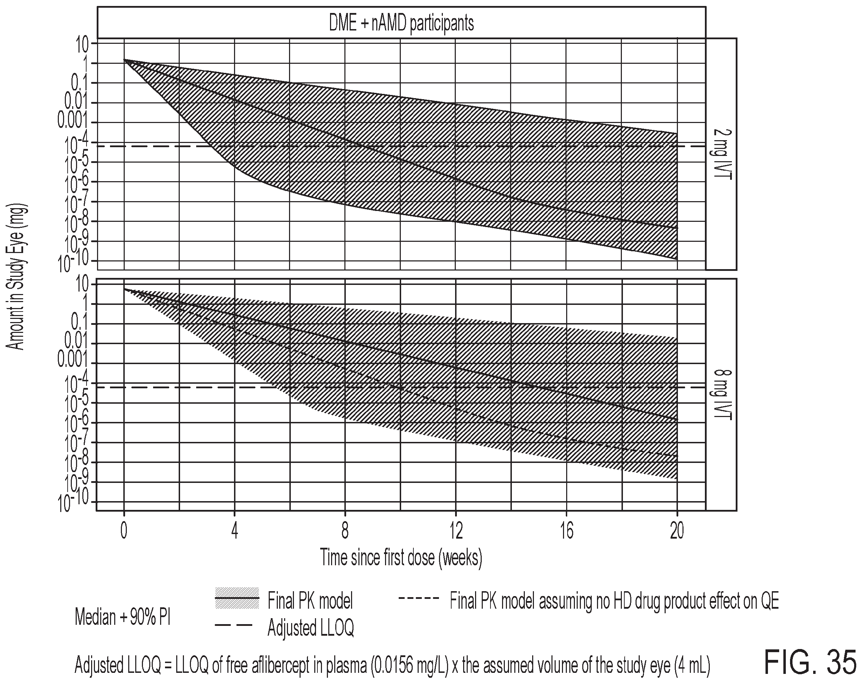

**Figure 35****:** Model-Predicted Amounts (mg) of Aflibercept Exposures After a Single IVT Injection, Stratified by Dosing Regimen in Combined Participants with nAMD and DME. DME = diabetic macular edema, HD = aflibercept 8 mg, IVT = intravitreal, nAMD = neurovascular age-related macular degeneration, PI = prediction interval, PK = pharmacokinetics, QE = inter-compartmental clearance between ocular compartment and central compartment of free aflibercept Adjusted LLOQ (0.0624 µg), set as the LLOQ of free aflibercept in plasma (that is, 0.0156 mg/L) times the assumed volume of the study eye compartment in the PK model (that is, 4 mL). Since the concentrations of (free or bound) aflibercept were not measured in the study eye in the clinical studies included in the Population PK analysis dataset, this target was selected arbitrarily on the basis of the LLOQ in plasma and was used as reference for comparison across dosing regimens and to assess the effect of the effect of HD aflibercept on QE.

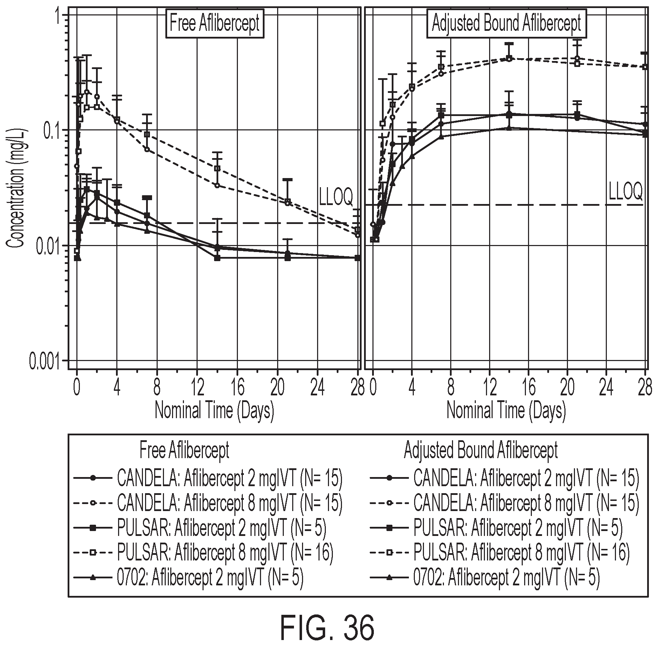

**Figure 36****:** Mean (±SD) Concentrations (mg/l) of Free and Adjusted Bound Aflibercept Over 28 Days for Single 2 mg and 8 mg IVT Doses of Aflibercept in Participants with nAMD in the Dense PK Sub-studies (DPKS, Log-Scaled) - No Outlier. DME = diabetic macular edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, PK = pharmacokinetic, SD = standard deviation Adjusted Bound Aflibercept = 0.717*Bound Aflibercept. Note: Concentrations below the LLOQ (0.0156 mg/L for Free and 0.0224 mg/L for Adjusted Bound Aflibercept) were set to LLOQ/2. Note: 8 mg data for the first 28 days (obtained from PULSAR or PHOTON) is a combination of data from participants who received HDq12 or HDq16 Data source: drug concentration from the week 48 lock for PULSAR and PHOTON and final lock for CANDELA. Records after fellow-eye treatment are excluded.

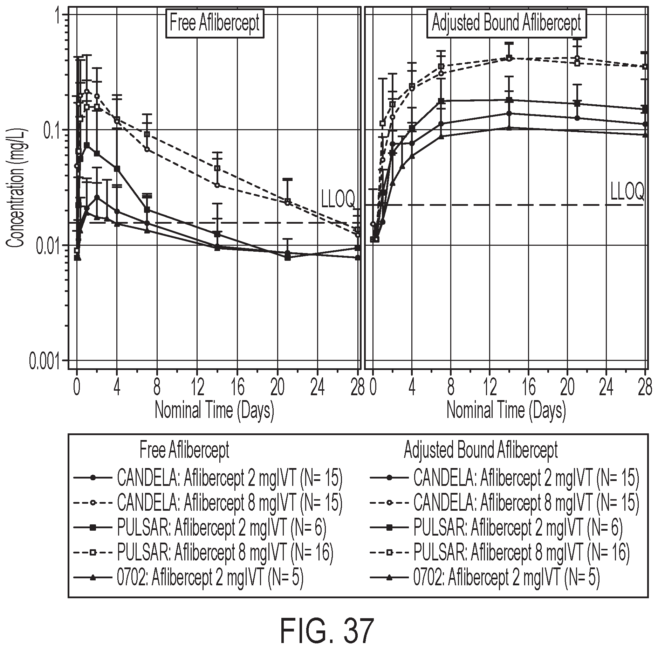

**Figure 37****:** Mean (+SD) Concentrations (mg/l) of Free and Adjusted Bound Aflibercept Over 28 Days for Single 2 mg and 8 mq IVT Doses of Aflibercept in Participants with nAMD in the Dense PK Sub-study (DPKS, Log-Scaled) -Outlier Included. DME = diabetic macular edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, PK = pharmacokinetic, SD = standard deviation Adjusted Bound Aflibercept = 0.717*Bound Aflibercept. Note: Concentrations below the lower limit of quantification (LLOQ, 0.0156 mg/L for Free and 0.0224 mg/L for Adjusted Bound Aflibercept) were set to LLOQ/2. Data source: drug concentration from the week 48 lock for PULSAR and final lock for CANDELA. Data from VGFTOD-0702 are included as a reference (the concentration in PK sub-study is subtracted by pre-dose concentration when it is >LLOQ). Records after fellow-eye treatment are excluded.

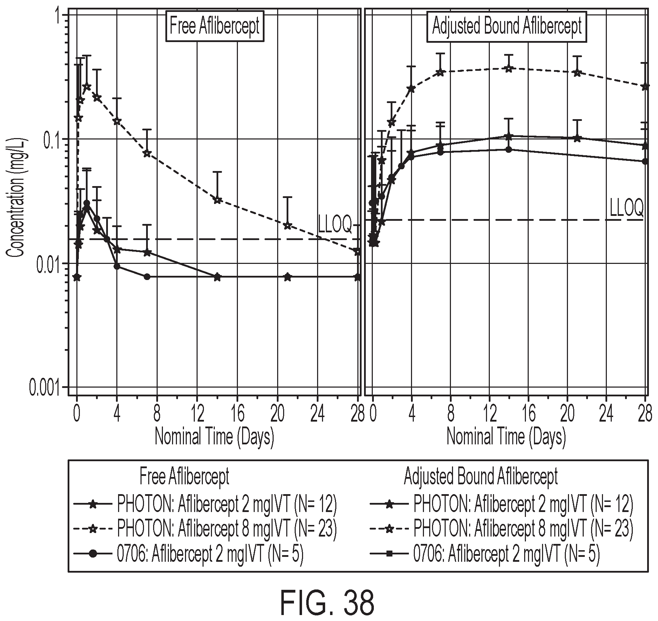

**Figure 38****:** Mean (+SD) Concentrations (mg/l) of Free and Adjusted Bound Aflibercept Over 28 Days for Single 2 mg and 8 mq IVT Doses of Aflibercept in Participants with DME in the Dense PK Sub-studies (DPKS, Log-Scaled). BLQ = below limit of quantification, DME = diabetic macular edema, DPKS = dense pharmacokinetic analysis set, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, SD = standard deviation Note: Concentrations below the LLOQ (0.0156 mg/L for Free and 0.0224 mg/L for Adjusted Bound Aflibercept) were set to LLOQ/2. Adjusted Bound Aflibercept = 0.717*Bound Aflibercept. Note: 8 mg data for the first 28 days (obtained from PULSAR or PHOTON) is a combination of data from participants who received HDq12 or HDq16. Note: The Concentration is subtracted by baseline concentration if participants took the Aflibercept prior to study drug started within 12 weeks and the baseline concentration is > BLQ. Data source: drug concentration data from the week 48 lock for PHOTON. Drug concentration data from VGFT-OD-0706 (historical data) are included as a reference. Records after fellow-eye treatment are excluded.

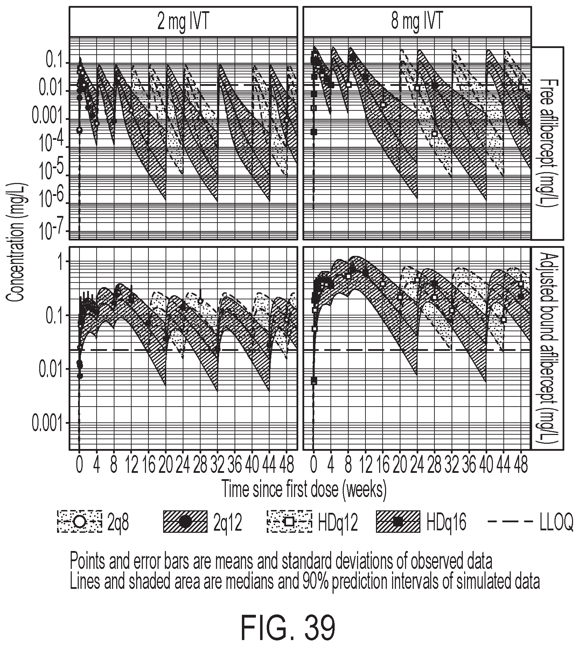

**Figure 39****:** Overlay of Observed and Model-Predicted Concentrations (mg/l) of Free and Adjusted Bound Aflibercept in Plasma for Participants with nAMD. 2q8 = aflibercept 2 mg administered every 8 weeks, after 3 initial injections at 4-week intervals, 2q12 = aflibercept 2 mg administered every 12 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantitation, nAMD = neovascular age-related macular degeneration, PK = pharmacokinetics Observed concentrations below the lower limit of quantitation (LLOQ; 0.0156 mg/L for free and 0.0224 mg/L for adjusted bound aflibercept) were set to LLOQ/2. Data from PULSAR and CANDELA.

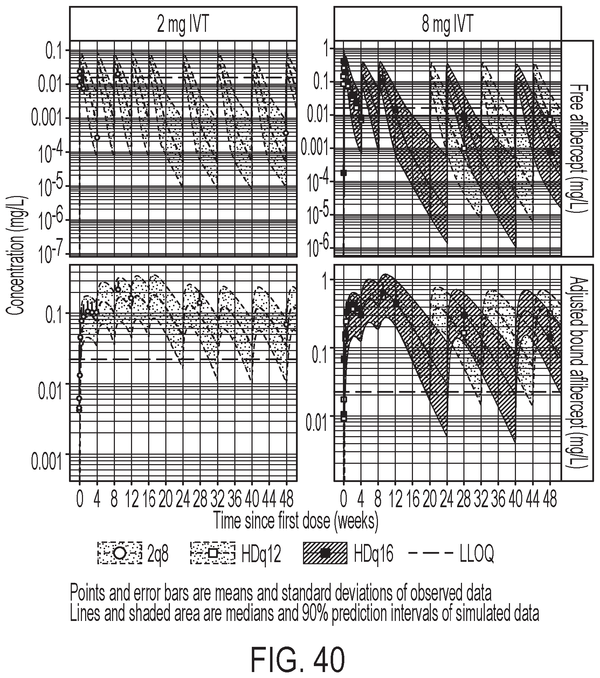

**Figure 40****:** Overlay of Observed and Model-Predicted Concentrations (mg/l) of Free and Adjusted Bound Aflibercept in Plasma for Participants with Diabetic Macular Edema in study PHOTON. 2q8 = aflibercept 2 mg administered every 8 weeks, after 3 initial injections at 4-week intervals, HDq12 = aflibercept 8 mg administered every 12 weeks, after 3 initial injections at 4-week intervals, HDq16 = aflibercept 8 mg administered every 16 weeks, after 3 initial injections at 4-week intervals, IVT = intravitreally, LLOQ = lower limit of quantitation Data from PHOTON.

### DETAILED DESCRIPTION OF THE INVENTION

The present invention provides, in part, a safe and effective high-dose aflibercept IVT injection which extends the maintenance dosing interval past 8 weeks, with at least similar functional and potentially improved anatomic outcomes. The regimen exhibited an unexpectedly high level of durability in subjects which exceeded that which would have been expected simply based on administration of more aflibercept.

EYLEA has become the standard-of-care for diabetic macular edema (DME) and diabetic retinopathy (DR). Eylea is prescribed for DME and DR at a dose of 2 mg once a month for 5 doses followed by maintenance dosing every 8 weeks. The dosing regimen of the present invention has demonstrated that a remarkably high percentage of subjects can be maintained on 12- and 16-week dosing intervals. In trials testing these dosing regimens, nearly 90% of subjects with diabetic macular edema were able to maintain a 16-week dosing regimen. These durability data coupled with a safety profile consistent with that of EYLEA support high-dose aflibercept as a potential new standard-of-care in angiogenic eye disorders such as DR or DME The data presented herein demonstrated that aflibercept 8 mg 12- and 16-week dosing regimens have achieved a high bar, sustaining improvements in visual acuity and anatomic measures of retinal fluid across 48 weeks in subjects with diabetic macular edema. These results were all achieved in subjects who were rapidly initiated on extended dosing intervals with the vast majority not requiring regimen modification. Altogether, the pivotal data support aflibercept 8 mg as providing a longer duration of action while maintaining a safety profile similar to EYLEA.

Prior to initiating the HD aflibercept clinical development program, pharmacokinetic simulations of free aflibercept concentration-time profiles in human vitreous using a 1-compartment ocular model predicted that an 8 mg IVT dose of aflibercept could extend the dosing interval by approximately 20 days (two half-lives) relative to a 2 mg IVT dose. The aflibercept HDq12 and HDq16 regimens exhibited a duration of efficacy in the HD clinical studies that was longer than predicted. A subsequent population PK analysis that integrated data from the CANDELA PHOTON and PULSAR phase 3 studies indicated that ocular clearance of free aflibercept was 34% slower for the HD aflibercept drug product compared to 2 mg IVT aflibercept administered as the Eylea drug product, and the slower ocular clearance for HD aflibercept was predicted to result in both a longer persistence of free aflibercept in the eye and an approximate 6-week longer duration of efficacy compared to 2 mg. The magnitude of reduction in ocular clearance for the HD aflibercept drug product compared to the 2 mg Eylea drug product was greater than expected and attributed to an "HD aflibercept drug product effect", a highly statistically significant effect in the population PK model that cannot be explained by just an increase in the dose from 2 mg to 8 mg.

The Population PK predicted median time for free aflibercept concentrations in plasma to reach the lower limit of quantification (LLOQ) following 2 mg IVT aflibercept was estimated to be 1.5 weeks compared to 3.50 weeks for 8 mg HD aflibercept. The longer duration of systemic exposure to free aflibercept, representative of the movement of free aflibercept from the eye, for the HD aflibercept regimen was attributed to not only a higher administered dose and nonlinear systemic target-mediated elimination, but also to a 34% slower ocular clearance of free aflibercept. The slower ocular clearance of the HD aflibercept drug product was predicted to provide a 6-week longer duration of efficacy compared to that of the 2 mg aflibercept drug product, as the population PK estimated time to achieve the free aflibercept amount in the ocular compartment for the 2q8 regimen at the end of an 8-week dosing interval occurs 6 weeks later for the HD aflibercept drug product. Exposure-response analyses estimated that the slower ocular clearance for 8 mg aflibercept, attributable to the HD drug product effect, resulted in a 20.6% lower rate of dosing regimen modification (DRM) than would have been expected if the HD drug product had the same ocular clearance as 2 mg aflibercept.

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, N.Y.; Sambrook and Russell (2001) Molecular Cloning, 3rd ed., Cold Spring Harbor Laboratory Press, Cold Spring Harbor, N.Y.; Wu (1993) Recombinant DNA, Vol. 217, Academic Press, San Diego, Calif.). Standard methods also appear in Ausbel, et al. (2001) Current Protocols in Molecular Biology, Vols. 1-4, John Wiley and Sons, Inc. New York, N.Y., which describes cloning in bacterial cells and DNA mutagenesis (Vol. 1), cloning in mammalian cells and yeast (Vol. 2), glycoconjugates and protein expression (Vol. 3), and bioinformatics (Vol. 4).

General methods for protein purification including immunoprecipitation, chromatography, electrophoresis, centrifugation, and crystallization are described (Coligan et al. (2000) Current Protocols in Protein Science, Vol. 1, John Wiley and Sons, Inc., New York). Chemical analysis, chemical modification, post-translational modification, production of fusion proteins, glycosylation of proteins are described (see *e.g*., Coligan et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, N.Y., pp. 16.0.5-16.22.17; Sigma-Aldrich, Co. (2001) Products for Life Science Research, St. Louis, Mo.; pp. 45-89; Amersham Pharmacia Biotech (2001) BioDirectory, Piscataway, N.J., pp. 384-391). Production, purification, and fragmentation of polyclonal and monoclonal antibodies are described (Coligan et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, N.Y.; Harlow and Lane, *supra).* Standard techniques for characterizing ligand/receptor interactions are available (see, *e.g*., Coligan et al. (2001) Current Protocols in Immunology, Vol. 4, John Wiley, Inc., New York).

Methods for flow cytometry, including fluorescence activated cell sorting (FACS), are available (see, *e.g*., Owens et al. (1994) Flow Cytometry Principles for Clinical Laboratory Practice, John Wiley and Sons, Hoboken, N.J.; Givan (2001) Flow Cytometry, 2nd ed.; Wiley-Liss, Hoboken, N.J.; Shapiro (2003) Practical Flow Cytometry, John Wiley and Sons, Hoboken, N.J.). Fluorescent reagents suitable for modifying nucleic acids, including nucleic acid primers and probes, polypeptides, and antibodies, for use, *e.g*., as diagnostic reagents, are available (Molecular Probes (2003) Catalogue, Molecular Probes, Inc., Eugene, Oreg.; Sigma-Aldrich (2003) Catalogue, St. Louis, Mo.).

Standard methods of histology of the immune system are described (see *e.g*., Muller-Harmelink (ed.) (1986) Human Thymus: Histopathology and Pathology, Springer Verlag, New York, N.Y.; Hiatt et al. (2000) Color Atlas of Histology, Lippincott, Williams, and Wilkins, Phila, Pa.; Louis et al. (2002) Basic Histology: Text and Atlas, McGraw-Hill, New York, N.Y.).

"Isolated" VEGF antagonists and VEGF receptor fusion proteins (*e.g*., aflibercept), polypeptides, polynucleotides and vectors, are at least partially free of other biological molecules from the cells or cell culture from which they are produced. Such biological molecules include nucleic acids, proteins, other VEGF antagonists and VEGF receptor fusion proteins, lipids, carbohydrates, or other material such as cellular debris and growth medium. An isolated VEGF antagonist or VEGF receptor fusion protein may further be at least partially free of expression system components such as biological molecules from a host cell or of the growth medium thereof. Generally, the term "isolated" is not intended to refer to a complete absence of such biological molecules (*e.g*., minor or insignificant amounts of impurity may remain) or to an absence of water, buffers, or salts or to components of a pharmaceutical formulation that includes the VEGF antagonists or VEGF receptor fusion proteins.

Subject and patient are used interchangeably herein. A subject or patient is a mammal, for example a human, mouse, rabbit, monkey or non-human primate, preferably a human. A subject or patient may be said to be "suffering from" an angiogenic eye disorder such as nAMD. Such a subject or patient has the disorder in one or both eyes. In an embodiment of the invention, a subject or patient (preferably a human) has one or more of the following characteristics (presently or in the past):
1. ≥50 years of age, *e.g*., 61, 62, 63, 74 or 75;
2. Has active subfoveal CNV secondary to nAMD, *e.g*., including juxtafoveal lesions that affect the fovea in an eye;
3. Has Best Corrected Visual Acuity (BCVA) Early Treatment Diabetic Retinopathy Study (ETDRS) letter score of about 78 to 24, 73-78, <73, 58, 59, 60, 61, 62 or 63 (Snellen equivalent of 20/40, 20/63, 20/50, 20/32 or 20/320), *e.g*., due to DME or wet AMD;
4. Central retinal thickness of ≥300 micrometers or ≥320 micrometers, or about 367, 368, 369, 370, 450, 451, 452, 453, 454 or 455 micrometers; or and/or DME with central involvement in an eye with CRT ≥300 micrometers (or ≥320 micrometers on Spectralis);
5. Total lesion area of about 6 or 7 mm², *e.g*., wherein the lesion type is occult, predominantly classic or minimally classic;
6. DRSS score of better or equal to Level 43, Level 47 or worse;
7. Type 1 or type 2 diabetes mellitus (insulin dependent or non-insulin dependent) (*e.g*., for about 15 or more years);
8. Hemoglobin A1C (%) of about 7 or 8 or more;
9. Body mass index of about 30 or 31 or more; and/or
10. A history of diabetic retinal oedema, diabetic retinopathy, dry eye, vitreous detachment, retihopathy hypersensitive, retinal hemorrhage, cataract operation, retinal laser coagulation, and intraocular lens implant, hypertension,
and/or, has or lacks any one or more of the following characteristics:
1. Evidence of macular edema due to any cause other than diabetes mellitus in an eye;
2. IOP ≥ 25 mmHg in an eye;
3. History of glaucoma filtration surgery in the past, or likely to need filtration surgery in the future in an eye;
4. Evidence of infectious blepharitis, keratitis, scleritis, or conjunctivitis in either eye within 4 weeks (28 days) of treatment;
5. Any intraocular inflammation and/or ocular infection in an eye within 12 weeks (84 days) of treatment;
6. History of idiopathic or autoimmune uveitis in an eye;
7. Vitreomacular traction or epiretinal membrane in an eye, *e.g*., as evident on biomicroscopy or OCT that is thought to affect central vision;
8. Preretinal fibrosis involving the macula in an eye;
9. Any history of macular hole of stage 2 and above in an eye;
10. Current iris neovascularization, vitreous hemorrhage, or tractional retinal detachment visible at the screening assessments in an eye;
11. History of corneal transplant or corneal dystrophy in an eye;
12. Any concurrent ocular condition in an eye which, in the opinion of the treating physician, could either increase the risk to the subject beyond what is to be expected from standard procedures of IVT injections, or which otherwise may interfere with the VEGF antagonist injection procedure;
13. History of other disease, metabolic dysfunction, physical examination finding, or clinical laboratory finding giving reasonable suspicion of a disease or condition that contraindicates the use of the VEGF antagonist;
14. Any prior systemic (IV) anti-VEGF administration;
15. Uncontrolled diabetes mellitus as defined by hemoglobin A1c (HbA1c) >12%;
16. Uncontrolled blood pressure (defined as systolic >160 mmHg or diastolic >95 mmHg);
17. History of cerebrovascular accident or myocardial infarction within 24 weeks (168 days) of treatment;
18. Renal failure, dialysis, or history of renal transplant;
19. Known sensitivity to any of the compounds to be administered in treatment; and/or
20. Pregnant or breastfeeding woman

Thus, the present invention includes a method for treating or preventing DR and/or DME, in a subject in need thereof
1. who is ≥50 years of age;
2. who has active subfoveal CNV;
3. who has Best Corrected Visual Acuity (BCVA) Early Treatment Diabetic Retinopathy Study (ETDRS) letter score of about 78 to 24;
4. who has a central retinal thickness of ≥300 micrometers or ≥320 micrometers;
5. who has a lesion area of about 6 or 7 mm²;
6. who has a DRSS score of better or equal to Level 43, Level 47 or worse; and/or
7. has Type 1 or type 2 diabetes mellitus;
and/or,
1. who lacks evidence of macular edema due to any cause other than diabetes mellitus in an eye;
2. does not have an IOP ≥ 25 mmHg in an eye;
3. who does not have a history of glaucoma filtration surgery in the past, or is not likely to need filtration surgery in the future in an eye;
4. who does not have evidence of infectious blepharitis, keratitis, scleritis, or conjunctivitis in either eye within 4 weeks (28 days) of treatment;
5. who does not have any intraocular inflammation and/or ocular infection in an eye within 12 weeks (84 days) of treatment;
6. who does not have a history of idiopathic or autoimmune uveitis in an eye;
7. who does not have vitreomacular traction or epiretinal membrane in an eye;
8. who does not have preretinal fibrosis involving the macula in an eye;
9. who does not have any history of macular hole of stage 2 and above in an eye;
10. who does not have current iris neovascularization, vitreous hemorrhage, and/or tractional retinal detachment;
11. who does not have a history of corneal transplant or corneal dystrophy in an eye;
12. who does not have any concurrent ocular condition in an eye which, in the opinion of the treating physician, could either increase the risk to the subject beyond what is to be expected from standard procedures of IVT injections, or which otherwise may interfere with the VEGF antagonist injection procedure;
13. who does not have a history of other disease, metabolic dysfunction, physical examination finding, or clinical laboratory finding giving reasonable suspicion of a disease or condition that contraindicates the use of the VEGF antagonist;
14. who has not had any prior systemic (IV) anti-VEGF administration;
15. who does not have uncontrolled diabetes mellitus as defined by hemoglobin A1c (HbA1c) >12%;
16. who does not have uncontrolled blood pressure (defined as systolic >160 mmHg or diastolic >95 mmHg);
17. who does not have a history of cerebrovascular accident or myocardial infarction within 24 weeks (168 days) of treatment;
18. who does not have renal failure, dialysis, or history of renal transplant;
19. who does not have a known sensitivity to any of the compounds to be administered in treatment; and/or
20. who is not pregnant or a breastfeeding woman;
comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks after the immediately preceding dose.

### VEGF Antagonists

The present invention includes methods for using a VEGF antagonist for treating or preventing angiogenic eye disorders. VEGF antagonists include molecules which interfere with the interaction between VEGF and a natural VEGF receptor, e.g., molecules which bind to VEGF or a VEGF receptor and prevent or otherwise hinder the interaction between VEGF and a VEGF receptor. Specific, exemplary VEGF antagonists include anti-VEGF antibodies, anti-VEGF receptor antibodies, and VEGF receptor fusion proteins. Though VEGF receptor fusion proteins, such as aflibercept, are preferred for use in connection with the methods set forth herein, the scope of the present invention includes such methods wherein any of the VEGF antagonists described herein (*e.g*., scFvs, DARPins, anti-VEGF antibodies) are used in place of such fusion proteins.

For purposes herein, a "VEGF receptor fusion protein" refers to a molecule that comprises one or more VEGF receptors or domains thereof, fused to another polypeptide, which interferes with the interaction between VEGF and a natural VEGF receptor, *e.g*., wherein two of such fusion polypeptides are associated thereby forming a homodimer or other multimer. Such VEGF receptor fusion proteins may be referred to as a "VEGF-Trap" or "VEGF Trap". VEGF receptor fusion proteins within the context of the present disclosure that fall within this definition include chimeric polypeptides which comprise two or more immunoglobulin (Ig)-like domains of a VEGF receptor such as VEGFR1 (also known as Flt1) and/or VEGFR2 (also known as Flk1 or KDR), and may also contain a multimerizing domain (for example, an Fc domain).

An exemplary VEGF receptor fusion protein is a molecule referred to as VEGF1R2-FcΔC1(a) which is encoded by the nucleic acid sequence of SEQ ID NO:1 or nucleotides 79-1374 or 79-1371 thereof.

VEGF1 R2-FcΔC1 (a) comprises three components:
(1) a VEGFR1 component comprising amino acids 27 to 129 of SEQ ID NO:2;
(2) a VEGFR2 component comprising amino acids 130 to 231 of SEQ ID NO:2; and
(3) a multimerization component ("FcΔC1(a)") comprising amino acids 232 to 457 of SEQ ID NO:2 (the C-terminal amino acids of SEQ ID NO:2, *i.e*., K458, may or may not be included in the VEGF receptor fusion proteins, see U.S. Patent No. 7,396,664 or 7,354,579, incorporated herein for all purposes). Note that amino acids 1 to 26 of SEQ ID NO:2 are the signal sequence.

If the multimerizing component (MC) of a VEGF receptor fusion protein is derived from an IgG (*e.g*., IgG1) Fc domain, then the MC has no fewer amino acids than are in amino acids 232 to 457 of SEQ ID NO:2. Thus, the IgG of the MC cannot be truncated to be shorter than 226 amino acids.

In an embodiment of the invention, the VEGF receptor fusion protein comprises amino acids 27-458 or 27-457 of SEQ ID NO: 2 (*e.g*., in the form of a homodimer).

atggtcagctactgggacaccggggtcctgctgtgcgcgctgctcagctgtctgcttctcacaggatctagttccgg
aagtgataccggtagacctttcgtagagatgtacagtgaaatccccgaaattatacacatgactgaaggaagggagc
tcgtcattccctgccgggttacgtcacctaacatcactgttactttaaaaaagtttccacttgacactttgatccct
gatggaaaacgcataatctgggacagtagaaagggcttcatcatatcaaatgcaacgtacaaagaaatagggcttct
gacctgtgaagcaacagtcaatgggcatttgtataagacaaactatctcacacatcgacaaaccaatacaatcatag
atgtggttctgagtccgtctcatggaattgaactatctgttggagaaaagcttgtcttaaattgtacagcaagaact
gaactaaatgtggggattgacttcaactgggaatacccttcttcgaagcatcagcataagaaacttgtaaaccgaga
cctaaaaacccagtctgggagtgagatgaagaaatttttgagcaccttaactatagatggtgtaacccggagtgacc
aaggattgtacacctgtgcagcatccagtgggctgatgaccaagaagaacagcacatttgtcagggtccatgaaaag
gacaaaactcacacatgcccaccgtgcccagcacctgaactcctggggggaccgtcagtcttcctcttccccccaaa
acccaaggacaccctcatgatctcccggacccctgaggtcacatgcgtggtggtggacgtgagccacgaagaccctg
aggtcaagttcaactggtacgtggacggcgtggaggtgcataatgccaagacaaagccgcgggaggagcagtacaac
agcacgtaccgtgtggtcagcgtcctcaccgtcctgcaccaggactggctgaatggcaaggagtacaagtgcaaggt
ctccaacaaagccctcccagcccccatcgagaaaaccatctccaaagccaaagggcagccccgagaaccacaggtgt
acaccctgcccccatcccgggatgagctgaccaagaaccaggtcagcctgacctgcctggtcaaaggcttctatccc

agcgacatcgccgtggagtgggagagcaatgggcagccggagaacaactacaagaccacgcctcccgtgctggactc
cgacggctccttcttcctctacagcaagctcaccgtggacaagagcaggtggcagcaggggaacgtcttctcatgct
ccgtgatgcatgaggctctgcacaaccactacacgcagaagagcctctccctgtctccgggtaaatga
(SEQ ID NO: 1)

MVSYWDTGVLLCALLSCLLLTGSSSGSDTGRPFVEMYSEIPEIIHMTEGRELVIPCRVTS
PNITVTLKKFPLDTLIPDGKRIIWDSRKGFIISNATYKEIGLLTCEATVNGHLYKTNYLT
HRQTNTIIDVVLSPSHGIELSVGEKLVLNCTARTELNVGIDFNWEYPSSKHQHKKLVNRD
LKTQSGSEMKKFLSTLTIDGVTRSDQGLYTCAASSGLMTKKNSTFVRVHEKDKTHTCPPC
PAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
(SEQ ID NO: 2)

In an embodiment of the invention, the VEGF receptor fusion protein comprises
(1) an immunoglobin-like (Ig) domain 2 of a first VEGF receptor (*e.g*., VEGFR1), and
(2) an Ig domain 3 of a second VEGF receptor (*e.g*., VEGFR2),
(3) and, optionally, further including an Ig domain 4 of the second VEGF receptor (*e.g*., VEGFR2) and
(4) a multimerizing component (*e.g*., Fc domain of IgG including the hinge, CH2 and CH3 domains).

For example, in an embodiment of the invention, the VEGF receptor fusion protein has the following arrangement of said domains:
- [VEGFR1 Ig domain 2]-[VEGFR2 Ig domain 3]-[MC] (*e.g*., a homodimer thereof) or
- [VEGFR1 Ig domain 2]-[VEGFR2 Ig domain 3]-[VEGFR2 Ig domain 4]-[MC] (*e.g*., a homodimer thereof).

Note that the present disclosure also includes, within its scope, high concentration formulations including, instead of a VEGF receptor fusion protein, a VEGF binding molecule or anti-VEGF antibody or antigen-binding fragments thereof or biopolymer conjugate thereof (*e.g*., KSI-301), *e.g*.,
- bevacizumab (*e.g*., at a concentration of about 80-90 or 88 mg/ml),
- ranibizumab (*e.g*., at a concentration of about 20-40 mg/ml, *e.g*., 21-35, 21 or 35 mg/ml),
- an anti-VEGF aptamer such as pegaptanib (*e.g*., pegaptanib sodium),
- a single chain (*e.g*., V_{L}-V_{H}) anti-VEGF antibody such as brolucizumab (*e.g*., at a concentration of about 200-400 or 200, 210, 400 or 420 mg/ml),
- an anti-VEGF DARPin such as the Abicipar Pegol DARPin (*e.g*., at a concentration of about 70-140, 70 or 140 mg/ml), or
- a bispecific anti-VEGF antibody, *e.g*., which also binds to ANG2, such as RG7716 (faricimab) (*e.g*., at a concentration of about 100-400, 100, 105, 400 or 420 mg/ml).

In order to minimize the repetitiveness of the embodiments discussed herein, it is contemplated that the scope of the present invention includes embodiments wherein any of the formulations discussed herein include, in place of a VEGF receptor fusion protein, an anti-VEGF antibody or antibody fragment or other VEGF binding molecule as discussed herein (*e.g*., substituted with an anti-VEGF DARPin) at any of the concentrations discussed herein. For example, the present invention includes a formulation having 35 or 80 mg/ml ranibizumab, a buffer, a thermal stabilizer, a viscosity reducing agent and a surfactant.

DARPins are Designed Ankyrin Repeat Proteins. DARPins generally contain three to four tightly packed repeats of approximately 33 amino acid residues, with each repeat containing a β-turn and two anti-parallel α-helices. This rigid framework provides protein stability whilst enabling the presentation of variable regions, normally comprising six amino acid residues per repeat, for target recognition.

An "anti-VEGF" antibody or antigen-binding fragment of an antibody refers to an antibody or fragment that specifically binds to VEGF.

Illustrative VEGF receptor fusion proteins include aflibercept (EYLEA^{®}, Regeneron Pharmaceuticals, Inc.) or conbercept (sold commercially by Chengdu Kanghong Biotechnology Co., Ltd.). See International patent application publication no. WO2005/121176 or WO2007/112675. The terms "aflibercept" and "conbercept" include biosimilar versions thereof. A biosimilar version of a reference product (*e.g*., aflibercept) generally refers to a product comprising the identical amino acid sequence, but includes products which are biosimilar under the U.S. Biologics Price Competition and Innovation Act.

The present invention also includes embodiments including administering one or more further therapeutic agents in addition to VEGF antagonist, for example, administering (one or more doses of) a second VEGF antagonist, an antibiotic, anesthetic (*e.g*., local anesthetic) to the eye receiving an injection, a non-steroidal anti-inflammatory drug (NSAID), a steroid (*e.g*., a corticosteroid, dexamethasone), triamcinolone acetonide (TA), methotrexate, rapamycin, an anti-tumor necrosis factor alpha drug (*e.g*., infliximab), daclizumab, and/or a complement component (*e.g*., C3 or C5) inhibitor.

### Pharmaceutical Formulations

The present invention includes methods in which the VEGF antagonist that is administered to the subject's eye is contained within a pharmaceutical formulation. The pharmaceutical formulation includes a VEGF antagonist along with a pharmaceutically acceptable carrier. Other agents may be incorporated into the pharmaceutical formulation to provide improved transfer, delivery, tolerance, and the like. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the VEGF antagonist is administered. A multitude of appropriate formulations can be found in the formulary known to all pharmaceutical chemists: Remington's Pharmaceutical Sciences (15^{th} ed, Mack Publishing Company, Easton, Pa., 1975), *e.g*., Chapter 87 by Blaug, Seymour, therein.

Pharmaceutical formulations for use in a method of the present invention can be "high concentration". High concentration pharmaceutical formulations of the present invention include VEGF antagonist, *e.g*., VEGF receptor fusion protein, at a concentration of at least 41 mg/ml, of at least 80 mg/ml, of at least 100 mg/ml, of at least 125 mg/ml, of at least 140 mg/ml, of at least 150 mg/ml, of at least 175 mg/ml, of at least 200 mg/ml, of at least 225 mg/ml, of at least 250 mg/ml, or of at least 275 mg/ml. "High concentration" can refer to formulations that include a concentration of VEGF antagonist of from about 140 mg/ml to about 160 mg/ml, at least about 140 mg/ml but less than 160 mg/ml, from about 41 mg/ml to about 275 mg/ml, from about 70 mg/ml to about 75 mg/ml or from about 80 mg/ml to about 250 mg/ml. In some aspects, the VEGF antagonist concentration in the formulation is about any of the following concentrations: 41 mg/ml; 42 mg/ml; 43 mg/ml; 44 mg/ml; 45 mg/ml; 46 mg/ml; 47 mg/ml; 48 mg/ml; 49 mg/ml; 50mg/ml; 51 mg/ml; 52 mg/ml; 53 mg/ml; 54 mg/ml; 55 mg/ml; 56 mg/ml; 57 mg/ml; 58 mg/ml; 59 mg/ml; 60 mg/ml; 61 mg/ml; 62 mg/ml; 63 mg/ml; 64 mg/ml; 65 mg/ml; 66 mg/ml; 67 mg/ml; 68 mg/ml; 69 mg/ml; 70 mg/ml; 71 mg/ml; 72 mg/ml; 73 mg/ml; 74 mg/ml; 75 mg/ml; 76 mg/ml; 77 mg/ml; 78 mg/ml; 79 mg/ml; 80 mg/ml; 81 mg/ml; 82mg/ml; 83 mg/ml; 84 mg/ml; 85 mg/ml; 86 mg/ml; 87mg/ml; 88 mg/ml; 89 mg/ml; 90 mg/ml; 91 mg/ml; 92 mg/ml; 93 mg/ml; 94 mg/ml; 95mg/ml; 96 mg/ml; 97 mg/ml; 98 mg/ml; 99 mg/ml; 100 mg/ml; 101 mg/ml; 102 mg/ml; 103 mg/ml; 104 mg/ml; 105 mg/ml; 106mg/ml; 107 mg/ml; 108 mg/ml; 109 mg/ml; 110 mg/ml; 111 mg/ml; 112 mg/ml; 113 mg/ml; 113.3 mg/ml; 114 mg/ml; 114.1 mg/ml; 114.2 mg/ml; 114.3 mg/ml; 114.4 mg/ml; 114.5 mg/ml; 114.6 mg/ml, 114.7 mg/ml, 114.8 mg/ml; 114.9 mg/ml; 115 mg/ml; 116 mg/ml; 117 mg/ml; 118 mg/ml; 119 mg/ml; 120 mg/ml; 121 mg/ml; 122 mg/ml; 123 mg/ml; 124 mg/ml; 125 mg/ml; 126 mg/ml; 127mg/ml; 128 mg/ml; 129 mg/ml; 130 mg/ml; 131 mg/ml; 132 mg/ml; 133 mg/ml; 133.3 mg/ml; 133.4 mg/ml, 134 mg/ml; 135 mg/ml; 136 mg/ml; 137 mg/ml; 138 mg/ml; 139 mg/ml; 140 mg/ml; 141 mg/ml; 142 mg/ml; 143 mg/ml; 144 mg/ml; 145 mg/ml; 146 mg/ml; 147 mg/ml; 148 mg/ml; 149 mg/ml; 150 mg/ml; 151 mg/ml; 152 mg/ml; 153 mg/ml; 154mg/ml; 155 mg/ml; 156 mg/ml; 157mg/ml; 158 mg/ml; 159 mg/ml; 160 mg/ml; 161 mg/ml; 162 mg/ml; 163 mg/ml; 164 mg/ml; 165 mg/ml; 166 mg/ml; 167 mg/ml; 168 mg/ml; 169 mg/ml; 170 mg/ml; 171 mg/ml; 172 mg/ml; 173 mg/ml; 174 mg/ml; 175 mg/ml; 176 mg/ml; 177 mg/ml; 178 mg/ml; 179 mg/ml; 180 mg/ml; 181 mg/ml; 182 mg/ml; 183 mg/ml; 184 mg/ml; 185 mg/ml; 186 mg/ml; 187 mg/ml; 188 mg/ml; 189 mg/ml; 190 mg/ml; 191 mg/ml; 192 mg/ml; 193 mg/ml; 194 mg/ml; 195 mg/ml; 196 mg/ml; 197 mg/ml; 198 mg/ml; 199 mg/ml; 200 mg/ml; 201 mg/ml; 202 mg/ml; 203 mg/ml; 204 mg/ml; 205 mg/ml; 206 mg/ml; 207 mg/ml; 208 mg/ml; 209 mg/ml; 210 mg/ml; 211 mg/ml; 212 mg/ml; 213 mg/ml; 214 mg/ml; 215 mg/ml; 216 mg/ml; 217 mg/ml; 218 mg/ml; 219 mg/ml; 220 mg/ml; 221 mg/ml; 222 mg/ml; 223 mg/ml; 224 mg/ml; 225 mg/ml; 226 mg/ml; 227 mg/ml; 228 mg/ml; 229 mg/ml; 230 mg/ml; 231 mg/ml; 232 mg/ml; 233 mg/ml; 234 mg/ml; 235 mg/ml; 236 mg/ml; 237mg/ml; 238 mg/ml; 239 mg/ml; 240 mg/ml; 241 mg/ml; 242 mg/ml; 243 mg/ml; 244 mg/ml; 245 mg/ml; 246 mg/ml; 247 mg/ml; 248 mg/ml; 249 mg/ml; 250 mg/ml; 251 mg/ml; 252 mg/ml; 253 mg/ml; 254 mg/ml; 255 mg/ml; 256 mg/ml; 257 mg/ml; 258 mg/ml; 259 mg/ml; 260 mg/ml; 261 mg/ml; 262 mg/ml; 263 mg/ml; 264 mg/ml; 265 mg/ml; 266 mg/ml; 267 mg/ml; 268 mg/ml; 269 mg/ml; 270 mg/ml; 271 mg/ml; 272 mg/ml; 273 mg/ml; 274 mg/ml; or 275 mg/ml. Other VEGF antagonist concentrations are contemplated herein, as long as the concentration functions in accordance with embodiments herein.

In an embodiment of the invention, a pharmaceutical formulation for use in a method of the present invention is of such a concentration as to contain about 4, 6, 8, 10, 12, 14, 16, 18 or 20 mg VEGF receptor fusion protein (*e.g*., aflibercept), or the amount of such protein in any of the acceptable doses thereof which are discussed herein, in about 100 µl or less, about 75 µl or less or about 70 µl or less, *e.g*., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl.

The present invention includes methods of using (as discussed herein) any of the formulations set forth under *"Illustrative Formulations"* herein, but wherein the concentration of the VEGF receptor fusion protein (*e.g*., aflibercept) is substituted with a concentration which is set forth in this section ("VEGF Receptor Fusion Proteins and Other VEGF inhibitors").

Buffers for use in pharmaceutical formulations herein that may be used in a method of the present invention refer to solutions that resist pH change by use of acid-base conjugates. Buffers are capable of maintaining pH in the range of from about 5.0 to about 6.8, and more typically, from about 5.8 to about 6.5, and most typically, from about 6.0 to about 6.5. In some cases, the pH of the formulation of the present invention is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, or about 6.8. Example buffers for inclusion in formulations herein include histidine-based buffers, for example, histidine and histidine hydrochloride or histidine acetate. Buffers for inclusion in formulations herein can alternatively be phosphate-based buffers, for example, sodium phosphate, acetate-based buffers, for example, sodium acetate or acetic acid, or can be citrate-based, for example, sodium citrate or citric acid. It is also recognized that buffers can be a mix of the above, as long as the buffer functions to buffer the formulations in the above described pH ranges. In some cases, the buffer is from about 5 mM to about 25 mM, or more typically, about 5 mM to about 15 mM. Buffers can be about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, or about 25 mM.

In an embodiment of the invention, a histidine-based buffer is prepared using histidine and histidine monohydrochloride.

Surfactant for use herein refers to ingredients that protect the higher concentration of VEGF antagonist, *e.g*., VEGF receptor fusion protein, from various surface and interfacial induced stresses. As such, surfactants can be used to limit or minimize VEGF receptor fusion protein aggregation, and promote protein solubility. Suitable surfactants herein have been shown to be non-ionic, and can include surfactants that have a polyoxyethylene moiety. Illustrative surfactants in this category include: polysorbate 20, polysorbate 80, poloxamer 188, polyethylene glycol 3350, and mixtures thereof. Surfactants in the formulations can be present at from about 0.02% to about 0.1% weight per volume (w/v), and more typically, about 0.02% to about 0.04% (w/v). In some cases, the surfactant is about 0.02% (w/v), about 0.03% (w/v), about 0.04% (w/v), about 0.05% (w/v), about 0.06% (w/v), about 0.07% (w/v), about 0.08% (w/v), about 0.09% (w/v), or about 0.1% (w/v).

Thermal stabilizers for use in pharmaceutical formulations that may be used in methods set forth herein refers to ingredients that provide thermal stability against thermal denaturation of the VEGF antagonist, *e.g*., VEGF receptor fusion protein, as well as protect against loss of VEGF receptor fusion protein potency or activity. Suitable thermal stabilizers include sugars, and can be sucrose, trehalose, sorbitol or mannitol, or can be amino acids, for example L-proline, L-arginine (*e.g*., L-arginine monohydrochloride), or taurine. Additionally, thermal stabilizers may also include substituted acrylamides or propane sulfonic acid, or may be compounds like glycerol.

In some cases, the pharmaceutical formulations for use in a method herein include both a sugar and taurine, a sugar and an amino acid, a sugar and propane sulfonic acid, a sugar and taurine, glycerol and taurine, glycerol and propane sulfonic acid, an amino acid and taurine, or an amino acid and propane sulfonic acid. In addition, formulations can include a sugar, taurine and propane sulfonic acid, glycerol, taurine and propane sulfonic acid, as well as L-proline, taurine and propane sulfonic acid.

Embodiments herein may have thermal stabilizers present alone, each independently present at a concentration of, or present in combination at a total concentration of, from about 2% (w/v) to about 10% (w/v) or 4% (w/v) to about 10% (w/v), or about 4% (w/v) to about 9% (w/v), or about 5% (w/v) to about 8% (w/v). Thermal stabilizers in the formulation can be at a concentration of about 2% (w/v), about 2.5% (w/v), about 3% (w/v), about 4% (w/v), about 5% (w/v), about 6% (w/v), about 7% (w/v), about 8% (w/v), about 9% (w/v), about 10% (w/v) or about 20% (w/v).

With respect to taurine and propane sulfonic acid, in an embodiment of the invention, these thermal stabilizers can be present in the formulations at about from 25 mM to about 100 mM, and more typically from about 50 mM to about 75 mM (as compared to the other thermal stabilizers).

Viscosity reducing agents typically are used to reduce or prevent protein aggregation. Viscosity reducing agents for inclusion herein include: sodium chloride, magnesium chloride, D-or L-arginine (*e.g*., L-arginine monohydrochloride), lysine, or mixtures thereof. When present herein, viscosity reducing agents can be present at from about 10 mM to about 100 mM, and more typically from about 30 mM to about 75 mM, and even more typically from about 40 mM to about 70 mM. In some cases, the viscosity reducing agent is present at about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM.

Pharmaceutical formulations for use in a method as set forth herein can also have a pharmaceutically acceptable viscosity for ocular administration, for example, intravitreal injection. Viscosity generally refers to the measure of resistance of a fluid which is being deformed by either shear stress or tensile stress (typically measured by techniques known in the art, viscometer or rheometer, for example). Typical viscosities of formulations for use in a method set forth herein are from about 5.0 cP (centipoise) to about 15 cP, from about 11 cP to about 14 cP, from about 12 cP to about 15 cP or from about 11 cP to about 12 cP. As such, formulation viscosity herein can be about 5.0 cP, about 6.0, about 7.1 cP, about 7.2 cP, about 7.3 cP, about 7.4 cP, about 7.5 cP, about 7.6 cP, about 10 cP, about 10.5 cP, about 11.0 cP, about 11.5 cP, about 12.0 cP, about 12.5 cP, about 13.0 cP, about 13.5 cP, about 14.0 cP, about 14.5 cP, or about 15.0 cP (e.g., when measured at 20°C).

Various embodiments herein do not require inclusion of an inorganic salt, or other viscosity reducing agent, to maintain these highly useful viscosities. Typically, high concentration protein solutions require viscosity reducing agents to avoid protein aggregation and higher viscosity, making the formulations difficult for intravitreal injection and reducing the potency of the VEGF receptor fusion protein. As such, embodiments herein include methods of using formulations that have had substantially no, or no added, sodium chloride (NaCl), magnesium chloride (MgCl₂), D- or L-arginine (*e.g*., L-arginine hydrochloride), lysine or other viscosity reducing agent.

Osmolality is a critical attribute for injectable pharmaceutical formulations for use in a method of the present invention. It is desirable to have products match physiological osmotic conditions. Furthermore, osmolality provides confirmation of soluble content in solution. In an embodiment of the invention, the osmolality of a formulation for use in a method of the present invention is less than or equal to about 506 mmol/Kg or from about 250 to about 506 mmol/Kg., e.g., about 250, 260, 270, 280, 290, 299, 300, 310, 314, 315, 316, 324, 343, 346, 349, 369, 384, 403, 426, 430 or 506 mmol/Kg. In an embodiment of the invention, the osmolality is lower than about 250 mmol/Kg.

Illustrative pharmaceutical formulations for use in the methods of the present invention include the following:
Formulation A: 80 mg/ml aflibercept, 10 mM histidine-based buffer, 5 % (w/v) sucrose, 0.03% (w/v) polysorbate 20, and 40 mM sodium chloride, with a pH of 5.8 to 6.2.
Formulation B: 80 mg/ml aflibercept, 10 mM phosphate-based buffer, 5 % (w/v) sucrose, 0.03% (w/v) polysorbate 20, and 40 mM sodium chloride, with a pH of 5.8 to 6.2.
Formulation C: 80 mg/ml aflibercept, 10 mM citrate-based buffer, 5 % (w/v) sucrose, 0.03% (w/v) polysorbate 20, and 40 mM sodium chloride, with a pH of 5.8 to 6.2.
Formulation D: 80 mg/ml aflibercept, 10 mM histidine-based buffer, 5 % (w/v) sucrose, 0.03 % (w/v) polysorbate 80, and 40 mM sodium chloride, with a pH of 6.2.
Formulation E: 80 mg/ml aflibercept, 10 mM phosphate-based buffer, 5 % (w/v) sucrose, 0.03 % (w/v) polysorbate 80, and 40 mM sodium chloride, with a pH of 5.8 to 6.2.
Formulation F: 80 mg/ml aflibercept, 10 mM citrate-based buffer, 5 % (w/v) sucrose, 0.03 % (w/v) polysorbate 80, and 40 mM sodium chloride, with a pH of 5.8 to 6.2.
Formulation G: 80 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation H: 80 mg/ml aflibercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation I: 80 mg/ml aflibercept, 10 mM citrate-based buffer, 8 % (w/v) sucrose, and 0.0 3% (w/v) polysorbate 20, with a pH of 5.8 to 6.2, and, optionally, specifically excluding a viscosity reducing agent.
Formulation J: 80 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation K: 80 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation L: 80 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation M: 150 mg/ml aflibercept, 10 mM histidine-based buffer, 5 % (w/v) sucrose, 0.03 % (w/v) polysorbate 20, and 40 mM sodium chloride, with a pH of 5.8 to 6.2.
Formulation N: 150 mg/ml aflibercept, 10 mM phosphate-based buffer, 5 % (w/v) sucrose, 0.03 % (w/v) polysorbate 20, and 40 mM sodium chloride, with a pH of 5.8 to 6.2.
Formulation O: 150 mg/ml aflibercept, 10 mM citrate-based buffer, 5 % (w/v) sucrose, 0.03 % (w/v) polysorbate 20, and 40 mM sodium chloride, with a pH of 5.8 to 6.2.
Formulation P: 150 mg/ml aflibercept, 10 mM histidine-based buffer, 5 % (w/v) sucrose, 0.03 % (w/v) polysorbate 80, and 40 mM sodium chloride, with a pH of 6.2.
Formulation Q: 150 mg/ml aflibercept, 10 mM phosphate-based buffer, 5 % (w/v) sucrose, 0.03 % (w/v) polysorbate 80, and 40 mM sodium chloride, with a pH of 5.8 to 6.2.
Formulation R: 150 mg/ml aflibercept, 10 mM citrate-based buffer, 5 % (w/v) sucrose, 0.03 % (w/v) polysorbate 80, and 40 mM sodium chloride, with a pH of 5.8 to 6.2.
Formulation S: 150 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation T: 150 mg/ml aflibercept, 10 mM phosphate-based buffer, 8 % (w/v) sucrose, and 0.03 % (w/v) polysorbate 20, with a pH of 5.8 to 6.2 (*e.g*., 6.2), and, optionally, specifically excluding a viscosity reducing agent.
Formulation U: 150 mg/ml aflibercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation V: 150 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation W: 150 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation X: 150 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation Y: 80 mg/ml conbercept, 10 mM histidine-based buffer, 5 % (w/v) sucrose, 0.03 % (w/v) polysorbate 20, and 40 mM sodium chloride, with a pH of 5.8 to 6.2.
Formulation Z: 80 mg/ml conbercept, 10 mM phosphate-based buffer, 5 % (w/v) sucrose, 0.03 % (w/v) polysorbate 20, and 40 mM sodium chloride, with a pH of 5.8 to 6.2.
Formulation AA: 80 mg/ml conbercept, 10 mM citrate-based buffer, 5 % (w/v) sucrose, 0.03 % (w/v) polysorbate 20, and 40 mM sodium chloride, with a pH of 5.8 to 6.2.
Formulation BB: 80 mg/ml conbercept, 10 mM histidine-based buffer, 5 % (w/v) sucrose, 0.03 % (w/v) polysorbate 80, and 40 mM sodium chloride, with a pH of 6.2.
Formulation CC: 80 mg/ml conbercept, 10 mM phosphate-based buffer, 5 % (w/v) sucrose, 0.03 % (w/v) polysorbate 80, and 40 mM sodium chloride, with a pH of 5.8 to 6.2.
Formulation DD: 80 mg/ml conbercept, 10 mM citrate-based buffer, 5 % (w/v) sucrose, 0.03 % (w/v) polysorbate 80, and 40 mM sodium chloride, with a pH of 5.8 to 6.2.
Formulation EE: 80 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation FF: 80 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation GG: 80 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation HH: 80 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation II: 80 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation JJ: 80 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation KK: 150 mg/ml conbercept, 10 mM histidine-based buffer, 5 % (w/v) sucrose, 0.03 % (w/v) polysorbate 20, and 40 mM sodium chloride, with a pH of 5.8 to 6.2.
Formulation LL: 150 mg/ml conbercept, 10 mM phosphate-based buffer, 5 % (w/v) sucrose, 0.03 % (w/v) polysorbate 20, and 40 mM sodium chloride, with a pH of 5.8 to 6.2.
Formulation MM: 150 mg/ml conbercept, 10 mM citrate-based buffer, 5 % (w/v) sucrose, 0.03 % (w/v) polysorbate 20, and 40 mM sodium chloride, with a pH of 5.8 to 6.2.
Formulation NN: 150 mg/ml conbercept, 10 mM histidine-based buffer, 5 % (w/v) sucrose, 0.03 % (w/v) polysorbate 80, and 40 mM sodium chloride, with a pH of 6.2.
Formulation OO: 150 mg/ml conbercept, 10 mM phosphate-based buffer, 5 % (w/v) sucrose, 0.03 % (w/v) polysorbate 80, and 40 mM sodium chloride, with a pH of 5.8 to 6.2.
Formulation PP: 150 mg/ml conbercept, 10 mM citrate-based buffer, 5 % (w/v) sucrose, 0.03 % (w/v) polysorbate 80, and 40 mM sodium chloride, with a pH of 5.8 to 6.2.
Formulation QQ: 150 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation RR: 150 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation SS: 150 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation TT: 150 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation UU: 150 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation VV: 150 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation WW: 140 mg/ml VEGF receptor fusion protein (*e.g*., aflibercept), 10 mM histidine-based buffer, 5 % (w/v) sucrose, 0.03 % (w/v) polysorbate 20, and 50 mM taurine, with a pH of 5.8.
Formulation XX: 140 mg/ml VEGF receptor fusion protein (*e.g*., aflibercept), 20 mM histidine-based buffer, 4 % (w/v) proline, 0.03 % (w/v) polysorbate 20, and 50 mM arginine (e.g., arginine hydrochloride), with a pH of 5.8.
Formulation YY: 140 mg/ml VEGF receptor fusion protein (*e.g*., aflibercept), 20 mM histidine-based buffer, 2.5 % (w/v) sucrose, 2.0 % (w/v) proline, 0.03 % (w/v) polysorbate 20, and 50 mM taurine, with a pH of 5.8.
Formulation ZZ: 140 mg/ml VEGF receptor fusion protein (*e.g*., aflibercept), 10 mM histidine-based buffer, 2.5 % (w/v) sucrose, 2.0 % (w/v) proline, 0.03 % (w/v) polysorbate 20, and 50 mM arginine (*e.g*., arginine hydrochloride), with a pH of 5.8.
Formulation AAA: 140 mg/ml VEGF receptor fusion protein (*e.g*., aflibercept), 20 mM histidine-based buffer, 5 % (w/v) sucrose, 0.03% (w/v) polysorbate 20, and 50 mM PSA, with a pH of 5.8.
Formulation BBB: 140 mg/ml VEGF receptor fusion protein (*e.g*., aflibercept), 20 mM histidine-based buffer, 2.5 % (w/v) sucrose, 2.0 % (w/v) proline, 0.03 % (w/v) polysorbate 20, and 50 mM PSA, with a pH of 5.8.
Formulation CCC: 80, 100, 120 or 140 mg/ml VEGF receptor fusion protein (*e.g*., aflibercept), 20 mM histidine-based buffer, 5 % (w/v) sucrose, 0.03 % (w/v) polysorbate 20, and 50 mM arginine (*e.g*., arginine hydrochloride), with a pH of 5.8.
Formulation DDD: 140 mg/ml VEGF receptor fusion protein (*e.g*., aflibercept), 10 mM histidine-based buffer, 4 % (w/v) proline, 0.03 % (w/v) polysorbate 20, and 50 mM PSA, with a pH of 5.8.
Formulation EEE: 140 mg/ml VEGF receptor fusion protein (*e.g*., aflibercept), 20 mM histidine-based buffer, 5 % (w/v) sucrose, and 0.03 % (w/v) polysorbate 20 and, optionally, no thermal stabilizer, with a pH of 5.8.
Formulation FFF: 140 mg/ml VEGF receptor fusion protein (*e.g*., aflibercept), 10mM sodium phosphate, 5 % (w/v) sucrose and 0.03 % polysorbate 20 with a pH of 6.2.
Formulation GGG: 140 mg/ml VEGF receptor fusion protein (*e.g*., aflibercept); 20 mM histidine, pH 5.8; 5% sucrose; 0.03 % polysorbate 20; 50 mM sodium sulfate
Formulation HHH: 140 mg/ml VEGF receptor fusion protein (*e.g*., aflibercept); 20 mM histidine, pH 5.8; 5% sucrose; 0.03 % polysorbate 20; 50 mM sodium thiocyanate
Formulation III: 140 mg/ml VEGF receptor fusion protein (*e.g*., aflibercept); 20 mM histidine, pH 5.8; 5 % sucrose, 0.03 % polysorbate 20; 40 mM sodium citrate
Formulation JJJ: 140 mg/ml VEGF receptor fusion protein (*e.g*., aflibercept); 20 mM histidine, pH 5.8; 5% Sucrose, 0.03 % polysorbate 20; 50 mM glycine
Formulation KKK: 140 mg/ml VEGF receptor fusion protein (*e.g*., aflibercept); 20 mM histidine, pH 5.8; 5 % sucrose, 0.03 % polysorbate 20; 50 mM sodium chloride
Formulation LLL: 140 mg/ml VEGF receptor fusion protein (*e.g*., aflibercept); 20 mM histidine, pH 5.8; 5 % sucrose; 0.03 % polysorbate 20; 50 mM lysine
Formulation MMM: 140 mg/ml VEGF receptor fusion protein (*e.g*., aflibercept); 20 mM histidine, pH 5.8; 5 % sucrose; 0.03% polysorbate 20; 50 mM sodium aspartate
Formulation NNN: 140 mg/ml VEGF receptor fusion protein (*e.g*., aflibercept); 20 mM histidine, pH 5.8; 5 % sucrose; 0.03 % polysorbate 20; 50 mM sodium glutamate
Formulation OOO: 140 mg/ml VEGF receptor fusion protein (*e.g*., aflibercept); 20 mM histidine, pH 5.8; 5 % sucrose; 0.03% polysorbate 20; 50 mM sodium citrate; 50 mM arginine (*e.g*., arginine hydrochloride)
Formulation PPP: 140 mg/ml VEGF receptor fusion protein (*e.g*., aflibercept); 20 mM histidine, pH 5.8; 5 % sucrose; 0.03% polysorbate 20; 50 mM glycine; 50 mM arginine (*e.g*., arginine hydrochloride)
Formulation QQQ: 140 mg/ml VEGF receptor fusion protein (*e.g*., aflibercept); 20 mM histidine, pH 5.8; 5 % sucrose; 0.03% polysorbate 20; 50 mM sodium aspartate; 50 mM arginine (*e.g.*, arginine hydrochloride)
Formulation RRR: 140 mg/ml VEGF receptor fusion protein (*e.g.*, aflibercept); 20 mM histidine, pH 5.8; 5 % sucrose; 0.03% polysorbate 20; 50 mM sodium glutamate; 50 mM arginine (*e.g*., arginine hydrochloride)
Formulation SSS: 140 mg/ml VEGF receptor fusion protein (*e.g*., aflibercept); 20 mM His, pH 5.8; 5 % sucrose; 0.03 % polysorbate 20; 10 mM L-arginine (*e.g*., L-arginine hydrochloride)
Formulation TTT: 140 mg/ml VEGF receptor fusion protein (*e.g*., aflibercept); 20 mM His, pH 5.8; 5 % sucrose; 0.03 % polysorbate 20; 100 mM L-arginine (*e.g*., L-arginine hydrochloride)
Formulation UUU: 30 mg/ml VEGF receptor fusion protein (*e.g*., aflibercept), 10 % sucrose, 10 mM phosphate, 0.03 % polysorbate 20, pH 6.2
Formulation VVV: 30 mg/ml VEGF receptor fusion protein (*e.g*., aflibercept), 20 % sucrose, 10 mM phosphate, 0.03 % polysorbate 20, pH 6.2
Formulation WWW: 60 mg/ml VEGF receptor fusion protein (*e.g*., aflibercept), 10 % sucrose, 10 mM phosphate, 0.03 % polysorbate 20, pH 6.2
Formulation XXX: 60 mg/ml VEGF receptor fusion protein (*e.g*., aflibercept), 20 % sucrose, 10 mM phosphate, 0.03 % polysorbate 20, pH 6.2
Formulation YYY: 120 mg/ml VEGF receptor fusion protein (*e.g*., aflibercept), 10 % sucrose, 10 mM phosphate, 0.03 % polysorbate 20, pH 6.2
Formulation ZZZ: 120 mg/ml VEGF receptor fusion protein (*e.g*., aflibercept), 20 % sucrose, 10 mM phosphate, 0.03 % polysorbate 20, pH 6.2
Formulation AAAA: 120 mg/ml VEGF receptor fusion protein (*e.g*., aflibercept), 10 % sucrose, 10 mM phosphate, 0.03 % polysorbate 20, 50 mM NaCl, pH 6.2
Formulation BBBB: 120 mg/ml VEGF receptor fusion protein (*e.g*., aflibercept), 20 % sucrose, 10 mM phosphate, 0.03 % polysorbate 20, 50 mM NaCl, pH 6.2
Formulation CCCC: 140 mg/ml VEGF receptor fusion protein (*e.g*., aflibercept), 10 mM sodium phosphate, 5 % sucrose, 40 mM sodium chloride, 0.03 % PS20, pH 6.2
Formulation DDDD: 80 mg/ml VEGF receptor fusion protein (*e.g*., aflibercept), 20 mM histidine-based buffer, 5 % (w/v) sucrose, 0.03 % (w/v) polysorbate 20, and 50 mM L-arginine (*e.g*., L-arginine hydrochloride), with a pH of 5.8.
Formulation EEEE: 120.0 mg/ml VEGF receptor fusion protein (*e.g*., aflibercept) (e.g., ± 12 mg/ml), 20 mM histidine-based buffer (e.g., ± 2 mM), 5 % (w/v) sucrose (*e.g*., ± 0.5%), 0.03 % (w/v) polysorbate 20 (e.g., 0.02-0.04%), and 50 mM L-arginine (*e.g*., L-arginine hydrochloride) *(e.g.,* ± 5 mM), with a pH of 5.8 (*e.g*., 5.6-6.0 or 5.5-6.1).
Formulation FFFF: 113.3 mg/ml VEGF receptor fusion protein (*e.g*., aflibercept) (*e.g*., 102-125 mg/ml), 20 mM histidine-based buffer (e.g., ± 2 mM), 5 % (w/v) sucrose (e.g., ± 0.5%), 0.03 % (w/v) polysorbate 20 (*e.g*., 0.02-0.04%), and 50 mM L-arginine (e.g., L-arginine monohydrochloride) (*e.g*., ± 5 mM), with a pH of 5.8 (*e.g*., 5.6-6.0 or 5.5-6.1).
Formulation GGGG: 114.3 mg/ml VEGF receptor fusion protein (*e.g*., aflibercept) (*e.g*., 103-126 mg/ml), 10 mM histidine-based buffer, for example, including Histidine and Histine-HCl (*e.g*., ± 1 mM), 5 % (w/v) sucrose (*e.g*., ± 0.5%), 0.03 % (w/v) polysorbate 20 (*e.g*., 0.02-0.04%), and 50 mM L-arginine (*e.g*., L-arginine monohydrochloride) (*e.g*., ± 5 mM), with a pH of 5.8 (*e.g*., 5.6-6.0 or 5.5-6.1).
Formulation HHHH: 100.0 mg/ml VEGF receptor fusion protein (*e.g*., aflibercept) (*e.g*., ± 10 mg/ml), 20 mM histidine-based buffer (*e.g*., ± 2 mM), 5 % (w/v) sucrose (*e.g*., ± 0.5%), 0.03 % (w/v) polysorbate 20 (*e.g*., 0.02-0.04%), and 50 mM L-arginine (*e.g*., L-arginine monohydrochloride) (*e.g*., ± 5 mM), with a pH of 5.8 (*e.g*., 5.6-6.0 or 5.5-6.1).
Formulation IIII: 133.3 mg/ml VEGF receptor fusion protein (*e.g*., aflibercept) (*e.g*., ± 13 mg/ml), 20 mM histidine-based buffer (*e.g*., ± 2 mM), 5 % (w/v) sucrose (*e.g*., ± 0.5%), 0.03 % (w/v) polysorbate 20 (*e.g*., 0.02-0.04%), and 50 mM L-arginine (*e.g*., L-arginine monohydrochloride) (*e.g.,* ± 5 mM), with a pH of 5.8 (*e.g*., 5.6-6.0 or 5.5-6.1).
Formulation JJJJ: 150 mg/ml aflibercept (*e.g*., aflibercept) (*e.g*., ± 15 mg/ml), 10 mM sodium phosphate, 8% (w/v) sucrose (*e.g*., ± 0.8%), 0.03% (w/v) polysorbate 20 (*e.g*., 0.02-0.04%) and 50 mM L-arginine (*e.g*., arginine hydrochloride), pH 6.2 (*e.g*., 6.0-6.4 or 5.9-6.5).
Formulation KKKK: 114.3 mg/ml VEGF receptor fusion protein (*e.g*., aflibercept) (*e.g*., ± 14 mg/ml), 20 mM histidine-based buffer (e.g., ± 2 mM), 5% (w/v) sucrose (*e.g*., ± 0.5%), 0.03% (w/v) polysorbate 20 (*e.g*., 0.02-0.04%), and 50 mM L-arginine (*e.g*., arginine monohydrochloride) (*e.g*., ± 5 mM), with a pH of 5.8 (*e.g*., 5.6-6.0 or 5.5-6.1);

See International Patent Application Publication No. WO2019/217927.

In an embodiment of the invention, the ≥8 mg VEGF receptor fusion protein, preferably aflibercept, when administered, is in an aqueous pharmaceutical formulation comprising: a VEGF receptor fusion protein comprising two polypeptides that each comprises an immunoglobin-like (Ig) domain 2 of VEGFR1, an Ig domain 3 of VEGFR2, and a multimerizing component (e.g., which comprises amino acids 27-457 of SEQ ID NO: 2) at a concentration of at least about 100 mg/ml; about 5% sucrose; L-arginine (*e.g*., L-arginine monohydrochloride); a histidine-based buffer (*e.g*., containing histidine HCl); and about 0.03% surfactant; wherein the formulation has a pH of about 5.0 to about 6.8 (*e.g*., 5.8 to 6.5, for example 5.8). Preferably the formulation is suitable for intravitreal administration. Other components that may be included are sodium sulfate, sodium thiocyanate, glycine, NaCl, sodium aspartate and/or sodium glutamate. In an embodiment of the invention, the VEGF receptor fusion protein is at a concentration of: about 100 mg/ml; about 111.5 mg/ml; about 112.0 mg/ml; about 113.3 mg/ml; about 114.3 mg/ml; about 115.6 mg/ml; about 116.3 mg/ml; about 120 mg/ml; about 133 mg/ml; about 140 mg/ml; about 150 mg/ml; about 200 mg/ml; or about 250 mg/ml. The formulation may be characterized by (i) an osmolality of about 299 to about 506 mmol/Kg; and/or (ii) a viscosity of from about 6-15 cP at 20°C. The surfactant may be a non-ionic surfactant such as polysorbate 20, polysorbate 80, poloxamer 188, polyethylene glycol 3350 or mixtures thereof. The histidine-based buffer may be at a concentration of about 10 mM to 20 mM. In an embodiment of the invention, the VEGF receptor fusion protein has less than about 3.5% high molecular weight species immediately after manufacture and purification and/or less than or equal to about 6% high molecular weight species after storage for about 24 months at about 2-8°C.

In an embodiment of the invention, the ≥8 mg VEGF receptor fusion protein is, when administered in an aqueous pharmaceutical formulation, comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component (e.g., aflibercept); about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

In an embodiment of the invention, the aqueous pharmaceutical formulation includes:
- ≥ about 100 mg/ml VEGF receptor fusion protein (*e.g*., aflibercept), histidine-based buffer and L-arginine;
- about 140 mg/ml aflibercept; 20 mM histidine-based buffer; 5% sucrose; 0.03 % polysorbate 20; 10 mM L-arginine; pH 5.8;
- about 150 ± 15 mg/ml aflibercept, 10 mM phosphate-based buffer, 8 ± 0.8% (w/v) sucrose, 0.02-0.04% (w/v) polysorbate 20 and 50 mM L-arginine, pH 5.9-6.5;
- about 103-126 mg/ml aflibercept, 10 ± 1 mM histidine-based buffer, 5 ± 0.5% (w/v) sucrose, 0.02-0.04% (w/v) polysorbate 20, and 50 ± 5 mM L-arginine, pH 5.5-6.1;
- about 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
- about 114.3 mg/ml aflibercept, 10 mM histidine-based buffer, 5% (w/v) sucrose, 0.03% (w/v) polysorbate 20 and 50 mM L-arginine, pH 5.8;
- ≥ about 100 mg/ml aflibercept, histidine-based buffer and L-arginine;
- ≥ about 100 mg/ml aflibercept at about pH 5.8, wherein the formulation forms <3% HMW aggregates after incubation at 5°C for 2 months;
- about 114.3 mg/mL aflibercept; 10 mM - 50 mM histidine-based buffer, sugar, non-ionic surfactant, L-Arginine, pH 5.8;
   or
- about 114.3 mg/mL aflibercept; 10 mM His/His-HCl-based buffer, 5% sucrose, 0.03% polysorbate-20, 50 mM L-Arginine, pH 5.8.

In an embodiment of the invention, the ≥8 mg VEGF receptor fusion protein is, when administered in an aqueous pharmaceutical formulation comprising
aflibercept at a concentration of at least about 100 mg/ml (e.g., about 111.5 mg/ml; 112.0 mg/ml; 113.3 mg/ml; about 114.3 mg/ml; about 115.6 mg/ml; or about 116.3 mg/ml);
a thermal stabilizer which is a sugar, an amino acid, sucrose, mannitol, sorbitol, trehalose, **L-**proline, glycine, glycerol, taurine or propane sulfonic acid (e.g., at about 2% (w/v) to about 10% (w/v), for example, 5% (w/v));
a buffer which is a histidine-based buffer, a phosphate-based buffer, an acetate-based buffer (e.g., at a concentration of about 5-25 mM, e.g., 10 mM or 20 mM); or a citrate-based buffer;
a non-ionic surfactant, such as for example, polyoxyethylene-based, polysorbate 20, polysorbate 80, poloxamer 188 or polyethylene glycol 3350 (e.g., at a concentration of about 0.02% to about 0.1 % (w/v), e.g., 0.03% (w/v)); and
a viscosity reducing agent which is NaCl, MgCl₂, D-arginine, L-arginine or L-lysine (e.g., at a concentration of about 10-100 mM, e.g., 50 mM),
wherein the formulation has a pH of about 5.0 to about 6.8 (e.g., 5.0-6.0 or 5.8).

In an embodiment of the invention, the aflibercept is at a concentration in the aqueous pharmaceutical formulation of about 100 mg/ml; 101 mg/ml; 102 mg/ml; 103 mg/ml; 104 mg/ml; 105 mg/ml; 106 mg/ml; 107 mg/ml; 108 mg/ml; 109 mg/ml; 110 mg/ml; 111 mg/ml; 112 mg/ml; 113 mg/ml; 113.3 mg/ml; 114 mg/ml; 114.1 mg/ml; 114.2 mg/ml; 114.3 mg/ml; 114.4 mg/ml; 114.5 mg/ml; 114.6 mg/ml, 114.7 mg/ml, 114.8 mg/ml; 114.9 mg/ml; 115 mg/ml; 116 mg/ml; 117 mg/ml; 118 mg/ml; 119 mg/ml; 120 mg/ml; 121 mg/ml; 122 mg/ml; 123 mg/ml; 124 mg/ml; 125 mg/ml; 126 mg/ml; 127 mg/ml; 128 mg/ml; 129 mg/ml; 130 mg/ml; 131 mg/ml; 132 mg/ml; 133 mg/ml; 133.3 mg/ml; 133.4 mg/ml, 134 mg/ml; 135 mg/ml; 136 mg/ml; 137 mg/ml; 138 mg/ml; 139 mg/ml; 140 mg/ml; 141 mg/ml; 142 mg/ml; 143 mg/ml; 144 mg/ml; 145 mg/ml; 146 mg/ml; 147 mg/ml; 148 mg/ml; 149 mg/ml; 150 mg/ml; 151 mg/ml; 152 mg/ml; 153 mg/ml; 154mg/ml; 155 mg/ml; 156 mg/ml; 157mg/ml; 158 mg/ml; 159 mg/ml; 160 mg/ml; 161 mg/ml; 162 mg/ml; 163 mg/ml; 164 mg/ml; 165 mg/ml; 166 mg/ml; 167 mg/ml; 168 mg/ml; 169 mg/ml; 170 mg/ml; 171 mg/ml; 172 mg/ml; 173 mg/ml; 174 mg/ml; 175 mg/ml; 176 mg/ml; 177 mg/ml; 178 mg/ml; 179 mg/ml; 180 mg/ml; 181 mg/ml; 182 mg/ml; 183 mg/ml; 184 mg/ml; 185 mg/ml; 186 mg/ml; 187 mg/ml; 188 mg/ml; 189 mg/ml; 190 mg/ml; 191 mg/ml; 192 mg/ml; 193 mg/ml; 194 mg/ml; 195 mg/ml; 196 mg/ml; 197 mg/ml; 198 mg/ml; 199 mg/ml; 200 mg/ml; 201 mg/ml; 202 mg/ml; 203 mg/ml; 204 mg/ml; 205 mg/ml; 206 mg/ml; 207 mg/ml; 208 mg/ml; 209 mg/ml; 210 mg/ml; 211 mg/ml; 212 mg/ml; 213 mg/ml; 214 mg/ml; 215 mg/ml; 216 mg/ml; 217 mg/ml; 218 mg/ml; 219 mg/ml; 220 mg/ml; 221 mg/ml; 222 mg/ml; 223 mg/ml; 224 mg/ml; 225 mg/ml; 226 mg/ml; 227 mg/ml; 228 mg/ml; 229 mg/ml; 230 mg/ml; 231 mg/ml; 232 mg/ml; 233 mg/ml; 234 mg/ml; 235 mg/ml; 236 mg/ml; 237mg/ml; 238 mg/ml; 239 mg/ml; 240 mg/ml; 241 mg/ml; 242 mg/ml; 243 mg/ml; 244 mg/ml; 245 mg/ml; 246 mg/ml; 247 mg/ml; 248 mg/ml; 249 mg/ml; 250 mg/ml; 251 mg/ml; 252 mg/ml; 253 mg/ml; 254 mg/ml; 255 mg/ml; 256 mg/ml; 257 mg/ml; 258 mg/ml; 259 mg/ml; 260 mg/ml; 261 mg/ml; 262 mg/ml; 263 mg/ml; 264 mg/ml; 265 mg/ml; 266 mg/ml; 267 mg/ml; 268 mg/ml; 269 mg/ml; 270 mg/ml; 271 mg/ml; 272 mg/ml; 273 mg/ml; 274 mg/ml; or 275 mg/ml.

In an embodiment of the invention, the aqueous pharmaceutical formulation includes aflibercept at a concentration of at least about 100 mg/ml; sucrose, mannitol, sorbitol, trehalose; a histidine-based buffer; polysorbate 20 or polysorbate 80; and L-arginine, at a pH of about 5.0 to about 6.8; wherein the aflibercept has less than about 3.5% high molecular weight species immediately after manufacture and purification and/or less than or equal to about 6% high molecular weight species after storage for about 24 months at about 2-8°C.

In an embodiment of the invention, the sucrose, mannitol, sorbitol or trehalose is at a concentration of about 2-10% (w/v); the L-arginine is at a concentration of about 10-100 mM; the polysorbate 20 or polysorbate 80 is at a concentration of about 0.02-0.1% (w/v); and the histidine-based buffer is at a concentration of about 5-25 mM; at a pH of about 5.0 to about 6.8.

### Treatment and Administration

The present invention provides methods for treating angiogenic eye disorders (*e.g*., DR and/or DME) by sequentially administering initial loading doses (*e.g*., 2 mg or more, 4 mg or more or, preferably, about 8 mg or more of VEGF antagonist or inhibitor, for example, a VEGF receptor fusion protein such as aflibercept) (*e*.*g*., about every 2-4 or 3-5 weeks) followed by additional doses every 12-20 weeks, preferably 12-16 weeks, 12 weeks, 16 weeks or 20 weeks. For example, the present invention provides methods for treating or preventing angiogenic eye disorders, such as diabetic macular edema (DME) and/or diabetic retinopathy (DR), by administering, sequentially, one or more (*e.g*., 3 or 4 or 5) doses of about 8 mg or more of VEGF antagonist (*e.g*., a VEGF receptor fusion protein such as aflibercept) about every 2-4 or 3-5 weeks, *e.g*., every month (or about every 28 days, 28 ± 5 days or about every 4 weeks), followed by one or more doses of about 8 mg or more VEGF antagonist (*e.g*., a VEGF receptor fusion protein such as aflibercept) every 12 weeks (or about every 3 months or about every quarter year or about every 84 days) or every 16 weeks (or about every 4 months or about every 1/3 years or about every 112 days) or every 20 weeks. The dosing regimen including the 12 week tertiary dosing interval may be referred to herein as a 12 week dosing regimen or 8q12 or HDq12; the dosing regimen including the 16 week tertiary dosing interval may be referred to herein as a 16 week dosing regimen or 8q16 or HDq16; and the dosing regimen including the 20 week tertiary dosing interval may be referred to herein as a 20 week dosing regimen or 8q20 or HDq20.

In addition, the present invention includes methods for treating angiogenic eye disorders (*e.g*., DR and/or DME) by administering, one or more times, ≥8 mg VEGF receptor fusion protein, preferably aflibercept, every 4 weeks, 8 weeks, 12-20 weeks, 12-16 weeks, 12 weeks or 16 weeks; as well as every 4 weeks for the first 3, 4 or 5 doses followed by dosing about every 8 weeks.

In an embodiment of the invention, a subject begins receiving the ≥8 mg maintenance doses of every 12 or 16 or 20 weeks after the 8 mg monthly loading doses with no intervening doses. The subject enters the maintenance dose phase rapidly/immediately after the loading dose phase. In an embodiment of the invention, the subject continues receiving the ≥8 mg 12 or 16 or 20 week doses without any intervening doses.

In an embodiment of the invention, the subject does not receive a dosing regimen modification (DRM) or does not terminate treatment for at least 1, 2, 3, 4 or 5 years.

The present invention also provides methods for improving visual acuity in subjects with type 1 or type 2 diabetes mellitus (*e.g*., subjects with diabetic macular edema or diabetic retinopathy), by administering, sequentially, one or more (e.g., 3 or 4 or 5) doses about every month (or about every 28 days, 28 ± 5 days or about every 4 weeks), followed by one or more doses every 12 weeks (or about every 3 months or about every quarter year or about every 84 days) or every 16 weeks (or about every 4 months or about every 1/3 years or about every 112 days) or every 20 weeks.

The terms "initial dose," "secondary doses," and "tertiary doses," refer to the temporal sequence of administration of the VEGF antagonist (e.g., a VEGF receptor fusion protein such as aflibercept). Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of VEGF antagonist (e.g., a VEGF receptor fusion protein such as aflibercept), but will generally differ from one another in terms of frequency of administration. In certain embodiments, however, the amount of VEGF antagonist (e.g., a VEGF receptor fusion protein such as aflibercept) contained in the initial, secondary and/or tertiary doses will vary from one another (e.g., adjusted up or down as appropriate) during the course of treatment.

Thus, a dosing regimen of the present invention may be expressed as follows:
a method for treating an angiogenic eye disorder (e.g., DME or DR) in a subject in need thereof including administering (e.g., intravitreally) to the subject in need thereof,
a single initial dose of about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of a VEGF antagonist (e.g., a VEGF receptor fusion protein such as aflibercept), followed by
one or more (e.g., 2, or 3 or 4, preferably 2) secondary doses of the VEGF antagonist (e.g., a VEGF receptor fusion protein such as aflibercept), followed by
one or more tertiary doses of the VEGF antagonist (e.g., a VEGF receptor fusion protein such as aflibercept);
wherein each secondary dose is administered 2 to 4 weeks (preferably, about 4 weeks) after the immediately preceding dose; and
wherein each tertiary dose is administered 12 or 16 or 20 weeks (preferably, about 12-16, 12 or 16 or 20 weeks) after the immediately preceding dose.

The present invention includes methods wherein one or more additional, non-scheduled doses, in addition to any of the scheduled initial, secondary and/or tertiary doses of VEGF antagonist (e.g., a VEGF receptor fusion protein such as aflibercept) are administered to a subject. Such doses are typically administered at the discretion of the treating physician depending on the particular needs of the subject. The present invention also provides methods for treating angiogenic eye disorders (e.g., DR or DME) by administering to a subject in need thereof about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of VEGF antagonist (e.g., a VEGF receptor fusion protein such as aflibercept) on a PRN basis.

A *pro re nata* (PRN) treatment protocol calls for intervals between doctor visits to remain fixed (e.g., once every 2, 3, 4, 8, 12, 16 or 20 weeks) and decisions to carry out an injection of VEGF receptor fusion protein to be based on the anatomic findings at each respective visit. A capped PRN dosing regimen is PRN wherein subjects must be treated at a certain minimal frequency, e.g., at least once every 2 or 3 or 4 months.

Treat & Extend (T&E) regimens call for the time interval between doctor visits to be adjusted based on the patient's clinical course-e.g., if a subject shows no sign of an active disease (e.g., the macula remains dry, without any leakage), the next one or more intervals can be extended; if there is fluid accumulation, the next interval will be shortened. At each visit following T&E, an injection of VEGF receptor fusion protein will be performed; the current clinical status only has an impact on the duration of the next injection interval.

The present invention includes embodiments wherein, at any point during a HDq12-20, HDq12, HDq16 or HDq20 treatment regimen, the patient can be switched to a PRN, capped PRN or T&E regimen. The PRN, capped PRN and/or T&E may be continued indefinitely or can be stopped at any point and then the HDq12-20, HDq12, HDq16 or HDq20 regimen is reinitiated at any phase thereof. Any HDq12-20, HDq12, HDq16 or HDq20 regimen can be preceded or followed by a period of PRN, capped PRN and/or T&E.

The present invention includes methods comprising administering the required doses of the HDq12 or HDq16 regimen, wherein each of the tertiary doses is administered 12 or 16 weeks after the immediately preceding dose, wherein the treatment interval between two tertiary doses is extended (e.g., from 12 weeks to 13, 14, 15, 16 or 20 weeks or from 16 weeks to 17, 18, 19, or 20 weeks), for example, until signs of disease activity or visual impairment deteriorate or recur and then either continuing dosing at the last tertiary interval used or the penultimate tertiary interval used.

The present invention includes methods comprising administering the required doses of the HDq12-20 or HDq12 or HDq16 or HDq20 regimen, wherein the treatment interval between any two tertiary doses is reduced (e.g., from 20 weeks to 19, 18, 17 or 16 weeks, from 16 weeks to 15, 14, 13, 12, 11, 10, 9, 8, 7, 6, 5, 4, 3, or 2 weeks or from 12 weeks to 11, 10, 9, 8, 7, 6, 5, 4, 3 or 2 weeks), for example, until signs of disease activity or visual impairment improve (e.g., BCVA stabilizes or improves and/or CRT stabilizes or reduces) whereupon, optionally, the interval between doses can be extended, e.g., back to a greater interval length.

For example, in an embodiment of the invention, the interval between doses, e.g., during the 12 week or 16 week dosing phase, can be lengthened, for example by 4 week intervals as appropriate (e.g., from 12 weeks to 16 or 16 weeks to 20 weeks), for example if:
- <5 letter loss in BCVA, e.g., from week 12; and/or
- CRT <300 µm on SD-OCT (or <320 µm on Spectralis SD-OCT).
In an embodiment of the invention, the subject receives the initial, secondary and, then, 12 or 16 week tertiary intervals and, then, after about 1 year, extending the tertiary intervals to about 20 weeks.

In an embodiment of the invention, a method of treating an angiogenic eye disorder such as DR or DME as set forth herein includes the step of evaluating BCVA and/or CRT and lengthening the interval as discussed if one or both of the criteria are met.

For example, in an embodiment of the invention, the interval between doses, e.g., during the 12-20 week or 12 week or 16 week or 20 week dosing phase, can be shortened (e.g., from 12 or 16 or 20 weeks to 8 weeks; from 16 or 20 weeks to 12 weeks or from 20 weeks to 16 weeks), for example if:
- greater than 5 or 10 letters are lost in BCVA (ETDRS or Snellen equivalent) (e.g., relative to the BCVA observed at about 12 or 16 weeks after treatment initiation) occurs, for example, due to or in association with persistent or worsening DR or DME; and/or
- greater than 25 or 50 micrometers increase in CRT is observed (e.g., relative to the CRT observed at about 12 weeks after treatment initiation).

In an embodiment of the invention, if the criteria for reducing the interval between doses is met in a subject receiving the HDq12 regimen, e.g., at week 16 or 20, the interval between doses is decreased to 8 weeks. In an embodiment of the invention, if the criteria for reducing the interval between doses is met in a subject receiving the HDq16 regimen, e.g., at week 16 or 20, the interval between doses is decreased to 8 weeks; and if the criteria for reducing the interval between doses is met in a subject receiving the HDq16 regimen, e.g., at week 24, the interval between doses is decreased to 12 weeks. In an embodiment of the invention, the interval is not decreased to anything shorter than 8 weeks. In an embodiment of the invention, a method of treating an angiogenic eye disorder such as DR or DME as set forth herein includes the step of evaluating BCVA and/or CRT and shortening the interval as discussed if one or both of the criteria are met.

See Figure 3 or Figure 4.

The present invention also provides methods for treating angiogenic eye disorders (preferably, DME or DR) by administering:
doses of about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) about once every 4, 5, 6, 7, 8, 12, 16 or 20 weeks;
   or
a single initial dose (e.g., about ≥8 mg, for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of a VEGF antagonist *(e.g.,* a VEGF receptor fusion protein such as aflibercept), followed by
one or more (e.g., 2, or 3 or 4 (preferably, 2 or 4)) secondary doses of the VEGF antagonist *(e.g.,* a VEGF receptor fusion protein such as aflibercept), followed by
one or more tertiary doses of the VEGF antagonist *(e.g.,* a VEGF receptor fusion protein such as aflibercept);
wherein each secondary dose is administered 2 to 4 (preferably, 4) weeks after the immediately preceding dose; and
wherein each tertiary dose is administered at about 4, 5, 6, 7 or 8 (e.g., 8) weeks after the immediately preceding dose;
   or
about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of VEGF antagonist *(e.g.,* a VEGF receptor fusion protein such as aflibercept) once every about 4 weeks (q4w);
   or
less than about 8 or 9 doses (e.g., about 5 doses or 6 doses) of about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of a VEGF antagonist *(e.g.,* a VEGF receptor fusion protein such as aflibercept) over the course of about 48 weeks.

Dosing every "month" or after a "month" refers to dosing after about 28 days, about 4 weeks, or about 28 ± 5 days and may encompass up to 5 weeks ± 5 days. Dosing every "4 weeks" or after "4 weeks" refers to dosing after about 28 days (± 5 days), about a month or about 28 (± 5 days), and may encompass up to every 5 weeks (± 5 days).

Dosing every "2-4 weeks" or after "2-4 weeks" refers to dosing after about 2 weeks (± 5 days), 3 weeks (± 5 days) or 4 weeks (± 5 days). Dosing every "8 weeks" or after "8 weeks" refers to dosing after about 2 months (± 5 days) or about 56 (± 5 days).

Dosing every "12 weeks" or after "12 weeks" refers to dosing after about 3 months, about 84 days (± 5 days), about 90 days (± 5 days) or about 84 (± 5 days). Dosing every "16 weeks" or after "16 weeks" refers to dosing after about 4 months or about 112 days (± 5 days).

Dosing every "12-20 weeks" or after "12-20 weeks" refers to dosing after 12, 13, 14, 15, 16, 17, 18, 19 or 20 weeks (± 5 days), preferably about 12-16 weeks (± 5 days), about 12 weeks (± 5 days), about 16 weeks (± 5 days) or about 20 weeks (± 5 days).

Dosing every "12-20 weeks" refers to dosing after about 12, 13, 14, 15, 16, 17, 18, 19 or 20 weeks (± 5 days), preferably about 12-16 weeks (± 5 days), about 12 weeks (± 5 days), about 16 weeks (± 5 days) or about 20 weeks (± 5 days).

A dose of ≥ 8 mg encompasses a dose of about 8 mg or doses exceeding 8 mg, for example, about 9, 10, 11, 12, 13, 14, 15, 16, 17, 18, 19 or 20 mg.

Any dosing frequency specified herein may, in an embodiment of the invention, be expressed as the specific frequency "± 5 days" (e.g., where "4 weeks" is stated, the present invention also includes embodiments such as 4 weeks ± 5 days). The term + 5 days includes ±1, ±2, ±3, ±4 and/or ±5 days.

"Sequentially administering" means that each dose of VEGF antagonist (e.g., a VEGF receptor fusion protein such as aflibercept) is administered to the eye of a subject at a different point in time, e.g., on different days separated by a predetermined interval (e.g., hours, days, weeks or months). The present invention includes methods which comprise sequentially administering to the eye of a subject a single initial dose of a VEGF antagonist (e.g., a VEGF receptor fusion protein such as aflibercept), followed by one or more secondary doses of the VEGF antagonist (e.g., a VEGF receptor fusion protein such as aflibercept), followed by one or more tertiary doses of the VEGF antagonist (e.g., a VEGF receptor fusion protein such as aflibercept).

An effective or therapeutically effective dose of VEGF antagonist (e.g., a VEGF receptor fusion protein such as aflibercept), for treating or preventing an angiogenic eye disorder refers to the amount of VEGF antagonist (e.g., a VEGF receptor fusion protein such as aflibercept) sufficient to alleviate one or more signs and/or symptoms of the disease or condition in the treated subject, whether by inducing the regression or elimination of such signs and/or symptoms or by inhibiting the progression of such signs and/or symptoms. In an embodiment of the invention, an effective or therapeutically effective dose of VEGF antagonist (e.g., a VEGF receptor fusion protein such as aflibercept) is about ≥8 mg every month, for 3 doses, followed by once every 12-20 weeks. In an embodiment of the invention, the alleviation of signs and/or symptoms is achievement, e.g., by 1 year, of a gain of ≥5, 10 or 15 letters BCVA (relative to baseline) (e.g., ≥5 letters improvement in a nAMD subject and/or 8-14 letters improvement in a DME patient/subject); achieving a BCVA ≥ 69 letters; achieving no fluid at foveal center; reduction in central retinal thickness (CRT) by about 150 micrometers or more (e.g., below 300 micrometers in an nAMD subject/patient; and/or reduction by at least about 200 micrometers in a DR or RVO patient/subject) or achievement of normal CRT (e.g., about 300 micrometers or less); and/or achievement of no leakage on fluorescein angiography.

Baseline values refer to values prior to initiation of a treatment (pre-dose).

An "angiogenic eye disorder" means any disease of the eye which is caused by or associated with the growth or proliferation of blood vessels or by blood vessel leakage. Nonlimiting examples of angiogenic eye disorders that are treatable or preventable using the methods of the present invention include:
- age-related macular degeneration (neovascular (nAMD)),
- macular edema (ME),
- macular edema following retinal vein occlusion (ME-RVO),
- retinal vein occlusion (RVO),
- central retinal vein occlusion (CRVO),
- branch retinal vein occlusion (BRVO),
- diabetic macular edema (DME),
- choroidal neovascularization (CNV),
- iris neovascularization,
- neovascular glaucoma,
- post-surgical fibrosis in glaucoma,
- proliferative vitreoretinopathy (PVR),
- optic disc neovascularization,
- corneal neovascularization,
- retinal neovascularization,
- vitreal neovascularization,
- pannus,
- pterygium,
- vascular retinopathy,
- diabetic retinopathies (DR) (e.g., non-proliferative diabetic retinopathy (e.g., characterized by a Diabetic Retinopathy Severity Scale (DRSS) level of about 47 or 53) or proliferative diabetic retinopathy; e.g., in a subject that does not suffer from DME), and
- Diabetic retinopathy in a subject who has diabetic macular edema (DME).

The present invention provides methods for treating angiogenic eye disorders (e.g., DR and/or DME) in a subject in need thereof, by sequentially administering initial loading doses *(e.g.,* 2 mg or more, 4 mg or more or, preferably, about 8 mg or more of VEGF antagonist or inhibitor, for example, a VEGF receptor fusion protein such as aflibercept) (e.g., about every 2-4 or 3-5 weeks, preferably every 4 weeks; preferably, three initial loading doses) followed by additional doses every 12-20 weeks, preferably 12-16 weeks, 12 weeks, 16 weeks or 20 weeks wherein the subject achieves and/or maintains, e.g., by week 4, 8, 12, 16, 20, 24, 28, 32, 36, 40, 44, 48, 52, 56, 60, 64, 68, 72, 76, 80, 84, 88, 92 or 96 weeks after treatment initiation:
- an improvement in Diabetic Retinopathy Severity Scale (DRSS), *e.g.,* by at least 2 or 3 steps;
- an improvement in best corrected visual acuity;
- a dry retina;
- a gain in best corrected visual acuity;
- a gain in best corrected visual acuity of 1, 2, 3, 4, 5, 6, 7, 8, 9 or 10 letters or ≥5, ≥10 or ≥15 letters;
- a BCVA of at least 69 letters;
- a decrease in central retinal thickness (CRT), e.g., by about 100, 125, 150, 175 or 200 micrometers;
- no vascular leakage as measured by fluorescein angiography (FA);
- an improvement from pre-treatment baseline in National Eye Institute Visual Function Questionnaire (NEI-VFQ-25) total score;
- a retina without fluid (total fluid, intraretinal fluid [IRF] and/or subretinal fluid [SRF]) at the foveal center and in center subfield;
- maintenance of a fluid-free retina (total fluid, IRF and/or SRF at foveal center and in the center subfield);
- a lack of macular edema;
- a retina free of fluid on spectral domain optical coherence tomography (SD-OCT); and/or
- Does not deviate from the HDq12 or HDq16 treatment regimen once started.

In an embodiment of the invention, a subject receiving a HDq12 or HDq16 or HDq20 treatment for an angiogenic eye disorder (e.g., DR and/or DME) as set forth herein achieves one or more of the following:
- Does not receive a dose regimen modification, e.g., wherein the interval between doses (e.g., tertiary doses) is reduced from the HDq12-20 or HDq12 or HDq16 or HDq20 treatment regimen once started, e.g., for at least 4, 8, 12, 16, 20, 24, 28, 32, 36, 40, 44, 48, 52, 56, 60, 64, 68, 72, 76, 80, 84, 88, 92 or 96 weeks;
- Receives 100% of all scheduled doses, e.g., for at least 4, 8, 12, 16, 20, 24, 28, 32, 36, 40, 44, 48, 52, 56, 60, 64, 68, 72, 76, 80, 84, 88, 92 or 96 weeks;
- Non-inferior BVCA compared to that of aflibercept which is intravitreally dosed at 2 mg approximately every 4 weeks for the first 3, 4 or 5 injections followed by 2 mg approximately once every 8 weeks or once every 2 months;
- Increase in BCVA (according to ETDRS letter score) of about 7, 8 or 9 letters by week 4, 8, 12, 16, 20, 24, 28, 32, 36, 40, 44, 48, 52, 56, 60, 64, 68, 72, 76, 80, 84, 88, 92 or 96, e.g., wherein the baseline BCVA is about 61, 62 and 63;
- Improvement in BCVA, by 4 weeks after initiation of treatment, of about 4 or 5 letters (ETDRS or Snellen equivalent) when on HDq12 regimen; or of about 4 or 5 letters (ETDRS or Snellen equivalent) when on HDq16 regimen;
- Improvement in BCVA, by 8 weeks after initiation of treatment, of about 6 letters (ETDRS or Snellen equivalent) when on HDq12 regimen; or of about 5 or 6 letters (ETDRS or Snellen equivalent) when on HDq16 regimen;
- Improvement in BCVA, by 12 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 16 weeks after initiation of treatment, of about 6 or 7 letters (ETDRS or Snellen equivalent) when on HDq12 regimen; or of 7 letters (ETDRS or Snellen equivalent) when on HDq16 regimen;
- Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
- Improvement in BCVA, by 24 weeks after initiation of treatment, of about 7 letters (ETDRS or Snellen equivalent) when on HDq12 regimen; or of about 5 or 6 letters (ETDRS or Snellen equivalent) when on HDq16 regimen;
- Improvement in BCVA, by 28 weeks after initiation of treatment, of about 7 or 8 letters (ETDRS or Snellen equivalent) when on HDq12 regimen; or of about 7 or 8 letters (ETDRS or Snellen equivalent) when on HDq16 regimen;
- Improvement in BCVA, by 32 weeks after initiation of treatment, of about 7 letters (ETDRS or Snellen equivalent) when on HDq12 regimen; or of about 7 or 8 letters (ETDRS or Snellen equivalent) when on HDq16 regimen;
- Improvement in BCVA, by 36 weeks after initiation of treatment, of 8 letters (ETDRS or Snellen equivalent) when on HDq12 regimen; or of about 6 or 7 letters (ETDRS or Snellen equivalent) when on HDq16 regimen;
- Improvement in BCVA, by 40 weeks after initiation of treatment, of about 8 letters (ETDRS or Snellen equivalent) when on HDq12 regimen; or of about 6 or 7 letters (ETDRS or Snellen equivalent) when on HDq16 regimen;
- Improvement in BCVA, by 44 weeks after initiation of treatment, of about 8 letters (ETDRS or Snellen equivalent) when on HDq12 regimen; or of about 7 or 8 letters (ETDRS or Snellen equivalent) when on HDq16 regimen;
- Improvement in BCVA, by 48 weeks after initiation of treatment, of about 8 or 9 letters (ETDRS or Snellen equivalent) when on HDq12 regimen; or of about 7 or 8 letters (ETDRS or Snellen equivalent) when on HDq16 regimen *e.g.,* wherein the baseline BCVA is about 61, 62 or 63 letters (ETDRS or Snellen equivalent);
- Improvement in BCVA, by 60 weeks after initiation of treatment, of about 8 or 9 letters (ETDRS or Snellen equivalent) when on HDq12 regimen; or of about 7 or 8 letters (ETDRS or Snellen equivalent) when on HDq16 regimen, *e.g.,* wherein the baseline BCVA is about 61, 62 or 63 letters (ETDRS or Snellen equivalent);
- An improvement in BCVA between weeks 48 and 60 of about 8 or 9 letters, or up to 40 letters (ETDRS or Snellen equivalent) when on the HDq12 regimen, e.g., when the basline BCVA is about 63 or 64; or an improvement in BCVA between weeks 48 and 60 of about 7 or 8 letters or up to 40 letters (ETDRS or Snellen equivalent) when on the HDq16 regimen e.g., when the basline BCVA is about 61 or 62;
- An improvement in BCVA by about week 8 after initiation of treatment which is maintained (e.g., within about ±1 or ±2 ETDRS letters or Snellen equivalent) thereafter during the treatment regimen, e.g., to at least week 48;
- Improvement in best corrected visual acuity (according to ETDRS letter score) (e.g., by week 12, 24, 36, 48, 60, 72, 84, 90 or 96 from start of treatment);
- Improvement in best corrected visual acuity (BVCA) by week 4, week 8, week 12, week 16, week 20, week 24, week 28, week 32, week 36, week 40, week 44, or week 48 from start of treatment;
- Increase in BCVA, e.g., as measured by the Early Treatment Diabetic Retinopathy Study (ETDRS) visual acuity chart or Snellen equivalent (e.g., by week 4, 8, 12, 16, 20, 24, 28, 32, 36, 40, 44 or 48 weeks from start of treatment) by ≥4 letters, ≥5 letters, ≥6 letters, ≥7 letters, ≥8 letters, ≥ 9 letters or ≥ 10 letters;
- Between weeks 36 and 48, a change in BCVA score (according to ETDRS letter score) from initiation of treatment of about 7, 8 or 9, e.g., wherein the BCVA at any point between week 36 to 48 is about 60 or 70;
- Between weeks 36 and 48, a change in BCVA score (according to ETDRS letter score) from initiation of treatment of up to 38 letters when on the HDq12 or HDq16 regimen, *e.g.,* wherein BCVA at baseline is between about 27 and 79;

- Between weeks 48 and 60, a change in BCVA score (according to ETDRS letter score) from initiation of treatment of about 7, 8 or 9; *e.g*., wherein the BCVA at any point between week 48 to 60 is about 69, 70, 71, 72 or 73;
- A BCVA improvement, *e.g*., by week 48 following treatment initiation, of about 9 or 10 letters (ETDRS or Snellen equivalent) when baseline BCVA is about ≤73 ETDRS letters when on HDq12 regimen;
- A BCVA improvement, *e.g*., by week 48 following treatment initiation, of about 5 or 6 letters (ETDRS or Snellen equivalent) when baseline BCVA is about >73 ETDRS letters when on HDq12 regimen;
- A BCVA improvement, *e.g*., by week 48 following treatment initiation, of about 8 or 9 letters (ETDRS or Snellen equivalent) when baseline BCVA is about ≤73 ETDRS letters when on HDq16 regimen;
- A BCVA improvement, *e.g*., by week 48 following treatment initiation, of about 4 or 5 letters (ETDRS or Snellen equivalent) when baseline BCVA is about >73 ETDRS letters when on HDq16 regimen;
- A BCVA improvement, *e.g*., by week 48 following treatment initiation, of about 7 or 8 letters (ETDRS or Snellen equivalent) when baseline CRT is < about 400 micrometers when on HDq12 regimen;
- A BCVA improvement, *e.g*., by week 48 following treatment initiation, of about 9 or 10 letters (ETDRS or Snellen equivalent) when baseline CRT is ≥400 micrometers when on HDq12 regimen;
- A BCVA improvement, *e.g*., by week 48 following treatment initiation, of about 5 or 6 letters (ETDRS or Snellen equivalent) when baseline CRT is < about 400 micrometers when on HDq16 regimen;
- A BCVA improvement, *e.g*., by week 48 following treatment initiation, of about 9 or 10 letters (ETDRS or Snellen equivalent) when baseline CRT is ≥ about 400 micrometers when on HDq16 regimen;
- Did not lose 5, 10 or 15 letters by week 4, 8, 12, 16, 20, 24, 28, 32, 36, 40, 44, 48, 52, 56, 60, 64, 68, 72, 76, 80, 84, 88, 92 or 96 (according to ETDRS letter score);
- Gains at least 5, 10 or 15 letter by week 4, 8, 12, 16, 20, 24, 28, 32, 36, 40, 44, 48, 52, 56, 60, 64, 68, 72, 76, 80, 84, 88, 92 or 96 (according to ETDRS letter score);
- Does not lose 5, 10, 15 or 69 letters or more BCVA (*e.g*., after week 4, 8, 12, 16, 20, 24, 28, 32, 36, 40, 44, 48, 52, 56, 60, 64, 68, 72, 76, 80, 84, 88, 92 or 96 from start of treatment);
- Between weeks 48 and 60, a BCVA score (according to ETDRS letter score) of about 69, 70, 71, 72 or 73;
- BCVA (according to ETDRS letter score) of at least about 69 letters, *e.g*., by week 48 or 60;
- A BCVA by 4 weeks after initiation of treatment of about 68 letters (ETDRS or Snellen equivalent) when on the HDq12 regimen; or a BCVA of about 66 letters (ETDRS or Snellen equivalent) when on the HDq16 regimen;
- A BCVA by 8 weeks after initiation of treatment of about 70 letters (ETDRS or Snellen equivalent) when on the HDq12 regimen; or a BCVA of about 67 letters (ETDRS or Snellen equivalent) when on the HDq16 regimen;
- A BCVA by 12 weeks after initiation of treatment of about 70 letters (ETDRS or Snellen equivalent) when on the HDq12 regimen; or a BCVA of about 68 letters (ETDRS or Snellen equivalent) when on the HDq16 regimen;
- A BCVA by 16 weeks after initiation of treatment of about 71 letters (ETDRS or Snellen equivalent) when on the HDq12 regimen; or a BCVA of about 69 letters (ETDRS or Snellen equivalent) when on the HDq16 regimen;
- A BCVA by 20 weeks after initiation of treatment of about 70 letters (ETDRS or Snellen equivalent) when on the HDq12 regimen; or a BCVA of about 68 letters (ETDRS or Snellen equivalent) when on the HDq16 regimen;
- A BCVA by 24 weeks after initiation of treatment of about 71 letters (ETDRS or Snellen equivalent) when on the HDq12 regimen; or a BCVA of about 67 letters (ETDRS or Snellen equivalent) when on the HDq16 regimen;
- A BCVA by 28 weeks after initiation of treatment of about 72 letters (ETDRS or Snellen equivalent) when on the HDq12 regimen; or a BCVA of about 70 letters (ETDRS or Snellen equivalent) when on the HDq16 regimen;
- A BCVA by 32 weeks after initiation of treatment of about 71 letters (ETDRS or Snellen equivalent) when on the HDq12 regimen; or a BCVA of about 70 letters (ETDRS or Snellen equivalent) when on the HDq16 regimen;
- A BCVA by 36 weeks after initiation of treatment of about 71 letters (ETDRS or Snellen equivalent) when on the HDq12 regimen; or a BCVA of about 68 letters (ETDRS or Snellen equivalent) when on the HDq16 regimen;
- A BCVA by 40 weeks after initiation of treatment of about 72 letters (ETDRS or Snellen equivalent) when on the HDq12 regimen; or a BCVA of about 69 letters (ETDRS or Snellen equivalent) when on the HDq16 regimen;
- A BCVA by 44 weeks after initiation of treatment of about 72 letters (ETDRS or Snellen equivalent) when on the HDq12 regimen; or a BCVA of about 70 letters (ETDRS or Snellen equivalent) when on the HDq16 regimen;
- A BCVA by 48 weeks after initiation of treatment of about 73 letters (ETDRS or Snellen equivalent) when on the HDq12 regimen; or a BCVA of about 70 letters (ETDRS or Snellen equivalent) when on the HDq16 regimen;
- A BCVA between weeks 36 and 48 of about 71, 72, 73 or 74 (ETDRS or Snellen equivalent) when on the HDq12 regimen, *e.g.,* when the basline BCVA is about 57, 58, 59, 60, 61, 62, 63 or 64; or a BCVA between weeks 36 and 48 of about 69, 70, 71, 72 or 73 (ETDRS or Snellen equivalent) when on the HDq16 regimen *e.g.,* when the basline BCVA is about 55, 56, 57, 58, 59, 60, 61, or 62;
- A BCVA between weeks 48 and 60 of about 69 or 70 or up to 94 (ETDRS or Snellen equivalent) when on the HDq12 regimen, *e.g*., when the basline BCVA is about 63 or 64; or a BCVA between weeks 48 and 60 of about 72 or 73 or up to 89 (ETDRS or Snellen equivalent) when on the HDq16 regimen *e.g*., when the basline BCVA is about 61 or 62;
- Gain of ≥5, ≥10 or ≥15 letters BCVA (according to ETDRS letter score) (*e.g*., by week 4, 8, 12, 16, 20, 24, 28, 32, 36, 40, 44, 48, 52, 56, 60, 64, 68, 72, 76, 80, 84, 88, 92 or 96 from start of treatment);
- ≥ 2 step improvement in Diabetic Retinopathy Severity Scale (DRSS) (*e.g*., by week 4, 8, 12, 16, 20, 24, 28, 32, 36, 40, 44, 48, 52, 56, 60, 72, 84, 90 or 96 from start of treatment);
- ≥ 3 step improvement in diabetic retinopathy severity scale (DRSS) (*e.g*., by week 4, 8, 12, 16, 20, 24, 28, 32, 36, 40, 44, 48, 52, 56, 60, 72, 84, 90 or 96 weeks from start of treatment);
- Retina without fluid (total fluid, intraretinal fluid [IRF] and/or subretinal fluid [SRF]) at the foveal center or center subfield (*e.g*., by week 4, 8, 12, 16, 20, 24, 28, 32, 36, 40, 44, 48, 52, 56, 60, 64, 68, 72, 76, 80, 84, 88, 92 or 96 from start of treatment) (*e.g*., as measured by optical coherence tomography (OCT);
- No vascular leakage in the retina as measured by fluorescein angiography (FA) (*e.g*., by week 4, 8, 12, 16, 20, 24, 28, 32, 36, 40, 44, 48, 52, 56, 60, 64, 68, 72, 76, 80, 84, 88, 92 or 96 from start of treatment);
- Maintenance of a fluid-free retina (total fluid, IRF and/or SRF at foveal center and in the center subfield) (*e.g*., by week 4, 8, 12, 16, 20, 24, 28, 32, 36, 40, 44, 48, 52, 56, 60, 64, 68, 72, 76, 80, 84, 88, 92 or 96 from start of treatment);
- Reduction in total area of fluorescein leakage within ETDRS grid (mm²) at week 48 or 60 by about 12, 12.6, 13, 13.6, 13.9 or 14 mm² or more, or up to abou 57 or 68 mm² (*e.g.,* as measured by fluorescein angiography);
- Reduction in total area of fluorescein leakage within ETDRS grid (mm²) at week 48 by about 13. 13.3, 13.9 or 14 mm² or more (*e.g*., up to about 52 mm²) (*e.g*., as measured by fluorescein angiography) when on the HDq12 regimen;
- Reduction in total area of fluorescein leakage within ETDRS grid (mm²) at week 48 by about 7, 7.7, 8, 9, 9.4 or 10 mm² or more (*e.g*., up to about 55 mm²) (*e.g*., as measured by fluorescein angiography) when on the HDq16 regimen;
- Retina free of fluid on spectral domain optical coherence tomography (SD-OCT) (*e.g*., by week 12, 24, 36, 48, 60, 72, 84, 90 or 96 from start of treatment);
- Retina without fluid (total fluid, intraretinal fluid [IRF] and/or subretinal fluid [SRF]) at the foveal center (*e.g*., by week 4, 8, 12, 16, 20, 24, 28, 32, 36, 40, 44 or 48 weeks from start of treatment);
- Dry retina (*e.g*., by week 4, 8, 12, 16, 20, 24, 28, 32, 36, 40, 44, 48, 52, 56, 60, 64, 68, 72, 76, 80, 84, 88, 92 or 96 from start of treatment);
- Foveal center without fluid (*e.g*., by week 4, 8, 12, 16, 20, 24, 28, 32, 36, 40, 44, 48, 52, 56, 60, 64, 68, 72, 76, 80, 84, 88, 92 or 96 from start of treatment) (*e.g.*, as measured by optical coherence tomography (OCT);
- A change in central retinal thickness, by 4 weeks after initiation of treatment of about - 118 or -118.3 micrometers (±17, 18 or 19 micrometers) when on the HDq12 regimen; or of about -124 or -125 or -124.9 or -125.5 micrometers (±17, 18 or 19 micrometers) when on the HDq16 regimen;
- A change in central retinal thickness, by 8 weeks after initiation of treatment of about - 137 or -137.4 micrometers (±17, 18 or 19 micrometers) when on the HDq12 regimen; or of about -139 or -140 or -139.6 or -140.3 micrometers (±17, 18 or 19 micrometers) when on the HDq16 regimen;
- A change in central retinal thickness, by 12 weeks after initiation of treatment of about -150 or -150.1 micrometers (±17, 18 or 19 micrometers) when on the HDq12 regimen; or of about -152 or -153 or -152.7 or -153.4 micrometers (±17, 18 or 19 micrometers) when on the HDq16 regimen;
- A change in central retinal thickness, by 16 weeks after initiation of treatment of about -139 or -139.4 micrometers (±17, 18 or 19 micrometers) when on the HDq12 regimen; or of about -145 or -146 or -145.5 or -146.4 micrometers (±17, 18 or 19 micrometers) when on the HDq16 regimen;
- A change in central retinal thickness, by 20 weeks after initiation of treatment of about -117 or -117.1 micrometers (±17, 18 or 19 micrometers) when on the HDq12 regimen; or of about -112 or -113 or -112.5 or -113.3 micrometers (±17, 18 or 19 micrometers) when on the HDq16 regimen;
- A change in central retinal thickness, by 24 weeks after initiation of treatment of about -158 or -158.1 micrometers (±17, 18 or 19 micrometers) when on the HDq12 regimen; or of about -103 or -104 or -103.8 or -104.3 micrometers (±17, 18 or 19 micrometers) when on the HDq16 regimen;
- A change in central retinal thickness, by 28 weeks after initiation of treatment of about -146 or -147 or -146.7 micrometers (±17, 18 or 19 micrometers) when on the HDq12 regimen; or of about -162 or -162.3 micrometers (±17, 18 or 19 micrometers) when on the HDq16 regimen;
- A change in central retinal thickness, by 32 weeks after initiation of treatment of about -132 micrometers (±17, 18 or 19 micrometers) when on the HDq12 regimen; or of about -145 or -146 or -145.8 micrometers (±17, 18 or 19 micrometers) when on the HDq16 regimen;
- A change in central retinal thickness, by 36 weeks after initiation of treatment of about -168 or -168.1 micrometers (±17, 18 or 19 micrometers) when on the HDq12 regimen; or of about -124 or -125 or -124.7 or -125.2 micrometers (±17, 18 or 19 micrometers) when on the HDq16 regimen;
- A change in central retinal thickness, by 40 weeks after initiation of treatment of about -163 micrometers (±17, 18 or 19 micrometers) when on the HDq12 regimen; or of about -122 or -123 or -122.5 or -123.1 micrometers (±17, 18 or 19 micrometers) when on the HDq16 regimen;
- A change in central retinal thickness, by 44 weeks after initiation of treatment of about -147 or -148 or -147.4 micrometers (±17, 18 or 19 micrometers) when on the HDq12 regimen; or of about -164 or -164.1 or -164.3 micrometers (±17, 18 or 19 micrometers) when on the HDq16 regimen;
- A change in central retinal thickness, by 48 weeks after initiation of treatment of about -171 or -172 or -171.7, -172, -173, -174, -175, -176 or -176.77 micrometers (± 5, 6, 7, 8, 9, 10, 11, 12, 13, 14, 15, 16, 17, 18 or 19 micrometers) when on the HDq12 regimen; or of about -148 or -149 or -148.3 or -149.4 micrometers (±9, 10, 11, 12, 13, 14, 15, 16, 17, 18 or 19 micrometers) when on the HDq16 regimen, *e.g*., wherein baseline CRT is about 449, 450, 455 or 460 micrometers;
- A change in central retinal thickness, by 60 weeks after initiation of treatment of about -181, -182, -181.95, -176, -176.24 or -177 (± 6, 10, 17, 18 or 19 micrometers) micrometers when on the HDq12 regimen (*e.g*., wherein the baseline CRT is about 460 micrometers); or of about -166, -166.26, -167 or -167.18 micrometers (±8, 9, 10, 17, 18 or 19 micrometers) when on the HDq16 regimen (*e.g*., wherein the baseline CRT is about 457 micrometers);
- A change in central retinal thickness of about -118 or -119 or -118.3 micrometers, between initiation of treatment (week 0) and week 4 when on the HDq12 regimen;
- A change in central retinal thickness of about -19, -20 or -19.1 micrometers, between weeks 4 and 8 when on the HDq12 regimen;
- A change in central retinal thickness of about -12, -13 or -12.7 micrometers, between weeks 8 and 12 when on the HDq12 regimen;
- A change in central retinal thickness of about -40, or -41 micrometers, between weeks 20 and 24 when on the HDq12 regimen;
- A change in central retinal thickness of about -36, -37 or -36.1 micrometers, between weeks 32 and 36 when on the HDq12 regimen;
- A change in central retinal thickness of about -24, -25 or -24.3 micrometers, between weeks 44 and 48 when on the HDq12 regimen;
- A change in central retinal thickness of about -124, -125 or -124.9 micrometers, between initiation of treatment (week 0) and week 4 when on the HDq16 regimen;
- A change in central retinal thickness of about -14, -15 or -14.7 micrometers, between weeks 4 and 8 when on the HDq16 regimen;
- A change in central retinal thickness of about -13, -14 or -13.1 micrometers, between weeks 8 and 12 when on the HDq16 regimen;
- A change in central retinal thickness of about -58, -59 or -58.5 micrometers, between weeks 24 and 28 when on the HDq16 regimen;
- A change in central retinal thickness of about -41, -42 or -41.6 micrometers, between weeks 40 and 44 when on the HDq16 regimen;
- A reduction in CRT by week 4, 5, 6, 7 or 8 after initiation of treatment which is maintained (*e.g*., within about ±17, ±18 or ±19 micrometers) thereafter during the treatment regimen, *e.g*., to at least week 48;
- Decrease in central retinal thickness (CRT), for example, by about 100, 125, 150, 175 or 200 micrometers (*e.g*., by week 12, 24, 36, 48, 60, 72, 84, 90 or 96 from start of treatment);
- Reduction in CRT of about 148-182 micrometers (*e.g*., 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183) by week 48 or 60 (*e.g*., as measured by optical coherence tomography (OCT)), for example, wherein the baseline CRT is about 449, 450, 455 or 460 micrometers;
- Decrease in central retinal thickness (CRT), for example, by at least about 100, 125, 130, 135, 140, 145, 149, 150, 155, 160, 165, 170, 171, 172, 173, 174 or 175 micrometers (*e.g*., by week 4, 8, 12, 16, 20, 24, 28, 32, 36, 40, 44 or 48 from start of treatment);
- Ocular (*e.g*., intraocular pressure) and non-ocular safety (*e.g*., hypertensive events or APTC events) or death rate, in a subject suffering from an angiogenic eye disorder, *e.g.*, DR or DME, similar to that of aflibercept which is intravitreally dosed at 2 mg approximately every 4 weeks for the first 3, 4 or 5 injections followed by 2 mg approximately once every 8 weeks or once every 2 months;
- At about 0.1667 days after the first dose, free aflibercept concentration in plasma of about 0.149 (±0.249) mg/l; *e.g*., wherein at baseline free aflibercept concentration in plasma not detectable, for example, wherein the subject has not received intravitreal VEGF inhibitor (*e.g*., aflibercept) treatment for at least 12 weeks;
- At about 0.3333 days after the first dose, free aflibercept concentration in plasma of about 0.205 (±0.250) mg/l; *e.g.*, wherein at baseline free aflibercept concentration in plasma not detectable, for example, wherein the subject has not received intravitreal VEGF inhibitor (*e.g*., aflibercept) treatment for at least 12 weeks;
- At about 1 day after the first dose, free aflibercept concentration in plasma of about 0.266 (±0.211) mg/l; *e.g*., wherein at baseline free aflibercept concentration in plasma not detectable, for example, wherein the subject has not received intravitreal VEGF inhibitor (*e*.*g*., aflibercept) treatment for at least 12 weeks;
- At about 2 days after the first dose, free aflibercept concentration in plasma of about 0.218 (±0.145) mg/l; *e.g*., wherein at baseline free aflibercept concentration in plasma not detectable, for example, wherein the subject has not received intravitreal VEGF inhibitor (*e.g*., aflibercept) treatment for at least 12 weeks;
- At about 4 days after the first dose, free aflibercept concentration in plasma of about 0.140 (±0.0741) mg/l; *e.g*., wherein at baseline free aflibercept concentration in plasma not detectable, for example, wherein the subject has not received intravitreal VEGF inhibitor (*e.g*., aflibercept) treatment for at least 12 weeks;
- At about 7 days after the first dose, free aflibercept concentration in plasma of about 0.0767 (±0.0436) mg/l; *e.g*., wherein at baseline free aflibercept concentration in plasma not detectable, for example, wherein the subject has not received intravitreal VEGF inhibitor (e.g., aflibercept) treatment for at least 12 weeks;
- At about 14 days after the first dose, free aflibercept concentration in plasma of about 0.0309 (±0.0241) mg/l; *e.g*., wherein at baseline free aflibercept concentration in plasma not detectable, for example, wherein the subject has not received intravitreal VEGF inhibitor (*e.g.*, aflibercept) treatment for at least 12 weeks;
- At about 21 days after the first dose, free aflibercept concentration in plasma of about 0.0171 (±0.0171) mg/l; *e.g*., wherein at baseline free aflibercept concentration in plasma not detectable, for example, wherein the subject has not received intravitreal VEGF inhibitor (*e.g*., aflibercept) treatment for at least 12 weeks;
- At about 28 days after the first dose, free aflibercept concentration in plasma of about 0.00730 (±0.0113) mg/l; *e.g*., wherein at baseline free aflibercept concentration in plasma not detectable, for example, wherein the subject has not received intravitreal VEGF inhibitor (*e.g*., aflibercept) treatment for at least 12 weeks;
- At about 0.1667 days after the first dose, adjusted bound aflibercept concentration in plasma of about 0.00698 (±0.0276) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 0.3333 days after the first dose, adjusted bound aflibercept concentration in plasma of about 0.00731 (±0.0279) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 1 days after the first dose, adjusted bound aflibercept concentration in plasma of about 0.0678 (±0.0486) mg/l; *e.g*., wherein at baseline there is about 0.00583 mg/l (±0.0280) adjusted bound aflibercept concentration, for example, wherein the subject has not received intravitreal VEGF inhibitor (*e*.*g*., aflibercept) treatment for at least 12 weeks;
- At about 2 days after the first dose, adjusted bound aflibercept concentration in plasma of about 0.138 (±0.0618) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 4 days after the first dose, adjusted bound aflibercept concentration in plasma of about 0.259 (±0.126) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 7 days after the first dose, adjusted bound aflibercept concentration in plasma of about 0.346 (±0.151) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 14 days after the first dose, adjusted bound aflibercept concentration in plasma of about 0.374 (±0.110) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 21 days after the first dose, adjusted bound aflibercept concentration in plasma of about 0.343 (±0.128) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- At about 28 days after the first dose, adjusted bound aflibercept concentration in plasma of about 0.269 (±0.149) mg/l; *e.g*., wherein at baseline there is about 0.00583 mg/l (±0.0280) adjusted bound aflibercept concentration, for example, wherein the subject has not received intravitreal VEGF inhibitor (*e.g*., aflibercept) treatment for at least 12 weeks;
- The maximum concentration of free aflibercept in the plasma is reached about 0.965 (*e.g*., about 1) day after the first dose;
- Reaches a maximum concentration of about 0.310 mg/l (±0.263) free aflibercept in the plasma;
- Individual free aflibercept concentration (Cₘₐₓ) in the plasma of from about 0 to about 1.08 mg/L (0.1, 0.2, 0.3, 0.4, 0.5, 0.6, 0.7, 0.8, 0.9, 1.0 or 1.1 mg/l);
- Free aflibercept in the plasma maximum (mg/l) per dose (mg) of aflibercept of about 0.0388 (±0.00328) mg/l/mg;
- The maximum concentration of adjusted bound aflibercept in the plasma is reached about 14 day after the first dose;
- Reaches a maximum concentration of about 0.387 mg/l (±0.135) adjusted bound aflibercept in the plasma;
- Adjusted bound aflibercept concentration in the plasma of from about 0.137 to about 0.774 mg/L (0.1, 0.2, 0.3, 0.4, 0.5, 0.6, 0.7, 0.8 mg/l);
- Adjusted bound aflibercept concentration in the plasma maximum (mg/l) per dose (mg) of aflibercept of about 0.0483 (±0.0168) mg/l/mg;
- Does not have anti-drug antibodies against aflibercept after 48 or 60 weeks of treatment;
- Improvement from pre-treatment baseline in National Eye Institute Visual Function Questionnaire (NEI-VFQ-25) total score (*e.g*., by week 12, 24, 36, 48, 60, 72, 84, 90 or 96 from start of treatment), *e.g*., by about 4, 5 or 6 when on the HDq12 regimen or by about 2, 3 or 4 when on the HDq16 regimen; *e.g*., wherein the baseline score is about 76 or 77;
- Lack of macular edema (*e.g*., by week 12, 24, 36, 48, 60, 72, 84, 90 or 96 from start of treatment); and/or
- Efficacy and/or safety, in a subject suffering from DR or DME, similar to that of aflibercept which is intravitreally dosed at 2 mg approximately every 4 weeks for the first 5 injections followed by 2 mg approximately once every 8 weeks or once every 2 months, *e.g*., wherein efficacy is measured as increase in BCVA and/or reduction in central retinal thickness, *e.g*., wherein safety is as measured as the incidence of adverse events (treatment-emergent adverse events occurring anytime within 30 days of any injection) such as blood pressure increase, intraocular pressure increase, visual impairment, vitreous floaters, vitreous detachment, iris neovascularization and/or vitreous hemorrhage.

Thus, the present invention provides the following:
- A method for achieving a non-inferior BVCA compared to that of aflibercept which is intravitreally dosed at 2 mg approximately every 4 weeks for the first 3, 4 or 5 injections followed by 2 mg approximately once every 8 weeks or once every 2 months; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an increase in BCVA (according to ETDRS letter score) of about 7, 8 or 9 letters by week 60 wherein the baseline BCVA is about 61, 62 and 63; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a BCVA (according to ETDRS letter score) of at least about 69 letters by week 48 or 60; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a BCVA wherein there is not a loss of 5, 10, 15 or 69 letters or more after week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an improvement in best corrected visual acuity (according to ETDRS letter score) by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an improvement in best corrected visual acuity (BVCA) by week 4, week 8, week 12, week 16, week 20, week 24, week 28, week 32, week 36, week 40, week 44, week 48 or week 60 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving between weeks 48 and 60 from treatment initiation, a BCVA score (according to ETDRS letter score) of about 69, 70, 71, 72 or 73; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving between weeks 36 and 48, a change in BCVA score (according to ETDRS letter score) from initiation of treatment of about 7, 8 or 9, wherein the BCVA at any point between week 36 to 48 is about 60 or 70; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving between weeks 48 and 60, a change in BCVA score (according to ETDRS letter score) from initiation of treatment of about 7, 8 or 9 wherein the BCVA at any point between week 48 to 60 is about 69, 70, 71, 72 or 73; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an increase in BCVA as measured by the Early Treatment Diabetic Retinopathy Study (ETDRS) visual acuity chart or Snellen equivalent by week 4, 8, 12, 16, 20, 24, 28, 32, 36, 40, 44, 48 or 60 weeks from start of treatment by ≥4 letters, ≥5 letters, ≥6 letters, ≥7 letters, ≥8 letters, ≥ 9 letters or ≥ 10 letters; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a BCVA wherein there is not a loss of 5, 10 or 15 letters by week 48 or 60 (according to ETDRS letter score); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an gain at least 5, 10 or 15 letter by week 48 or 60 (according to ETDRS letter score); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an improvement in BCVA, by 4 weeks after initiation of treatment, of about 4 or 5 letters (ETDRS or Snellen equivalent) when on a HDq12 regimen; or of about 4 or 5 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 8 weeks after initiation of treatment, of about 6 letters (ETDRS or Snellen equivalent) when on a HDq12 regimen; or of about 5 or 6 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 12 weeks after initiation of treatment, of about 6 or 7 letters (ETDRS or Snellen equivalent) when on aHDq12 regimen; or of about 6 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 16 weeks after initiation of treatment, of about 6 or 7 letters (ETDRS or Snellen equivalent) when on a HDq12 regimen; or of 7 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 20 weeks after initiation of treatment, of about 6 letters (ETDRS or Snellen equivalent) when on a HDq12 regimen; or of about 6 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 24 weeks after initiation of treatment, of about 7 letters (ETDRS or Snellen equivalent) when on a HDq12 regimen; or of about 5 or 6 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 28 weeks after initiation of treatment, of about 7 or 8 letters (ETDRS or Snellen equivalent) when on a HDq12 regimen; or of about 7 or 8 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 32 weeks after initiation of treatment, of about 7 letters (ETDRS or Snellen equivalent) when on a HDq12 regimen; or of about 7 or 8 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 36 weeks after initiation of treatment, of 8 letters (ETDRS or Snellen equivalent) when on a HDq12 regimen; or of about 6 or 7 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 40 weeks after initiation of treatment, of about 8 letters (ETDRS or Snellen equivalent) when on a HDq12 regimen; or of about 6 or 7 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 44 weeks after initiation of treatment, of about 8 letters (ETDRS or Snellen equivalent) when on a HDq12 regimen; or of about 7 or 8 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 48 weeks after initiation of treatment, of about 8 or 9 letters (ETDRS or Snellen equivalent) when on a HDq12 regimen; or of about 7 or 8 letters (ETDRS or Snellen equivalent) when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA by about week 8 after initiation of treatment which is maintained (within about ±1 or ±2 ETDRS letters or Snellen equivalent) thereafter during the treatment regimen to at least week 48 or 60; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a BCVA by 4 weeks after initiation of treatment of about 68 letters (ETDRS or Snellen equivalent) when on the a HDq12 regimen; or a BCVA of about 66 letters (ETDRS or Snellen equivalent) when on the a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 8 weeks after initiation of treatment of about 70 letters (ETDRS or Snellen equivalent) when on the a HDq12 regimen; or a BCVA of about 67 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 12 weeks after initiation of treatment of about 70 letters (ETDRS or Snellen equivalent) when on the HDq12 regimen; or a BCVA of about 68 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 16 weeks after initiation of treatment of about 71 letters (ETDRS or Snellen equivalent) when on the HDq12 regimen; or a BCVA of about 69 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 20 weeks after initiation of treatment of about 70 letters (ETDRS or Snellen equivalent) when on the HDq12 regimen; or a BCVA of about 68 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 24 weeks after initiation of treatment of about 71 letters (ETDRS or Snellen equivalent) when on the HDq12 regimen; or a BCVA of about 67 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 28 weeks after initiation of treatment of about 72 letters (ETDRS or Snellen equivalent) when on the HDq12 regimen; or a BCVA of about 70 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 32 weeks after initiation of treatment of about 71 letters (ETDRS or Snellen equivalent) when on the HDq12 regimen; or a BCVA of about 70 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 36 weeks after initiation of treatment of about 71 letters (ETDRS or Snellen equivalent) when on the HDq12 regimen; or a BCVA of about 68 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 40 weeks after initiation of treatment of about 72 letters (ETDRS or Snellen equivalent) when on the HDq12 regimen; or a BCVA of about 69 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 44 weeks after initiation of treatment of about 72 letters (ETDRS or Snellen equivalent) when on the HDq12 regimen; or a BCVA of about 70 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 48 weeks after initiation of treatment of about 73 letters (ETDRS or Snellen equivalent) when on the HDq12 regimen; or a BCVA of about 70 letters (ETDRS or Snellen equivalent) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA improvement, by week 48 following treatment initiation, of about 9 or 10 letters (ETDRS or Snellen equivalent) when baseline BCVA is about ≤73 ETDRS letters when on HDq12 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen after the immediately preceding dose.
- A method for achieving a BCVA improvement by week 48 following treatment initiation, of about 5 or 6 letters (ETDRS or Snellen equivalent) when baseline BCVA is about >73 ETDRS letters when on HDq12 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) after the immediately preceding dose.
- A method for achieving a BCVA improvement, by week 48 following treatment initiation, of about 8 or 9 letters (ETDRS or Snellen equivalent) when baseline BCVA is about ≤73 ETDRS letters when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks (HDq16 regimen) after the immediately preceding dose.
- A method for achieving a BCVA improvement, by week 48 following treatment initiation, of about 4 or 5 letters (ETDRS or Snellen equivalent) when baseline BCVA is about >73 ETDRS letters when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks (HDq16 regimen) after the immediately preceding dose.
- A method for achieving a BCVA improvement, by week 48 following treatment initiation, of about 7 or 8 letters (ETDRS or Snellen equivalent) when baseline CRT is < about 400 micrometers when on a HDq12 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) after the immediately preceding dose.
- A method for achieving a BCVA improvement, by week 48 following treatment initiation, of about 9 or 10 letters (ETDRS or Snellen equivalent) when baseline CRT is ≥400 micrometers when on a HDq12 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) after the immediately preceding dose.
- A method for achieving a BCVA improvement, by week 48 following treatment initiation, of about 5 or 6 letters (ETDRS or Snellen equivalent) when baseline CRT is < about 400 micrometers when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks (HDq16 regimen) after the immediately preceding dose.
- A method for achieving a BCVA improvement, by week 48 following treatment initiation, of about 9 or 10 letters (ETDRS or Snellen equivalent) when baseline CRT is ≥ about 400 micrometers when on a HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks (HDq16 regimen) after the immediately preceding dose.
- A method for achieving a gain of ≥5, ≥10 or ≥15 letters BCVA (according to ETDRS letter score) by week 12, 24, 36, 48, 60, 72, 84, 90 or 96 from start of treatment); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) or 20 weeks (HDq20 regimen) after the immediately preceding dose.
- A method for achieving a ≥ 2 or ≥3 step improvement in Diabetic Retinopathy Severity Scale (DRSS) by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a ≥ 2 step improvement in diabetic retinopathy severity scale (DRSS) by 4, 8, 12, 16, 20, 24, 28, 32, 36, 40, 44, 48 or 60 weeks from start of treatment); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a retina without fluid (total fluid, intraretinal fluid [IRF] and/or subretinal fluid [SRF]) at the foveal center and in center subfield by week 12, 24, 36, 48, 60, 72, 84, 90 or 96 from start of treatment as measured by optical coherence tomography (OCT); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a no vascular leakage from the retina as measured by fluorescein angiography (FA) by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maintenance of a fluid-free retina (total fluid, IRF and/or SRF at foveal center and in the center subfield) by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a reduction in total area of fluorescein leakage within ETDRS grid (mm²) at week 48 or 60 by about 12, 13 or 14 mm² or more as measured by fluorescein angiography; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a retina free of fluid on spectral domain optical coherence tomography (SD-OCT) by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a retina without fluid (total fluid, intraretinal fluid [IRF] and/or subretinal fluid [SRF]) at the foveal center by week 4, 8, 12, 16, 20, 24, 28, 32, 36, 40, 44 or 48 weeks from start of treatment); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a dry retina by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a foveal center without fluid by week 12, 24, 36, 48, 60, 72, 84, 90 or 96 from start of treatment as measured by optical coherence tomography (OCT); in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a change in central retinal thickness, by 4 weeks after initiation of treatment of about -118 or -118.3 micrometers (±17, 18 or 19 micrometers) when on the HDq12 regimen; or of about -124 or -125 or -124.9 or -125.5 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 8 weeks after initiation of treatment of about -137 or -137.4 micrometers (±17, 18 or 19 micrometers) when on the HDq12 regimen; or of about -139 or -140 or -139.6 or -140.3 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 12 weeks after initiation of treatment of about -150 or -150.1 micrometers (±17, 18 or 19 micrometers) when on the HDq12 regimen; or of about -152 or -153 or -152.7 or -153.4 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 16 weeks after initiation of treatment of about -139 or -139.4 micrometers (±17, 18 or 19 micrometers) when on the HDq12 regimen; or of about -145 or -146 or -145.5 or -146.4 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 20 weeks after initiation of treatment of about -117 or -117.1 micrometers (±17, 18 or 19 micrometers) when on the HDq12 regimen; or of about -112 or -113 or -112.5 or -113.3 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 24 weeks after initiation of treatment of about -158 or -158.1 micrometers (±17, 18 or 19 micrometers) when on the HDq12 regimen; or of about -103 or -104 or -103.8 or -104.3 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 28 weeks after initiation of treatment of about -146 or -147 or -146.7 micrometers (±17, 18 or 19 micrometers) when on the HDq12 regimen; or of about -162 or-162.3 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 32 weeks after initiation of treatment of about -132 micrometers (±17, 18 or 19 micrometers) when on the HDq12 regimen; or of about -145 or -146 or -145.8 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 36 weeks after initiation of treatment of about -168 or -168.1 micrometers (±17, 18 or 19 micrometers) when on the HDq12 regimen; or of about -124 or -125 or -124.7 or -125.2 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 40 weeks after initiation of treatment of about -163 micrometers (±17, 18 or 19 micrometers) when on the HDq12 regimen; or of about -122 or -123 or -122.5 or -123.1 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 44 weeks after initiation of treatment of about -147 or -148 or -147.4 micrometers (±17, 18 or 19 micrometers) when on the HDq12 regimen; or of about -164 or-164.1 or -164.3 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 48 weeks after initiation of treatment of about -171 or -172 or -171.7 micrometers (±17, 18 or 19 micrometers) when on the HDq12 regimen; or of about -148 or -149 or -148.3 or -149.4 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 60 weeks after initiation of treatment of about -181.95 or -176.24 (±17, 18 or 19 micrometers) micrometers when on the HDq12 regimen; or of about -166.26 or -167.18 micrometers (±17, 18 or 19 micrometers) when on the HDq16 regimen; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a reduction in CRT by week 4, 5, 6, 7 or 8 after initiation of treatment which is maintained (within about ±17, ±18 or ±19 micrometers) thereafter during the treatment regimen to at least week 48 or 60; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a decrease in central retinal thickness (CRT) by about 100, 125, 150, 175 or 200 micrometers by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a reduction in CRT of about 148-182 micrometers by week 48 or 60 as measured by optical coherence tomography (OCT) wherein the baseline CRT is about 449, 450, 455 or 460 micrometers; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a decrease in central retinal thickness (CRT) by at least about 100, 125, 130, 135, 140, 145, 149, 150, 155, 160, 165, 170, 171, 172, 173, 174 or 175 micrometers by week 4, 8, 12, 16, 20, 24, 28, 32, 36, 40, 44, 48 or 60 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 0.1667 days after the first dose, free aflibercept in plasma of about 0.149 (±0.249) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 0.3333 days after the first dose, free aflibercept in plasma of about 0.205 (±0.250) mg/l; wherein at baseline free aflibercept in plasma not detectable, for example, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 1 day after the first dose, free aflibercept in plasma of about 0.266 (±0.211) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 2 days after the first dose, free aflibercept in plasma of about 0.218 (±0.145) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 4 days after the first dose, free aflibercept in plasma of about 0.140 (±0.0741) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 7 days after the first dose, free aflibercept in plasma of about 0.0767 (±0.0436) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 14 days after the first dose, free aflibercept in plasma of about 0.0309 (±0.0241) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 21 days after the first dose, free aflibercept in plasma of about 0.0171 (±0.0171) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 28 days after the first dose, free aflibercept in plasma of about 0.00730 (±0.0113) mg/l; wherein at baseline free aflibercept in plasma not detectable, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 0.1667 days after the first dose, adjusted bound aflibercept in plasma of about 0.00698 (±0.0276) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 0.3333 days after the first dose, adjusted bound aflibercept in plasma of about 0.00731 (±0.0279) mg/l; wherein at baseline there is about 0.00583 mg/l (±0.0280) adjusted bound aflibercept, for example, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 1 days after the first dose, adjusted bound aflibercept in plasma of about 0.0678 (±0.0486) mg/l; wherein at baseline there is about 0.00583 mg/l (±0.0280) adjusted bound aflibercept, for example, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 2 days after the first dose, adjusted bound aflibercept in plasma of about 0.138 (±0.0618) mg/l; wherein at baseline there is about 0.00583 mg/l (±0.0280) adjusted bound aflibercept, wherein the subject has not received intravitreal aflibercept protein treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 4 days after the first dose, adjusted bound aflibercept in plasma of about 0.259 (±0.126) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 7 days after the first dose, adjusted bound aflibercept in plasma of about 0.346 (±0.151) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 14 days after the first dose, adjusted bound aflibercept in plasma of about 0.374 (±0.110) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 21 days after the first dose, adjusted bound aflibercept in plasma of about 0.343 (±0.128) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving at about 28 days after the first dose, adjusted bound aflibercept in plasma of about 0.269 (±0.149) mg/l; wherein at baseline there is about 0.00583 mg/l (±0.0280) adjusted bound aflibercept, wherein the subject has not received intravitreal aflibercept treatment for at least 12 weeks; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maximum level of free aflibercept in the plasma that is reached about 0.965 day after the first dose; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maximum level of about 0.310 mg/l (±0.263) free aflibercept in the plasma; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving free aflibercept in the plasma of from about 0 to about 1.08 mg/L (0.1, 0.2, 0.3, 0.4, 0.5, 0.6, 0.7, 0.8, 0.9, 1.0 or 1.1 mg/l); in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving free aflibercept in the plasma maximum (mg/l) per dose (mg) of aflibercept of about 0.388 (±0.0328) mg/l/mg; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maximum level of adjusted bound aflibercept in the plasma that is reached about 14 days after the first dose; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maximum level of about 0.387 mg/l (±0.135) adjusted bound aflibercept in the plasma; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving adjusted bound aflibercept in the plasma of from about 0.137 to about 0.774 mg/L; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving adjusted bound aflibercept in the plasma maximum (mg/l) per dose (mg) of aflibercept of about 0.483 (±0.0168) mg/l/mg; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving undetectable plasma concentrations of anti-drug antibodies against aflibercept after 48 or 60 weeks of treatment; in a subject in need thereof having an angiogenic eye disorder (preferably DR and/or DME) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving an improvement from pre-treatment baseline in National Eye Institute Visual Function Questionnaire (NEI-VFQ) total score by week 12, 24, 36, 48, 60, 72, 84, 90 or 96 from start of treatment; in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a lack of macular edema by week 12, 24, 36, 48, 60, 72, 84, 90 or 96 from start of treatment in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving:
   a change in central retinal thickness of about -118 or -119 or -118.3 micrometers, between initiation of treatment (week 0) and week 4 when on the HDq12 regimen;
   a change in central retinal thickness of about -19, -20 or -19.1 micrometers, between weeks 4 and 8 when on the HDq12 regimen;
   a change in central retinal thickness of about -12, -13 or -12.7 micrometers, between weeks 8 and 12 when on the HDq12 regimen;
   a change in central retinal thickness of about -40, or -41 micrometers, between weeks 20 and 24 when on the HDq12 regimen;
   a change in central retinal thickness of about -36, -37 or -36.1 micrometers, between weeks 32 and 36 when on the HDq12 regimen;
   a change in central retinal thickness of about -24, -25 or -24.3 micrometers, between weeks 44 and 48 when on the HDq12 regimen;
   a change in central retinal thickness of -4, -5 or -4.5 micrometers, between weeks 48 and 60 when on the HDq12 regimen;
   a change in central retinal thickness of about -124, -125 or -124.9 micrometers, between initiation of treatment (week 0) and week 4 when on the HDq16 regimen;
   a change in central retinal thickness of about -14, -15 or -14.7 micrometers, between weeks 4 and 8 when on the HDq16 regimen;
   a change in central retinal thickness of about -13, -14 or -13.1 micrometers, between weeks 8 and 12 when on the HDq16 regimen;
   a change in central retinal thickness of about -58, -59 or -58.5 micrometers, between weeks 24 and 28 when on the HDq16 regimen;
   a change in central retinal thickness of about -41, -42 or -41.6 micrometers, between weeks 40 and 44 when on the HDq16 regimen; and/or
   a change in central retinal thickness of -18, -19 or -18.9 micrometers, between weeks 48 and 60 when on the HDq16 regimen,
   in a subject in need thereof having an angiogenic eye disorder (preferably DR and/or DME) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.

The molecular weight adjusted concentration of bound aflibercept (adjusted bound aflibercept) is calculated by multiplying the observed concentrations by 0.717 to account for the target VEGF weight in the complex in plasma in the concentration-time profiles discussed herein.

In an embodiment of the invention, CRT and/or retinal fluid is as measured on spectral domain optical coherence tomography (SD-OCT). In an embodiment of the invention, any of such achievements are maintained as long as the subject is receiving the treatment regimen. In an embodiment of the invention, a subject receiving a treatment for an angiogenic eye disorder, *e.g*., diabetic macular edema (DME) and/or diabetic retinopathy (DR), does not experience or is no more likely to experience than a subject receiving Eylea according to the prescribed dosage regimen:
- Ocular serious TEAE (*e.g*., through week 48 after treatment initiation), for example, cataract subcapsular, retinal detachment, ulcerative keratitis, vitreous haemorrhage or increased intraocular pressure, angle closure glaucoma, cataract, choroidal detachment, retinal detachment, retinal haemorrhage, skin laceration and/or vitreous haemorrhage;
- TEAE intraocular inflammation (*e.g*., through week 48 after treatment initiation), for example, chorioretinitis, iridocyclitis, iritis, uveitis, vitreal cells and/or vitritis;
- Non-ocular serious TEAEs (*e.g*., through week 48 after treatment initiation), for example, acute left ventricular failure, acute myocardial infarction, cardiac arrest, coronary artery disease, myocardial infarction, covid-19 pneumonia, gangrene, pneumonia, hyponatraemia, cerebrovascular accident, acute kidney injury, acute respiratory failure, angina pectoris, chest pain, cellulitis, pneumonia, pyelonephritis acute, urinary tract infection, upper limb fracture, hyponatraemia, osteoarthritis, bladder neoplasm and/or cerebrovascular accident;
- Treatment emergent APTC event (*e.g*., through week 48 after treatment initiation), for example, non-fatal myocardial infarction, non-fatal stroke and/or vascular death;
- Treatment emergent hypertension events (*e.g*., through week 48 after treatment initiation), for example, blood pressure increased (diastolic and/or systolic), hypertension, diastolic hypertension, systolic hypertension, hypertensive crisis, hypertensive emergency, hypertensive urgency, labile hypertension and/or white coat hypertension;
- Potentially clinically significant values (*e.g.*, through week 48 after treatment initiation), for example, systolic blood pressure≥160 mmHg and an increase from baseline of ≥20 mmHg; and/or diastolic blood pressure ≥110 mmHg and an increase from baseline of ≥10 mmHg; and/or
- Death (*e.g*., through week 48 after treatment initiation), for example, due to cardiac arrest, cardio-respiratory arrest, left ventricular failure, myocardial infarction, death, sudden death, covid-19, pneumonia, diabetic metabolic decompensation, endometrial cancer, acute kidney injury, abdominal strangulated hernia, pneumonia aspiration, skull fracture, metastatic neoplasm and/or non-small cell lung cancer

The present invention further includes methods for achieving a pharmacokinetic effect in a subject suffering from DR and/or DME comprising administering to an eye of the subject, at least one dose (*e*.*g*., a first dose) of about ≥8 mg VEGF antagonist (*e.g*., a VEGF receptor fusion protein such as aflibercept). The pharmacokinetic effect can be one or more set forth below:
- Decreasing ocular clearance of free VEGF antagonist (*e.g*., a VEGF receptor fusion protein such as aflibercept), *e.g*., by about 34% relative to that of a dose of 2 mg of the VEGF antagonist (*e.g*., a VEGF receptor fusion protein such as aflibercept);
- 0.2-0.3 (*e.g*., 0.310 or 0.245) mg/l free VEGF antagonist (*e.g*., a VEGF receptor fusion protein such as aflibercept) in the plasma, *e.g*., within about 1, 2 or 3 days of the first dose;
- About 0.38 or 0.5 mg/l adjusted bound VEGF antagonist (*e.g*., a VEGF receptor fusion protein such as aflibercept) in the plasma, *e.g*., within about 14, 15 or 16 days of the first dose;
- Reaches LLOQ of free VEGF antagonist (*e.g*., a VEGF receptor fusion protein such as aflibercept) (about 15.6 ng/ml) in the plasma by about 3 or 4 weeks of the first dose;
- Reaches LLOQ of free VEGF antagonist (*e.g*., a VEGF receptor fusion protein such as aflibercept) (about 15.6 ng/ml) in the ocular compartment by about 15 weeks of the first dose;
- Reaches LLOQ of free VEGF antagonist (*e.g*., a VEGF receptor fusion protein such as aflibercept) (about 15.6 ng/ml) that is about 6 weeks longer than achieved for a 2 mg dose of the antagonist;
- A clearance of the VEGF antagonist (*e.g*., a VEGF receptor fusion protein such as aflibercept) from the ocular compartment of about 0.41 ml/day;
for example, wherein the subject is administered:
a single initial dose of about ≥8 mg or more of a VEGF antagonist (*e.g*., a VEGF receptor fusion protein such as aflibercept), followed by one or more secondary doses of about ≥8 mg or more of the VEGF antagonist (*e.g*., a VEGF receptor fusion protein such as aflibercept), followed by one or more tertiary doses of about ≥8 mg or more of the VEGF antagonist (*e.g.*, a VEGF receptor fusion protein such as aflibercept); wherein each secondary dose is administered about 2 to 4 weeks (preferably, 4 weeks) after the immediately preceding dose; and wherein each tertiary dose is administered about 12-20 weeks (preferably, 12, 16 or 20 weeks) after the immediately preceding dose.

In an embodiment of the invention, the method for treating or preventing diabetic macular edema, in a subject in need thereof comprises administering ≥8 mg aflibercept (0.07 mL or 70 microliters) administered by intravitreal injection every 4 weeks (approximately every 28 days +/- 7 days, monthly) for the first three doses, followed by ≥8 mg aflibercept (0.07 mL) via intravitreal injection once every 8 - 16 weeks (2 - 4 months, +/- 7 days).

In an embodiment of the invention, the method for treating or preventing diabetic retinopathy (DR), in a subject in need thereof comprises administering ≥8 mg aflibercept (0.07 mL or 70 microliters) administered by intravitreal injection every 4 weeks (approximately every 28 days +/- 7 days, monthly) for the first three doses, followed by ≥8 mg aflibercept (0.07 mL) via intravitreal injection once every 8 - 16 weeks (2 - 4 months, +/- 7 days).

Some subject may be excluded from administration based, for example, on the existence of certain exclusion criteria. For example, in an embodiment of the invention, the criteria are one or more of ocular infection, periocular infection; active intraocular inflammation; and/or hypersensitivity, *e.g*., to aflibercept or any component of a formulation thereof. The method presented herein may include the step of evaluating the subject for such exclusion criteria and excluding the subject from said administration if any one or more if found in the subject; and proceeding with administration if exclusion criteria are not found.

In an embodiment of the invention, a subject receiving VEGF antagonist (*e.g*., a VEGF receptor fusion protein such as aflibercept) is monitored for adverse events (AEs) such as conjunctival hemorrhage, cataract, vitreous detachment, vitreous floaters, corneal epithelium defect and/or increased intraocular pressure. If an AE is found, the AE can be treated in the subject and treatment can either be discontinued or continued.

The methods of present invention can include preparatory steps that include use of
- one single-dose glass vial having a protective plastic cap and a stopper containing an aqueous formulation comprising ≥8 mg aflibercept in about 70 microliters;
- one 18-gauge x 1½-inch, 5-micron, filter needle that includes a tip and a bevel;
- one 30-gauge x ½-inch injection needle; and
- one 1-mL Luer lock syringe having a graduation line marking for 70 microliters of volume;
packaged together (kits including such items form part of the present invention). The steps can include, for example: (1) visually inspecting the aqueous formulation in the vial and, if particulates, cloudiness, or discoloration are visible, then using another vial of aqueous formulation containing the aflibercept; (2) removing the protective plastic cap from the vial; and (3) cleaning the top of the vial with an alcohol wipe; then, using aseptic technique the following steps: (4) removing the 18-gauge x 1½-inch, 5-micron, filter needle and the 1 mL syringe from their packaging; (5) attaching the filter needle to the syringe by twisting it onto the Luer lock syringe tip; (6) pushing the filter needle into the center of the vial stopper until the needle is completely inserted into the vial and the tip touches the bottom or a bottom edge of the vial; (7) withdrawing all of the aflibercept vial contents into the syringe, keeping the vial in an upright position, slightly inclined, while ensuring the bevel of the filter needle is submerged into the liquid; (8) continuing to tilt the vial during withdrawal keeping the bevel of the filter needle submerged in the formulation; (9) drawing the plunger rod sufficiently back when emptying the vial in order to completely empty the filter needle; (10) removing the filter needle from the syringe and disposing of the filter needle; (11) removing the 30-gauge x ½-inch injection needle from its packaging and attaching the injection needle to the syringe by firmly twisting the injection needle onto the Luer lock syringe tip; (12) holding the syringe with the needle pointing up, and checking the syringe for bubbles, wherein if there are bubbles, gently tapping the syringe with a finger until the bubbles rise to the top; and (13) slowly depressing the plunger so that the plunger tip aligns with the graduation line that marks 70 microliters on the syringe. Preferably injection of VEGF antagonist (*e.g*., a VEGF receptor fusion protein such as aflibercept) as performed in methods of the present invention is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.

### Switching

The present invention includes embodiments wherein a subject has a history of receiving one or more doses of aflibercept or any other VEGF antagonist (*e.g*., 2 mg aflibercept such as Eylea (*e.g*., 2q8 regimen) or one or more doses of about 8 mg aflibercept) and is then switched to a dosing regimen of the present invention, *e.g*., HDq12, HDq16, HDq20, HDq12-20 or HDq16-20, starting at any step in the regimen.

For example, a subject may have been initially administered aflibercept manufactured by a first process (a first aflibercept) and then is switched to aflibercept manufactured by a different process (*e.g*., a second aflibercept; *e.g*., a biosimilar aflibercept); for example, wherein each process is carried out by a different manufacturer.

Subjects may initially be receiving aflibercept according to a 2q8 dosing regimen comprising administering 5 initial monthly doses followed by one or more maintenance doses every 8 weeks (*e.g*., Eylea) and then switch to a HDq12-20 or HDq12 or HDq16 or HDq20 dosing regimen. The aflibercept administered in the HDq12-20 or HDq12 or HDq16 or HDq20 dosing regimen may have been manufactured by a different process, *e.g*., by a different manufacturer.

In addition, a subject may be receiving a HDq12-20 or HDq12 or HDq16 or HDq20 dosing regimen with aflibercept and then switch to aflibercept manufactured by a different process, *e.g*., by a different manufacturer, while remaining on the HDq12-20 or HDq12 or HDq16 or HDq20 dosing regimen.

The present invention encompasses, but is not limited to, methods for treating an angiogenic eye disorder, preferably DR and/or DME, wherein a subject is switched, from a first aflibercept (manufactured by one process) for use in a HDq12-20 or HDq12 or HDq16 or HDq20 regimen to a second aflibercept (manufactured by another process) for use in a HDq12-20 or HDq12 or HDq16 or HDq20 regimen. The present invention includes embodiments wherein the subject initiates treatment of the second aflibercept HDq12-20 or HDq20 or HDq12 or HDq16 regimen at any dosing phase-initial, secondary or tertiary/maintenance-after having received the initial dose, one or more secondary doses or one or more tertiary/maintenance doses of the first aflibercept HDq12-20 or HDq20 or HDq12 or HDq16 regimen. Thus, the present invention includes embodiments wherein, the subject is switched from any phase of the first HDq12-20 or HDq20 or HDq12 or HDq16 regimen into any phase of the second HDq12-20 or HDq20 or HDq12 or HDq16 regimen. Preferably, the subject will pick up receiving the second aflibercept HDq12-20 or HDq20 or HDq12 or HDq16 regimen at the dosing phase that corresponds to where dosing was stopped with the first HDq12-20 or HDq20 or HDq12 or HDq16 regimen, *e.g.*, if a particular secondary dose was due with the first aflibercept therapy, the subject would timely receive the same secondary dose with the second aflibercept and, for example, continue receiving the second aflibercept according to the HDq12-20 or HDq20 or HDq12 or HDq16 regimen as needed thereafter.

The present invention also encompasses, but is not limited to, methods for treating an angiogenic eye disorder wherein a subject is switched, from a first aflibercept for use in a 2q8 regimen to a second aflibercept for use in a HDq12-20 or HDq20 or HDq12 or HDq16 regimen. The present invention includes embodiments wherein the subject initiates treatment of the second aflibercept HDq12-20 or HDq20 or HDq12 or HDq16 regimen at any dosing phase-initial, secondary or tertiary/maintenance-after having received the initial dose, one or more secondary doses or one or more tertiary/maintenance doses of the first aflibercept 2q8 regimen. Thus, the present invention includes embodiments wherein, for example, the subject is switched directly to the maintenance phase of the HDq12-20 or HDq20 or HDq12 or HDq16 regimen with second aflibercept after having received the initial and a single secondary dose in the 2q8 regimen with the first aflibercept.

In an embodiment of the invention, a subject who has received an initial, one or more secondary doses and/or one or more tertiary doses of 2 mg aflibercept (*e*.*g.*, Eylea) therapy (*e.g*., 2q8) according to the prescribed dosing regimen may receive an ≥8 mg dose of aflibercept, undergo an evaluation by a treating physician in about 8 or 10 or 12 weeks and, if, in the judgment of a treating physician, dosing every 12 weeks or every 16 weeks or every 20 weeks is appropriate (*e.g.,* there is no undue loss in BCVA and/or increase in CRT), then continuing to dose the subject every 12-20 weeks, 12 weeks or 16 weeks or 20 weeks with ≥8 mg aflibercept.

The present invention includes methods for treating or preventing an angiogenic eye disorder, preferably DR or DME, in a subject in need thereof, by administering to said subject ≥8 mg aflibercept, wherein:
- the subject has received an initial ≥8 mg dose of aflibercept then the method comprises, after 1 month, administering to the subject the first ≥8 mg secondary dose of aflibercept and 1 month thereafter, administering the 2^{nd} ≥8 mg secondary dose of aflibercept; and then, every 12-20, 12 or 16 or 20 weeks thereafter, administering one or more ≥8 mg maintenance doses of aflibercept according to the HDq12-20 or HDq12 or HDq16 or HDq20 dosing regimen;
   or
- the subject has received an initial ≥8 mg dose of aflibercept & 1^{st} ≥8 mg secondary dose of aflibercept after 1 month, then the method comprises, after another 1 month, administering to the subject the 2^{nd} ≥8 mg secondary dose of aflibercept; and then, every 12-20, 12 or 16 or 20 weeks thereafter, one or more ≥8 mg maintenance doses of aflibercept according to the HDq12-20 or HDq12 or HDq16 or HDq20 dosing regimen;
   or
- the subject has received an initial ≥8 mg dose of aflibercept & 1^{st} ≥8 mg secondary dose of aflibercept after 1 month & the 2^{nd} ≥8 mg secondary dose of aflibercept after another month, then the method comprises, after 12-20 or 12 or 16 or 20 weeks administering to the subject the 1^{st} ≥8 mg maintenance dose of aflibercept and all further ≥8 mg maintenance doses of aflibercept every 12-20 or 12 or 16 or 20 weeks according to the HDq12-20 or HDq12 or HDq16 or HDq20 dosing regimen;
   or
- the subject has received an initial ≥8 mg dose of aflibercept & a 1^{st} ≥8 mg secondary dose of aflibercept after 1 month & the 2^{nd} ≥8 mg secondary dose of aflibercept after another month, then every 12-20 or 12 or 16 or 20 weeks thereafter, the subject has received one or more ≥8 mg maintenance doses of aflibercept; and, then the method comprises, after 12-20 or 12 or 16 or 20 weeks from the last maintenance dose of aflibercept, administering to the subject one or more ≥8 mg maintenance doses of aflibercept and all further ≥8 mg maintenance doses of aflibercept every 12-20 or 12 or 16 or 20 weeks according to the HDq12-20 or HDq12 or HDq16 or HDq20 dosing regimen.

Patients may switch from a reference 2 mg aflibercept dosing regimen to a particular step in the HDq12-20 or HDq12 or HDq16 or HDq20 dosing regimen. For example, a subject may receive only the initial 2mg dose of reference, and then, skipping the initial and secondary doses of the HDq12-20 or HDq12 or HDq16 or HDq20 dosing regimen, begin receiving the HDq12-20 or HDq12 or HDq16 or HDq20 maintenance doses. The present invention includes methods for treating or preventing DR and/or DME, in a subject in need thereof, by administering to said subject ≥8 mg aflibercept, wherein:
(1) the subject has received an initial 2 mg dose of aflibercept, then the method comprises, after 1 month, administering to the subject the initial ≥8 mg dose of aflibercept and, 1 month thereafter, the 1^{st} ≥8 mg secondary dose of aflibercept; and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(2) the subject has received an initial 2 mg dose of aflibercept, then the method comprises, after 1 month, administering to the subject the first ≥8 mg secondary dose of aflibercept and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(3) the subject has received an initial 2 mg dose of aflibercept, then the method comprises, after 1 month, administering to the subject the 2^{nd} ≥8 mg secondary dose of aflibercept and then, every 12-20 or 12 or 16 or 20 weeks thereafter, one or more ≥8 mg maintenance doses of aflibercept according to the HDq12-20 or HDq12 or HDq16 or HDq20 dosing regimen;
(4) the subject has received an initial 2 mg dose of aflibercept, then the method comprises, after 1 month, administering to the subject the 1^{st} ≥8 mg maintenance dose of aflibercept and all further ≥8 mg maintenance doses of aflibercept every 12-20 or 12 or 16 or 20 weeks according to the HDq12-20 or HDq12 or HDq16 or HDq20 dosing regimen;
(5) the subject has received an initial 2 mg dose of aflibercept and a 1^{st} 2 mg secondary dose of aflibercept after 1 month, then the method comprises, after another 1 month, administering to the subject the initial ≥8 mg dose of aflibercept and, 1 month thereafter, the 1^{st} ≥8 mg secondary dose of aflibercept; and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(6) the subject has received an initial 2 mg dose of aflibercept and a 1^{st} 2 mg secondary dose of aflibercept after 1 month, then the method comprises, after another 1 month, administering to the subject a first ≥8 mg secondary dose of aflibercept and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(7) the subject has received an initial 2 mg dose of aflibercept and a 1^{st} 2 mg secondary dose of aflibercept after 1 month, then the method comprises, after another 1 month, administering to the subject the 2^{nd} ≥8 mg secondary dose of aflibercept and then, every 12-20 or 12 or 16 or 20 weeks thereafter, one or more ≥8 mg maintenance doses of aflibercept according to the HDq12-20 or HDq12 or HDq16 or HDq20 dosing regimen;
(8) the subject has received an initial 2 mg dose of aflibercept and a 1^{st} 2 mg secondary dose of aflibercept after 1 month, then the method comprises, after another 1 month, administering to the subject the 1^{st} ≥8 mg maintenance dose of aflibercept and all further ≥8 mg maintenance doses of aflibercept every 12-20 or 12 or 16 or 20 weeks according to the HDq12-20 or HDq12 or HDq16 or HDq20 dosing regimen;
(9) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the initial ≥8 mg dose of aflibercept and, 1 month thereafter, the 1^{st} ≥8 mg secondary dose of aflibercept; and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(10) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the first ≥8 mg secondary dose of aflibercept and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(11) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the 2^{nd} ≥8 mg secondary dose of aflibercept and then, every 12-20 or 12 or 16 or 20 weeks thereafter, one or more ≥8 mg maintenance doses of aflibercept according to the HDq12-20 or HDq12 or HDq16 or HDq20 dosing regimen;
(12) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after 2 months, administering to the subject the 1^{st} ≥8 mg maintenance dose of aflibercept and, all further ≥8 mg maintenance doses of aflibercept every 12-20 or 12 or 16 or 20 weeks according to the HDq12-20 or HDq12 or HDq16 or HDq20 dosing regimen;
(13) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, then the method comprises, after 1 month, administering to the subject the initial ≥8 mg dose of aflibercept and 1 month thereafter, the 1^{st} ≥8 mg secondary dose of aflibercept; and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(14) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, then the method comprises, after 1 month, administering to the subject the first ≥8 mg secondary dose of aflibercept and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(15) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, then the method comprises, after 1 month, administering to the subject the 2^{nd} ≥8 mg secondary dose of aflibercept and then, every 12-20 or 12 or 16 or 20 weeks thereafter, one or more ≥8 mg maintenance doses of aflibercept according to the HDq12-20 or HDq12 or HDq16 or HDq20 dosing regimen;
(16) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, then the method comprises, after 2 months, administering to the subject the 1^{st} ≥8 mg maintenance dose of aflibercept and all further ≥8 mg maintenance doses of aflibercept every 12-20 or 12 or 16 or 20 weeks according to the HDq12-20 or HDq12 or HDq16 or HDq20 dosing regimen;
(17) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month; and a 4^{th} 2 mg secondary dose of aflibercept after 1 month; thereafter, then the method comprises, after 2 months, administering to the subject the initial ≥8 mg dose of aflibercept and, 1 month thereafter, the 1^{st} ≥8 mg secondary dose of aflibercept; and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(18) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month; and a 4^{th} 2 mg secondary dose of aflibercept after 1 month; thereafter, then the method comprises, after 2 months, administering to the subject the first ≥8 mg secondary dose of aflibercept and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(19) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, and a 4^{th} 2 mg secondary dose of aflibercept after 1 month; thereafter, then the method comprises, after 2 months, administering to the subject the 2^{nd} ≥8 mg secondary dose of aflibercept and, 12-20 or 12 or 16 or 20 weeks thereafter, one or more 12-20 or 12 or 16 or 20 weekly ≥8 mg maintenance doses of aflibercept according to the HDq12-20 or HDq12 or HDq16 or HDq20 dosing regimen;
(20) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, and a 4^{th} 2 mg secondary dose of aflibercept after 1 month, thereafter, then the method comprises, after 2 months, administering to the subject the 1^{st} ≥8 mg maintenance dose of aflibercept and, all further ≥8 mg maintenance doses of aflibercept every 12-20 or 12 or 16 or 20 weeks according to the HDq12-20 or HDq12 or HDq16 or HDq20 dosing regimen;
(21) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month, and a 4^{th} 2 mg secondary dose of aflibercept after 1 month; and one or more 2 mg maintenance doses every 8 weeks thereafter, then the method comprises, 2 months after the last aflibercept maintenance dose, administering to the subject the initial ≥8 mg dose of aflibercept and, 1 month thereafter, the 1^{st} ≥8 mg secondary dose of aflibercept; and 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(22) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month; and a 4^{th} 2 mg secondary dose of aflibercept after 1 month; and one or more 2 mg maintenance doses every 8 weeks thereafter, then the method comprises, 2 months after the last aflibercept maintenance dose administering to the subject the first ≥8 mg secondary dose of aflibercept and, 1 month thereafter, the 2^{nd} ≥8 mg secondary dose of aflibercept; and then, every 12-20 or 12 or 16 or 20 weeks thereafter, one or more ≥8 mg maintenance doses of aflibercept according to the HDq12-20 or HDq12 or HDq16 or HDq20 dosing regimen;
(23) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month; and a 4^{th} 2 mg secondary dose of aflibercept after 1 month; and one or more 2 mg maintenance doses every 8 weeks thereafter, then the method comprises, 2 months after the last aflibercept maintenance dose, administering to the subject the 2^{nd} ≥8 mg secondary dose of aflibercept and, 12-20 or 12 or 16 or 20 weeks thereafter, one or more 12-20 or 12 or 16 or 20 weekly ≥8 mg maintenance doses of aflibercept according to the HDq12-20 or HDq12 or HDq16 or HDq20 dosing regimen;
   or
(24) the subject has received an initial 2 mg dose of aflibercept and a 1^{st} 2 mg secondary dose of aflibercept after 1 month and a 2^{nd} 2 mg secondary dose of aflibercept after another 1 month and a 3^{rd} 2 mg secondary dose of aflibercept after 1 month; and a 4^{th} 2 mg secondary dose of aflibercept after 1 month; and one or more 2 mg maintenance doses every 8 weeks thereafter, then the method comprises, 2 months after the last aflibercept maintenance dose, administering to the subject the 1^{st} ≥8 mg maintenance dose of aflibercept and, all further ≥8 mg maintenance doses of aflibercept every 12-20 or 12 or 16 or 20 weeks according to the HDq12-20 or HDq12 or HDq16 or HDq20 dosing regimen.

### Precision Dose Drug Delivery

The present invention provides methods as set forth herein wherein a VEGF antagonist (*e.g.,* aflibercept) is delivered with a high amount of precision, *e.g.,* with a drug delivery device (DDD) (*e.g.,* with a 0.5 mL volume), whether pre-filled or capable of being filled from a vial, and delivering a volume of between 70 and 100 microliter with an average volume of about 81 or 82 or 81-82 microliters, *e.g.,* with a standard deviation of about 4 or 5 or 4-5 microliters (*e.g.,* about 4.5 or 4.46 microliters) or less. In an embodiment of the invention, the DDD is a syringe, *e.g.,* with a 30 gauge, ½ inch needle.

One means for ensuring precision of a dose to be delivered with a device, such as a syringe, is by employing a syringe wherein the dose volume is device-determined. If the dose volume is device-determined, the device is designed only to deliver a single volume (*e.g.,* 87 microliters) or a single volume with a limited amount of acceptable error (± 4-5 microliters). Thus, if used properly, the user cannot deliver the wrong dose (*e.g.,* cannot deliver more than the intended volume from the device).

The present invention includes embodiments wherein, a precise dosage of about 8 mg or more is a dose of about 9, 9.3, 9.33, 9.7, 9.8, 9.9, 9.7-9.9 mg or more ± about 0.5, or ± about 0.51 mg is delivered to a subject's eye. The volume in which a dose is delivered can be, for example, about 70, 81, 82, 81.7, 85, 86, 87, 85-87 microliters ± about 4, 4.45, 4.5, or 5 microliters. Doses may be delivered with a dose delivery device (DDD) which is a syringe.

Highly precise doses of VEGF antagonist (*e.g.,* aflibercept) may be delivered, for example, in a volume that is device-determined (wherein the device is a syringe), by a method that includes the steps: (a) priming the syringe (*e.g.,* a pre-filled syringe), thereby removing air from the syringe and, thus avoiding injection of air into the eye, by advancing the plunger rod by a predetermined distance into the syringe body until advancement of the plunger rod is resisted by a stop; (b) rotating the plunger rod about a longitudinal axis; and (c) actuating the plunger rod to dispense a predetermined (device-determined) volume (*e.g.,* about 70, 81, 82, 81.7, 85, 86, 87, 85-87 microliters, ± about 4, 4.45, 4.5, or 5 microliters) of the formulation.

In an embodiment of the invention, the drug delivery device (DDD), comprises:
- a barrel including a longitudinal axis, a proximal end region, and a distal end region, the proximal end region including an opening, wherein the barrel is configured to receive a drug therein;
- a plunger rod disposed at least partially inside the barrel and protruding from the opening, wherein the plunger rod includes a rack having a plurality of teeth; and
- a pinion having a plurality of teeth configured to engage with the plurality of teeth of the rack,
wherein rotation of the pinion against the rack moves at least a part of the plunger rod along the longitudinal axis of the barrel; for example, which further comprises a shaft affixed to the pinion, wherein rotation of the shaft rotates the pinion against the rack which may include a knob affixed to the shaft. In an embodiment of the invention, the DDD further includes a magnifier disposed on the distal end region of the barrel. In an embodiment of the invention, the DDD further includes a stopper inside the barrel, wherein the stopper is affixed to a distal end of the plunger rod. In an embodiment of the invention, the DDD further includes a circular ratchet disposed coaxially with the pinion, wherein the circular ratchet has a diameter smaller than a diameter of the pinion; a spring-loaded pawl disposed on an internal circumference of the pinion, wherein the pawl is configured to engage the ratchet; and a shaft affixed to the ratchet, wherein rotation of the shaft in one direction causes rotation of the pinion, and rotation of the shaft in a second direction does not cause rotation of the pinion for example wherein the ratchet is disposed inside the pinion. In an embodiment of the invention, the pinion includes a plurality of teeth having a first height, and a stopper tooth having a second height greater than the first height, for example, wherein the second height of the stopper tooth prevents the pinion from engaging the plurality of teeth of the rack, and/or wherein the second height of the stopper tooth is configured to contact one of the plunger rod and the rack to stop rotation of the pinion. In an embodiment of the invention, the plunger rod includes an inner column and an outer lumen, and wherein the rack is disposed on the inner column, *e.g.,* wherein rotation of the pinion against the rack moves the inner column of the plunger rod independently of the outer lumen, and/or further including a shaft removably affixed to the pinion, wherein the shaft prevents movement of the outer lumen of the plunger rod relative to the barrel, and wherein removal of the shaft allows for movement of the outer lumen of the plunger rod relative to the barrel. In an embodiment of the invention, the plunger rod further includes a body and a flange, the flange extending partially along a longitudinal length of the body and having a width greater than a width of the body; wherein the barrel further comprises a plunger lock, the plunger lock including a through hole configured to allow the flange to pass through the second plunger lock in a specific orientation.

In an embodiment of the invention, the drug delivery device (DDD), comprises:
- a barrel including a longitudinal axis, a proximal end region, a distal end region, and an interior, the proximal end region including an opening and the interior including a threaded region; and
- a plunger rod disposed at least partially inside the barrel and protruding from the opening, the plunger rod including a threaded region configured to engage the threaded region of the barrel interior,
wherein rotation of the plunger rod about the longitudinal axis of the drug delivery device moves the plunger rod along the longitudinal axis. In an embodiment of the invention, the plunger rod further includes a tab protruding from the plunger rod in a first direction and located proximally from the threaded region of the plunger rod, and wherein the threaded region in the interior of the barrel further includes a slot sized and configured to allow for the tab to pass through the threaded region in the interior of the barrel, *e.g.,* wherein the slot includes a first segment parallel to the longitudinal axis of the drug delivery device and a second segment perpendicular to the longitudinal axis of the drug delivery device-the slot may include a third segment parallel to the longitudinal axis of the drug delivery device, wherein the second segment is in between the first segment and the third segment. In an embodiment of the invention, the tab is a first tab, and wherein the plunger rod further includes a second tab protruding from the plunger rod in a second direction opposite to the first direction, and wherein the threaded region in the interior of the barrel further includes a second slot sized and configured to allow for the second tab to pass through the threaded region in the interior of the barrel.

In an embodiment of the invention, the drug delivery device, includes:
- a barrel having a proximal end region, a distal end region, an opening in the proximal end region, an interior, and a threaded region in the interior;
- a sleeve disposed partly inside the barrel and protruding from the opening in the proximal end region of the barrel, the sleeve including a threaded region engaged with the threaded region of the barrel interior;
- a plunger rod disposed at least partially inside the sleeve; and
- a stopper inside the barrel and located distally from the sleeve, the stopper connected to a distal end of the plunger rod,
wherein rotation of the sleeve in a first direction around a longitudinal axis of the drug delivery device moves the sleeve towards the distal end region of the barrel. In an embodiment of the invention, rotation of the sleeve in the first direction moves the stopper towards the distal end region of the barrel. In an embodiment of the invention; the sleeve includes an inner passage, and the stopper has a diameter larger than a diameter of the inner passage; and/or the sleeve includes a tab disposed on an exterior of the sleeve, the tab located proximally from the threaded region of the barrel interior, and wherein the tab stops movement of the sleeve towards the distal end region of the barrel, *e.g.,* wherein the tab is configured to stop movement of the sleeve towards the distal end region of the barrel after the drug delivery device has been primed or wherein the tab is a first tab, and wherein the sleeve further includes a second tab disposed on an exterior of the sleeve, the second tab located distally from the threaded region of the barrel interior, wherein the second tab stops movement of the sleeve towards the proximal end region of the barrel.

In an embodiment of the invention, the drug delivery device, comprises:
- a barrel including a proximal end region and a distal end region, the proximal end region including an opening;
- a plunger rod including a body and a flange, the flange extending partially along a longitudinal length of the body and having a width greater than a width of the body, the plunger rod disposed at least partially inside the barrel and protruding from the opening;
- a first plunger lock disposed on the barrel, the first plunger lock configured to block the flange from entering the barrel; and
- a second plunger lock disposed in the barrel, the second plunger lock including a through hole configured to allow the flange to pass through the second plunger lock in a specific orientation.

For example, in an embodiment of the invention, the first plunger lock is removable and/or frangible. In an embodiment of the invention, a distance between the first plunger lock and the second plunger lock is equivalent to the distance that the stopper must travel to prime the drug delivery device; and/or the plunger rod is rotatable around a longitudinal axis of the drug delivery device.

Substances from such a DDD (*e.g.,* a formulation including aflibercept as described herein), having a plunger rod and a barrel, may be dispensed as follows:
- advancing the plunger rod by a predetermined distance into the barrel until advancement of the plunger rod is resisted by a stop;
- deactivating the stop; and
- actuating the plunger rod (*e.g.,* which includes a flange, wherein the stop includes a lock that prevents the flange from entering the barrel; or which includes a flange, wherein the stop comprises a lock that prevents the flange from entering the barrel) to deliver the substance.

Advancing the plunger rod may include the step of rotating a pinion against a rack disposed on the plunger rod, *e.g.,* wherein the stop comprises a shaft removably affixed to the pinion, and wherein deactivating the stop comprises removing the shaft from the pinion. Deactivating the stop may include the step of rotating the plunger rod. In an embodiment of the invention, deactivating the stop includes the step of removing the lock and/or breaking the lock.

In an embodiment of the invention, the drug delivery device, includes:
- a barrel including a longitudinal axis, a proximal end region, and a distal end region, the proximal end region including an opening and a rack disposed on the interior of the barrel, the rack having a plurality of teeth, wherein the barrel is configured to receive a drug therein;
- a plunger rod disposed at least partially inside the barrel and protruding from the opening, wherein the plunger rod includes a rack having a plurality of teeth; a pinion having a plurality of teeth configured to engage with the plurality of teeth of the plunger rod rack; and
- an inner plunger coupled to the pinion by a rod, wherein rotation of the pinion against the plunger rod rack results in movement of the inner plunger along the longitudinal axis of the barrel;
for example, wherein the teeth of the pinion are further configured to engage with the plurality of teeth of the rack disposed on the barrel. In an embodiment of the invention, the pinion is a first pinion, and further includes: a second pinion disposed coaxially with the first pinion, the second pinion having a diameter smaller than a diameter of the first pinion and a plurality of teeth configured to engage with the plurality of the teeth of the rack disposed on the barrel, wherein rotation of the first pinion results in rotation of the second pinion against the rack disposed on the barrel and in movement of the inner plunger along the longitudinal axis of the barrel.

See International patent application publication no. WO2019/118588.

In an embodiment of the invention, the drug delivery device (DDD), includes:
- a body;
- a plunger rod disposed partially inside the body;
- a protrusion extending from the plunger rod; and
- a blocking component coupled to a proximal end portion of the body, wherein the blocking component is a flange piece,
wherein, when the protrusion is in a first position relative to the blocking component, the blocking component restricts distal movement of the plunger rod to a first stopping point, and when the protrusion is in a second position relative to the blocking component, the blocking component restricts distal movement of the plunger rod to a second stopping point. In an embodiment of the invention, the DDD further includes: a stopper disposed in the body, wherein distal movement of the plunger rod distally moves the stopper; and a drug substance disposed in the body in between the stopper and a distal end of the body, wherein distal movement of the plunger rod to the first stopping point primes the drug delivery device, and distal movement of the plunger rod to the second stopping point dispenses a predetermined volume of the drug substance from a distal end of the device.

In an embodiment of the invention, moving the protrusion from the first position to the second position includes twisting the plunger rod relative to the blocking component. In an embodiment of the invention, the DDD further includes: a cavity in a proximal side of the blocking component, the cavity sized and configured to receive a portion of the protrusion, wherein when the protrusion is in the second position relative to the blocking component, the protrusion is positioned proximally from the cavity, such that distal movement of the plunger rod moves the protrusion into the cavity; *e.g.,* wherein the cavity is a first cavity, and further includes: a second cavity in a proximal side of the blocking component, the second cavity sized and configured to receive a portion of the protrusion, wherein the first and second cavity are located on opposite sides of a central longitudinal axis of the drug delivery device. In an embodiment of the invention, the plunger rod passes through an opening in the blocking component. In an embodiment of the invention the DDD further includes an actuation portion at a proximal end portion of the plunger rod, wherein the protrusion extends from the actuation portion, *e.g.,* wherein the actuation portion includes a generally cylindrical shape having a diameter greater than a width of the remainder of the plunger rod, wherein the protrusion extends from a side of the generally cylindrical shape, and wherein the actuation portion further comprises: a thumb pad on a proximal end of the actuation portion; and a ring on an exterior surface on the side of the generally cylindrical shape; *e.g.,* further including a proximal collar on the blocking component, wherein the actuation portion partially fits inside the proximal collar; *e.g.,* wherein the plunger rod further includes a pair of extensions protruding distally from the actuation portion and the blocking component (*e.g.,* which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes a pair of internal grooves formed along a sidewall of the blocking component; and wherein a portion of each extension is configured to be received by at least one of the pair of internal grooves upon rotation of the plunger rod relative to the blocking component to expand the extensions radially-outward from a compressed state to a relaxed state) includes a pair of openings; and wherein a portion of each extension is configured to be received by one of the pair of openings in the first stopping point. In an embodiment of the invention, the protrusion is a first protrusion, and further includes a second protrusion extending from the plunger rod in a direction opposite to the first protrusion. In an embodiment of the invention, the blocking component is slidably coupled to the body and includes a third cavity and a pair of ribs that extend into the third cavity, wherein the body includes a top flange and the pair of ribs are configured to engage the top flange received in the third cavity; wherein the pair of internal ribs are configured to apply a distally-directed force onto the top flange. In an embodiment of the invention, the blocking component is slidably coupled to the body and includes a pair of movable tabs that are configured to engage the body; and the pair of movable tabs are laterally deflectable upon receiving the body in the blocking component and are configured to apply a radially-inward directed force onto the body. In an embodiment of the invention, the blocking component further includes a pair of finger flanges, and each of the finger flanges includes a textured surface having a predefined pattern that increases a grip of the blocking component.

In an embodiment of the invention, the drug delivery device (DDD), includes:
- a body;
- a plunger rod having a distal end contacting a stopper inside the body, and a proximal end including an actuation portion with a thumb pad;
- a plurality of protrusions extending from the actuation portion; and
- a blocking component disposed on the body, the blocking component including a proximal collar having a plurality of slots,

wherein, when the protrusions and the slots are in a first configuration relative to one another, the blocking component restricts distal movement of the plunger rod to a first stopping point, and when the protrusions and the slots are in a second configuration, the blocking component restricts distal movement of the plunger rod to a second stopping point, wherein, in the second configuration, the slots are configured to receive the protrusions upon distal movement of the plunger rod. In an embodiment of the invention, the protrusions and the slots are movable from the first configuration to the second configuration by rotation of the actuation portion about a longitudinal axis in relation to the blocking component, and wherein when the protrusions and the slots are in the second configuration, the protrusions and the slots are not movable to the first configuration; and/or
a difference between the first stopping point and the second stopping point is equivalent to a distance that the stopper must travel to expel a predetermined volume of a drug product from a distal end of the body, and wherein the plunger rod is prevented from moving from the second stopping point to the first stopping point; and/or the plurality of protrusions includes two protrusions disposed symmetrically about the actuation portion; and/or the blocking component further comprises a pair of finger flanges; and/or the drug delivery device is a pre-filled syringe; and/or the drug delivery device is changeable: (a) from a pre-use state to a primed state, by longitudinally moving the plunger rod (*e.g*., wherein the plunger rod includes a neck disposed distally from the actuation portion, wherein the neck interfaces with an opening in the blocking component to prevent proximal movement of the plunger rod, for example, wherein the neck further interfaces with the opening in the blocking component to prevent movement of the drug delivery device from the delivery state to the primed state) until the plunger rod reaches the first stopping point; (b) from the primed state to a delivery state by rotating the plunger rod in relation to the blocking component until the protrusions and the blocking component are in the second configuration; and (c) from a delivery state to a used state by longitudinally moving the plunger rod until the plunger reaches the second stopping point, wherein the drug delivery device is not changeable from the used state to the delivery state, from the delivery state to the primed state, or from the primed state to the pre-use state. In an embodiment of the invention, when the plunger rod is at the second stopping point, the stopper does not contact a distal end of the body.

In an embodiment of the invention, a drug delivery device, includes:
- a body;
- a plunger rod, including:
- a distal portion contacting a stopper inside the body;
- a proximal end including a generally cylindrical actuation portion disposed outside of the body; and
- two protrusions extending from opposite sides of the actuation portion in a symmetrical configuration; and
- a blocking component coupled to the body, the blocking component including: a collar configured to accept a distal part of the actuation portion; and two cavities in the collar having proximally-facing openings, wherein each cavity is configured to accept a distal portion of one of the two protrusions;

wherein the plunger rod is longitudinally movable and rotatable about a longitudinal axis relative to the blocking component, and
wherein, when the drug delivery device is in a pre-use state, the protrusions and the cavity openings are not longitudinally aligned, and when the drug delivery device is in a delivery state, the protrusions and the cavity openings are longitudinally aligned. In an embodiment of the invention, the blocking component further includes a finger flange, and further includes a ribbed surface on a side of the actuation portion. In an embodiment of the invention, the plunger rod further includes: two extensions protruding distally from the actuation portion; and a plurality of openings in the collar of the blocking component, wherein a portion of each extension is configured to be received by one of the plurality of openings upon distal movement of the plunger rod relative to the blocking component.

In an embodiment of the invention, a drug delivery device includes:
- a body;
- a stopper disposed inside the body;
- a sleeve having a proximal end and a distal end, the distal end being disposed inside the body, proximally from the stopper; and
- a plunger rod disposed at least partially inside the sleeve;
wherein, when the stopper is in a ready position, distal advancement of one of (a) only the sleeve, (b) only the plunger rod, or (c) both the sleeve and the plunger rod together, relative to the body advances the stopper to a primed position, and wherein, when the stopper is in the primed position, distal advancement of another of (a) only the sleeve, (b) only the plunger rod, or (c) both the sleeve and the plunger rod together, relative to the body advances the stopper to a dose completion position. For example, in an embodiment of the invention, a DDD further includes a removable blocking component (*e.g*., wherein the blocking component is a clip removably secured around at least a portion of the sleeve) disposed between a proximal portion of the sleeve and a proximal end of the body, the blocking component obstructing distal advancement of the sleeve relative to the body, wherein distal advancement of the sleeve relative to the body after removal of the blocking component advances the stopper to the primed position. In an embodiment of the invention, the DDD further includes a removable locking component (*e.g*., a pin, a tab, or a bar) that couples the plunger rod to the sleeve, wherein distal advancement of both the sleeve and the plunger rod together relative to the body advances the stopper to the primed position, wherein distal advancement of only the plunger rod relative to the body after removal of the locking component advances the stopper to the dose completion position. In an embodiment of the invention, in the dose completion position, a proximal end of the plunger rod abuts against a distal end of the sleeve, such that the plunger rod is prevented from advancing distally any further relative to the body. In an embodiment of the invention, the DDD further includes a protrusion disposed on the plunger rod; and an inner protrusion disposed on an interior wall of the sleeve distally to the protrusion of the plunger rod, wherein distal advancement of only the plunger rod relative to the body advances the stopper to the primed position and causes the protrusion of the plunger rod to contact the inner protrusion of the sleeve, and wherein distal advancement of both the plunger rod and the sleeve relative to the body, after the protrusion of the plunger rod has contacted the inner protrusion of the sleeve, advances the stopper to the dose completion position. In an embodiment of the invention, the sleeve includes a finger flange. In an embodiment of the invention, the DDD further includes a stop disposed at a proximal end of the body, the stop sized to block distal advancement of the sleeve or the plunger rod once the stopper is in the completion position.

In an embodiment of the invention, a drug delivery device, includes:
- a body;
- a plunger rod having a distal portion disposed inside the body and a proximal portion disposed outside a proximal end of the body, the proximal portion having a width greater than a width of the distal portion; and
- an obstruction that, in an obstructing position relative to the plunger rod, prevents distal advancement of the plunger rod from a primed position to a dose completion position,
wherein displacement of the obstruction from the obstructing position permits distal advancement of the plunger rod to the dose completion position, for example, further including a collar affixed to a proximal end portion of the body, the collar surrounding the proximal portion of the plunger rod; and a collar projection extending radially inward from the collar, wherein the proximal portion of the plunger rod includes a channel into which the collar projection protrudes, the channel including a circumferential path and an axial dose completion path, wherein the obstruction comprises the collar projection, which, when disposed in the circumferential path of the channel, prevents distal advancement of the plunger rod to the dose completion position, and wherein displacement of the obstruction from the obstructing position comprises twisting the plunger rod about a longitudinal axis to align the collar projection with the axial dose completion path. For example, in an embodiment of the invention, the channel further includes an axial priming path offset from the axial dose completion path, and connected to the axial dose completion path by the circumferential path, and distal movement of the plunger rod such that the collar projection travels on the axial priming path advances the plunger rod to the primed position. In an embodiment of the invention, the DDD further includes a finger flange. In an embodiment of the invention, the proximal portion of the plunger rod includes a projection extending radially outward, and the drug delivery device further includes: a rotatable alignment component disposed in between the proximal portion of the plunger rod and the body, the alignment component including a channel, the channel sized and configured to accommodate the plunger rod projection, wherein the obstruction comprises a wall of the channel that blocks a distal axial path of the plunger rod projection when the plunger rod is in the primed position, and wherein displacement of the obstruction from the obstructing position comprises rotating the alignment component to remove the wall of the channel from the distal axial path of the plunger rod projection, *e.g.,* further including a finger flange coupled to a proximal end portion of the body, wherein the rotatable alignment component is disposed between the finger flange and the proximal portion of the plunger rod. In an embodiment of the invention, the DDD further includes a flange piece disposed at the proximal end of the body, wherein the obstruction includes a removable cap that, when in the obstructing position relative to the plunger rod, is disposed partially in between the proximal portion of the plunger rod and the flange piece. In an embodiment of the invention, removal of the cap allows the proximal portion of the plunger rod to advance to a dose completion position, wherein, in the dose completion position, the proximal portion of the plunger rod contacts the flange piece. In an embodiment of the invention, the removable cap covers the proximal portion of the plunger rod when in the obstructing position. In an embodiment of the invention, the DDD further includes a collar disposed between the proximal end of the body and the proximal portion of the plunger rod, the collar defining an opening sized to accommodate the proximal portion of the plunger rod upon distal advancement of the plunger rod beyond a primed position; wherein the obstruction comprises a tab protruding radially outward from the proximal portion of the plunger rod, the tab preventing the proximal portion of the plunger rod from fitting into the opening of the collar, and wherein a depth of the collar opening coincides with a distance the plunger rod must travel to advance distally to the dose completion position, *e.g.,* wherein displacement of the obstruction from the obstructing position comprises either removing the tab or compressing the tab into a side of the proximal portion of the plunger rod; and/or wherein the tab is a first tab, and wherein the obstruction further comprises a second tab protruding radially outward from the proximal portion of the plunger rod in a direction opposite the protruding direction of the first tab; and/or wherein the obstruction comprises a tab that, when in the obstructing position, is disposed between the body and the proximal portion of the plunger rod, and wherein the plunger rod includes a geometry disposed proximally from the tab, wherein the geometry cannot advance distally past the tab when the tab is in the obstructing position. For example, displacement of the obstruction may include removing the tab from the drug delivery device by pulling the tab. In an embodiment of the invention, the DDD further includes a flange piece, wherein a portion of the tab is disposed inside a cavity of the flange piece. In an embodiment of the invention, displacement of the obstruction comprises removing the tab from the drug delivery device by breaking the tab. In an embodiment of the invention, the obstruction includes a flange piece that, in the obstructing position, is disposed proximally from the proximal end of the body, between the proximal portion of the plunger rod and the body, and is spaced from the proximal end of the body by a removable blocking component, and wherein displacement of the obstruction from the obstructing position comprises: removing the blocking component; and shifting the flange piece distally towards the proximal end of the body. In an embodiment of the invention, the plunger rod includes a projection extending radially outward, wherein the obstruction includes a lever having an end that, in the obstructing position, is located distally from the projection and blocks distal movement of the projection and thereby distal movement of the plunger rod, and wherein displacement of the obstruction from the obstructing position comprises actuating the lever to remove the end of the lever from its location distal from the projection. In an embodiment of the invention, distal advancement of the plunger rod beyond the dose completion position is prevented by contact between the proximal portion of the plunger rod and a portion of a flange piece coupled to the body.

In an embodiment of the invention, the drug delivery device, includes:
- a body;
- a sleeve affixed to the body, the sleeve including a proximal end, a distal end, and an opening disposed in a circumferential wall of the sleeve;
- a plunger rod passing through the sleeve, the plunger rod including a distal end portion disposed inside the body, and a radially-extending protrusion;
wherein the plunger rod may be distally advanced into the body from a ready position to a primed position, wherein, in the primed position, the protrusion of the plunger rod is disposed inside the opening, and further distal advancement of the plunger rod is resisted by contact between the protrusion and a wall of the opening, and wherein pressure may be exerted on the protrusion to overcome the resistance to further distal advancement of the plunger rod. In an embodiment of the invention, the opening in the sleeve is a second opening, and the sleeve further includes a first opening disposed in the circumferential wall of the sleeve proximally from the second opening, and a third opening disposed in the circumferential wall of the sleeve distally from the second opening, wherein, in the ready position, the protrusion of the plunger rod is disposed in the first opening, and further distal advancement of the plunger rod is resisted by contact between the protrusion and a wall of the first opening, and wherein, after further distal advancement of the plunger rod past the primed position, the protrusion of the plunger rod is disposed in the third opening, and further distal advancement of the plunger rod is prevented. In an embodiment of the invention, the radially-extending protrusion is a first protrusion, and wherein the plunger rod further includes a second radially-extending protrusion opposite the first protrusion, and wherein squeezing the first and second protrusions towards one another while applying axial pressure in the distal direction on the plunger rod overcomes the resistance to further distal advancement of the plunger rod. In an embodiment of the invention, the protrusion includes a distally-tapering profile to aid in distal advancement of the plunger rod.

In an embodiment of the invention, a drug delivery device, includes:
- a body;
- a plunger rod including a distal end portion disposed inside the body and a rotatable element; and
- a sleeve affixed to the body, the sleeve including a proximal opening into which the plunger rod may be advanced,
wherein rotating the rotatable element causes distal advancement of the plunger rod to a primed position, and wherein once the plunger rod is in the primed position, further rotation of the rotatable element is resisted. In an embodiment of the invention, the DDD further includes a collar disposed at a proximal end of the body, an interior of the collar including a proximal threaded portion forming a proximal helical path, wherein the rotatable element comprises a proximal portion of the plunger rod including a protrusion, wherein the proximal portion of the plunger rod may be rotated about a longitudinal axis to cause the protrusion to travel distally along the proximal helical path, and wherein once the protrusion reaches the end of the proximal threaded portion of the collar, the plunger rod is in the primed position, *e.g*., wherein once the plunger rod is in the primed position, the plunger rod may be depressed axially into the body to distally advance the plunger rod to a dose completion position; and/or wherein the interior of the collar further includes a distal threaded portion, wherein threads of the distal threaded portion form a distal helical path offset from, and opposite to, the proximal helical path, wherein alignment of the protrusion with the distal helical path places the plunger rod in the primed position, and wherein rotation of the proximal portion of the plunger rod to cause the protrusion to travel distally along the distal helical path causes distal advancement of the plunger rod to a dose completion position.

A substance may be dispensed using such a DDD having a plunger rod and a body, may be done by a method including:
(a) advancing the plunger rod by a predetermined distance into the body until advancement of the plunger rod is resisted by a stop;
(b) rotating the plunger rod about a longitudinal axis; and
(c) actuating the plunger rod to dispense a predetermined volume of the substance,
wherein none of steps (a), (b), and (c) are reversible. In an embodiment of the invention, the DDD further includes a flange piece having a collar, and advancing the plunger rod and actuating the plunger rod comprise pressing an actuation portion of the plunger rod into the collar of the flange piece; for example, wherein the plunger rod comprises a protrusion, and wherein the collar of the flange piece abuts against the protrusion to resist advancement of the plunger rod. For example, in an embodiment of the invention, wherein rotating the plunger rod comprises twisting an actuation portion of the plunger rod relative to the flange piece, until a protrusion on the plunger rod becomes longitudinally aligned with a cavity in the collar of the flange piece, which may further include advancing the protrusion into the cavity until the protrusion abuts a distal side of the cavity, wherein the predetermined volume of the substance is dispensed when the protrusion abuts the distal side of the cavity.

See International patent application publication no. WO2020/247686.

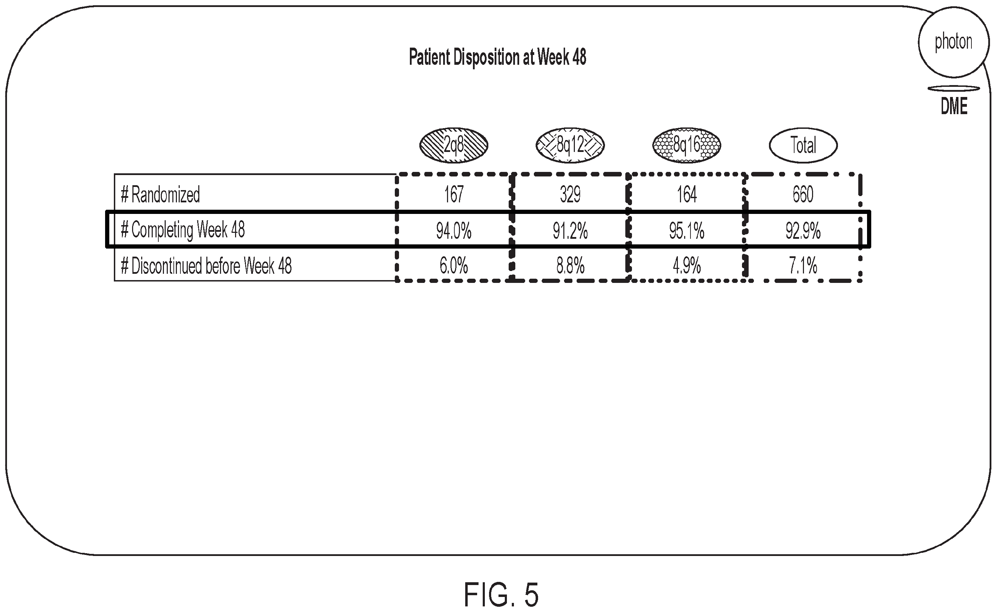

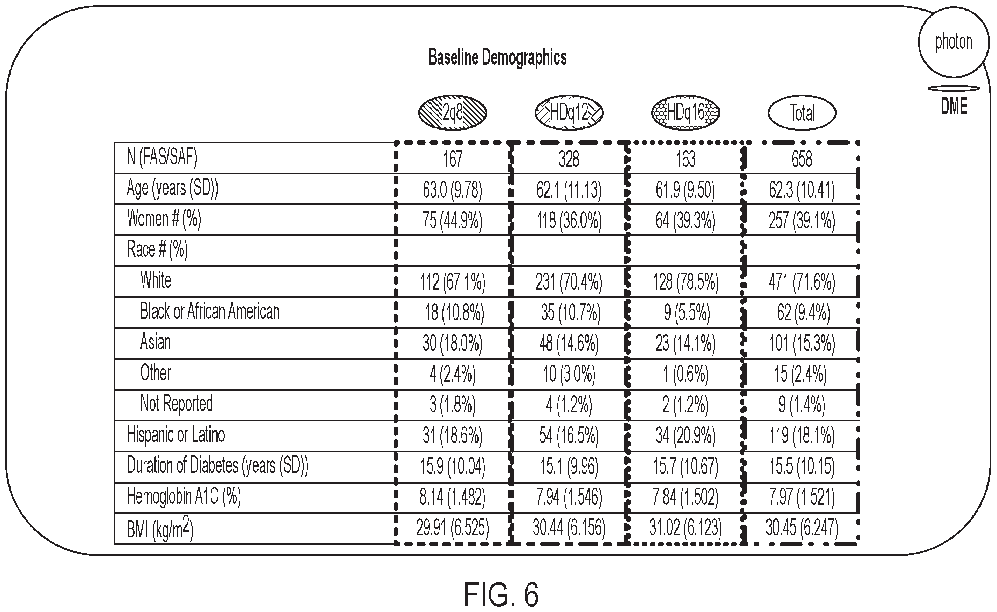

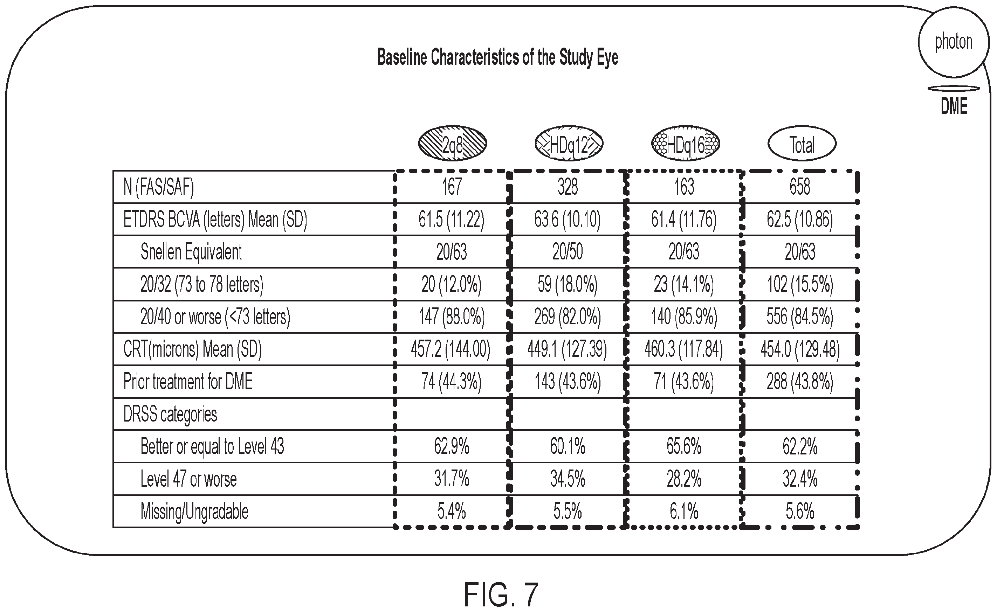

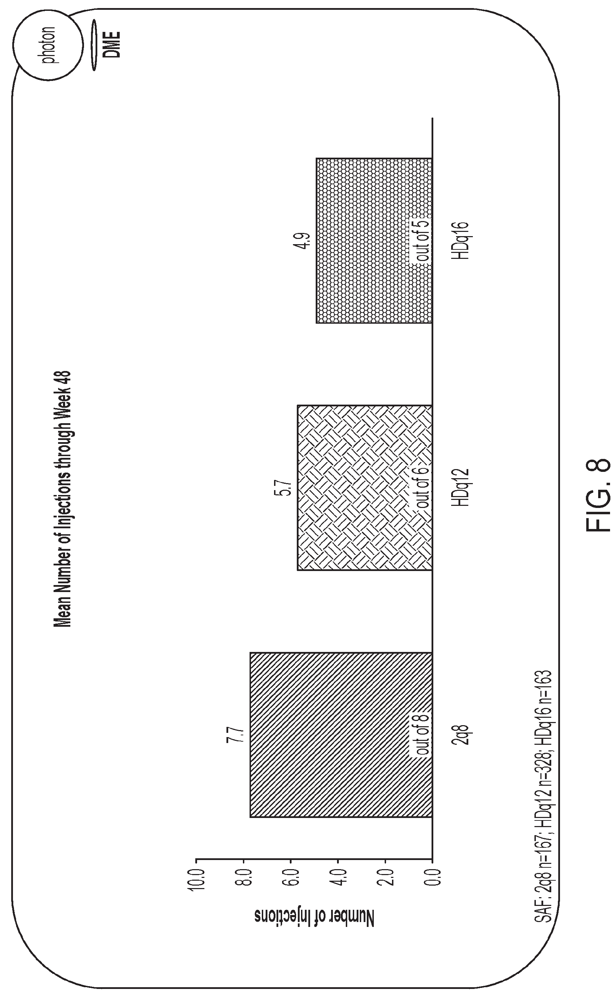

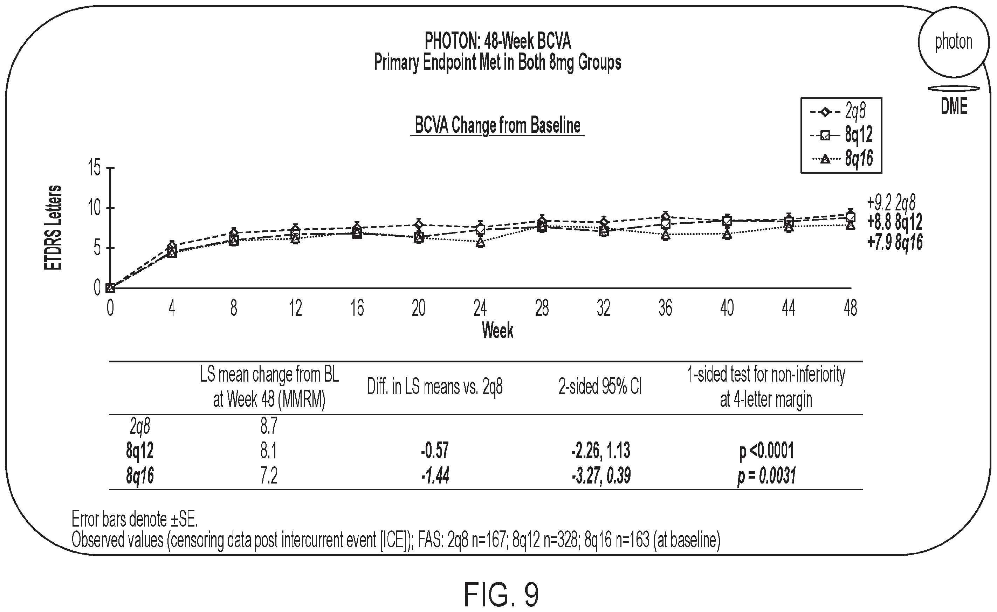

Data from the PHOTON trial through week 48 are provided. Patient disposition data in the PHOTON trial are set forth in Figure 5 and baseline demographics data are in Figures 6 and Figure 7. The mean patient exposure to injections per week is summarized in Figure 8. Efficacy data with respect to changes in visual acuity (BC VA; Figure 9); durability (Figure 10); changes to DRSS (Figure 11); retinal fluid (Figure 12); and changes to central retinal thickness (CRT) (Figure 13) are also provided. In addition, safety data are set forth in Figure 14-Figure 24.

### EXAMPLES

The present invention includes methods for achieving any of the individual results or PK points, for example, by the period of time after treatment initiation that is indicated (*e.g*., improvement in BCVA by X ETDRS letters by Y days after treatment initiation) as is set forth in the Examples section in a subject having DR and/or DME by administering an HDq12-20, HDq12, HDq16 or HDq20 treatment regimen to the subject.

### Example 1: A Randomized, Double-Masked, Active-Controlled Phase 2/3 Study of the Efficacy and Safety of High-Dose Aflibercept in Patients with Diabetic Macular Edema (PHOTON).

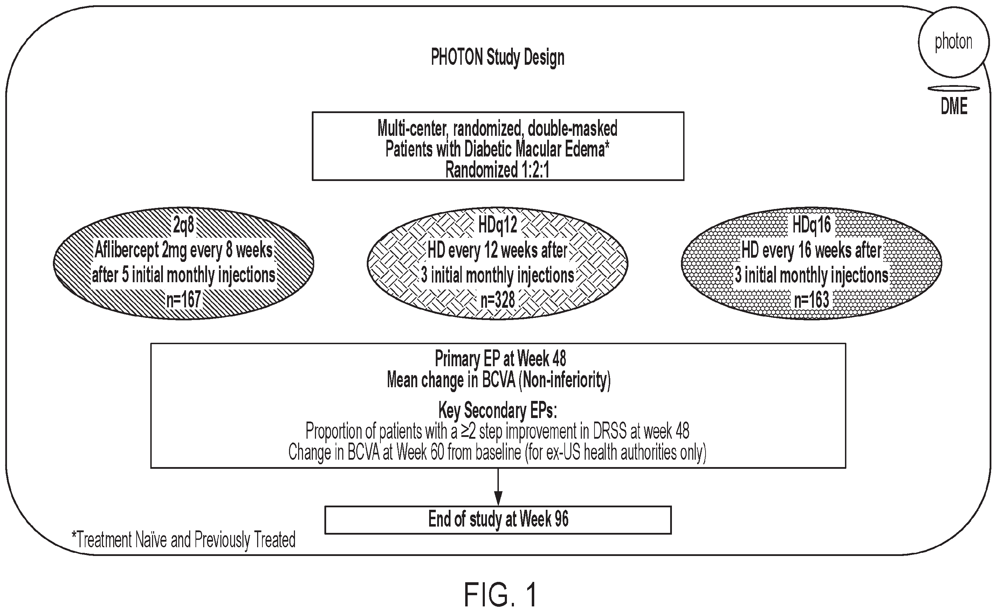

This is a phase 2/3, multi-center, randomized, double-masked study in patients with DME involving the center of the macula to investigate the efficacy and safety of HD versus 2 mg aflibercept. Approximately 640 eligible patients randomized into 3 treatment groups in a 1:2:1 ratio to the following 3 treatment groups:
1) **2q8:** 2 mg aflibercept every 8 weeks, following 5 initial monthly doses (n=160),
2) **HDq12:** HD aflibercept (8 mg) every 12 weeks, following 3 initial monthly doses (n=320),
3) **HDq16:** HD aflibercept (8 mg) every 16 weeks following 3 initial monthly doses (n=160). (see Figure 1)

Approximately 24 patients will be included in a dense PK sub-study (n=8 per group, with half Japanese and half non-Japanese per group). In all patients, blood samples for measurement of drug concentrations (PK) and anti-drug antibody (ADA) will be obtained prior to the first treatment and at prespecified time points throughout the course of the study.

### Dosing Schedule

The dosing schedule is set forth in Figure 3.

### Primary Endpoints

The primary endpoint is the change from baseline in BCVA at week 48.

### Secondary Endpoints

The key secondary efficacy endpoints are:
- Proportion of patients without retinal fluid at the foveal center at week 12
- Proportion of patients with a ≥2 step improvement in Diabetic Retinopathy Severity Scale (DRSS) at week 48
- Change from baseline in BCVA at week 60

The additional secondary efficacy endpoints are:
- Proportion of patients gaining ≥15 letters at week 48
- Proportion of patients with BCVA ≥69 letters at week 48
- Proportion of patients without fluid at foveal center at week 48
- Change from baseline in central retinal thickness (CRT) at week 48
- Proportion of patients without leakage on fluorescein angiography (FA) at week 48
- Change from baseline in National Eye Institute Visual Function Questionnaire (NEI-VFQ) total score at week 48
- Systemic pharmacokinetics of aflibercept as assessed from baseline through week 48
- Assessment of immunogenicity to aflibercept through end of study (EOS) week (week 96).

The secondary safety endpoint is:
- Safety evaluation by assessment of AEs and serious adverse events (SAEs) through weeks 48, 60, and 96

### Exploratory Endpoints

The exploratory endpoints are:
- Proportion of patients without retinal fluid (total fluid, intraretinal fluid [IRF] and/or subretinal fluid [SRF]) at the foveal center and in center subfield at week 48 and week 96
- Time to fluid-free retina over 48 weeks and 96 weeks (total fluid, IRF and/or SRF at foveal center and in the center subfield)
- Proportion of patients with sustained fluid-free retina over 48 weeks and 96 weeks (total fluid, IRF and/or SRF at foveal center and in the center subfield)
- Proportion of patients without CSME (clinically significant macular edema) at week 48 and week 96
- Fluid on spectral domain optical coherence tomography (SD-OCT)
- Proportion of patients with a ≥3 step improvement in DRSS at week 48 and week 96
- Proportions of patients gaining and losing ≥5 or ≥10 letters at week 48 and week 96
- Proportion of patients losing ≥15 letters at week 48 and week 96
- Proportion of patients randomized to HDq16 maintaining q16 dosing interval or longer through weeks 48, 60, and 96
- Proportion of patients randomized to HDq12 maintaining q12 dosing interval or longer through weeks 48, 60 and 96
- Proportion of patients with an assigned injection interval of ≥16 or ≥20 weeks based on assessment at the last injection visit
In addition to those specified above, all primary, secondary, and exploratory endpoints may be analyzed in an exploratory manner at weeks 60 and 96.

### Efficacy Variables

The efficacy variable relevant to the primary efficacy endpoint is visual acuity.

The efficacy variables relevant to the secondary endpoints are:
- BCVA
- Assessment of retinal fluid levels and retinal thickness on spectral domain optical coherence tomography (SD-OCT)
- Dosing interval
- Quality of life using the NEI VFQ-25
- Diabetic retinopathy severity level using the DRSS

The efficacy variables relevant to the exploratory endpoints are:
- Assessment of retinal fluid levels and retinal thickness on SD-OCT
- BCVA
- Dosing interval

### Safety Variables

Safety will be evaluated by assessment of AEs and SAEs, ocular exams, IOP, vital signs (including BP, heart rate, and temperature), and clinical laboratory values.

### Pharmacokinetic Variables

The PK variables are the concentrations of free, bound, adjusted bound, and total aflibercept in plasma at each time point.

### Immunogenicity Variables

The immunogenicity variables are anti-drug antibody (ADA) status, titer, and neutralizing antibody (NAb) status at each study visit time point.

### Number of Patients Planned

The study will enroll approximately 640 patients to be randomized 1:2:1 (160 patients each in the 2q8 and the HDq16 groups, and 320 patients in the HDq12 group).

### Study Population

The study population will comprise patients with DME with central involvement.

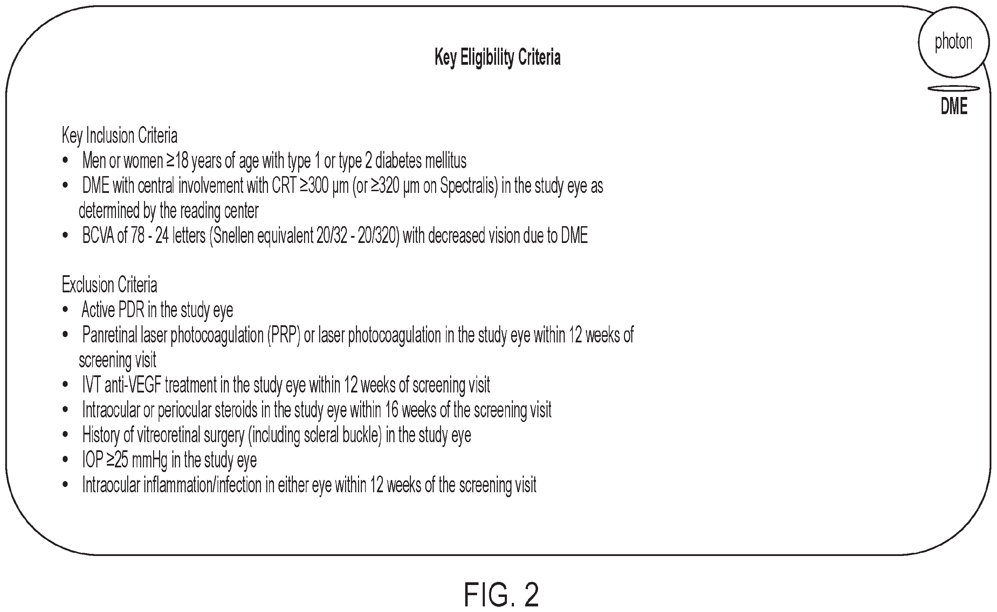

### Inclusion Criteria (See Figure 2)

A patient must meet the following criteria at both the screening and randomization visits (except where indicated) to be eligible for inclusion in the study:
1. Men or women ≥18 years of age (or country's legal age of adulthood if the legal age is >18 years) with type 1 or type 2 diabetes mellitus
2. DME with central involvement in the study eye with CRT ≥300 *µ* m (or ≥320 µm on Spectralis) as determined by the reading center at the screening visit
3. BCVA early treatment diabetic retinopathy study (ETDRS) letter score of 78 to 24 (approximate Snellen equivalent of 20/32 to 20/320) in the study eye with decreased vision determined to be primarily the result of DME
4. Willing and able to comply with clinic visits and study-related procedures
5. Provide informed consent signed by study patient or legally acceptable representative

### Exclusion Criteria (See Figure 2)

Patients who meets any of the following criteria at either the screening or randomization visits will be excluded from the study:
1. Evidence of macular edema due to any cause other than diabetes mellitus in either eye
2. Active proliferative diabetic retinopathy in the study eye
3. Panretinal laser photocoagulation (PRP) or macular laser photocoagulation in the study eye within 12 weeks (84 days) of the screening visit
4. IVT anti-VEGF treatment (aflibercept, ranibizumab, bevacizumab, brolucizumab, pegaptanib sodium) in the study eye within 12 weeks (84 days) of the screening visit
5. Prior IVT investigational agents in either eye (e.g., anti-ang-2/anti-VEGF bispecific monoclonal antibodies, gene therapy, etc.) at any time
6. Treatment with ocriplasmin (JETREA^{®}) in the study eye at any time
7. Previous use of intraocular or periocular corticosteroids in the study eye within 16 weeks (112 days) of the screening visit, or ILUVIEN^{®} or OZURDEX^{®} IVT implants at any time
8. History of vitreoretinal surgery (including scleral buckle) in the study eye
9. Any other intraocular surgery within 12 weeks (84 days) before the screening visit
10. Yttrium-aluminum-garnet (YAG) laser capsulotomy in the study eye within 4 weeks (28 days) of the screening visit
11. IOP ≥25 mmHg in the study eye
12. History of glaucoma filtration surgery in the past, or likely to need filtration surgery in the future in the study eye
13. Evidence of infectious blepharitis, keratitis, scleritis, or conjunctivitis in either eye within 4 weeks (28 days) of the screening visit
14. Any intraocular inflammation/infection in either eye within 12 weeks (84 days) of the screening visit
15. History of idiopathic or autoimmune uveitis in the study eye
16. Vitreomacular traction or epiretinal membrane in the study eye evident on biomicroscopy or OCT that is thought to affect central vision
17. Preretinal fibrosis involving the macula in the study eye
18. Any history of macular hole of stage 2 and above in the study eye
19. Current iris neovascularization, vitreous hemorrhage, or tractional retinal detachment visible at the screening assessments in the study eye
20. History of corneal transplant or corneal dystrophy in study eye
21. Any concurrent ocular condition in the study eye which, in the opinion of the investigator, could either increase the risk to the patient beyond what is to be expected from standard procedures of IVT injections, or which otherwise may interfere with the injection procedure or with evaluation of efficacy or safety
22. Only 1 functional eye, even if that eye was otherwise eligible for the study (e.g., BCVA of counting fingers or less in the eye with worse vision)
23. Structural damage to the center of the macula in the study eye that is likely to preclude improvement in BCVA following the resolution of macular edema including atrophy of the retinal pigment epithelium, subretinal fibrosis or scar, significant macular ischemia, or organized hard exudates
24. Ocular conditions with poorer prognosis in the fellow eye
25. Inability to obtain photographs, FA, or SD-OCT in the study eye, e.g., due to media opacity, allergy to fluorescein dye, or lack of venous access
26. History of other disease, metabolic dysfunction, physical examination finding, or clinical laboratory finding giving reasonable suspicion of a disease or condition that contraindicates the use of an investigational drug or that might affect interpretation of the results of the study or render the patient at high risk for treatment complications
27. Any prior systemic (IV) anti-VEGF administration
28. Uncontrolled diabetes mellitus as defined by hemoglobin A1c (HbA1c) >12%
29. Uncontrolled blood pressure (defined as systolic >160 mmHg or diastolic >95 mmHg). Patients may be treated with up to 3 agents known to have anti-hypertensive effects for arterial hypertension to achieve adequate blood pressure control. This limit applies to drugs that could be used to treat hypertension even if their primary indication in the patient was not for blood pressure control. Any recent changes in medications known to affect blood must be stable for 12 weeks (84 days) prior to screening.
30. History of cerebrovascular accident or myocardial infarction within 24 weeks (168 days) of screening visit
31. Renal failure, dialysis, or history of renal transplant
32. Known sensitivity to any of the compounds of the study formulation
33. Participation in an investigational study within 30 days prior to screening visit that involved treatment with any drug (excluding vitamins and minerals) or device
34. Members of the clinical site study team and/or his/her immediate family, unless prior approval granted by the sponsor
35. Pregnant or breastfeeding women
36. Men or women of childbearing potential (WOCBP)* who are unwilling to practice highly effective contraception prior to the initial dose/start of the first treatment, during the study, and for at least 3 months after the last dose. Highly effective contraceptive measures include:
   a. stable use of combined (estrogen and progestogen-containing) hormonal contraception (oral, intravaginal, transdermal) or progestogen-only hormonal contraception (oral, injectable, implantable) associated with inhibition of ovulation initiated 2 or more menstrual cycles prior to screening
   b. intrauterine device (IUD); intrauterine hormone-releasing system (IUS)
   c. bilateral tubal ligation
   d. vasectomy**
   e. condom plus contraceptive sponge, foam, or jelly, or diaphragm plus contraceptive sponge, foam, or jelly
   f. and/or sexual abstinence†, ‡.

   *Postmenopausal women must be amenorrhoeic for at least 12 months without an alternative medical cause in order not to be considered of childbearing potential. Pregnancy testing and contraception are not required for women with documented hysterectomy or tubal ligation.
   **Vasectomized partner or vasectomized study participant must have received medical assessment of the surgical success.
   †Sexual abstinence is considered a highly effective method only if defined as refraining from heterosexual intercourse during the entire period of risk associated with the study treatments. The reliability of sexual abstinence needs to be evaluated in relation to the duration of the clinical trial and the preferred and usual lifestyle of the subject.
   ‡Periodic abstinence (calendar, symptothermal, post-ovulation methods), withdrawal (coitus interruptus), spermicides only, and lactational amenorrhea method (LAM) are not acceptable methods of contraception. Female condom and male condom should not be used together.

Additional Exclusion Criteria for the PK sub-study:
1. Prior treatment with IVT aflibercept in the fellow eye within 12 weeks (84 days) of the screening visit
2. Other IVT anti-VEGF treatment (ranibizumab, bevacizumab, brolucizumab, pegaptanib sodium) in the fellow eye within 4 weeks (28 days) of the screening visit
3. Patients with SBP >140 mmHg or diastolic blood pressure (DBP) >90 mmHg
4. Patients with known cardiac arrhythmia
5. Patients who, in the opinion of the investigator, are unlikely to have stable BP over the course of the study (e.g., due to known or suspected non-compliance with medication)
6. Variation by more than 10% in the 3 pre-randomization BP measures recorded at the screening visits and at randomization

### Investigational and Reference Treatments

The HD drug product will be supplied for this study as an aqueous solution in sterile, sealed, single-use vials for IVT administration at a concentration of 114.3 mg/mL aflibercept which will be delivered in an injection volume of 70 µl (0.07 mL). Intravitreal aflibercept injection 2 mg will be supplied for this study as an aqueous solution in sterile, sealed, single-use vials for IVT administration at a concentration of 40 mg/mL delivered in an injection volume of 50 µL (0.05 mL).

### Study Assessments and Procedures

Study procedures and their timing are summarized in the following tables.

**Table 1-1. Baseline to Week 48**

| **Study Procedure** | **Screening Visit 1** | **Baseline Visit 2** | **Visit 3** | **Visit 4** | **Optional Visit 4.1'** | **Visit 5** | **Visit 6** | **Visit 7** | **Visit 8** | **Visit 9** | **Visit 10** | **Visit 11** | **Visit 12** | **Visit 13** | **Visit 14** |
|---|---|---|---|---|---|---|---|---|---|---|---|---|---|---|---|
| **Week** | | 0 | 4 | 8 | | 12 | 16 | 20 | 24 | 28 | 32 | 36 | 40 | 44 | 48 |
| Day | -21 to -1 | 1 | 29 | 57 | 60-64 | 85 | 113 | 141 | 169 | 197 | 225 | 253 | 281 | 309 | 337 |
| Window (day) | | | ±5 | ±5 | | ±5 | ±5 | ±5 | ±5 | ±5 | ±5 | ±5 | ±5 | ±5 | ±5 |

| **Screening/Baseline:** | | | | | | | | | | | | | | | |
|---|---|---|---|---|---|---|---|---|---|---|---|---|---|---|---|
| Informed consent form(ICF) | X | | | | | | | | | | | | | | |
| Dense PK substudy ICF² | X | | | | | | | | | | | | | | |
| Genomic substudy ICF³ | X | | | | | | | | | | | | | | |
| Future Biomedical Research ICF⁴ | X | | | | | | | | | | | | | | |
| Inclusion/Exclusion | X | X | | | | | | | | | | | | | |
| Medical history | X | | | | | | | | | | | | | | |
| Demographics | X | | | | | | | | | | | | | | |
| Concomitant Medications | X | X | X | X | X | x | X | x | X | X | X | X | x | X | x |
| Randomization | | X | | | | | | | | | | | | | |

| **Administer Study Drug⁵** | | | | | | | | | | | | | | | |
|---|---|---|---|---|---|---|---|---|---|---|---|---|---|---|---|
| Study drug (active or sham) | | X | X | X | | X | X | X | X | X | X | X | X | X | X |
| DRM assessment | | | | | | | X⁶ | X⁶ | X⁶ | X⁶ | X⁶ | X⁶ | X⁶ | X⁶ | X⁶ |

| **Ocular Efficacy and Safety (bilateral unless indicated):** | | | | | | | | | | | | | | | |
|---|---|---|---|---|---|---|---|---|---|---|---|---|---|---|---|
| BCVA (ETDRS) and Refraction⁷ | X | X | X | X | | X | X | X | X | X | X | X | X | X | X |
| IOP⁸ | X | X | X | X | | X | X | X | X | X | X | X | X | X | X |
| Slit lamp examination | X | X | X | X | | X | X | X | X | X | X | X | X | X | X |
| Indirect ophthalmoscopy⁹ | X | X | X | X | | X | X | X | X | X | X | X | X | X | X |
| FA, FP¹⁰ | X | | | | | X | | | X | | | X | | | X |
| SD-OCT¹⁰ | X | X | X | X | | X | X | X | X | X | X | X | X | X | X |

**Table 1-2. Baseline to Week 48 (continued)**

| **Study Procedure** | **Screen ing Visit 1** | **Baseline Visit 2** | **Visit 3** | **Visit 4** | **Optional Visit 4.1¹** | **Visit 5** | **Visit 6** | **Visit 7** | **Visit 8** | **Visit 9** | **Visit 10** | **Visit 11** | **Visit 12** | **Visit 13** | **Visit 14** |
|---|---|---|---|---|---|---|---|---|---|---|---|---|---|---|---|
| Week | | 0 | 4 | 8 | | 12 | 16 | 20 | 24 | 28 | 32 | 36 | 40 | 44 | 48 |
| Day | -21 to -1 | 1 | 29 | 57 | 60-64 | 85 | 113 | 141 | 169 | 197 | 225 | 253 | 281 | 309 | 337 |
| Window (day) | | | ±5 | ±5 | | ±5 | ±5 | ±5 | ±5 | ±5 | ±5 | ±5 | ±5 | ±5 | ±5 |
| OCTA substudies¹¹ | X | | | | | X | | | X | | | X | | | X |
| NEI-VFQ-25 | X | | | | | | | | X | | | | | | X |

| **Nonocular Safety:** | | | | | | | | | | | | | | | |
|---|---|---|---|---|---|---|---|---|---|---|---|---|---|---|---|
| Physical examination | X | | | | | | | | | | | | | | |
| Vital signs¹² | X | X | X | X | X¹ | X | X | X | X | X | X | X | X | X | X |
| ECG | X | | | | | | | | | | | | | | X |
| Adverse events | X | X | X | X | X | X | X | X | X | X | X | X | X | X | X |

| **Laboratory Testing¹³** | | | | | | | | | | | | | | | |
|---|---|---|---|---|---|---|---|---|---|---|---|---|---|---|---|
| Hematology | X | | | | | | | | | | | | | | X |
| Blood chemistry | X | | | | | | | | | | | | | | X |
| HbAlc | X | | | | | | | | | | | | | | X |
| Pregnancy test (women of childbearing potential)¹⁴ | X Serum | X Urine | X Urine | X Urine | | X Urine | X Urine | X Urine | X Urine | X Urine | X Urine | X Urine | X Urine | X Urine | X Urine |
| Urinalysis/UPCR | X | | | | | | | | | | | | | | X |

| **Pharmacokinetics and Other Sampling** | | | | | | | | | | | | | | | |
|---|---|---|---|---|---|---|---|---|---|---|---|---|---|---|---|
| PK samples (Dense)^{15,17} | | See schedule below | X | | X¹ | X | | | | X | | | | | X |
| PK samples (Sparse)^{16,17} | | X | X | | X¹ | X | | | | X | | | | | X |
| Genomic DNA sample³ | | X | | | | | | | | | | | | | |
| Immunogenicity samples^{16, 17} | | X | | | | | | | | | | | | | X |

**Table 1-3. Week 52 to Week 96**

| **Study Procedure** | **Visit 15** | **Visit 16** | **Visit 17** | **Visit 18** | **Visit 19** | **Visit 20** | **Visit 21** | **Visit 22** | **Visit 23** | **Visit 24** | **Visit 25** | **EOS Visit¹⁸ 26** |
|---|---|---|---|---|---|---|---|---|---|---|---|---|
| Week | 52 | 56 | 60 | 64 | 68 | 72 | 76 | 80 | 84 | 88 | 92 | 96 |
| Day | 365 | 393 | 421 | 449 | 477 | 505 | 533 | 561 | 589 | 617 | 645 | 673 |
| Window (day) | ±5 | ±5 | ±5 | ±5 | ±5 | ±5 | ±5 | ±5 | ±5 | ±5 | ±5 | ±5 |

| Screening/Baseline: | | | | | | | | | | | | |
|---|---|---|---|---|---|---|---|---|---|---|---|---|
| Concomitant medications | X | X | X | X | X | X | X | X | X | X | X | X |

| **Administer Study Drug⁵** | | | | | | | | | | | | |
|---|---|---|---|---|---|---|---|---|---|---|---|---|
| Study Drug (active or sham) | X | X | X | X | X | X | X | X | X | X | X | |
| DRM assessment | X⁶ | X⁶ | X⁶ | X⁶ | X⁶ | X⁶ | X⁶ | X⁶ | X⁶ | X⁶ | X⁶ | |

| **Ocular Efficacy and Safety (bilateral unless indicated):** | | | | | | | | | | | | |
|---|---|---|---|---|---|---|---|---|---|---|---|---|
| BCVA (ETDRS) and refraction⁷ | X | X | X | X | X | X | X | X | X | X | X | X |
| IOP⁸ | X | X | X | X | X | X | X | X | X | X | X | X |
| Slit lamp examination | X | X | X | X | X | X | X | X | X | X | X | X |
| Indirect ophthalmoscopy⁹ | X | X | X | X | X | X | X | X | X | X | X | X |
| FA, FP¹⁰ | | | X | | | X | | | X | | | X |
| SD-OCT¹⁰ | X | X | X | X | X | X | X | X | X | X | X | X |
| OCTA Substudies¹¹ | | | X | | | X | | | X | | | X |
| NEI VFQ-25 | | | X | | | | | | | | | X |

| **Nonocular Safety:** | | | | | | | | | | | | |
|---|---|---|---|---|---|---|---|---|---|---|---|---|
| Physical examination | | | | | | | | | | | | |
| Vital signs¹² | X | X | X | X | X | X | X | X | X | X | X | X |
| ECG | | | | | | | | | | | | X |
| Adverse events | X | X | X | X | X | X | X | X | X | X | X | X |

| **Laboratory Testing¹³** | | | | | | | | | | | | |
|---|---|---|---|---|---|---|---|---|---|---|---|---|
| Hematology | | | | | | | | | | | | X |
| Blood chemistry | | | | | | | | | | | | X |
| HbAlc | | | | | | | | | | | | X |

**Table 1-4. Study Procedure**

| **Study Procedure** | **Visit 15** | **Visit 16** | **Visit 17** | **Visit 18** | **Visit 19** | **Visit 20** | **Visit 21** | **Visit 22** | **Visit 23** | **Visit 24** | **Visit 25** | **EOS Visit¹⁸ 26** |
|---|---|---|---|---|---|---|---|---|---|---|---|---|
| Week | 52 | 56 | 60 | 64 | 68 | 72 | 76 | 80 | 84 | 88 | 92 | 96 |
| Day | 365 | 393 | 421 | 449 | 477 | 505 | 533 | 561 | 589 | 617 | 645 | 673 |
| Pregnancy test (women of childbearing potential)¹⁴ | X Urine | X Urine | X Urine | X Urine | X Urine | X Urine | X Urine | X Urine | X Urine | X Urine | X Urine | |
| Urinalysis/UPCR | | | | | | | | | | | | X |

| **Pharmacokinetics and Other Sampling** | | | | | | | | | | | | |
|---|---|---|---|---|---|---|---|---|---|---|---|---|
| PK samples(Dense)^{15,17} | | | | | | | | | | | | |
| PK samples(Sparse)^{16,17} | | | | | | | | | | | | |
| Genomic DNA sample³ | | | | | | | | | | | | |
| Immunogenicity sample^{16, 17} | | | | | | | | | | | | X |

| | | | | | | | | | | | | |
|---|---|---|---|---|---|---|---|---|---|---|---|---|
| BCVA=Best corrected visual acuity, DRM = Dose regimen modification, ECG=electrocardiogram, ETDRS=Early Treatment Diabetic Retinopathy Study, FA=fluorescein angiography, FP=fundus photography, IOP=Intraocular pressure, OCTA = optical coherence tomography angiography, PK=pharmacokinetics, SD-OCT=spectral domain optical coherence tomography, UPCR=urine protein:creatinine ratio, UWFA = ultra-widefield fluorescein angiography. | | | | | | | | | | | | |

Footnotes for Tables 1-1 to 1-3:
1. An optional visit for all patients on days 60 to 64 (after the third injection) to collect a PK sample and assess heart rate and BP (no temperature measures required) as well as concomitant medications and AEs
2. Signed only by patients participating in the dense PK sub-study and in addition to the study ICF
3. The optional genomic sub-study ICF should be presented to patients at the screening visit and may be signed at any subsequent visit at which the patient chooses to participate after screening. The genomic DNA sample should be collected on day 1/baseline (pre-dose) or at any study visit from patients who have signed the sub-study ICF.
4. The optional future biomedical research sub-study ICF should be presented to patients and signed at the screening visit.
5. Refer to pharmacy manual for study drug injection guidelines. Following study drug injection, patients will be observed for approximately 30 minutes.
6. Assessments for DRM criteria will occur in all patients at all visits for masking purposes beginning at week 16.
7. Patients enrolled at sites participating in the optional visual function sub-study may undergo additional visual function tests. See study procedure manual for details.
8. Intraocular pressure will be measured at all study visits (bilateral). On days when study drug is administered, IOP should be measured pre-dose (bilaterally) by the masked investigator (or designee) and approximately 30 minutes after administration of study drug (study eye only) by the unmasked investigator. IOP will be measured using Goldman applanation tonometry or Tono-pen^{™} and the same method of measurement must be used in each patient throughout the study.
9. Indirect ophthalmoscopy will be performed bilaterally at all visits by the masked investigator. On days when study drug is administered, it should also be performed immediately after administration of study drug (study eye only).
10. The same SD-OCT/FA/FP imaging system used at screening and day 1 must be used at all follow-up visits in each patient. Images will be taken in both eyes before dosing at each required visit. For FA, the study eye will be the transit eye and images should be collected using the widest field available. If available, sites should also submit an optional ultra-widefield color photograph.
11. Details on an optional sub-study evaluating OCTA are provided in study procedure manual. Images will be collected at the same timepoints as FA/FP.
12. Vital signs (BP, heart rate, temperature) should be measured prior to injection and any blood sampling. When possible, timing of all BP assessments should be within 2 hours of clock time of dosing on day 1.
13. All samples collected for laboratory assessments should be obtained prior to administration of fluorescein and prior to administration of study drug.
14. For women of childbearing potential, a negative serum pregnancy test at screening is required for eligibility. A negative urine pregnancy test is required before treatment is administered at subsequent visits.
15. Dense PK sampling will be performed in approximately 24 patients (n=8/group) as indicated in Table 1-2. Additional samples will be drawn according to the dense PK sub-study schedule defined in Table 1-3. On dosing visits, PK sampling should be performed prior to the administration of study drug and within 2 hours of the clock time of dosing on day 1.
16. On dosing visits, PK and ADA sampling will be performed prior to dosing.
17. PK and ADA samples may also be drawn at any non-specified scheduled visit or any unscheduled visit if a patient experiences an unexpected SAE.
18. The EOS will also represent the early termination visit.

Schedule of events for the Dense PK sub-study are presented in Table 1-5, below.

**Table 1-5. Schedule of Events for Dense PK sub-study**

| **Visit** | **Dose** | **Assessment Day** | **Assessment Time (h)** | **PK Sample** | **Heart Rate and Blood Pressure³** |
|---|---|---|---|---|---|
| Screening 2¹ | | -20 to -1 | ±2h | | X² |
| Visit 2 | | 1 | Predose³ | X | X² |
| | X | | 4h ±30min | X | |
| | | | 8h ±2h | X | |
| | | 2 | ±2h³ | X | X² |
| | | 3 | ±2h³ | X | X² |
| | | 5 | ±2h³ | X | X² |
| | | 8 | ±2h³ | X | X² |
| | | 15 | ±2h³ | X | X² |
| | | 22 | ±2h³ | X | X² |

Footnotes for the Dense PK sub-study
1. Additional BP assessment to confirm eligibility for patients in the dense PK sub-study between screening and baseline
2. Timing of all BP assessments must be within 2 hours of the clock time of dosing on day 1. Blood pressure assessments for patients in the dense PK sub-study will be obtained prior to blood sample collection, using automated office blood pressure (AOBP) measurement with the Omron Model HEM 907XL (or comparable). Measures displayed by the device will be recorded in the electronic data capture (EDC). Detailed instructions can be found in the study procedure manual.
3. PK sampling is to be performed within ±2 hours of the clock time of dosing on day 1.

### Ocular Procedures

The ocular study procedures (efficacy and safety) include the following.

*Intraocular Pressure* - Intraocular pressure of the study eye will be measured in both eyes at every visit using Goldmann applanation tonometry or Tono-pen^{®}. The same method of IOP measurement must be used throughout the study for each individual patient. Intraocular pressure will be measured pre-dose (bilateral) by the masked physician (or designee), and at approximately 30 minutes post-dose (study eye) by the unmasked physician (or designee). *Slit Lamp Examination -* Patients' anterior eye structure and ocular adnexa will be examined bilaterally pre-dose at each study visit using a slit lamp by the masked investigator.

*Indirect Ophthalmoscopy -* Patients' posterior pole and peripheral retina will be examined by indirect ophthalmoscopy at each study visit pre-dose (bilateral) by the masked investigator and post-dose (study eye) by the unmasked investigator, as specified in Table 2. Post-dose evaluation must be performed immediately after injection.

*Fundus Photography*/*Fluorescein Angiography -* The anatomical state of the retinal vasculature, including the DRSS level, leakage, and perfusion status will be evaluated by FP and FA. Fundus photography and FA will be captured and transmitted to an independent reading center for both eyes. For FA, the study eye will be the transit eye and images should be collected using the widest field available. If available, sites should also submit an optional ultra-widefield color photograph. Fundus and angiographic images will be sent to an independent reading center where images will be read by masked readers.

*Spectral Domain Optical Coherence Tomography -* Retinal characteristics will be evaluated at each study visit specified in Table 2 using SD-OCT. Images will be captured and transmitted for both eyes. Images will be sent to an independent reading center where they will be read by masked readers.

*Best Corrected Visual Acuity -* Visual function of the study eye and the fellow eye will be assessed using the ETDRS protocol (Early Treatment Diabetic Retinopathy Study Research Group, 1985) at 4 meters at each study visit. Best corrected visual acuity should be assessed before any other ocular procedures are performed.

*Quality of Life Questionnaire* - Vision-related quality of life (QoL) will be assessed using the NEI VFQ-25 in the interviewer-administered format at visits.

### Dosing Regimen Modifications/Rescue Regimen

For masking purposes, assessments for dose regimen modifications (DRMs) will be performed in all participants at all visits (through the IWRS) beginning at week 16. Based on these assessments, patients in the HD groups may have their treatment intervals shortened (year 1 and year 2) or extended (year 2). The minimum interval between injections will be 8 weeks which is considered a rescue regimen for patients randomized to HD aflibercept and unable to tolerate a dosing interval greater than every 8 weeks. Patients in the aflibercept 2 mg group will remain on fixed q8 dosing throughout the study (ie, will not have modifications of their treatment intervals regardless of the outcomes of the DRM assessments).

### Year 1: Baseline to Week 52

Beginning at week 16, patients in the HD groups will have the dosing interval shortened (at the visits described below) if BOTH of the following criteria are met:
1. >10 letter loss in BCVA from week 12 in association with persistent or worsening DME; AND
2. >50 µm increase in CRT from week 12 (It should be noted that the change in CRT for these criteria will be assessed at the site.)

If a patient in the HDq12 group or the HDq16 group meets both criteria at week 16 or week 20, the patient will be dosed with 8 mg aflibercept at that visit and will continue on a rescue regimen (aflibercept 8 mg, every 8 weeks). If a patient in the HDq16 group who has not met the criteria at week 16 or 20 meets both criteria at week 24, the patient will be dosed with 8 mg aflibercept at that visit and will continue on q12 week dosing.

For patients whose interval was not shortened to q8 dosing at or before week 24, the interval will be shortened if the DRM criteria are met at a subsequent dosing visit. Patients in the HDq12 group who meet the criteria will receive the planned dose at that visit and will then continue on a rescue regimen (aflibercept 8 mg, every 8 weeks). Patients in the HDq16 group who meet these criteria will receive the planned dose at that visit and will then continue to be dosed every 12 weeks if they were on a 16-week interval, or switch to the rescue regimen (aflibercept 8mg, every 8 weeks) if they were previously shortened to a 12-week interval. Therefore, a patient randomized to HDq16 whose injection interval has been shortened to q12 will have their injection interval further shortened to q8 if these criteria are met at any subsequent dosing visit.

### Year 2: Week 52 to Week 96 (End of Study)

From week 52 through the end of study (year 2), all patients in the HD groups will continue to have the interval shortened in 4-week intervals if the DRM criteria for shortening are met at dosing visits using the DRM criteria described above for year 1. As in year 1, the minimum dosing interval for patients in all treatment groups is every 8 weeks.

In addition to shortening of the interval, all patients in the HD groups (including patients whose interval was shortened during year 1) may be eligible for interval extension (by 4-week increments), if BOTH the following criteria are met at dosing visits in year 2:
1. <5 letter loss in BCVA from week 12; AND
2. CRT <300 µm for Cirrus SD-OCT (or <320 µm on Spectralis SD-OCT)

For patients who do not meet the criteria for shortening or extension of the interval, the dosing interval will be maintained.

As in year 1, all patients in all treatment groups (including the 2q8 group) will be evaluated against both DRM criteria at all visits through the IWRS for masking purposes. However, changes to dosing schedule will only be implemented as described above for those patients randomized to HDq12 or HDq16 treatment groups. No changes to the dosing schedule will be made to the 2q8 treatment group at any time.

### Results at Week 48

Aflibercept HDq12 and HDq16 dosing regimens achieved the high bar of sustaining improvements in visual acuity and anatomic measures of retinal fluid across 48 weeks in patients with diabetic macular edema. The vast majority of patients did not require regimen modification. The data also support these regimens while maintaining a safety profile similar to EYLEA.

### Visual Outcomes

Both HDq12 and HDq16 demonstrated non-inferiority to 2q8 with respect to the primary efficacy endpoint (change from baseline in BCVA at week 48) using the non-inferiority margin of 4 letters with LSₘₑₐₙ change from baseline in BCVA of 8.10 letters (HDq12) and 7.23 letters (HDq16) versus 8.67 letters in the 2q8 group (Table 1-6). The differences in LSₘₑₐₙ changes from baseline in BCVA (95% CI) were -0.57 (- 2.26, 1.13) and -1.44 (-3.27, 0.39) for HDq12 and HDq16, respectively compared to 2q8 (Table 1-6). The p-values for the non-inferiority test at a margin of 4 letters were <0.0001 for HDq12 vs. 2q8, and 0.0031 for HDq16 vs. 2q8. The lower confidence limits were greater than -4, allowing the conclusion of non-inferiority at week 48 timepoint.

**Table 1-6. Primary Endpoint -- Change from Baseline in BCVA (ETDRS Letters) at Week 48 in the Study Eye, MMRM (Full Analysis Set)**

| **Treatment** | **LSmean (SE) change from BL** | **Mean (SD) change form BL** | **BL mean** | **Number of patients with week 48 data** | **DF** | **Contrast^{a}** | **t-value** | **1-sided NI p-value^{b}** | **1-sided superiority p-value** | **Estimate for contrast and 2-sided 95% CI^{c}** |
|---|---|---|---|---|---|---|---|---|---|---|
| HDq12 (N=328) | 8.10 (0.61) | 8.77 (8.95) | 63.63 | 277 | 351.5 | HDq12 - 2q8 | 3.9881 | <0.0001 | 0.7447 | -0.57 (-2.26, 1.13) |
| HDq16 (N=163) | 7.23 (0.71) | 7.86 (8.38) | 61.44 | 149 | 315.0 | HDq16 - 2q8 | 2.7533 | 0.0031 | 0.9388 | -1.44 (-3.27, 0.39) |
| 2q8 (N=167) | 8.67 (0.73) | 9.21 (8.99) | 61.47 | 150 | | | | | | |

| | | | | | | | | | | |
|---|---|---|---|---|---|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; BCVA=Best corrected visual acuity; CRT=Central retinal thickness; DME=Diabetic macular edema; ETDRS=Early Treatment Diabetic Retinopathy Study; EDC=electronic data capture; BL=baseline; CI=confidence interval; DF=degrees of freedom; NI=non-inferiority; LS=least square; SAP=statistical analysis plan; SE=standard error; SD=standard deviation A mixed model for repeated measurements (MMRM) was used with baseline BCVA measurement as a covariate, treatment group and the stratification variables (geographic region [Japan vs. Rest of World]; baseline CRT from reading center [<400µm vs. ≥400µm], prior treatment for DME per EDC; [yes vs. no]) as fixed factors, and terms for the interaction between baseline and visit and the interaction between treatment and visit. A Kenward-Roger approximation was used for the denominator degrees of freedom. a The contrast also included the interaction term for treatment x visit. b p-value for the 1-sided non-inferiority (NI) test at a margin of 4 letters. c Estimate based on the MMRM model, was computed for the differences of HDq12 minus 2q8 and HDq16 minus 2q8, respectively with 2-sided 95% CIs. | | | | | | | | | | |

The mean values of BCVA score averaged from week 36 to week 48 were similar across treatment groups, and the change from baseline was similar across treatment groups (Table 1-7).

**Table 1-7. Summary of Averaged BCVA Score: Week 36 to Week 48 (OC) (Full Analysis Set)**

| **Value at Visit** | | | | | | | | | **Change from Baseline** | | | | | | | | |
|---|---|---|---|---|---|---|---|---|---|---|---|---|---|---|---|---|---|
| **Treatment** | **Visit** | **n** | **Mean** | **SD** | **SE** | **Q1** | **Median** | **Q3** | **Min, Maxn** | | **Mean** | **SD** | **SE** | **Q1** | **Median** | **Q3** | **Min, Max** |
| 2q8 (N=167) | BASELINE | 167 | 61.5 | 11.22 | 0.87 | 54.0 | 63.0 | 70.0 | 24, 78 | | | | | | | | |
| WEEK 36 TO 48 | | 155 | 70.5 | 11.75 | 0.94 | 64.0 | 73.0 | 79.0 | 23, 90 | 155 | 8.8 | 8.60 | 0.69 | 4.0 | 9.3 | 13.5 | -26, 48 |
| HDq12 (N=328) BASELINE | | 328 | 63.6 | 10.10 | 0.56 | 57.0 | 65.0 | 72.0 | 27, 79 | | | | | | | | |
| WEEK 36 TO 48 | | 286 | 71.8 | 11.35 | 0.67 | 65.3 | 73.9 | 80.0 | 22, 92 | 286 | 8.1 | 8.93 | 0.53 | 3.0 | 7.9 | 12.5 | -50, 38 |
| HDq16 (N=163) BASELINE | | 163 | 61.4 | 11.76 | 0.92 | 55.0 | 64.0 | 71.0 | 29, 78 | | | | | | | | |
| WEEK 36 TO 48 | | 154 | 69.1 | 12.77 | 1.03 | 62.5 | 72.6 | 78.3 | 21, 92 | 154 | 7.2 | 7.95 | 0.64 | 2.8 | 6.3 | 10.3 | -12, 38 |

| | | | | | | | | | | | | | | | | | |
|---|---|---|---|---|---|---|---|---|---|---|---|---|---|---|---|---|---|
| Abbreviations: 2q8= Aflibercept 2 mg administered every 8 weeks after 5 initial injections at 4-week intervals, HDq12= High dose aflibercept 8 mg administered every 12 weeks after 3 initial injections at 4-week intervals; HDq16= High dose aflibercept 8 mg administered every 16 weeks after 3 initial injections at 4-week intervals, ICE= intercurrent events; n= number; Q= quartile; SAP= statistical analysis plan; SD= standard deviation; SE= standard error; OC: observations after an intercurrent event (ICE) defined for the primary estimand were excluded. | | | | | | | | | | | | | | | | | |

The proportion of participants who gained ≥ 15 letters in BCVA from baseline to week 48 was 18.7% and 16.6% in the HDq12 and HDq16 groups, respectively, compared with 23.0% in the 2q8 group (Table 1-8).

Sensitivity analysis for the proportion of participants who gained ≥ 15 letters in BCVA from baseline at week 48 using OC was consistent with the LOCF analysis.

**Table 1-8. Proportion of Participants who Gained ≥ 15 Letters in BCVA from Baseline at Week 48 (LOCF) (Full Analysis Set)**

| **Treatment** | **Patients with ≥ 15 Letters gain in BCVA from baseline to Week 48, n (%)** | **Adjusted Difference (%) (95% CI)^{a}** | **CMH test^{b} p-value** |
|---|---|---|---|
| HDq12 (N=328) | 61/326 (18.7%) | -4.64 ( -12.30 , 3.02) | 0.2231 |
| HDq16 (N=163) | 27/163 (16.6%) | -7.14 ( -15.45 , 1.17) | 0.0960 |
| 2q8 (N=167) | 38/165 (23.0%) | | |

| | | | |
|---|---|---|---|
| Abbreviations: 2q8=Aflibercept 2 mg administered every 8 weeks after 5 initial injections at 4-week intervals; HDq12=High dose aflibercept 8 mg administered every 12 weeks after 3 initial injections at 4-week intervals; HDq16=High dose aflibercept 8 mg administered every 16 weeks after 3 initial injections at 4-week intervals. BCVA=best corrected visual acuity; CI=confidence interval; CMH=Cochran-Mantel-Haenszel; CRT= central retinal thickness (or, central subfield retinal thickness); DME= diabetic macular edema; ICE=intercurrent events; LOCF=last observation carried forward; N,n=number of patients; SAP= statistical analysis plan. LOCF: the last observation prior to an ICE defined for the primary estimand was used to impute subsequent and/or missing or non-gradable data. Missing data were not included in the denominator. a Difference with CI was calculated using Mantel-Haenszel weighting scheme adjusted for stratification factors (baseline CRT (from reading center) [<400 µm, ≥400 µm], prior DME treatment [yes, no], geographical region [Rest of world, Japan]). b Nominal p-value for the 2-sided CMH superiority test. | | | |

The proportion of participants who achieved ≥ 69 letters in BCVA (≥ 20/40 Snellen equivalent) at week 48 was similar across treatment groups (63.0 to 65.3% participants) (Table 1-9).

Sensitivity analyses for the proportion of participants who achieved ≥ 69 letters in BCVA at week 48 using OC were consistent with the LOCF analysis.

**Table 1-9. Proportion of Participants with BCVA ≥ 69 letters at Week 48 (LOCF) (FAS)**

| **Treatment** | **Patients with BCVA >= 69 letters at Week 48, n (%)** | **Adjusted Difference (%) (95% CI) ^{a}** | **CMH test ^{b} p-value** |
|---|---|---|---|
| HDq12 (N=328) | 213/326 (65.3%) | 2.45 ( -6.47 , 11.36) | 0.5917 |
| HDq16 (N=163 ) | 102/163 (62.6%) | -0.67 ( -11.16 , 9.82) | 0.8998 |
| 2q8 (N=167 ) | 104/165 (63.0%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. BCVA: best corrected visual acuity; CMH=Cochran-Mantel-Haenszel; CRT= central retinal thickness (or, central subfield retinal thickness); DME= diabetic macular edema; FAS= Full analysis set; LOCF: the last observation prior to an ICE defined for the primary estimand was used to impute subsequent and/or missing data; N,n=number of patients. a Difference with confidence interval (CI) was calculated using Mantel-Haenszel weighting scheme adjusted for stratification factors (baseline CRT (from reading center) [<400µm, >=400 µm], prior DME treatment [yes, no], geographical region [Rest of world, Japan]) b Nominal p-value for the 2-sided Cochran-Mantel-Haenszel | | | |

The proportion of participants who gained or lost ≥ 5, ≥ 10, or ≥ 15 letters from baseline through week 48 is presented in Table 1-10. Across all treatment groups, more participants gained letters, with the greatest proportion gaining ≥ 5 letters (approximately 65 to 71% across all treatment groups). A numerically lower proportion of participants in the HDq12 and HDq16 groups gained ≥ 10 letters or ≥ 15 letters compared to the 2q8 group. Few participants (approximately 1 to 6%) lost 5 or more letters through week 48 regardless of treatment group.

**Table 1-10. Proportion of Participants who Gained or Lost ≥ 5, 10, or 15 Letters in BCVA from Baseline by Visit through Week 48 (LOCF) (Full Analysis Set)**

| **Endpoint** | | **2q8 (N=167)** | **HDq12 (N=328)** | **HDq16 (N=163)** |
|---|---|---|---|---|
| Gained >= 5 letters | Week 48 | 113/165 (68.5%) | 231/326 (70.9%) | 107/163 (65.6%) |
| Gained >= 10 letters | Week 48 | 81/165 (49.1%) | 132/326 (40.5%) | 57/163 (35.0%) |
| Gained >= 15 letters | Week 48 | 38/165 (23.0%) | 61/326 (18.7%) | 27/163 (16.6%) |
| Lost >= 5 letters | Week 48 | 5/165 (3.0%) | 21/326 (6.4%) | 10/163 (6.1%) |
| Lost >= 10 letters | Week 48 | 2/165 (1.2%) | 11/326 (3.4%) | 2/163 (1.2%) |
| Lost >= 15 letters | Week 48 | 2/165 (1.2%) | 7/326 (2.1%) | 1/163 (0.6%) |

| | | | | |
|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; BCVA= best corrected visual acuity; ICE=intercurrent events; LOCF=last observation carried forward; SAP=statistical analysis plan. LOCF: the last observation prior to an ICE defined for the primary estimand was used to impute subsequent and/or missing data. Missing data were not included in the denominator. | | | | |

### DRSS

The proportion of participants with a ≥ 3-step improvement in DRSS at week 48 was 11.9% and 9.2% in the HDq12 and HDq16 groups, respectively, compared with 14.6% in the 2q8 group (Table 1-11).

Sensitivity analyses for the proportion of participants with a ≥ 3-step improvement in DRSS score at week 48 using OC were consistent with the LOCF analysis.

**Table 1-11. Proportion Analysis of Participants with a ≥ 3-step Improvement from Baseline in DRSS at Week 48 (LOCF) (Full Analysis Set)**

| **Treatment** | **Patients with a ≥ 3-step improvement from baseline in DRSS at Week 48, n (%)** | **Adjusted Difference (%) (95% CI) ^{a}** | **CMH test** ^{b} **p-value** |
|---|---|---|---|
| HDq12 (N=328) | 37/310 (11.9%) | -2.79 ( -9.50, 3.91) | 0.3920 |
| HDq16 (N=163) | 14/153 (9.2%) | -5.59 ( -12.88, 1.70) | 0.1310 |
| 2q8 (N=167) | 23/158 (14.6%) | | |

| | | | |
|---|---|---|---|
| 2q8=Aflibercept 2 mg administered every 8 weeks after 5 initial injections at 4-week intervals; HDq12=High dose aflibercept 8 mg administered every 12 weeks after 3 initial injections at 4-week intervals; HDq16=High dose aflibercept 8 mg administered every 16 weeks after 3 initial injections at 4-week intervals; DRSS=Diabetic Retinopathy Severity Scale; CRT=Central retinal thickness; DME=Diabetic macular edema. LOCF: the last observation prior to an ICE defined for the primary estimand was used to impute subsequent and/or missing or non-gradable data. Patients were considered as non-responders if all post-baseline measurements were missing or non-gradable. Missing or ungradable baseline was not included in the denominator. a. Difference with confidence interval (CI) was calculated using Mantel-Haenszel weighting scheme adjusted for stratification factors (baseline CRT (from reading center) [<400µm, >=400 µm], prior DME treatment [yes, no], geographical region [Rest of world, Japan]) b. p-value for the two-sided Cochran-Mantel-Haenszel (CMH) superiority test | | | |

HDq12 was non-inferior to 2q8 with respect to this endpoint (≥2 step improvement in DRSS). However, this could not be shown for the HDq16 group. The non-inferiority margin was prespecified at 15%, however HDq12 also met a 10% NI margin. The proportion of participants with ≥ 2-step improvement in DRSS score was 25.7%, 24.7% and 20.7% at week 12 and 26.6%, 29.0%, and 19.6% at week 48 in the 2q8, HDq12, and HDq16 groups respectively. In Cochran-Mantel-Haenszel (CMH)-weighted estimates, the adjusted difference (95% CI) was 1.98% (-6.61, 10.57) for HDq12 and -7.52% (- 16.88, 1.84) for HDq16, respectively versus 2q8 (Table 1-12). Sensitivity analysis using observed cases (OC) was performed and was consistent with the primary analysis.

**Table 1-12. Key Secondary Endpoint - Proportion of Participants with a ≥ 2-Step Improvement from Baseline in DRSS at Week 48 (LOCF) (Full Analysis Set)**

| **Treatment** | **Patients with a ≥ 2-step Improvement From Baseline in DRSS, n (%)** | **Adjusted Difference (%) 2-sided (95% CI) ^{a}** |
|---|---|---|
| HDq12 (N=328) | 90/310 (29.0%) | 1.98 ( -6.61 , 10.57) |
| HDq16 ( N=163 ) | 30/153 (19.6%) | -7.52 ( -16.88 , 1.84) |
| 2q8 ( N=167 ) | 42/158 (26.6%) | |

| | | |
|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI=confidence interval; CRT= central retinal thickness; DRSS=Diabetic Retinopathy Severity Scale; N,n=number of patients; SAP = statistical analysis plan Intercurrent events (ICE) were handled according to Table 1 of SAP (Appendix 16.1.9.1). LOCF= the last observation prior to an ICE defined for the primary estimand was used to impute subsequent and/or missing or nongradable data. Patients were considered as non-responders if all post-baseline measurements were missing or nongradable. a Difference with confidence interval (CI) was calculated using Mantel-Haenszel weighting scheme adjusted for stratification factors (baseline CRT (from reading center) [<400 µm, ≥400 µm], prior DME treatment [yes, no], geographical region [Rest of world, Japan]). The non-inferiority margin was set at 15%. Missing or ungradable baseline was not included in the denominator. | | |

As the tests for the primary endpoint and change from baseline in BCVA at Week 60 (key secondary endpoint) were significant for both HD groups, the test sequence could be continued with testing the key secondary efficacy endpoint, the proportion of participants with ≥ 2-step improvement in DRSS score (as assessed by the central reading center) at week 48. HDq12 was non-inferior to 2q8 with respect to this endpoint. However, this could not be shown for the HDq16 group. The non-inferiority margin was prespecified at 15%, however HDq12 also met a 10% NI margin. The proportion of participants with ≥ 2-step improvement in DRSS score was 25.7%, 24.7% and 20.7% at week 12 and 26.6%, 29.0%, and 19.6% at week 48 in the 2q8, HDq12, and HDq16 groups respectively. In Cochran-Mantel-Haenszel (CMH)-weighted estimates, the adjusted difference (95% CI) was 1.98% (-6.61, 10.57) for HDq12 and 7.52% (-16.88, 1.84) for HDq16, respectively versus 2q8.

### Retinal Fluid

The proportion of participants without fluid (no IRF and no SRF) at the foveal center (as assessed by the central reading center) at week 48 was 58.5% and 43.8% in the HDq12 and HDq16 groups, respectively, compared with 54.5% in the 2q8 group (Table 1-13).

Sensitivity analyses for the proportion of participants without fluid (no IRF and no SRF) at the foveal center at week 48 using OC were consistent with the LOCF analysis.

**Table 1-13. Proportion of Participants without Fluid (no IRF and no SRF) at the Foveal Center at Week 48 (LOCF) (Full Analysis Set)**

| **Treatment** | **Patients without fluid, n(%)** | **Adjusted Difference (%) (95% CI)^{a}** | **CMH test^{b} p-value** |
|---|---|---|---|
| HDq12 (N=328) | 190/325 (58.5%) | 4.32 ( -4.72, 13.36) | 0.3491 |
| HDq16 (N=163) | 71/162 (43.8%) | -9.73 ( -20.34, 0.87) | 0.0757 |
| 2q8 (N=167) | 90/165 (54.5%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI=confidence interval; CMH=Cochran-Mantel-Haenszel; DME= diabetic macular edema; ICE=intercurrent events; IRF=intraretinal fluid; LOCF=last observation carried forward; N=number of participants; SRF=subretinal fluid. LOCF= the last observation prior to an ICE defined for the primary estimand was used to impute subsequent and/or missing or non-gradable data. | | | |

Missing or undetermined data were not included in the denominator.
a. Difference with CI was calculated using Mantel-Haenszel weighting scheme adjusted for stratification factors (baseline CRT (from reading center) [<400 µm, ≥400 µm], prior DME treatment [yes, no], geographical region [Rest of world, Japan]).
b. Nominal p-value for the 2-sided CMH superiority test.

The proportion of participants without fluid (no IRF and no SRF) in the center subfield at week 48 was 27.4% and 14.8% in the HDq12 and HDq16 groups, respectively, compared with 21.8% in the 2q8 group (Table 1-14).

Sensitivity analyses for the subset of participants without fluid (no IRF and no SRF) in the center subfield at week 48 using OC were consistent with the LOCF analysis.

**Table 1-14. Proportion of Participants Without Fluid (no IRF and no SRF) at the Central Subfield at Week 48 (LOCF) (Full Analysis Set)**

| **Treatment** | **Patients without fluid, n (%)** | **Adjusted Difference (%) (95% CI) ^{a}** | **CMH test** ^{b} **p-value** |
|---|---|---|---|
| HDq12 (N=328) | 89/325 (27.4%) | 5.84 ( -2.02 , 13.71) | 0.1610 |
| HDq16 (N=163) | 24/162 (14.8%) | -6.75 ( -14.94 , 1.44) | 0.1110 |
| 2q8 (N=167) | 36/165 (21.8%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; CI=confidence interval; CMH=Cochran-Mantel-Haenszel; CRT = central retinal thickness; DME = diabetic macular edema; ICE= intercurrent events; IRF=intraretinal fluid; LOCF=last observation carried forward; N,n=number of patients; SAP = Statistical analysis plan; SRF=subretinal fluid. LOCF: the last observation prior to an ICE defined for the primary estimand was to be used to impute subsequent and/or missing data Missing or undetermined data were not included in the denominator. a Difference with CI was calculated using Mantel-Haenszel weighting scheme adjusted for stratification factors (baseline CRT (from reading center) [<400µm, >=400 µm], prior DME treatment [yes, no], geographical region [Rest of world, Japan]). b Nominal p-value for the 2-sided CMH superiority test. | | | |

### Anatomical Outcomes

Overall, the LSₘₑₐₙ (SE) change from baseline in CRT (as assessed by the central reading center) at week 48 was -176.77 (5.73) and -148.84 (9.45) in the HDq12 and HDq16 groups, respectively, compared with -164.85 (8.79) in the 2q8 group (Table 1-15).

The mean changes from baseline in CRT using OC, are graphically displayed in Figure 26A; the corresponding LSₘₑₐₙ changes from baseline in CRT using MMRM in the FAS, are displayed in Figure 26B.

Sensitivity analyses for change from baseline in CRT at week 48 using LOCF were consistent with the MMRM analysis.

**Table 1-15. Statistical Analysis of Change from Baseline in Central Retinal Thickness (microns) at Week 48 (MMRM)(FAS)**

| **Treatment** | **LSₘₑₐₙ (SE) change from BL** | **Mean (SD) change from BL** | **BL Mean** | **Number of patients with Week 48 data** | **DF** | **Contrast ^{a}** | **t-value** | **p-value b** | **Estimate for contrast and 2-sided 95% CI ^{c}** |
|---|---|---|---|---|---|---|---|---|---|
| HDq12 (N=328) | -176.77 (5.73) | -171.65 (141.52) | 449.15 | 276 | 254.9 | HDq12 - 2q8 | -1.2768 | 0.2028 | -11.92 (-30.30, 6.47) |
| HDql6 (N=163) | -148.84 (9.45) | -148.30 (133.20) | 460.32 | 149 | 295.3 | HDql6 - 2q8 | 1.3386 | 0.1817 | 16.01 (-7.53, 39.54) |
| 2q8 (N=167) | -164.85 (8.79) | -165.31 (140.22) | 457.25 | 148 | | | | | |

| | | | | | | | | | |
|---|---|---|---|---|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI = Confidence interval. CRT = Central retinal thickness. SE = Standard error. SD = Standard deviation. DF = Degrees of freedom. FAS = Full analysis set. LS = Least Square. BL = Baseline. MMRM = mixed model for repeated measurements. SAP = statistical analysis plan; DME=Diabetic macular edema; EDC=electronic data capture. A mixed model for repeated measurements (MMRM) was used with baseline CRT measurement as a covariate, treatment group and the stratification variables (geographic region [Japan vs. Rest of World]; baseline CRT (from reading center) [<400µm vs. >=400µm], prior treatment for DME (per EDC) [yes vs. no]) as fixed factors, and terms for the interaction between baseline and visit and the interaction between treatment and visit. A Kenward-Roger approximation was used for the denominator degrees of freedom. An unstructured covariance structure was used to model the within-subject error. a The contrast also included the interaction term for treatment x visit. b Nominal p-value for the 2-sided superiority test c Estimate based on the MMRM model, was computed for the differences of HDq12 minus 2q8 and HDq16 minus 2q8, respectively with 2-sided 95% CIs. | | | | | | | | | |

### Fluid Leakage

Overall, the proportion of participants without leakage on fluorescein angiography (as assessed by the central reading center) at week 48 (LOCF) was very low in all 3 treatment groups: 7.6% and 0.7% in the HDq12 and HDq16 groups, respectively, compared with 2.5% in the 2q8 group.

Sensitivity analyses for the proportion of participants without leakage on fluorescein angiography at week 48 using OC were consistent with the LOCF analysis.

A summary of the change from baseline in total area of fluorescein leakage within the ETDRS grid at week 48 is shown in Table 1-16.

**Table 1-16. Summary of the Change from Baseline in Total Area of Fluorescein Leakage within ETDRS Grid (mm²) at Week 48 (OC) (Full Analysis Set)**

| **Statistic** | **2q8 N=167** | **HDq12 N=328** | **HDq16 N=163** |
|---|---|---|---|
| Baseline n | 164 | 319 | 153 |
| Baseline mean (SD) | 24.6 (13.20) | 24.4 (13.22) | 24.6 (11.73) |
| Week 48 n | 131 | 224 | 129 |
| Mean (SD) change from baseline at week 48 | -9.2 (12.11) | -13.9 (13.91) | -9.4 (11.50) |
| Median change from baseline at week 48 | -6.8 | -13.3 | -7.7 |
| Min, Max | -85, 18 | -88, 52 | -39, 55 |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; SAP=statistical analysis plan. ETDRS: Early Treatment Diabetic Retinopathy Study; OC: observations after an ICE defined for the primary estimand were excluded; SD; standard deviation. | | | |

### Patient Reported Outcomes

Overall, the LSₘₑₐₙ change from baseline in NEI-VFQ-25 total score at week 48 was 4.06 and 2.94 in the HDq12, and HDq16 groups, respectively, compared with 2.82 in the 2q8 group (Table 1-17).

Sensitivity analyses for the change from baseline in NEI-VFQ-25 total score at week 48 using ANCOVA, LOCF and were consistent with the MMRM analysis.

**Table 1-17. Change from Baseline in NEI-VFQ-25 Total Score at Week 48 (MMRM) (Full Analysis Set)**

| **Treatment** | **LSₘₑₐₙ (SE) change from BL** | **Mean (SD) change from BL** | **BL Mean** | **Number of patients with Week 48 data** | **DF** | **Contrast ^{a}** | **t-value** | **p-value ^{b}** | **Estimate for contrast and 2-sided 95% CI ^{c}** |
|---|---|---|---|---|---|---|---|---|---|
| HDq12 (N=328) | 4.06 (0.80) | 5.64 (12.56) | 76.79 | 276 | 251.4 | HDq12 - 2q8 | 1.0515 | 0.2941 | 1.25 (-1.09, 3.58) |
| HDql6 (N=163) | 2.94 (0.93) | 4.16 (10.94) | 77.86 | 149 | 261.8 | HDql6 - 2q8 | 0.0995 | 0.9208 | 0.13 (-2.37, 2.62) |
| 2q8 (N=167) | 2.82 (1.10) | 4.41 (13.84) | 76.65 | 150 | | | | | |

| | | | | | | | | | |
|---|---|---|---|---|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; CI = Confidence interval. SE = Standard error. SD = Standard deviation. DF = Degrees of freedom. LS = Least Square. BL = Baseline. MMRM = mixed model for repeated measurements. SAP = statistical analysis plan; DME = diabetic macular edema; EDC = electronic data capture A mixed model for repeated measurements (MMRM) was used with baseline NEI-VFQ-25 total score measurement as a covariate, treatment group and the stratification variables (geographic region [Japan vs. Rest of World]; baseline CRT (from reading center) [<400µm vs. >=400µm], prior treatment for DME (per EDC) [yes vs. no]) as fixed factors, and terms for the interaction between baseline and visit and the interaction between treatment and visit. A Kenward-Roger approximation was used for the denominator degrees of freedom. An unstructured covariance structure was used to model the within-subject error. a The contrast also included the interaction term for treatment x visit b Nominal p-value for the 2-sided superiority test c Estimate based on the MMRM model, was computed for the differences of HDq12 minus 2q8 and HDq16 minus 2q8, respectively with 2-sided 95% CIs. | | | | | | | | | |

### Safety

Overall, a similar proportion of participants had TEAEs in the HD groups, 74.7% (245 participants; HDq12) and 77.3% (126 participants; HDq16), compared to 73.7% (123 participants) in the 2q8 group.

The proportions of participants with ocular TEAEs were similar across the groups and were 43.7% (73 participants), 44.8% (147 participants), and 44.8% (73 participants) in the 2q8, HDq12, and HDq16 groups, respectively. There were very few study-drug-related ocular and non-ocular TEAEs across all treatment groups. The proportion of participants with study conduct-related TEAEs and TEAEs related to 2 mg aflibercept in the fellow eye were minimally reported across groups (< 2.0% overall across groups). The proportion of participants with injection-procedure-related ocular TEAEs was similar across treatment groups (< 14% across groups).

The majority of serious AEs reported were non-ocular TEAEs (19.2% [32 participants], 18.6% [61 participants], and 16.6% [27 participants] in the 2q8, HDq12 and HDq16 groups, respectively). One injection-procedure-related ocular serious TEAE (Intraocular pressure increased) in the study eye was reported and occurred in the HDq12 group (0.3%). There were no reported study-drug-related serious TEAEs, study-conduct-related serious TEAEs, or serious TEAEs related to 2 mg aflibercept in the fellow eye.

Three (1.8%) participants in the 2q8 group, 9 (2.7%) participants in the HDq12 group, and 2 (1.2%) participants in the HDq16 group discontinued study drug due to TEAEs. Of these, 2 participants discontinued study drug due to ocular TEAEs (both in the HDq12 group).

Five deaths were reported in the 2q8 group (3.0%), 9 deaths in the HDq12 group (2.7%), and 4 deaths in the HDq16 group (2.5%). All deaths were considered unrelated to study treatment by the investigator.

The proportion of participants with treatment-emergent adjudicated Antiplatelet Trialists' Collaboration (APTC) events was low and generally similar across treatment groups: 3.6% (6 participants), 4.0% (13 participants), and 5.5% (9 participants) in the 2q8, HDq12, and HDq16 groups, respectively.

A slightly higher frequency of participants reported Hypertension in the HDq16 group (17.2%; 28 participants) compared to the 2q8 group (13.8%; 23 participants) and the HDq12 group (12.8%; 42 participants); however, this was not interpreted as clinically meaningful as there was no apparent dose relationship (ie, HDq16 versus HDq12).

There were no treatment-emergent nasal mucosal events reported through week 60. Table 39 summarizes the number of participants with ocular TEAEs in the study eye occurring in ≥ 1.0% of participants in any treatment group through week 60.

Ocular TEAEs in the study eye were reported at similar frequencies in all 3 groups (29.3% [49 participants], 36.0% [118 participants], and 34.4% [56 participants] in the 2q8, HDq12, and HDq16 groups, respectively). No clinically meaningful differences were observed in type of TEAEs or their frequencies between the HD and 2q8 treatment groups, and reported events were consistent with the known safety profile of IVT aflibercept.

Overall, ocular TEAEs in the fellow eye were reported in 52 (31.1%) participants in the 2q8 group, 91 (27.7%) participants in the HDq12 group, and 52 (31.9%) participants in the HDq16 group.

All ocular TEAEs in the fellow eye were reported in < 6.0% of participants in each treatment group. The most frequent PTs were Cataract (4.2% [7 participants], 3.0% [10 participants], and 5.5% [9 participants] in the 2q8, HDq12, and HDq16 groups, respectively), Vitreous floaters (4.3%; 7 participants in the HDq16 group), Diabetic retinal oedema (3.4% [11 participants] in the HDq12 group), and Diabetic retinopathy (3.6% [6 participants] in the 2q8 group and 3.7% [6 participants] in the HDq16 group).

Ocular TEAEs were generally balanced across the 3 treatment groups.

Non-ocular TEAEs were reported in a similar proportion of participants in the 2q8 group (57.5%; 96 participants) and the Pooled HD group (60.9%; 299 participants). The majority of the TEAEs were in the SOC (system organ class) of Infections and infestations; however, the most common TEAE was Hypertension. A slightly higher frequency of participants reported Hypertension in the HDq16 group (15.3%; 25 participants) compared to the 2q8 group (10.8%; 18 participants) and the HDq12 group (9.1%; 30 participants); however, this was not interpreted as clinically meaningful as there was no apparent dose relationship (ie, HDq16 versus HDq12).

Other non-ocular TEAEs were reported ≤ 5.0% of participants in the 2q8 and the Pooled HD group except for COVID-19 (8.6%; 42 participants in the Pooled HD group).

Ocular study-drug-related TEAEs in the study eye were reported in 3 (1.8%) participants in the 2q8 group, 6 (1.8%) participants in the HDq12 group, and no participants in the HDq16 group.

Intraocular pressure increased was the only PT reported in more than 1 participant (3 [0.9%] participants in the HDq12 group).

All ocular study-drug-related TEAEs in the study eye were reported in < 1.0% of participants. There were no ocular study drug-related TEAEs in the fellow eye through week 60 reported in any treatment group.

One non-ocular study drug-related TEAE was reported through week 60: Lacunar infarction reported in 1 (0.6%) participant in the HDq16 group.

There were no non-ocular study-drug-related TEAEs reported through week 60 in the 2q8 or HDq12 groups.

Ocular IVT-injection-related TEAEs were reported in 16 (9.6%) participants in the 2q8 group, 42 (12.8%) participants in the HDq12 group, and 13 (8.0%) participants in the HDq16 group. Ocular IVT-injection-related TEAEs that were reported in > 2 participants in any of the 3 treatment groups included Conjunctival haemorrhage, Vitreous floaters, Eye pain, and Intraocularpressure increased which were reported in similar proportions of participants across the 3 treatment groups.

All other ocular IVT-injection-related TEAEs in the study eye were reported in ≤ 2 participants in each group. Ocular IVT-injection-related TEAEs in the fellow eye through week 60 were reported in 5 (3.0%) participants in the 2q8 group, 7 (2.1%) participants in the HDq

Non-ocular IVT-injection-related TEAEs through week 60 were reported in 3 (0.6%) participants in the Pooled HD group. The TEAEs reported in the HD groups included Nausea, Vomiting, and Headache. No participants reported non-ocular IVT-injection-related TEAEs in the 2q8 group.

Ocular and Non-ocular Study Conduct-Related TEAEs Through Week 60 The relationship of TEAEs to other study procedures were assessed by the masked investigator, and was a clinical decision based on all available information.

Study-conduct-related TEAEs were reported in 2 (0.6%) participants in the HDq12 group. These TEAEs were Conjunctival haemorrhage and Injection site irritation. No study-conduct-related TEAEs were reported in the 2q8 or HDq16 groups.

There were no ocular study-conduct-related TEAEs in the fellow eye through week 60 reported in any treatment group.

Non-ocular TEAEs Related to Study Conduct Non-ocular study-conduct-related TEAEs through week 60 were reported in 3 (1.8%) participants in the 2q8 group and 4 (0.8%) participants in the Pooled HD group. These TEAEs were Nausea, Vessel puncture site haematoma, Contrast media allergy, Post procedural pruritus, Rash, and Vein rupture

Ocular and Non-ocular TEAEs related to 2-mg Aflibercept in the Fellow Eye Once the fellow eye received 2-mg aflibercept treatment during the study, TEAEs and serious TEAEs were also assessed as related/not related to 2-mg aflibercept treatment in the fellow eye, assessed as related/not related to the study drug (delivered to the study eye), IVT injection, and other protocol-specified procedures.

No ocular TEAEs in the study eye related to 2-mg aflibercept in the fellow eye through week 60 were reported in any treatment group

Ocular TEAEs in the fellow eye related to 2-mg aflibercept in the fellow eye through week 60 were reported in few participants, 2 (1.2%) participants in the 2q8 group, 1 (0.3%) participant in the HDq12 group, and 2 (1.2%) participants in the HDq16 group. These TEAEs were Conjunctival haemorrhage, Halo vision, and Intraocular pressure increased.

One non-ocular TEAE related to 2-mg aflibercept in the fellow eye was reported through week 60: Lacunar infarction was reported in 1 (0.6%) participant in the HDq16 group. The same event was also considered to be related to study drug.

No non-ocular TEAEs related to 2-mg aflibercept in the fellow eye were reported through week 60 in the 2q8 or HDq12 groups.

Intensity of Ocular and Non-ocular TEAEs Through Week 60 'Intensity' is used in parallel and synonymously with 'severity' of AEs herein.

The majority of ocular TEAEs in the study eye were mild (22.8% [38 participants; 2q8 group], 26.2% [86 participants; HDq12 group], and 28.2% [46 participants; HDq16 group]) to moderate (6.0% [10 participants; 2q8 group], 9.1% [30 participants; HDq12 group], and 5.5% [9 participants; HDq16 group]).

Severe ocular TEAEs in the study eye were reported in few participants, 1 (0.6%) participant in the 2q8 group, 2 (0.6%) participants in the HDq12 group, and 1 (0.6%) participant in the HDq16 group. The ocular TEAEs that were reported as being severe in the study eye were Cataract nuclear and Cataract subcapsular (reported by 1 participant in the 2q8 group), Cataract subcapsular and Retinal vascular disorder (reported by 1 participant each in the HDq12 group), and Retinal detachment and Vitreous haemorrhage (reported by 1 participant in the HDq16 group).

The majority of ocular TEAEs in the fellow eye were mild (22.2% [37 participants; 2q8 group], 21.0% [69 participants; HDq12 group], and 22.1% [36 participants; HDq16 group]) to moderate (7.2% [12 participants; 2q8 group], 5.8% [19 participants; HDq12 group], and 9.8% [16 participants; HDq16 group]).

Severe ocular TEAEs in the fellow eye were reported in few participants, 3 (1.8%) participants in the 2q8 group, 3 (0.9%) participants in the HDq12 group, and no participants in the HDq16 group. Severe ocular TEAEs in the fellow eye reported by the 3 participants in the 2q8 group were Cataract subcapsular, Cataract nuclear, Diabetic retinopathy, and Retinal artery occlusion (reported by 1 participant each); Diabetic retinopathy (reported by 1 participant) and Vitreous haemorrhage (reported by 3 participants) in the HDq12 group.

The majority of non-ocular TEAEs were mild (25.7% [43 participants; 2q8 group] and 26.9% [132 participants; Pooled HD group]) to moderate (18.0% [30 participants; 2q8 group] and 21.6% [106 participants; Pooled HD group]). Severe non-ocular TEAEs were reported in 23 (13.8%) participants in the 2q8 group, and 61 (12.4%) participants in the Pooled HD group.

Ocular Serious TEAEs in the Study Eye Through Week 60 A total of 5 ocular serious TEAEs in the study eye were reported in 4 participants. Serious TEAEs in the study eye were Ulcerative keratitis (1 [0.6%] participant; 2q8 group), Cataract subcapsular, and Intraocular pressure increased (1 [0.3%] participant each; both in the HDq12 group), and Retinal detachment and Vitreous haemorrhage (1 [0.6%] participant; HDq16 group). None of the events were considered related to the study drug and 1 event (Intraocular pressure increased) was considered related to injection procedure.

A total of 11 ocular serious TEAEs of the fellow eye were reported in 9 participants. None of these events were considered related to the study drug. The majority of these TEAEs were reported in single participants only. Across the 2q8 and Pooled HD groups, the most frequent non-ocular serious TEAEs (reported in ≥3 participants) were Acute left ventricular failure (3 [1.8%] participants) in the 2q8 group; and Acute myocardial infarction (7 [1.4%] participants), Cardiac arrest (3 [0.6%] participants), Coronary artery disease (4 [0.8%] participants), Myocardial infarction (7 [1.4%] participants), COVID-19 (4 [0.8%] participants), Covid-19 pneumonia (3 [0.6%] participants), Pneumonia (4 [0.8%] participants), Hypoglycaemia (3 [0.6%] participants), Cerebrovascular accident (5 [1.0%] participants), Acute kidney injury (6 [1.2%] participants), and Acute respiratory failure (3 [0.6%] participants) in the Pooled HD group. None of these events were considered related to the study

There were no ocular TEAEs in the fellow eye reported resulting in the discontinuation of the study drug.

Non-ocular TEAEs reported that resulted in the discontinuation of the study drug for 3 (1.8%) participants in the 2q8 group and 9 (1.8%) participants in the Pooled HD group Non-ocular TEAEs leading to discontinuation of the study drug included Blood loss anaemia, Acute myocardial infarction, Cardiac arrest, Death, Multiple organ dysfunction syndrome, Cholecystitis acute, Hip fracture, Endometrial cancer, Gastrointestinal neoplasm, Cerebrovascular accident, Encephalopathy, Acute kidney injury, Nephropathy toxic, and Aortic stenosis. No specific safety trend was observed, and most events were reported in single participants.

Ocular IVT-injection-related TEAEs in the fellow eye were generally balanced between the 3 treatment groups.

Through week 60, there were 18 deaths reported in this study, evenly distributed across the treatment groups, and all were associated with an SAE. None of the deaths were considered related to study drug or study procedure. Overall, the deaths reported were consistent with concurrent medical conditions and the complications of these conditions associated with an older population.

TEAEs related to Intraocular Inflammation were reported in 1 (0.6%) participant in the 2q8 group who reported Iridocyclitis, 4 (1.2%) participants in the HDq12 group who each reported 1 of the following: Iritis, Uveitis, Vitreal cells, and Vitritis, and 1 (0.6%) participant in the HDq16 group who reported Iridocyclitis. None of the events were serious.

Potential arterial thromboembolic events were evaluated by a masked adjudication committee according to criteria formerly applied and published by the APTC. Arterial thromboembolic events as defined by the APTC criteria include Nonfatal myocardial infarction, Nonfatal stroke (ischemic or hemorrhagic), or Death resulting from vascular or unknown causes. Low (< 6.0%) and similar proportions of participants reported adjudicated APTC events across the treatment groups.

Treatment-emergent hypertension events were reported in fewer than 20% of participants in any treatment group. A slightly higher portion of participants reported Hypertension in the HDq16 compared to the 2q8 group and the HDq12 group; however, this was not interpreted as clinically meaningful as there was no apparent dose relationship (ie, HDq16 versus HDq12). Approximately 76% of participants in all treatment groups had a medical history of Hypertension.

Due to findings from the preclinical toxicology studies for HD, an assessment was performed in the clinical program for events related to nasal mucosa. None of the participants experienced a TEAE consistent with Nasal mucosal findings.

Overall, the treatment-emergent ocular surgeries reported were consistent with the medical history and the concurrent clinical medical conditions of the population enrolled in this study. No specific safety concern was observed. Ocular treatment-emergent surgeries in the study eye were reported in 6 (3.6%) and 20 (4.1%) participants in the 2q8 and Pooled HD groups, respectively. The most frequent surgery was Cataract operation (3 [1.8%], 10 [3.0%], and 2 [1.2%] participants in the 2q8, HDq12, and HDq16 groups, respectively). Fellow Eye Ocular treatment-emergent surgeries in the fellow eye were reported in 15 (9.0%) and 53 (10.8%) participants in the 2q8 and Pooled HD groups, respectively. The most frequent surgery across all treatment groups was Retinal laser coagulation (5 [3.0%], 15 [4.6%], and 7 [4.3%] participants in the 2q8, HDq12, and HDq16 groups, respectively).

Non-ocular Treatment-Emergent Surgeries Non-ocular treatment-emergent surgeries were reported in 38 (22.8%) and 72 (14.7%) participants in the 2q8 and Pooled HD groups, respectively (Post-text Table 14.3.3.3a). The most frequent treatment-emergent surgeries were Tooth extraction (4 [2.4%], 3 [0.9%], and 2 [1.2%] participants in the 2q8, HDq12, and HDq16 groups, respectively); Catheterisation cardiac (3 [1.8%], 4 [1.2%], and 0 participants in the 2q8, HDq12, and HDq16 groups, respectively); and Coronary artery bypass (3 [1.8%], 2 [0.6%], and 2 [1.2%] in the 2q8, HDq12, and HDq16 groups, respectively).

At 48 weeks, PHOTON met the primary endpoints of non-inferiority of aflibercept 8 mg to EYLEA, with BCVA improvements from baseline demonstrated across dosing groups (all p=≤0.003). The EYLEA outcomes in DME were consistent with previous clinical trial experience. In the every 16-week dosing regimen group, 89% of DME patients in PHOTON maintained this dosing interval with an average of 5 injections in the first year. In the every 12-week dosing regimen groups, 91% of DME patients in PHOTON maintained this dosing interval with an average of 6 injections in the first year. In a pooled analysis of aflibercept 8 mg dosing groups, 93% of DME patients in PHOTON maintained 12-week dosing or longer.

Key efficacy findings at 48 weeks are set forth in Table 1-18.

**Table 1-18. Key 48 Week Efficacy Findings**

| | **High-dose aflibercept** | **High-dose aflibercept** | **EYLEA** |
|---|---|---|---|
| | **12-week regimen** | **16-week regimen** | **8-week regimen** |
| **PHOTON (DME)** | **n=328** | **n=163** | **n=167** |
| Mean BCVA improvement, primary endpoint | 8.8 letters | 7.9 letters | 9.2 letters |
| Non-inferiority p-value | <0.0001 | 0.0031 | N/A |
| Absolute BCVA | 72.6 letters | 69.8 letters | 71.0 letters |
| Patients maintained on dosing interval | 91% | 89% | N/A |
| Patients with ≥2-step DRSS, key secondary endpoint | 29%* | 20%* | 27% |

| | | | |
|---|---|---|---|
| DRSS: diabetic retinopathy severity scale; N/A: not applicable *the 12-week high-dose aflibercept group met the non-inferiority margin of 15%, while the 16-week group did not. | | | |

The safety of high-dose aflibercept was similar to EYLEA and consistent with the safety profile of EYLEA from previous clinical trials. There were no new safety signals for high-dose aflibercept and EYLEA, and no cases of retinal vasculitis, occlusive retinitis or endophthalmitis. Comparing pooled data for the 12- and 16-week high-dose aflibercept groups to the EYLEA groups, the following rates were observed:
- Serious ocular adverse events (AE): 0.6% versus 0.6% in PHOTON.
- Intraocular inflammation: 0.8% versus 0.6% in PHOTON.
- Patients meeting intraocular pressure criteria: 3.7% versus 2.4% in PHOTON.
- Serious non-ocular AEs: 14.7% versus 15.6% in PHOTON.

### Results at Week 60

This study was conducted at 138 centers that randomized participants various countries. A total of 970 participants were screened; 310 of them were screen failures with failure to meet inclusion/exclusion criteria being the most frequent reason for screen failure. Overall, 660 participants were randomized as displayed in Table 1-19. Most participants in each of the 3 groups (2q8: 92.8%, HDq12: 87.8%, and HDq16: 92.7%) completed their week 60 analysis visit (Table 1-19). Numbers of participants who discontinued the study with reasons for discontinuation by treatment group are presented in Table 1-19. The most common reasons for discontinuation were death and withdrawal of consent by participant.

**Table 1-19. Summary of Participant Disposition through Week 60 (All Randomized Participants)**

| | **2q8 (N=167)** | **HDq12 (N=329)** | **HDq16 (N=164)** | **All HD (N=493)** | **Total (N=660)** |
|---|---|---|---|---|---|
| **Week 48** | 157 (94.0%) | 300 (91.2%) | 156 (95.1%) | 456 (92.5%) | 613 (92.9%) |
| Number of patients who completed Week 48 | | | | | |
| Number of patients who discontinued prior to Week 48 | 10 (6.0%) | 29 (8.8%) | 8 (4.9%) | 37 (7.5%) | 47 (7.1%) |

| *Reasons for discontinuation prior to Week 48* | | | | | |
|---|---|---|---|---|---|
| Noncompliance with protocol by the subject | 1 (0.6%) | 0 | 0 | 0 | 1 (0.2%) |
| Adverse event | 0 | 4 (1.2%) | 1 (0.6%) | 5 (1.0%) | 5 (0.8%) |
| Decision by the investigator/sponsor | 0 | 4 (1.2%) | 1 (0.6%) | 5 (1.0%) | 5 (0.8%) |
| Withdrawal of consent by subject | 4 (2.4%) | 7 (2.1%) | 2 (1.2%) | 9 (1.8%) | 13 (2.0%) |
| Lost to follow-up | 1 (0.6%) | 5 (1.5%) | 1 (0.6%) | 6 (1.2%) | 7 (1.1%) |
| Death | 4 (2.4%) | 9 (2.7%) | 3 (1.8%) | 12 (2.4%) | 16 (2.4%) |
| Due to COVID-19 | 0 | 0 | 0 | 0 | 0 |
| **Week 60** | 155 (92.8%) | 289 (87.8%) | 152 (92.7%) | 441 (89.5%) | 596 (90.3%) |
| Number of patients who completed Week 60 | | | | | |
| Number of patients who discontinued prior to Week 60 | 12 (7.2%) | 40 (12.2%) | 12 (7.3%) | 52 (10.5%) | 64 (9.7%) |

| *Reasons for discontinuation prior to Week 60* | | | | | |
|---|---|---|---|---|---|
| Noncompliance with protocol by the subject | 1 (0.6%) | 1 (0.3%) | 0 | 1 (0.2%) | 2 (0.3%) |
| Adverse event | 0 | 4 (1.2%) | 2 (1.2%) | 6 (1.2%) | 6 (0.9%) |
| Decision by the investigator/sponsor | 0 | 6 (1.8%) | 2 (1.2%) | 8 (1.6%) | 8 (1.2%) |
| Withdrawal of consent by subject | 4 (2.4%) | 12 (3.6%) | 2 (1.2%) | 14 (2.8%) | 18 (2.7%) |
| Lost to follow-up | 2 (1.2%) | 8 (2.4%) | 2 (1.2%) | 10 (2.0%) | 12 (1.8%) |
| Death | 5 (3.0%) | 9 (2.7%) | 4 (2.4%) | 13 (2.6%) | 18 (2.7%) |
| Due to COVID-19 | 0 | 0 | 0 | 0 | 0 |

| | | | | | |
|---|---|---|---|---|---|
| Abbreviations: 2q8=Aflibercept 2 mg administered every 8 weeks after 5 initial injections at 4-week intervals; HDq12=High dose aflibercept 8 mg administered every 12 weeks after 3 initial injections at 4-week intervals; HDq16=High dose aflibercept 8 mg administered every 16 weeks after 3 initial injections at 4-week intervals; All HD=Pooled HDq12 and HDq16 groups; COVID-19=Coronavirus Disease 2019. The percentage was based on the number of patients in each treatment group as denominator. Definition of completed Week 48 = did not answer NO to the question "Did the subject complete the study?" on the "Study Completion" form prior to Week 48 visit. Definition of completed Week 60 = did not answer NO to the question "Did the subject complete the study?" on the "Study Completion" form prior to Week 60 visit. | | | | | |

### Protocol Deviations

A summary of important protocol deviations by treatment group through week 48 is presented in Table 1-20. No additional important protocol deviations were identified between week 48 and week 60 database locks. Overall, there were 36 important protocol deviations reported for 36 participants. The proportion of participants with important deviations was similar across all treatment groups. The most common important protocol deviation was initiation of study procedures without re-consenting participants to the amended informed consent form (ICF) (17 participants overall), followed by initiation of study procedures without consenting/prior to consenting of participants to the ICF (9 participants overall). All other important protocol deviations were reported in ≤ 5 participants in any treatment group and involved inclusion/exclusion criteria that were not met (Table 1-20).

**Table 1-20. Summary of Important Protocol Deviations Through Week 48 (All Randomized Participants)**

| | | **2q8 (N=167)** | **HDq12 (N=329)** | **HDq16 (N=164)** | **All HD (N=493)** | **Total (N=660)** |
|---|---|---|---|---|---|---|
| Number of Important Protocol Deviations | | 7 | 18 | 11 | 29 | 36 |
| Patients with Any Important Protocol Deviation Type of Important Protocol Deviation | | 7 (4.2%) | 18 (5.5%) | 11 (6.7%) | 29 (5.9%) | 36 (5.5%) |
| Subject did not re-consent to amended ICF and study procedures initiated (never signed amended ICF or signed after procedure) | | 4 (2.4%) | 6 (1.8%) | 7 (4.3%) | 13 (2.6%) | 17 (2.6%) |
| | Ex #07 met but subject randomized. ^{a} | 3 (1.8%) | 2 (0.6%) | 0 | 2 (0.4%) | 5 (0.8%) |
| | Ex #08 met but subject randomized. ^{b} | 0 | 1 (0.3%) | 0 | 1 (0.2%) | 1 (0.2%) |
| | Inc #03 not met but subject randomized. ^{c} | 0 | 3 (0.9%) | 1 (0.6%) | 4 (0.8%) | 4 (0.6%) |
| Subject did not sign ICF and study procedures were initiated (never signed ICF or signed after procedure performed) | | 0 | 6 (1.8%) | 3 (1.8%) | 9 (1.8%) | 9 (1.4%) |

| | | | | | | |
|---|---|---|---|---|---|---|
| Abbreviations: 2q8=Aflibercept 2 mg administered every 8 weeks after 5 initial injections at 4-week intervals; HDq12=8 mg aflibercept administered every 12 weeks after 3 initial injections at 4-week intervals; HDq16=8 mg aflibercept administered every 16 weeks after 3 initial injections at 4-week intervals; All HD=Pooled HDq12 and HDq16 groups; BCVA =best corrected visual acuity; DME= diabetic macular edema; ETDRS= Early Treatment Diabetic Retinopathy Study; Ex= exclusion criterion; ICF= informed consent form; Inc= inclusion criterion; IVT= intravitreal. The percentage for each analysis set was based on the number of randomized patients in each treatment group as denominator. a Exclusion criterion #7: Prior use of intraocular or periocular corticosteroids in study eye within 16 weeks/112 days of screening or ILUVIEN^{®} or OZURDEX^{®} IVT implants at any time b Exclusion criterion #8: History of vitreoretinal surgery (including scleral buckle) in the study eye c Inclusion criterion #3: Subject didn't satisfy BCVA ETDRS score of 78-24 (Snellen equivalent of 20/32 - 20/320) in study eye with decreased vision determined to be result of DME | | | | | | |

In addition to the above-mentioned important deviations, the following minor deviations regarding eligibility criteria were also reported:
- 1 participant in HDq16 met exclusion criterion #01- Evidence of macular edema due to any cause other than diabetes mellitus in the fellow eye;
- 5 participants met exclusion criterion #28- Uncontrolled diabetes mellitus as defined by HbA1c > 12% (3 in 2q8, 1 in HDq12, 1 in HDq16);
   o All 5 participants had values undetermined at baseline.
- 52 participants met exclusion criterion #29- Uncontrolled BP (systolic > 160 mmHg or diastolic > 95 mmHg); treated with up to 3 agents known to have anti-hypertensive effects for arterial hypertension to achieve adequate blood pressure control; changes in BP medications must be stable for 12 weeks (84 days prior to screening)
   ∘ 26 participants were randomized despite having SBP or DBP above of the protocol specified range (3 in 2q8, 14 in HDq12, 9 in HDq16);
   ∘ 25 participants were randomized despite being treated with > 3 BP medication (7 in 2q8, 10 in HDq12, 7 in HDq16);
   ∘ 1 participant in 2q8 group was randomized despite having BP medication regimen changed within 12 weeks of screening

This study was not substantially impacted by the COVID-19 pandemic. A total of 18 visits in 17 participants were not performed, 1 visit was conducted as hybrid visit (partial face to face and remote visit) due to participants not being able to travel due to COVID-19 or participants/guardian under quarantine due to COVID-19. None of the participants withdrew due to COVID-19.

### Visual Outcomes

Both HDq12 and HDq16 demonstrated non-inferiority to 2q8 with respect to this key secondary endpoint (change from baseline in BCVA at week 60) using the non-inferiority margin of 4 letters with LSₘₑₐₙ change from baseline in BCVA of 8.52 letters (HDq12) and 7.64 letters (HDq16) versus 9.40 letters in the 2q8 group (Table 1-21). The differences in LSₘₑₐₙ changes from baseline in BCVA (95% CI) were -0.88 (-2.67, 0.91) and -1.76 (-3.71, 0.19) for HDq12 and HDq16, respectively, compared to 2q8. The p-values for the non-inferiority test at a margin of 4 letters were 0.0003 for HDq12 vs. 2q8, and 0.0122 for HDq16 vs. 2q8. The lower confidence limits were greater than -4, allowing the conclusion of non-inferiority at the week 60 timepoint.

The mean changes from baseline in BCVA measured by the ETDRS letter score by visit using OC, are graphically displayed in Figure 25A; the corresponding LSₘₑₐₙ changes from baseline in BCVA using MMRM in the FAS, are displayed in Figure 25B. The mean increases in BCVA over time were similar across all groups and minor numerical differences were not considered clinically relevant.

The mean Change from baseline in BCVA score (ETDRS Letters) in the study eye through week 60, OC (Full Analysis Set) is set forth graphically in Figure 25A.

Results of the analysis in the PPS were consistent with the FAS and LSₘₑₐₙ change from baseline in BCVA by visit in the PPS was also consistent with the FAS.

**Table 1-21. Key Secondary Endpoint -- Change from Baseline in BCVA (ETDRS Letters) at Week 60 in the Study Eye, MMRM (Full Analysis Set)**

| | | | | **Number of patients with Week 60 data** | | | | | | |
|---|---|---|---|---|---|---|---|---|---|---|
| **Treatment** | **LS Mean (SE) change from BL** | **Mean (SD) change from BL** | **BL Mean** | | **DF** | **Contrast [a]** | **t-value** | **1-sided NI p-value [b]** | **1-sided superiority p-value** | **Estimate for contrast and 2-sided 95% CI [c]** |
| HDq12 (N=328) | 8.52 (0.63) | 9.05 (9.27) | 63.63 | 252 | 342.4 | HDq12 - 2q8 | 3.4242 | 0.0003 | 0.8325 | -0.88 (-2.67, 0.91) |
| HDq16 (N=163) | 7.64 (0.75) | 7.96 (9.14) | 61.44 | 138 | 315.5 | HDq16 - 2q8 | 2.2625 | 0.0122 | 0.9619 | -1.76 (-3.71, 0.19) |
| 2q8 (N=167) | 9.40 (0.77) | 9.62 (9.58) | 61.47 | 133 | | | | | | |

| | | | | | | | | | | |
|---|---|---|---|---|---|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. BL=baseline; CI=confidence interval; CRT= Central retinal thickness (or, central subfield retinal thickness); DF=degrees of freedom; DME=diabetic macular edema; NI=non-inferiority; LS=least square; SAP=statistical analysis plan; SE=standard error; SD=standard deviation A mixed model for repeated measurements (MMRM) was used with baseline BCVA measurement as a covariate, treatment group and the stratification variables (geographic region [Japan vs. Rest of World]; baseline CRT from reading center [<400µm vs. ≥400µm], prior treatment for DME per EDC; [yes vs. no]) as fixed factors, and terms for the interaction between baseline and visit and the interaction between treatment and visit. A Kenward-Roger approximation was used for the denominator degrees of freedom. [a] The contrast also included the interaction term for treatment x visit. [b] p-value for the 1-sided non-inferiority (NI) test at a margin of 4 letters. [c] Estimate based on the MMRM model, was computed for the differences of HDq12 minus 2q8 and HDq16 minus 2q8, respectively with 2-sided 95% CIs. | | | | | | | | | | |

The proportion of participants who gained ≥ 15 letters in BCVA from baseline to week 60 was 21.5% and 16.0% in the HDq12 and HDq16 groups, respectively, compared with 26.1% in the 2q8 group (Table 1-22). The lower values observed for this parameter are potentially due to a ceiling effect created by inclusion of participants with baseline BCVA up to 78 letters. Although lower values were seen in the HDq16 group (16.0% compared to >20.0% in 2q8), considering non-inferiority was achieved between HDq16 and 2q8 for the primary endpoint, the overall picture of letters gained/lost among the treatment groups must be taken into consideration.

Sensitivity analysis for the proportion of participants who gained ≥ 15 letters in BCVA from baseline at week 60 using OC was consistent with the LOCF analysis.

**Table 1-22. Proportion of Participants who Gained ≥ 15 Letters in BCVA from Baseline at Week 60 (LOCF) (Full Analysis Set)**

| **Treatment** | **Patients with ≥ 15 Letters gain in BCVA from baseline to Week 48, n (%)** | **Adjusted Difference (%) (95% CI)^{a}** | **CMH test**^{b} **p-value** |
|---|---|---|---|
| HDq12 (N=328) | 70/326 (21.5%) | -5.01 ( -13.04 , 3.02) | 0.2112 |
| HDq16 (N=163) | 26/163 (16.0%) | -10.78 ( -19.27 , -2.29) | 0.0143 |
| 2q8 (N=167) | 43/165 (26.1%) | | |

| | | | |
|---|---|---|---|
| Abbreviations: 2q8=Aflibercept 2 mg administered every 8 weeks after 5 initial injections at 4-week intervals; HDq12=High dose aflibercept 8 mg administered every 12 weeks after 3 initial injections at 4-week intervals; HDq16=High dose aflibercept 8 mg administered every 16 weeks after 3 initial injections at 4-week intervals. BCVA=best corrected visual acuity; CI=confidence interval; CMH=Cochran-Mantel-Haenszel; ICE=intercurrent events; LOCF=last observation carried forward; N=number of participants. LOCF: the last observation prior to an ICE defined for the primary estimand was used to impute subsequent and/or missing or non-gradable data. Missing data were not included in the denominator. a. Difference with CI was calculated using Mantel-Haenszel weighting scheme adjusted for stratification factors (baseline CRT (from reading center) [<400 µm, ≥400 µm], prior DME treatment [yes, no], geographical region [Rest of world, Japan]). b. Nominal p-value for the 2-sided CMH superiority test. | | | |

The proportion of participants who gained or lost ≥ 5, ≥ 10, or ≥ 15 letters from baseline through week 60 is presented in Table 1-23. Across all treatment groups, more participants gained letters, with the greatest proportion gaining ≥ 5 letters (approximately 64% to 72% across all treatment groups). A numerically lower proportion of participants in the HDq12 and HDq16 groups gained ≥ 10 letters or ≥ 15 letters compared to the 2q8 group. Few participants (approximately 3% to 6%) lost 5 or more letters through week 60 regardless of treatment group.

**Table 1-23. Proportion of Participants who Gained or Lost ≥ 5, 10, or 15 Letters in BCVA from Baseline by Visit through Week 60 (LOCF) (Full Analysis Set)**

| | | **2q8 (N=167)** | **HDq12 (N=328)** | **HDq16 (N=163)** |
|---|---|---|---|---|
| Gained >= 5 letters | Week 60 | 119/165 (72.1%) | 227/326 (69.6%) | 105/163 (64.4%) |
| Gained >= 10 letters | Week 60 | 82/165 (49.7%) | 133/326 (40.8%) | 56/163 (34.4%) |
| Gained >= 15 letters | Week 60 | 43/165 (26.1%) | 70/326 (21.5%) | 26/163 (16.0%) |
| Lost >= 5 letters | Week 60 | 10/165 (6.1%) | 21/326 (6.4%) | 5/163 (3.1%) |
| Lost >= 10 letters | Week 60 | 4/165 (2.4%) | 11/326 (3.4%) | 2/163 (1.2%) |
| Lost >= 15 letters | Week 60 | 1/165 (0.6%) | 7/326 (2.1%) | 1/163 (0.6%) |

| | | | | |
|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; BCVA= best corrected visual acuity; ICE= intercurrent events; LOCF= last observation carried forward; SAP= statistical analysis plan. LOCF: the last observation prior to an ICE defined for the primary estimand was used to impute subsequent and/or missing data. Missing data were not included in the denominator. | | | | |

The proportion of participants who achieved ≥ 69 letters in BCVA (≥ 20/40 Snellen equivalent) at week 60 was similar across treatment groups (60.6 to 64.7% participants). Sensitivity analyses for the proportion of participants who achieved ≥ 69 letters in BCVA at week 60 using OC were consistent with the LOCF analysis. See Table 1-24.

**Table 1-24. Proportion of Patients with BCVA ≥ 69 letters at Week 60 (LOCF) (FAS)**

| **Treatment** | **Patients with BCVA >= 69 letters at Week 60, n (%)** | **Adjusted Difference (%) (95% CI) ^{a}** | **CMH test ^{b} p-value** |
|---|---|---|---|
| HDq12 (N=328) | 211/326 (64.7%) | 4.34 ( -4.72, 13.40) | 0.3479 |
| HDq16 (N=163) | 101/163 (62.0%) | 1.63 ( -8.91, 12.17) | 0.7620 |
| 2q8 (N=167) | 100/165 (60.6%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. BCVA: best corrected visual acuity; CMH=Cochran-Mantel-Haenszel ; DME= diabetic macular edema; FAS= Full analysis set; LOCF: the last observation prior to an ICE defined for the primary estimand was used to impute subsequent and/or missing data; N=number of participants. a. Difference with confidence interval (CI) was calculated using Mantel-Haenszel weighting scheme adjusted for stratification factors (baseline CRT (from reading center) [<400µm, >=400 µm], prior DME treatment [yes, no], geographical region [Rest of world, Japan]) b. p-value for the two-sided Cochran-Mantel-Haenszel (CMH) superiority test. | | | |

Missing data were not included in the denominator.

The mean values of BCVA score averaged from week 48 to week 60 were similar across treatment groups, and the change from baseline was similar across treatment groups (Table 1-25).

Sensitivity analysis for the BCVA as measured by ETDRS letter score averaged over the period from week 48 to week 60 using LOCF analysis in the FAS was consistent with the OC analysis.

**Table 1-25. Summary of Averaged BCVA Score: Week 48 to Week 60 (OC) (Full Analysis Set)**

| | | **Value at Visit** | | | | | | | | **Change from Baseline** | | | | | | | |
|---|---|---|---|---|---|---|---|---|---|---|---|---|---|---|---|---|---|
| **Treatment** | **Visit** | **n** | **Mean** | **SD** | **SE** | **Q1** | **Median** | **Q3** | **Min , Max** | **n** | **Mean** | **S D** | **S E** | **Q1** | **Median** | **Q3** | **Min, Max** |
| 2q8 (N=167) | BASELINE | 167 | 61.5 | 11.2 2 | 0.87 | 54.0 | 63.0 | 70.0 | 24, 78 | | | | | | | | |
| | WEEK 48 TO 60 | 150 | 71.2 | 11.7 1 | 0.96 | 65.0 | 72.0 | 79.8 | 20, 88 | 150 | 9.3 | 8.81 | 0.72 | 4.3 | 9.3 | 14. 8 | -30, 45 |
| HDq12 (N=328) | BASELINE | 328 | 63.6 | 10.1 0 | 0.56 | 57.0 | 65.0 | 72.0 | 27, 79 | | | | | | | | |
| | WEEK 48 TO 60 | 277 | 72.5 | 10.7 2 | 0.64 | 66.0 | 74.3 | 81.0 | 30, 94 | 277 | 8.7 | 8.61 | 0.52 | 3.8 | 8.0 | 13. 0 | -22, 40 |
| HDq16 (N=163) | BASELINE | 163 | 61.4 | 11.7 6 | 0.92 | 55.0 | 64.0 | 71.0 | 29, 78 | | | | | | | | |
| | WEEK 48 TO 60 | 149 | 69.5 | 12.9 5 | 1.06 | 62.5 | 73.3 | 79.3 | 20, 89 | 149 | 7.6 | 8.35 | 0.68 | 3.0 | 7.5 | 11. 3 | -14, 40 |

| | | | | | | | | | | | | | | | | | |
|---|---|---|---|---|---|---|---|---|---|---|---|---|---|---|---|---|---|
| Abbreviations: 2q8= Aflibercept 2 mg administered every 8 weeks after 5 initial injections at 4-week intervals, HDq12= High dose aflibercept 8 mg administered every 12 weeks after 3 initial injections at 4-week intervals; HDq16= High dose aflibercept 8 mg administered every 16 weeks after 3 initial injections at 4-week intervals, ICE= intercurrent events; max= maximum; min= minimum; N,n= number of participants; Q1= quartile 1; Q3= quartile 3; SAP= statistical analysis plan; SD= standard deviation; SE= standard error OC: observations after an intercurrent event (ICE) defined for the primary estimand were excluded. | | | | | | | | | | | | | | | | | |

### Visual Outcomes Sub-group Analysis

The treatment effects of HDq12 and HDq16 versus 2q8 on the primary endpoint, the mean change from baseline in best-corrected visual acuity (BCVA) at Week 48, were evaluated by baseline demographics (sex, age, race, and ethnicity).

Mean BCVA change from baseline at Week 48 with 2q8, HDq12, and HDq16, respectively, was +8.7, +8.4, and +8.3 letters in male patients (n=401); +9.8, +9.6, and +7.2 letters in female patients (n=257); +13.0, +10.2, and +11.1 letters in patients aged <55 years (n=144); +10.3, +8.0, and +7.1 letters in patients aged ≥55-<65 years (n=225); +6.9, +9.2, and +7.0 letters in patients aged ≥65-<75 years (n=218). The results were generally comparable by race (White [n=471]: +9.3, +9.5, and +8.3 letters; Asian [n=101]: +7.3, +5.9, and +6.6 letters) and ethnicity (Hispanic or Latino [n=119]: +8.9, +8.3, and +7.6 letters; non-Hispanic or Latino [n=525]: +9.4, +8.8, and +7.9 letters). Select subgroups (≥75 years and Black or African American) could not be evaluated due to small sample size.

Aflibercept 8 mg achieved meaningful BCVA gains from baseline at Week 48 in patients with DME across evaluable subgroups of sex, age, race, and ethnicity.

### Diabetic Retinopathy Severity Score (DRSS)

The proportion of participants with ≥ 2-step improvement in DRSS score was 25.7%, 24.6%, and 20.7% at week 12 and 29.1%, 31.3%, and 22.2% at week 60 in the 2q8, HDq12, and HDq16 groups, respectively. In CMH-weighted estimates, the adjusted difference (95% CI) was 1.87(-6.88, 10.63) for HDq12 and -7.47 (-17.05, 2.12) for HDq16, respectively, versus 2q8 (Table 1-26). Sensitivity analysis using OC was performed and was consistent with the primary analysis.

**Table 1-26. Exploratory Endpoint - Proportion of Participants with a ≥ 2-Step Improvement from Baseline in DRSS at Week 60 (LOCF) (Full Analysis Set)**

| **Treatment** | **Patients with a ≥ 2-step Improvement From Baseline in DRSS, n (%)** | **Adjusted Difference (%)** |
|---|---|---|
| | | **2-sided (95% CI) ^{a}** |
| HDq12 (N=328) | 97/310 (31.3%) | 1.87 ( -6.88, 10.63) |
| HDq16 ( N=163 ) | 34/153 (22.2%) | -7.47 ( -17.05, 2.12) |
| 2q8 ( N=167 ) | 46/158 (29.1%) | |

| | | |
|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI=confidence interval; CRT= central retinal thickness; DRSS=Diabetic Retinopathy Severity Scale; N=number of participants; SAP = Statistical analysis plan LOCF= the last observation prior to an ICE defined for the primary estimand was used to impute subsequent and/or missing or non-gradable data. Participants were considered as non-responders if all post-baseline measurements were missing or non-gradable. a. Difference with confidence interval (CI) was calculated using Mantel-Haenszel weighting scheme adjusted for stratification factors (baseline CRT (from reading center) [<400 µm, ≥400 µm], prior DME treatment [yes, no], geographical region [Rest of world, Japan]). The non-inferiority margin was set at 15%. Missing or ungradable baseline was not included in the denominator. | | |

The proportion of participants with a ≥ 3-step improvement in DRSS at week 60 was 15.2% and 10.5% in the HDq12 and HDq16 groups, respectively, compared with 17.7% in the 2q8 group (Table 1-27).

Sensitivity analyses for the proportion of participants with a ≥ 3-step improvement in DRSS score at week 60 using OC were consistent with the LOCF analysis.

**Table 1-27. Proportion Analysis of Participants with a ≥3-step Improvement from Baseline in DRSS at Week 60 (LOCF) (Full Analysis Set)**

| **Treatment** | **Patients with** a **≥ 3-step improvement from baseline in DRSS at Week 60, n (%)** | **Adjusted Difference (%) (95% CI) ^{a}** | **CMH test** ^{b} **p-value** |
|---|---|---|---|
| HDq12 (N=328) | 47/310 (15.2%) | -2.73 (-9.90, 4.44) | 0.4412 |
| HDq16 (N=163) | 16/153 (10.5%) | -7.34 (-15.16, 0.47) | 0.0660 |
| 2q8 (N=167) | 28/158 (17.7%) | | |

| | | | |
|---|---|---|---|
| 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; DRSS = Diabetic Retinopathy Severity Scale. LOCF: the last observation prior to an ICE defined for the primary estimand was used to impute subsequent and/or missing or non-gradable data. Patients were considered as non-responders if all post-baseline measurements were missing or non-gradable. Missing or ungradable baseline was not included in the denominator. a. Difference with confidence interval (CI) was calculated using Mantel-Haenszel weighting scheme adjusted for stratification factors (baseline CRT (from reading center) [<400µm, >=400 µm], prior DME treatment [yes, no], geographical region [Rest of world, Japan]) b. p-value for the two-sided Cochran-Mantel-Haenszel (CMH) superiority test | | | |

### Retinal Fluid

The proportion of participants without fluid (no IRF and no SRF) at the foveal center (as assessed by the central reading center) at week 60 was 61.8% and 58.0% in the HDq12 and HDq16 groups, respectively, compared with 68.5% in the 2q8 group. Sensitivity analyses for the proportion of participants without fluid (no IRF and no SRF) at the foveal center at week 60 using OC were consistent with the LOCF analysis. See Table 1-28.

**Table 1-28. Proportion of Participants without Fluid (no IRF and no SRF) at the Foveal Center at Week 60 (LOCF) (Full Analysis Set)**

| **Treatment** | **Patients without fluid, n(%)** | **Adjusted Difference (%) (95% CI)^{a}** | **CMH test^{b} p-value** |
|---|---|---|---|
| HDq12 (N=328) | 201/325 (61.8%) | -5.98 ( -14.71 , 2.75) | 0.1878 |
| HDq16 (N=163) | 94/162 (58.0%) | -9.88 ( -20.31 , 0.56) | 0.0647 |
| 2q8 (N=167) | 113/165 (68.5%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI=confidence interval; CMH=Cochran-Mantel-Haenszel; DME= diabetic macular edema; ICE=intercurrent events; IRF=intraretinal fluid; LOCF=last observation carried forward; N=number of participants; SRF=subretinal fluid. LOCF= the last observation prior to an ICE defined for the primary estimand was used to impute subsequent and/or missing or non-gradable data. Missing or undetermined data were not included in the denominator. a. Difference with CI was calculated using Mantel-Haenszel weighting scheme adjusted for stratification factors (baseline CRT (from reading center) [<400 µm, ≥400 µm], prior DME treatment [yes, no], geographical region [Rest of world, Japan]). b. Nominal p-value for the 2-sided CMH superiority test. | | | |

The proportion of participants without fluid (no IRF and no SRF) in the center subfield at week 60 was 23.1% and 15.4% in the HDq12 and HDq16 groups, respectively, compared with 29.7% in the 2q8 group (Table 1-29).

Sensitivity analyses for the subset of participants without fluid (no IRF and no SRF) in the center subfield at week 60 using OC were consistent with the LOCF analysis.

**Table 1-29. Proportion of Participants without Fluid (no IRF and no SRF) at the Central Subfield at Week 60 (LOCF) (Full Analysis Set)**

| **Treatment** | **Patients without fluid, n (%)** | **Adjusted Difference (%) (95% CI)** ^{a} | **CMH test** ^{b} **p-value** |
|---|---|---|---|
| HDq12 (N=328) | 75/325 (23.1%) | -6.45 ( -14.78, 1.87) | 0.1217 |
| HDq16 (N=163) | 25/162 (15.4%) | -14.19 ( -23.03, -5.36) | 0.0021 |
| 2q8 (N=167) | 49/165 (29.7%) | | |

| | | | |
|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; CI=confidence interval; CMH=Cochran-Mantel-Haenszel; CRT = central retinal thickness; DME = diabetic macular edema; ICE= intercurrent events; IRF=intraretinal fluid; LOCF=last observation carried forward; N=number of participants; SAP = Statistical analysis plan; SRF=subretinal fluid. LOCF: the last observation prior to an ICE defined for the primary estimand was to be used to impute subsequent and/or missing data Missing or undetermined data were not included in the denominator. a. Difference with CI was calculated using Mantel-Haenszel weighting scheme adjusted for stratification factors (baseline CRT (from reading center) [<400µm, >=400 µm], prior DME treatment [yes, no], geographical region [Rest of world, Japan]). b. p-value for the two-sided CMH superiority test. | | | |

### Central Retinal Thickness (CRT)

Overall, the LSₘₑₐₙ (SE) change from baseline in CRT (as assessed by the central reading center) at week 60 was -181.95 (6.09) and -166.26 (8.56) in the HDq12 and HDq16 groups, respectively, compared with -194.16 (7.15) in the 2q8 group (Table 1-30).

The mean changes from baseline in CRT using OC are graphically displayed in Figure 26A; the corresponding LSₘₑₐₙ changes from baseline in CRT using MMRM in the FAS are displayed in Figure 26B. Both the mean and LSₘₑₐₙ changes in CRT over time were similar across all groups. Although reductions from baseline in CRT were consistently observed at all timepoints, some fluctuation in mean CRT was seen in all treatment groups with attenuation in magnitude over the course of 60 weeks. The small fluctuations that are observed in all treatment groups over time are not considered to be clinically relevant given the demonstration of the non-inferiority in visual acuity.

Sensitivity analyses for change from baseline in CRT at week 60 using LOCF were consistent with the MMRM analysis.

**Table 1-30. Statistical Analysis of Change from Baseline in Central Retinal Thickness (microns) at Week 60 (MMRM)(FAS)**

| | LSₘₑₐₙ **(SE) change from BL** | **Mean (SD) change from BL** | **BL Mean** | **Number of patients with Week 60 data** | **DF** | **Contrast ^{a}** | **t-value** | **p-value ^{b}** | **Estimate for contrast and 2-sided 95% CI ^{c}** |
|---|---|---|---|---|---|---|---|---|---|
| HDq12 (N=328) | -181.95 (6.09) | -176.24 (144.71) | 449.15 | 251 | 346.8 | HDq12 - 2q8 | 1.5051 | 0.1332 | 12.21 (-3.74, 28.16) |
| HDq16 (N=163) | -166.26 (8.56) | -167.18 (127.18) | 460.32 | 137 | 283.4 | HDq16 - 2q8 | 2.7685 | 0.0060 | 27.90 (8.06, 47.74) |
| 2q8 (N=167) | -194.16 (7.15) | -191.31 (142.00) | 457.25 | 131 | | | | | |

| | | | | | | | | | |
|---|---|---|---|---|---|---|---|---|---|
| 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. CI = Confidence interval. CRT = Central retinal thickness. SE = Standard error. SD = Standard deviation. DF = Degrees of freedom. FAS = Full analysis set. LS = Least Square. BL = Baseline. MMRM = mixed model for repeated measurements. SAP = statistical analysis plan A mixed model for repeated measurements (MMRM) was used with baseline CRT measurement as a covariate, treatment group and the stratification variables (geographic region [Japan vs. Rest of World]; baseline CRT (from reading center) [<400µm vs. >=400µm], prior treatment for DME (per EDC) [yes vs. no]) as fixed factors, and terms for the interaction between baseline and visit and the interaction between treatment and visit. A Kenward-Roger approximation was used for the denominator degrees of freedom. An unstructured covariance structure was used to model the within-subject error. a. The contrast also included the interaction term for treatment x visit. b. p-value for the two-sided superiority test c. Estimate based on the MMRM model, was computed for the differences of HDq12 minus 2q8 and HDq16 minus 2q8, respectively with two-sided 95% CIs. | | | | | | | | | |

### Fluid Leakage

Overall, the proportion of participants without leakage on fluorescein angiography (as assessed by the central reading center) at week 60 (LOCF) was very low in all 3 treatment groups: 7.9% and 2.0% in the HDq12 and HDq16 groups, respectively, compared with 4.3% in the 2q8 group.

Sensitivity analyses for the proportion of participants without leakage on fluorescein angiography at week 60 using OC were consistent with the LOCF analysis.

A summary of the change from baseline in total area of fluorescein leakage within the ETDRS grid at week 60 is shown in Table 1-31.

**Table 1-31. Summary of the Change from Baseline in Total Area of Fluorescein Leakage within ETDRS Grid (mm²) at Week 60 (OC) (Full Analysis Set)**

| **Statistic** | **2q8 N=167** | **HDq12 N=328** | **HDq16 N=163** |
|---|---|---|---|
| Baseline n | 164 | 319 | 153 |
| Baseline mean (SD) | 24.6 (13.20) | 24.4 (13.22) | 24.6 (11.73) |
| Week 60 n | 112 | 202 | 110 |
| Mean (SD) change from baseline at week 60 | -14.4 (12.89) | -13.9 (13.54) | -12.0 (13.26) |
| Median change from baseline at week 60 | -12.3 | -13.6 | -12.6 |
| Min, Max | -86, 4 | -80, 57 | -37, 68 |

| | | | |
|---|---|---|---|
| 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals. ETDRS: Early Treatment Diabetic Retinopathy Study; OC: observations after an ICE defined for the primary estimand were excluded; SD; standard deviation. | | | |

### Safety

Overall, a similar proportion of participants had TEAEs in the HD groups, 74.7% (245 participants; HDq12) and 77.3% (126 participants; HDq16), compared to 73.7% (123 participants) in the 2q8 group. The proportions of participants with ocular TEAEs were similar across the groups and were 43.7% (73 participants), 44.8% (147 participants), and 44.8% (73 participants) in the 2q8, HDq12, and HDq16 groups, respectively. There were very few study-drug-related ocular and non-ocular TEAEs across all treatment groups. The proportion of participants with study conduct-related TEAEs and TEAEs related to 2 mg aflibercept in the fellow eye were minimally reported across groups (< 2.0% overall across groups). The proportion of participants with injection-procedure-related ocular TEAEs was similar across treatment groups (< 14% across groups). The majority of serious AEs reported were non-ocular TEAEs (19.2% [32 participants], 18.6% [61 participants], and 16.6% [27 participants] in the 2q8, HDq12 and HDq16 groups, respectively). One injection-procedure-related ocular serious TEAE (Intraocular pressure increased) in the study eye was reported and occurred in the HDq12 group (0.3%). There were no reported study-drug-related serious TEAEs, study-conduct-related serious TEAEs, or serious TEAEs related to 2 mg aflibercept in the fellow eye. Three (1.8%) participants in the 2q8 group, 9 (2.7%) participants in the HDq12 group, and 2 (1.2%) participants in the HDq16 group discontinued study drug due to TEAEs. Of these, 2 participants discontinued study drug due to ocular TEAEs (both in the HDq12 group).

Five deaths were reported in the 2q8 group (3.0%), 9 deaths in the HDq12 group (2.7%), and 4 deaths in the HDq16 group (2.5%). All deaths were considered unrelated to study treatment by the investigator.

The proportion of participants with treatment-emergent adjudicated Antiplatelet Trialists' Collaboration (APTC) events was low and generally similar across treatment groups: 3.6% (6 participants), 4.0% (13 participants), and 5.5% (9 participants) in the 2q8, HDq12, and HDq16 groups, respectively. A slightly higher frequency of participants reported Hypertension in the HDq16 group (17.2%; 28 participants) compared to the 2q8 group (13.8%; 23 participants) and the HDq12 group (12.8%; 42 participants); however, this was not interpreted as clinically meaningful as there was no apparent dose relationship (i.e., HDq16 versus HDq12).

There were no treatment-emergent nasal mucosal events reported through week 60.

Ocular TEAEs in the study eye were reported at similar frequencies in all 3 groups (29.3% [49 participants], 36.0% [118 participants], and 34.4% [56 participants] in the 2q8, HDq12, and HDq16 groups, respectively). No clinically meaningful differences were observed in type of TEAEs or their frequencies between the HD and 2q8 treatment groups, and reported events were consistent with the known safety profile of IVT aflibercept. Overall, ocular TEAEs in the fellow eye were reported in 52 (31.1%) participants in the 2q8 group, 91 (27.7%) participants in the HDq12 group, and 52 (31.9%) participants in the HDq16 group. All ocular TEAEs in the fellow eye were reported in < 6.0% of participants in each treatment group. The most frequent PTs were Cataract (4.2% [7 participants], 3.0% [10 participants], and 5.5% [9 participants] in the 2q8, HDq12, and HDq16 groups, respectively), Vitreous floaters (4.3%; 7 participants in the HDq16 group), Diabetic retinal oedema (3.4% [11 participants] in the HDq12 group), and Diabetic retinopathy (3.6% [6 participants] in the 2q8 group and 3.7% [6 participants] in the HDq16 group). Ocular TEAEs were generally balanced across the 3 treatment groups.

Non-ocular TEAEs were reported in a similar proportion of participants in the 2q8 group (57.5%; 96 participants) and the Pooled HD group (60.9%; 299 participants). The majority of the TEAEs were in the SOC of Infections and infestations; however, the most common TEAE was Hypertension. A slightly higher frequency of participants reported Hypertension in the HDq16 group (15.3%; 25 participants) compared to the 2q8 group (10.8%; 18 participants) and the HDq12 group (9.1%; 30 participants); however, this was not interpreted as clinically meaningful as there was no apparent dose relationship (i.e., HDq16 versus HDq12).

Other non-ocular TEAEs were reported ≤ 5.0% of participants in the 2q8 and the Pooled HD group except for COVID-19 (8.6%; 42 participants in the Pooled HD group)

Ocular study-drug-related TEAEs in the study eye were reported in 3 (1.8%) participants in the 2q8 group, 6 (1.8%) participants in the HDq12 group, and no participants in the HDq16 group. Intraocular pressure increased was the only PT (Preferred term) reported in more than 1 participant (3 [0.9%] participants in the HDq12 group). All ocular study-drug-related TEAEs in the study eye were reported in < 1.0% of participants. One non-ocular study drug-related TEAE was reported through week 60: Lacunar infarction reported in 1 (0.6%) participant in the HDq16 group. There were no non-ocular study-drug-related TEAEs reported through week 60 in the 2q8 or HDq12 groups.

Ocular IVT-injection-related TEAEs were reported in 16 (9.6%) participants in the 2q8 group, 42 (12.8%) participants in the HDq12 group, and 13 (8.0%) participants in the HDq16 group. Ocular IVT-injection-related TEAEs that were reported in > 2 participants in any of the 3 treatment groups included Conjunctival haemorrhage, Vitreous floaters, Eye pain, and Intraocularpressure increased which were reported in similar proportions of participants across the 3 treatment groups. All other ocular IVT-injection-related TEAEs in the study eye were reported in ≤ 2 participants in each group. Ocular IVT-injection-related TEAEs in the fellow eye through week 60 were reported in 5 (3.0%) participants in the 2q8 group, 7 (2.1%) participants in the HDq12 group, and 5 (3.1%) participants in the HDq16 group. Ocular IVT-injection-related TEAEs in the fellow eye were generally balanced between the 3 treatment groups.

Non-ocular IVT-injection-related TEAEs through week 60 were reported in 3 (0.6%) participants in the Pooled HD group. The TEAEs reported in the HD groups included Nausea, Vomiting, and Headache. No participants reported non-ocular IVT-injection-related TEAEs in the 2q8 group

The relationship of TEAEs to other study procedures were assessed by the masked investigator, and was a clinical decision based on all available information.

Study-conduct-related TEAEs were reported in 2 (0.6%) participants in the HDq12 group. These TEAEs were Conjunctival haemorrhage and Injection site irritation. No study-conduct-related TEAEs were reported in the 2q8 or HDq16 groups.

There were no ocular study-conduct-related TEAEs in the fellow eye through week 60 reported in any treatment group.

Non-ocular study-conduct-related TEAEs through week 60 were reported in 3 (1.8%) participants in the 2q8 group and 4 (0.8%) participants in the Pooled HD group. These TEAEs were Nausea, Vessel puncture site haematoma, Contrast media allergy, Post procedural pruritus, Rash, and Vein.

Once the fellow eye received 2-mg aflibercept treatment during the study, TEAEs and serious TEAEs were also assessed as related/not related to 2-mg aflibercept treatment in the fellow eye, assessed as related/not related to the study drug (delivered to the study eye), IVT injection, and other protocol-specified procedures.

No ocular TEAEs in the study eye related to 2-mg aflibercept in the fellow eye through week 60 were reported in any treatment group.

Ocular TEAEs in the fellow eye related to 2-mg aflibercept in the fellow eye through week 60 were reported in few participants, 2 (1.2%) participants in the 2q8 group, 1 (0.3%) participant in the HDq12 group, and 2 (1.2%) participants in the HDq16 group. These TEAEs were Conjunctival haemorrhage, Halo vision, and Intraocular pressure increased.

One non-ocular TEAE related to 2-mg aflibercept in the fellow eye was reported through week 60: Lacunar infarction was reported in 1 (0.6%) participant in the HDq16 group. The same event was also considered to be related to study drug.

No non-ocular TEAEs related to 2-mg aflibercept in the fellow eye were reported through week 60 in the 2q8 or HDq12 groups.

The majority of ocular TEAEs in the study eye were mild (22.8% [38 participants; 2q8 group], 26.2% [86 participants; HDq12 group], and 28.2% [46 participants; HDq16 group]) to moderate (6.0% [10 participants; 2q8 group], 9.1% [30 participants; HDq12 group], and 5.5% [9 participants; HDq16 group]). Severe ocular TEAEs in the study eye were reported in few participants, 1 (0.6%) participant in the 2q8 group, 2 (0.6%) participants in the HDq12 group, and 1 (0.6%) participant in the HDq16 group. The ocular TEAEs that were reported as being severe in the study eye were Cataract nuclear and Cataract subcapsular (reported by 1 participant in the 2q8 group), Cataract subcapsular and Retinal vascular disorder (reported by 1 participant each in the HDq12 group), and Retinal detachment and Vitreous haemorrhage (reported by 1 participant in the HDq16 group).

The majority of ocular TEAEs in the fellow eye were mild (22.2% [37 participants; 2q8 group], 21.0% [69 participants; HDq12 group], and 22.1% [36 participants; HDq16 group]) to moderate (7.2% [12 participants; 2q8 group], 5.8% [19 participants; HDq12 group], and 9.8% [16 participants; HDq16 group]). Severe ocular TEAEs in the fellow eye were reported in few participants, 3 (1.8%) participants in the 2q8 group, 3 (0.9%) participants in the HDq12 group, and no participants in the HDq16 group.

Severe ocular TEAEs in the fellow eye reported by the 3 participants in the 2q8 group were Cataract subcapsular, Cataract nuclear, Diabetic retinopathy, and Retinal artery occlusion (reported by 1 participant each); Diabetic retinopathy (reported by 1 participant) and Vitreous haemorrhage (reported by 3 participants) in the HDq12 group.

The majority of non-ocular TEAEs were mild (25.7% [43 participants; 2q8 group] and 26.9% [132 participants; Pooled HD group]) to moderate (18.0% [30 participants; 2q8 group] and 21.6% [106 participants; Pooled HD group]).

Severe non-ocular TEAEs were reported in 23 (13.8%) participants in the 2q8 group, and 61 (12.4%) participants in the Pooled HD group. Severe non-ocular TEAEs were primarily reported in the SOC of Cardiac disorders.

A total of 5 ocular serious TEAEs in the study eye were reported in 4 participants. Serious TEAEs in the study eye were Ulcerative keratitis (1 [0.6%] participant; 2q8 group), Cataract subcapsular, and Intraocular pressure increased (1 [0.3%] participant each; both in the HDq12 group), and Retinal detachment and Vitreous haemorrhage (1 [0.6%] participant; HDq16 group). None of the events were considered related to the study drug and 1 event (Intraocular pressure increased) was considered related to injection procedure

A total of 11 ocular serious TEAEs of the fellow eye were reported in 9 participants. None of these events were considered related to the study drug

The majority of these Non-ocular Serious TEAEs Through Week 60 were reported in single participants only. Across the 2q8 and Pooled HD groups, the most frequent non-ocular serious TEAEs (reported in ≥3 participants) were Acute left ventricular failure (3 [1.8%] participants) in the 2q8 group; and Acute myocardial infarction (7 [1.4%] participants), Cardiac arrest (3 [0.6%] participants), Coronary artery disease (4 [0.8%] participants), Myocardial infarction (7 [1.4%] participants), COVID-19 (4 [0.8%] participants), Covid-19 pneumonia (3 [0.6%] participants), Pneumonia (4 [0.8%] participants), Hypoglycaemia (3 [0.6%] participants), Cerebrovascular accident (5 [1.0%] participants), Acute kidney injury (6 [1.2%] participants), and Acute respiratory failure (3 [0.6%] participants) in the Pooled HD group. None of these events were considered related to the study drug.

Ocular TEAEs in the study eye leading to discontinuation of the study drug were Iritis and Visual impairment. There were no ocular TEAEs in the fellow eye reported resulting in the discontinuation of the study drug.

Non-ocular TEAEs reported that resulted in the discontinuation of the study drug for 3 (1.8%) participants in the 2q8 group and 9 (1.8%) participants in the Pooled HD group. Non-ocular TEAEs leading to discontinuation of the study drug included Blood loss anaemia, Acute myocardial infarction, Cardiac arrest, Death, Multiple organ dysfunction syndrome, Cholecystitis acute, Hip fracture, Endometrial cancer, Gastrointestinal neoplasm, Cerebrovascular accident, Encephalopathy, Acute kidney injury, Nephropathy toxic, and Aortic stenosis. No specific safety trend was observed, and most events were reported in single participants.

Through week 60, there were 18 deaths reported in this study, evenly distributed across the treatment groups, and all were associated with an SAE. None of the deaths were considered related to study drug or study procedure. Overall, the deaths reported were consistent with concurrent medical conditions and the complications of these conditions associated with an older population.

TEAEs related to Intraocular Inflammation were reported in 1 (0.6%) participant in the 2q8 group who reported Iridocyclitis, 4 (1.2%) participants in the HDq12 group who each reported 1 of the following: Iritis, Uveitis, Vitreal cells, and Vitritis, and 1 (0.6%) participant in the HDq16 group who reported Iridocyclitis. None of the events were serious.

Potential arterial thromboembolic events were evaluated by a masked adjudication committee according to criteria formerly applied and published by the APTC. Arterial thromboembolic events as defined by the APTC criteria include Nonfatal myocardial infarction, Nonfatal stroke (ischemic or hemorrhagic), or Death resulting from vascular or unknown causes.

Low (< 6.0%) and similar proportions of participants reported adjudicated APTC events across the treatment groups.

Treatment-emergent hypertension events were reported in fewer than 20% of participants in any treatment group. A slightly higher portion of participants reported Hypertension in the HDq16 compared to the 2q8 group and the HDq12 group; however, this was not interpreted as clinically meaningful as there was no apparent dose relationship (i.e., HDq16 versus HDq12). Approximately 76% of participants in all treatment groups had a medical history of Hypertension.

Due to findings from the preclinical toxicology studies for HD, an assessment was performed in the clinical program for events related to nasal mucosa. None of the participants experienced a TEAE consistent with Nasal mucosal findings.

Overall, the treatment-emergent ocular surgeries reported were consistent with the medical history and the concurrent clinical medical conditions of the population enrolled in this study. No specific safety concern was observed.

### Pharmacokinetics- observed systemic concentration-time profiles and associated PK parameters

Choice of Dose and Dosing Regimen. Prior to the conduct of the HD aflibercept phase 2 and 3 studies, the choice of dose and dosing regimens was supported by an empirical modeling approach (Eissing *et al.,* Durability of VEGF Suppression With Intravitreal Aflibercept and Brolucizumab: Using Pharmacokinetic Modeling to Understand Clinical Outcomes, Transl Vis Sci Technol. 2021 Apr 1;10(4):9). PK simulations, based on the simple linear 1-compartment model, predicted that an 8 mg IVT dose of aflibercept may provide up to 20 days longer duration of pharmacological effect than a 2 mg IVT dose of aflibercept. However, the observed clinical data from the phase 2 CANDELA and phase 3 (PULSAR, PHOTON) studies indicate a longer than expected duration of pharmacological effect for HD aflibercept than that based on this initial empirical model.

Initiation of the HD aflibercept clinical program was based on the continuing need for patients with proliferative neovascular eye disease who are currently being treated with, or indicated for, anti-VEGF therapy to have treatment options with less frequent IVT dosing without inferior efficacy. Given the well-characterized and favorable risk/benefit profile of 2 mg aflibercept, a higher dose of aflibercept able to extend the treatment interval was proposed. A novel drug product (High Dose Aflibercept [HD aflibercept]) able to deliver via IVT 8 mg of aflibercept was developed. Pharmacokinetic simulations of free aflibercept concentration-time profiles in human vitreous using a 1-compartment ocular model demonstrate 8 mg IVT dose of aflibercept extending the dosing interval by approximately 20 days (two half-lives) relative to a 2 mg IVT dose.

Summaries of free and adjusted bound aflibercept concentrations in plasma for participants in the dense PK analysis set (DPKS) are presented by treatment in Table 1-32 and Table 1-33. Mean (SD) concentrations of free and adjusted bound aflibercept are presented by nominal time for participants in the DPKS in Figure 27 and Figure 28 (log scale). After the initial IVT aflibercept dose of 2 mg or pooled HD aflibercept (HDq12+HDq16 aflibercept, referred to in this section as 8 mg aflibercept), the concentration-time profiles of free aflibercept were characterized by an initial phase of increasing concentrations as the drug moved from the ocular space into systemic circulation followed by a mono-exponential elimination phase (Figure 27). The concentration-time profiles of adjusted bound aflibercept in plasma was characterized by a slower attainment of peak concentration (Cₘₐₓ) compared to free aflibercept. Following attainment of Cₘₐₓ, a sustained plateau of the concentration-time profiles of adjusted bound aflibercept in plasma was observed until approximately the end of the dosing interval for both dose groups (Figure 28).

For participants enrolled in the dense PK sub-study who received 2 mg aflibercept (n = 12), concentrations of free aflibercept were detectable in 4 participants at day 7 and were undetectable in all participants by day 14. Adjusted bound aflibercept concentrations were undetectable by day 28 in 1 participant in the 2 mg aflibercept treatment (n = 12). For the 8 mg aflibercept treatment (n = 21), free aflibercept concentrations were undetectable in 4 participants at day 28, and adjusted bound aflibercept concentrations were undetectable in 1 participant at day 28 (Table 1-32, Table 1-33).

After the initial aflibercept dose of 2 mg or 8 mg aflibercept, the concentration-time profiles for free aflibercept in plasma were similar for Japanese and non-Japanese populations in participants enrolled in the dense PK sub-study.

Following the third initial monthly IVT dose of aflibercept, based on the ratio of aflibercept concentration in plasma at week 12 to week 4 (C_{week12}/C_{week4}), the accumulation of free aflibercept was 2.0 and 1.8 for HDq12 and HDq16. The accumulation of free aflibercept could not be determined for 2q8 since all aflibercept concentration values at week 12 were below the limit of quantitation (BLQ). The accumulation of adjusted bound aflibercept was 1.7 for 2q8 and 1.5 for both HDq12 and HDq16.

For the participants in the PKAS, the concentrations of free and adjusted bound aflibercept in plasma were, on average, higher for the HDq12 and HDq16 treatment groups than the 2q8 treatment group (Figure 29 and Figure 30).

The impact of fellow eye treatment with 2 mg aflibercept and DRMs on free and adjusted bound aflibercept concentrations was assessed by comparing the mean concentrations over time for all participants in PKAS with the mean concentrations over time from participants in the PKAS who only received unilateral aflibercept injections without DRMs through week 48. Concentrations of free and adjusted bound aflibercept were lower in participants who received unilateral aflibercept injections with no DRMs than in the overall population. Of note, the dosing interval was only allowed to be shortened in the first year of treatment.

**Table 1-32. Summary of Concentrations of Free Aflibercept in Plasma by Time and Treatment in Participants with DME with Unilateral Treatment in the Dense PK Sampling Sub-study**

| **Concentrations of Free Aflibercept in Plasma (mg/L)** | | | | |
|---|---|---|---|---|
| | **2q8 (N=12)** | | **HDq12+HDq16 (N=23)** | |
| **Sampling Time Post First Dose (Days)** | **n** | **Mean (SD)** | **n** | **Mean (SD)** |
| 0 | 11 | 0 (0) | 23 | 0 (0) |
| 0.1667 | 12 | 0.00834 (0.0154) | 22 | 0.149 (0.249) |
| 0.3333 | 11 | 0.0147 (0.0229) | 22 | 0.205 (0.250) |
| 1 | 12 | 0.0229 (0.0324) | 22 | 0.266 (0.211) |
| 2 | 12 | 0.0146 (0.0171) | 22 | 0.218 (0.145) |
| 4 | 12 | 0.00858 (0.0107) | 22 | 0.140 (0.0741) |
| 7 | 12 | 0.00713 (0.0114) | 19 | 0.0767 (0.0436) |
| 14 | 12 | 0 (0) | 21 | 0.0309 (0.0241) |
| 21 | 12 | 0 (0) | 21 | 0.0171 (0.0171) |
| 28 | 12 | 0 (0) | 21 | 0.00730 (0.0113) |

| | | | | |
|---|---|---|---|---|
| Abbreviations: N,n=Number of patients; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses; DPKS=Dense PK Sampling Substudy Note: HDq12+HDq16 = combined data from treatment groups HDq12 and HDq16. Patients in the dense PK substudy only received aflibercept injections unilaterally. | | | | |

**Table 1-33. Summary of Concentrations of Adjusted Bound Aflibercept in Plasma by Time and Treatment in Participants with DME with Unilateral Treatment in the Dense PK Sampling Sub-study (Study VGFTe-HD-DME-1934, [DPKS])**

| **Concentrations of Adjusted Bound Aflibercept in Plasma (mg/L)** | | | | |
|---|---|---|---|---|
| **Sampling Time Post First Dose (Days)** | **2q8 (N=12)** | | **HDq12+HDq16 (N=23)** | |
| | **n** | **Mean (SD)** | **n** | **Mean (SD)** |
| 0 | 11 | 0.00451 (0.0150) | 23 | 0.00583 (0.0280) |
| 0.1667 | 12 | 0.00452 (0.0156) | 22 | 0.00698 (0.0276) |
| 0.3333 | 11 | 0.00426 (0.0141) | 22 | 0.00731 (0.0279) |
| 1 | 12 | 0.0131 (0.0260) | 22 | 0.0678 (0.0486) |
| 2 | 12 | 0.0452 (0.0357) | 22 | 0.138 (0.0618) |
| 4 | 12 | 0.0765 (0.0412) | 22 | 0.259 (0.126) |
| 7 | 12 | 0.0885 (0.0392) | 19 | 0.346 (0.151) |
| 14 | 12 | 0.105 (0.0414) | 21 | 0.374 (0.110) |
| 21 | 12 | 0.101 (0.0423) | 21 | 0.343 (0.128) |
| 28 | 12 | 0.0873 (0.0506) | 21 | 0.269 (0.149) |

| | | | | |
|---|---|---|---|---|
| Abbreviations: N,n=Number of patients; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16 = High-dose (8 mg) intravitreal aflibercept every 16 weeks following 3 initial monthly doses. Note: Adjusted bound aflibercept = 0.717*bound aflibercept. HDq12+HDq16 = combined data from treatment groups HDq12 and HDq16. Patients in the dense PK substudy only received aflibercept injections unilaterally. | | | | |

Summaries of PK parameters from observed free and adjusted bound aflibercept concentrations for participants in the DPKS are presented by treatment in Table 1-35 and Table 1-35. After the initial monthly aflibercept dose of 2 mg (2q8) or 8 mg, free aflibercept median time to peak concentration (tₘₐₓ) was 0.268 and 0.965 days for the 2 mg and 8 mg aflibercept treatments, respectively. The attainment of tₘₐₓ for adjusted bound aflibercept concentrations was slower when compared to free aflibercept. For adjusted bound aflibercept, the median tₘₐₓ was 14 days for the 2 mg and 8 mg aflibercept treatments. As the IVT dose of aflibercept increased from 2 mg to 8 mg (a 4-fold increase in dose), the mean peak concentration (Cₘₐₓ) and mean area under the concentration-time curve from time zero to the time of the last measurable concentration (AUCₗₐₛₜ) for free aflibercept increased in a greater than dose-proportional manner (approximately 12 to 14 fold). Conversely, for adjusted bound aflibercept, mean AUCₗₐₛₜ and Cₘₐₓ increased slightly less than to dose proportionally (approximately 3 to 4-fold). These results are consistent with historical data and the known nonlinear kinetics of aflibercept (Table 1-34 and Table 1-35).

**Table 1-34. Summary of Pharmacokinetic Parameters Calculated from Concentrations of Free Aflibercept in Plasma by Treatment in Participants with DME with Unilateral Treatment in the Dense PK Sampling Substudy (Study VGFTe-HD-DME-1934, [DPKS])**

| | | **2q8 (N=12)** | | | | **HDq12+HDq16 (N=23)** | | | |
|---|---|---|---|---|---|---|---|---|---|
| **PK Parameters** | **Unit** | **n** | **Mean (SD)** | **Median** | **Min : Max** | **n** | **Mean (SD)** | **Median** | **Min : Max** |
| AUC_{inf} | day*mg/L | 0 | | | | 13 | 2.26 (0.714) | 2.29 | 1.14 : 3.36 |
| AUC_{inf}/Dose | day*mg/L/mg | 0 | | | | 13 | 0.283 (0.0892) | 0.287 | 0.143 : 0.420 |
| AUCₗₐₛₜ | day*mg/L | 7 | 0.126 (0.0660) | 0.146 | 0.0244 : 0.202 | 21 | 1.71 (0.816) | 1.85 | 0.230 : 2.82 |
| AUCₗₐₛₜ/Dose | day*mg/L/mg | 7 | 0.0628 (0.0330) | 0.0732 | 0.0122 : 0.101 | 21 | 0.214 (0.102) | 0.232 | 0.0287 : 0.352 |
| AUC₀₋₂₈ | day*mg/L | 6 | 0.170 (0.124) | 0.162 | 0 : 0.358 | 19 | 1.86 (0.840) | 1.97 | 0 : 2.89 |
| Cₘₐₓ | mg/L | 12 | 0.0266 (0.0333) | 0.0198 | 0 : 0.109 | 23 | 0.310 (0.263) | 0.245 | 0 : 1.08 |
| Cₘₐₓ/Dose | mg/L/mg | 12 | 0.0133 (0.0167) | 0.00990 | 0 : 0.0545 | 23 | 0.0388 (0.0328) | 0.0306 | 0 : 0.135 |
| Cₗₐₛₜ | mg/L | 7 | 0.0217 (0.00619) | 0.0195 | 0.0157 : 0.0335 | 21 | 0.0390 (0.0399) | 0.0223 | 0.0158 : 0.169 |
| C_{trough28} | mg/L | 12 | 0 (0) | 0 | 0:0 | 21 | 0.00730 (0.0113) | 0 | 0 : 0.0371 |
| t_{1/2} | day | 1 | NR¹ | NR¹ | NR¹ | 15 | 8.43 (4.55) | 8.48 | 1.41 : 20.4 |
| tₘₐₓ | day | 12 | -- | 0.268 | 0 : 7.04 | 23 | -- | 0.965 | 0 : 4.01 |
| tₗₐₛₜ | day | 7 | -- | 6.94 | 1.98 : 7.04 | 21 | -- | 20.9 | 2.00 : 32.0 |

| | | | | | | | | | |
|---|---|---|---|---|---|---|---|---|---|
| PK=Pharmacokinetic; N=Number of patients; n=Number of patients contributing to each PK parameter; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses Note: HDq12+HDq16=combined data from treatment groups HDq12 and HDq16. Patients in the dense PK sub-study only received aflibercept injections unilaterally. Data presented were collected during the dense PK sub-study (from pre-dose Day 0 through Day 28). 1 NR = not reported; N=1 participant had a reportable t_{1/2} value of 17.3 days. | | | | | | | | | |

**Table 1-35. Summary of Pharmacokinetic Parameters Calculated from Concentrations of Adjusted Bound Aflibercept in Plasma by Treatment in Participants with DME with Unilateral Treatment in the Dense PK Sampling Substudy (Study VGFTe-HD-DME-1934, [DPKS])**

| | | **2q8 (N=12)** | | | | **HDq12+HDq16 (N=23)** | | | |
|---|---|---|---|---|---|---|---|---|---|
| **Parameters** | **Unit** | **n** | **Mean (SD)** | **Median** | **Min : Max** | **n** | **Mean (SD)** | **Median** | **Min : Max** |
| AUCinf | day*mg/L | 0 | | | | 0 | | | |
| AUCinf/Dose | day*mg/L/mg | 0 | | | | 0 | | | |
| AUCₗₐₛₜ | day*mg/L | 11 | 2.48 (0.607) | 2.52 | 1.33 : 3.67 | 23 | 8.30 (3.05) | 8.03 | 4.07 : 17.6 |
| AUCₗₐₛₜ/Dose | day*mg/L/mg | 11 | 1.24 (0.303) | 1.26 | 0.664 : 1.83 | 23 | 1.04 (0.382) | 1.00 | 0.509 : 2.20 |
| AUC₀₋₂₈ | day*mg/L | 4 | 2.53 (0.360) | 2.49 | 2.14 : 3.00 | 14 | 8.71 (3.65) | 8.42 | 3.27 : 17.7 |
| Cₘₐₓ | mg/L | 12 | 0.115 (0.0442) | 0.124 | 0 : 0.154 | 23 | 0.387 (0.135) | 0.380 | 0.137 : 0.774 |
| Cₘₐₓ/Dose | mg/L/mg | 12 | 0.0575 (0.0221) | 0.0618 | 0 : 0.0771 | 23 | 0.0483 (0.0168) | 0.0475 | 0.0171 : 0.0968 |
| Cₗₐₛₜ | mg/L | 11 | 0.104 (0.0314) | 0.0961 | 0.0551 : 0.154 | 23 | 0.287 (0.136) | 0.260 | 0.0649 : 0.599 |
| C_{trough28} | mg/L | 12 | 0.0873 (0.0506) | 0.0929 | 0 : 0.154 | 21 | 0.269 (0.149) | 0.252 | 0 : 0.599 |
| tₘₐₓ | day | 12 | -- | 14.0 | 0: 23.9 | 23 | -- | 14.0 | 3.96 : 37.1 |
| tₗₐₛₜ | day | 11 | -- | 27.0 | 21.0 : 30.1 | 23 | -- | 27.9 | 20.9 : 43.0 |

| | | | | | | | | | |
|---|---|---|---|---|---|---|---|---|---|
| Abbreviations: PK=Pharmacokinetic; N,n=Number of patients; SD=Standard deviation; DME=Diabetic macular edema; 2q8=2 mg intravitreal aflibercept every 8 weeks following 5 initial monthly doses; HDq12=High-dose (8 mg) intravitreal aflibercept every 12 weeks following 3 initial monthly doses; HDq16=High-dose (8 mg) intravitreal aflibercept every 16 weeks following 3 initial monthly doses Note: Adjusted bound aflibercept = 0.717*bound aflibercept. HDq12+HDq16 = combined data from treatment groups HDq12 and HDq16. Patients in the dense PK substudy only received aflibercept injections unilaterally. Data presented were collected during the dense PK substudy (from pre-dose day 0 through day 28). | | | | | | | | | |

### Expanded Population PK Analysis (referred to as the Population PK model, or PopPK).

With availability of the free and adjusted bound aflibercept concentration data from the CANDELA, PULSAR, and PHOTON along PK data from the other studies listed herein, a comprehensive PopPK model was developed, In this latter PopPK model, the PK of free and adjusted bound aflibercept following IV, SC, or IVT administration was adequately described by a 3-compartment PopPK model with the binding of free aflibercept from the central compartment to VEGF described by Michaelis-Menten kinetics. An additional tissue compartment that could represent platelets (Sobolewska *et al.,* Human Platelets Take up Anti-VEGF Agents. J Ophthalmol 2021; 2021:8811672) was added where the rate of elimination from the central compartment of free aflibercept to the platelet compartment was dependent on the number of platelets that were able to uptake anti-VEGF agents such as ranibizumab, bevacizumab, and aflibercept (Figure 31).

Although PK parameters for free and adjusted bound aflibercept in plasma were determined by noncompartmental analysis (NCA) and reported at the level of the individual study reports, the PK parameters determined by population PK analysis are considered to be the more accurate estimate and therefore the definitive PK parameters are those assessed by the population PK model.

Across all 3 studies (CANDELA, PULSAR, and PHOTON), the pharmacokinetic analysis set (PKAS) includes all treated participants who received any amount of study drug (aflibercept or HD aflibercept) and had at least 1 non-missing aflibercept or adjusted bound aflibercept measurement following the first dose of study drug. The PKAS is based on the actual treatment received (as treated), rather than as randomized. The PKAS-dense (PK-dense) analysis set is a subset of the PKAS and includes participants who had dense blood sample collection for systemic drug concentrations.

CANDELA, PULSAR, and PHOTON each included a PK substudy where drug concentration data were collected using dense blood sample collection schedules during the first dosing interval and sparse PK sampling thereafter in up to approximately 30 participants. Drug concentration data were also collected in each study for all participants using a sparse sampling schedule throughout the 44 weeks (CANDELA) or 48 weeks (PHOTON, PULSAR) of treatment.

Pharmacokinetic parameters for individual studies were calculated by non-compartmental analysis for free and adjusted bound aflibercept concentration data collected from participants with dense sampling schedules in these 3 studies.

Additionally, all concentration data from these 3 studies were incorporated into the Population PK data set.

The concentration time profiles of free and adjusted bound aflibercept in plasma after the initial dose of HD aflibercept by IVT administration were consistent between all studies in participants with nAMD or DME. The consistency of the concentration-time profiles for free and adjusted bound aflibercept in plasma between the nAMD and DME populations is further supported by population PK analysis (Figure 33 and Figure 34).

Population PK estimated post-hoc concentration-time profiles and PK parameters for combined nAMD and DME populations following single IVT administration of 2 mg aflibercept or HD aflibercept are provided in Figure 33 and Figure 34 and in Table 1-36 and Table 1-39.

Following single IVT administration of aflibercept 2 mg or HD aflibercept, the concentration-time profiles of free and adjusted bound aflibercept in plasma in participants who underwent dense sample collection for systemic drug concentrations (dense PK substudy) after the initial dosing of aflibercept 2 mg or HD aflibercept, respectively, were consistent between the 3 studies in participants with nAMD or DME (Figure 32). Notably, there was one excluded participant in the PULSAR dense-sampling PK substudy that had free aflibercept concentrations over time that were approximately 10-fold higher than the mean concentration-time profiles in that study.

The consistency of the concentration-time profiles for free and adjusted bound aflibercept between the nAMD and DME populations is further supported by Population PK analysis (Figure 33). Population PK estimated post-hoc concentration-time profiles and PK parameters for combined nAMD and DME populations following single IVT administration of 2 mg aflibercept or HD aflibercept are provided below in Figure 33 and in Table 1-37 and Table 1-38.

The corresponding observed and Population PK estimated post-hoc concentration-time profiles and PK parameters for participants with nAMD and DME are provided in Figure 36, Figure 37, and Figure 38 and Table 1-39, Table 1-40.

Following single IVT administration of 2 mg aflibercept or HD aflibercept, the concentration-time profiles of free aflibercept are characterized by an initial phase of increasing concentrations, as the drug moved from the ocular space into systemic circulation, followed by a mono-exponential elimination phase. The concentration time profiles of adjusted bound aflibercept in plasma are characterized by a slower attainment of Cmax compared to free aflibercept. Following attainment of Cₘₐₓ, a sustained plateau of the concentration-time profiles of adjusted bound aflibercept in plasma was observed until approximately the end of the first dosing interval (Figure 32, Figure 33).

For participants who underwent dense blood sample collection for systemic drug concentrations across the CANDELA, PULSAR, and PHOTON studies, after the initial dosing of 2 mg IVT aflibercept (n = 34), observed concentrations of free aflibercept were detectable in 15 (44.1%) participants by week 1 and in 3 (8.8%) participants by week 2.

For participants who underwent dense blood sample collection for systemic drug concentrations after the initial dosing of 8 mg IVT aflibercept (n = 54), observed concentrations of free aflibercept were detectable in 46 (85.2%) participants by week 1 and in 44 (77.8%) participants by week 2. The observed and Population PK simulated free and adjusted bound aflibercept concentrations in plasma for up to 48 weeks are presented for the combined nAMD and DME population (Figure 34), and the nAMD (Figure 39) and DME (Figure 40) populations. Based on the Population PK analysis, the median time for free aflibercept concentrations to reach LLOQ in plasma following HDq12 or HDq16 was more than double (3.50 weeks versus 1.5 weeks) the median time needed to reach LLOQ following aflibercept 2q8 (Table 1-41).

The longer duration of systemic exposure to free aflibercept following HDq12 and HDq16 compared to the 2 mg aflibercept is attributed to not only a higher administered dose and nonlinear systemic target-mediated elimination, but also to a 34% slower ocular clearance of free aflibercept. The slower ocular clearance of free aflibercept for HD aflibercept is attributed to a HD drug product effect which was identified as a statistically significant covariate in the Population PK model.

Population PK analysis confirmed no relevant differences in PK between the nAMD and DME populations, and therefore all subsequent analyses are presented for the combined nAMD and DME population.

The pharmacokinetic (PK) data set forth above summarize the observed systemic concentration-time profiles and associated PK parameters for free and adjusted bound aflibercept for each individual study. The analyses utilized to estimate the PK parameters in each individual study were performed by non-compartmental analysis. While the individual PHOTON study results describe the observed systemic concentration-time profiles and associated PK parameters of free and adjusted bound aflibercept in plasma, they do not specifically identify PK characteristics of the HD 8 mg aflibercept drug product contributing to the unexpected pharmacodynamic (PD) and efficacy results for HD aflibercept observed in the CANDELA (NCT04126317), PULSAR (NCT04423718), and PHOTON (European Clinical Trials Database (EudraCT): 2019-003851-12) studies.

An expanded PopPK analysis that utilized free and adjusted bound concentration in plasma data from the HD clinical studies, as well as 13 prior studies:

### DME population

- VGFT-OD-0307: A double-blind, randomised, dose-escalating study evaluating safety, tolerability and bio-effect after intravenous (IV) administration of VEGF Trap in subjects with DME. Subjects were planned to receive 4 IV infusions of VEGF Trap, once every 2 weeks (day 1, day 15, day 29, and day 43), at dose levels of 0.3 mg/kg, 1 mg/kg, or 3 mg/kg, or placebo. However, dosing was stopped before the planned sequential dose escalation when dose-limiting toxicities (grade 2 proteinuria in a single subject and grade 4 treatment-related malignant hypertension in a single subject) were observed in another phase 1 dose-escalation study in subjects with AMD (VGFT-OD-0305). The dose limiting toxicities observed in study VGFT-OD-0305 occurred at the 3 mg/kg IV dose. Therefore, only the lowest dose (0.3 mg/kg) of study drug was investigated. Nine subjects were randomised and treated (3 placebo, 6 VEGF Trap 0.3 mg/kg). Concentrations of free and bound VEGF Trap were determined at selected time intervals following dose administration (screening, day 1[pre-dose], day 8, day 15, day 29, day 43, day 57, day 71, day 85, and day 133 [3 months post-last dose]);
- VGFT-OD-0512: An open-label, proof-of-concept study evaluating safety, tolerability and bio-effect of VEGF Trap IVT administration in subjects with DME. Five (5) subjects with DME were enrolled. Subjects received a single IVT injection of 4 mg VEGF Trap into the study eye. During the first 6 weeks after the injection, vital signs as well as ocular and systemic adverse events (AEs) were recorded. Blood samples for analysis of free and bound VEGF Trap concentrations in plasma were collected at screening, day 1 (pre-dose), day 3, day 8, day 15, day 29, day 43 and day 155 following the single IVT administration;
- VGFT-OD-0706.PK (PK sub-study of VGFT-OD- 0706): DME And VEGF Trap-Eye [Intravitreal Aflibercept Injection (IAI;EYLEA^{®};BAY86-5321)] INvestigation of Clinical Impact (DA VINCI)-Clinicaltrials.gov Identifier NCT00789477; and
- 91745: Intravitreal Aflibercept Injection in Vision Impairment Due to DME (VIVID-DME)-ClinicalTrials.gov Identifier NCT01331681;

### AMD population

- VGFT-OD-0305: A double-masked, placebo controlled, sequential group, dose escalating, (0.3 mg/kg, 1 mg/kg, 3 mg/kg, 5 mg/kg, 7 mg/kg, and 10 mg/kg) study of safety and bioeffect. The study included subjects with a diagnosis of visual impairment associated with neovascular AMD. Subjects were required to have visual loss due to subfoveal choroidal neovascularization (CNV) secondary to AMD, be 50 years of age or older, with no history of Type I or Type II diabetes, without significant cardiac, liver or kidney disease, or congestive heart failure (CHF); and without confounding ophthalmic issues. The study treatments were: 1. VEGF Trap 0.3 mg/kg. 2. VEGF Trap 1 mg/kg, and 3. VEGF Trap 3 mg/kg;
- VGFT-OD-0306: An open label, long term safety and tolerability extension study of intravenous VEGF Trap in subjects with neovascular AMD who had been included in Study VEGF-OD-0305. The study treatments were VEGF Trap at the same dose level the subjects had been treated with in Study VEGF-OD-0305: either 0.3 mg/kg or 1 mg/kg, by intravenous administration every 2 weeks. Placebo patients from Study VGFT-OD-0305 were assigned to VEGF Trap at the dose level at which they were enrolled in Study VGFT-OD-0305. The efficacy outcome measures were: Visual acuity (ETDRS), Retinal thickness (OCT), Funduscopic examination, Fundus photography, and FA. The safety outcome measures were: AEs, Clinical laboratory tests, and Ophthalmic exam. Treatment duration was for up to 106 days. There were seven subjects: four subjects treated with 0.3 mg/kg, 3 subjects treated with 1 mg/kg. There were five females, two males and the age range was 68 to 84 years. Six of the seven subjects had a slight reduction in ERT in the study eye and six of seven subjects had slight reductions in macular volume in the study eye;
- VGFT-OD-0502 Part A: Safety and Tolerability Study of Intravitreal VEGF-Trap Administration in Patients With Neovascular AMD-ClinicalTrials.gov Identifier: NCT00320775;
- VGFT-OD-0502 Part C: ClinicalTrials.gov Identifier: NCT00320775;
- VGFT-OD-0603: Safety and Tolerability of Intravitreal VEGF Trap Formulations in Subjects With Neovacular AMD-ClinicalTrials.gov Identifier: NCT00383370;
- VGFT-OD-0702.PK (PK sub-study of VGFT-OD-0702): Randomized, Single-Masked, Long-Term, Safety and Tolerability Study of VEGF Trap-Eye in AMD-ClinicalTrials.gov Identifier: NCT00527423; and
- 311523 (VIEW2): A multicentre, double masked, randomised, active controlled, parallel group, non-inferiority efficacy and safety study. The study was almost identical in design to Study VGFT-OD-0605/14393 (VIEW 1). The submission contained the report of the first 52 weeks of the study. The study was conducted at 186 centres in 26 countries.

The inclusion criteria, exclusion criteria and study treatments were identical to Study VGFT-OD-0605/14393 (VIEW 1). The efficacy outcome measures were the same, except for the additional outcome measure: change in scores of the EQ-5D questionnaire from screening at Week 52;

### Oncology population

- VGFT-ST-0103, (also known as TED6113): VEGF Trap in Treating Patients With Relapsed or Refractory Solid Tumors or Non-Hodgkin's Lymphoma-ClinicalTrials.gov Identifier: NCT00036946;

### Healthy participant population

- PDY6655: A Phase I, single centre, randomised, single dose, crossover, pharmacokinetic (PK) study in healthy volunteers to compare the pharmacokinetics and pharmacodynamic (PD) of intravenous and subcutaneous administration of aflibercept. The study included 40 healthy male subjects aged 18 to 45 years. The study treatments were: aflibercept 2.0 mg/kg as an intravenous infusion over 1 hour, and as a subcutaneous injection. The aflibercept was presented as 4 mL of 25 mg/mL solution. The treatments were administered as single doses followed by 6 week observation period. The treatment periods were separated by 1 to 2 weeks. The PK outcome measures were: Cmax, AUC, apparent volume of distribution at steady state (Vss), clearance and half life (t½). The PD outcome measures were: systolic blood pressure, diastolic blood pressure, heart rate, mean arterial pressure, plasma renin activity, angiotensin I, aldosterone, and free endogenous VEGF. The safety outcome measures were: AEs, clinical laboratory test, injection site reactions, and anti-aflibercept antibodies. AUC and Cmax were slightly higher for Period 2, indicating some carry over. For Period 1, for free aflibercept mean (co-variance (CV%)) AUC was 177 (33) µg•day/mL and peak plasma concentration (Cmax) was 44.4 (36) µg/mL for intravenous and AUC was 84.9 (30) µg•day/mL and Cmax was 7.76 (39) µg/mL for subcutaneous. For Period 1, for bound aflibercept mean (CV%) AUC was 57.7 (19) µg•day/mL and Cmax was 1.84 (22) µg/mL for intravenous and AUC was 47.3 (27) µg•day/mL and Cmax was 1.60 (27) µg/mL for subcutaneous. The mean (90% CI) ratio for AUC, subcutaneous/ intravenous, was 0.51 (0.46 to 0.56) [(range)], and
- PDY6656: A single centre, Phase I, randomised, double blind, placebo controlled, sequential ascending dose study of intravenous aflibercept. The study included healthy male subjects 18 to 45 years of age; non-smoker; 18≤ body mass index (BMI) ≤28 kg/m²; with normal vital signs and no symptomatic hypotension. The study treatments were aflibercept 1 mg/kg, 2 mg/kg and 4 mg/kg, and placebo. There were three cohorts of 16 subjects: twelve treated with aflibercept and four treated with placebo. The treatments were administered as a single dose by intravenous infusion over 1 hour. The pharmacodynamic outcome measures were: systolic blood pressure (SBP), diastolic blood pressure (DBP), mean arterial pressure (MAP), plasma active renin, aldosterone and angiotensin I; markers of endothelium dysfunction (plasma endothelin-1, E-selectin, cyclic guanosine 3'5' monophosphate (cGMP), and urine nitrites/nitrates); renal function; and VEGF. The safety outcome measures were: AEs and laboratory tests. The study included 48 subjects: 12 treated with 1 mg/kg, 12 with 2 mg/kg, 12 with 4 mg/kg and 12 with placebo. The age range was 21 to 45 years. For free aflibercept mean (CV%) Cmax was 18.2 (18) µg/mL for the 1 mg/kg dose, 39.7 (27) µg/mL for the 2 mg/kg dose and 78.6 (15) µg/mL for the 4 mg/kg dose; and mean (CV%) AUC was 64.8 (20) µg.day/mL for the 1 mg/kg dose, 180 (20) for the 2 mg/kg dose and 419 (21) for the 4 mg/kg dose. Bound aflibercept concentrations were not dose dependent and the proportion of bound aflibercept decreased with increasing dose. However, Cmax and AUC for total aflibercept were dose proportional [(range)];(See Australian Public Assessment Report for Afibercept, AusPAR Eylea Aflibercept Bayer Australia Ltd; PM-2010-03802-3-5 Final 30 July 2012; and Assessment Report, Eylea, Committee for Medicinal Products for Human Use (CHMP) 26 June 2014 EMA/430291/2014; FDA, Center for Drug Evaluation and Research, Approval Package for: APPLICATION NUMBER: 125387Orig1s048, Eylea, March 25, 2015) of aflibercept 2 mg in the DME and nAMD populations, healthy participants, and participants with oncology diseases after intravenous (IV), subcutaneous (SC), or intravitreal (IVT) administration was performed to: characterize the concentration-time profiles of free and adjusted bound aflibercept in plasma; estimate population and individual PK parameters of aflibercept in patients with nAMD and DME; investigate the effects of relevant covariates which may explain variability in aflibercept PK parameters; and derive *post-hoc* estimates of individual exposure metrics in the nAMD and DME patients from the final PopPK model that formed the basis for pharmacokinetic/pharmacodynamic (PK/PD) analyses.

A key finding from this expanded PopPK analysis is that clearance of free aflibercept from the ocular compartment (ocular clearance) is 34.3% slower for HD drug product than for 2 mg IVT aflibercept reference drug product, and is attributed to an "HD aflibercept drug product effect". Ultimately, it is this HD drug product effect on slowing the ocular clearance that resulted in a longer than expected ocular residence time, and the greater than expected proportion of patients able to be maintained on the longer dosing intervals of q12 and q16.

The consequences of the slower ocular clearance for HD (8 mg) aflibercept, as identified in the PopPK analysis, were further evaluated via PopPK model-based simulations to predict the time-course of free aflibercept in the eye (ocular compartment) under different dosing scenarios, and via exposure-response analyses to assess whether PopPK estimates of ocular clearance are predictive of the time required for dose regimen modification (DRM).

Efficacy data from the phase 3 PULSAR study in the nAMD population confirmed that the HDq12 and HDq16 regimens provide durable efficacy over the 48-week treatment period, as both regimens met the primary endpoint for efficacy of non-inferior change from baseline in BCVA at week 48 compared to 2q8. A majority of participants randomized to HDq12 or HDq16 maintained their 12-week (79%) and 16-week (77%) dosing intervals, without the need for DRM, through 48 weeks.

Results from the phase 2/3 PHOTON study also confirmed efficacy of the HDq12 and HDq16 regimens in participants with DME and DR as both met the primary endpoint for efficacy of noninferior change from baseline in BCVA at week 48 compared to 2q8, with a majority of participants maintaining their HDq12 (91%) and HDq16 (89%) regimens, without the need for DRM, through the end of the 48-week treatment period.

As the vast majority of participants enrolled in the PHOTON study had underlying DR, they were also assessed for efficacy endpoints associated with the improvement of their underlying retinopathy. The HDq12 regimen met the key secondary efficacy endpoint of noninferiority for the proportion of participants with a ≥2-step improvement in DRSS score compared to 2q8 at the prespecified margin of 15%. Additionally, noninferiority was demonstrated using the FDA recommended 10% margin. Non-inferiority was not established for HDq16 at the 15% margin. The HDq16 group had more participants with mild to moderate disease than both the HDq12 and the 2q8 group, which may have contributed to these findings.

Regarding safety, similar ocular and systemic safety profiles for HDq12 and HDq16 compared to 2q8 aflibercept were observed in all 3 studies, with no new safety signals identified for HD aflibercept.

Residual variability was modeled separately for free and adjusted bound aflibercept using an additive + proportional error model. Estimated bioavailability for free aflibercept was 71.9% following IVT administration (Table 1-36). Parameter estimates for the Population PK model are presented in Table 1-36.

**Table 1-36. Population Pharmacokinetic Parameter Estimates for the Final Model for Aflibercept**

| **Parameter** | **Estimate** | **C.I.95** | **RSE %** | **CV %** |
|---|---|---|---|---|
| K20 (1/day) [run431 Estimate] | 0.0807 | 0.0438 - 0.136 | 29.5 | - |
| V2 V4 (L) [run431 Estimate] | 4.99 | 4.71 - 5.25 | 2.79 | - |
| V3 (L) [run431 Estimate] | 1.08 | 0.816 - 1.58 | 17 | - |
| QF1 (L/day) [run431 Estimate] | 0.849 | 0.435 - 1.33 | 29.2 | - |
| V8 (L) [run431 Estimate] | 1.18 | 0.281 - 0.541 | 16.8 | - |
| QF2 (L/day) [run431 Estimate] | 0.0763 | 0.147 - 0.186 | 5.93 | - |
| KM (mg/L) [run431 Estimate] | 0.411 | 0.293 - 0.442 | 10.5 | - |
| VMK24 (mg/day/L) [run431 Estimate] | 0.167 | 0.482 - 0.593 | - | - |
| K40 (1/day) [run463 Estimate] | 0.035 | - | - | - |
| F1 & F5 | 0.719 | 0.706 - 0.731 | - | - |
| QE (L/day) | 0.000624 | 0.000577 - 0.000674 | 3.97 | - |
| K62 (1/day) [run431 Estimate] | 0.368 | 0.0165 - 0.0632 | 35.3 | - |
| F6 [run431 Estimate] | 0.536 | 0.00584 - 0.149 | 99 | - |
| VMK27 (mg/day/L) [run431 Estimate] | 0.031 | 4.17 - 340 | 159 | - |
| K70 (1/day) [run431 Estimate] | 0.0265 | 0.632 - 2.84 | 39.8 | - |
| KMK27 (mg) [run431 Estimate] | 42.7 | 0.0481 - 0.12 | 23.7 | - |
| TWGT V2+V4 [run431 Estimate] | 0.872 | 0.55 - 1.22 | 19.3 | - |
| TWGT V3 [run431 Estimate] | 1.08 | -0.00762 - 2.45 | 51.3 | - |
| TWGT V8 [run431 Estimate] | 1.16 | -2.97 - 6.58 | 135 | - |
| TWGT K20 [run431 Estimate] | -0.192 | -1.28 - 0.819 | 234 | - |
| TWGT K40 [run463 Estimate] | -0.153 | - | - | - |
| HD QE | 0.657 | 0.607 - 0.712 | 4.08 | - |
| AGE QE | -1.53 | -1.76 - -1.3 | 7.68 | - |
| TALB K40 [run463 Estimate] | -0.767 | - | - | - |
| IIV K20 [run431 Estimate] | 0.207 | -0.0147 - 0.508 | 54.1 | 48 |
| IIV covariance(K20, V2 & V4) [run431 Estimate] | -0.0727 | -0.136 - -0.0183 | 38.9 | - |
| IIV V2 & V4 [run431 Estimate] | 0.0618 | 0.0198 - 0.105 | 34.7 | 25.3 |
| IIV VMK24 [run431 Estimate] | 0.305 | -0.083 - 0.67 | 65.4 | 59.8 |
| IIV K40 [run463 Estimate] | 0.0452 | - | - | 21.5 |
| IIV QE | 0.297 | 0.257 - 0.336 | 6.86 | 58.8 |
| IIV K62 [run431 Estimate] | 0.852 | 0.124 - 1.45 | 43 | 116 |
| IIV F6 [run431 Estimate] | 0.629 | 0.288 - 0.905 | 26.4 | 93.6 |
| SD ADD LLOQ 0.0313 (Free,IV+SC) [run463 Estimate] | 0.025 | - | - | - |
| SD PROP LLOQ 0.0313 (Free,IV+SC) [run463 Estimate] | 0.403 | - | - | - |
| SD ADD LLOQ 0.0156 (Free) | 0.00779 | 0.00624 - 0.00973 | 11.4 | - |
| SD PROP LLOQ 0.0156 (Free) | 0.433 | 0.418 - 0.448 | 1.72 | - |
| SD ADD LLOQ 0.0315 (Adj.Bound) | 0.0216 | 0.0177 - 0.0264 | 10.2 | - |
| SD PROP LLOQ 0.0315 (Adj.Bound) | 0.159 | 0.12 - 0.197 | 12.4 | - |
| SD ADD LLOQ 0.0224 (Adj.Bound) | 0.0291 | 0.0202 - 0.0419 | 18.8 | - |
| SD PROP LLOQ 0.0224 (Adj.Bound) | 0.214 | 0.194 - 0.234 | 4.83 | |

| | | | | |
|---|---|---|---|---|
| ADD = additive, age = baseline age, C.I.95 = 95% confidence intervals, CV = coefficient of variation, F1 and F5 = bioavailability of intravitreal injections in ocular compartments, F6 = bioavailability of subcutaneous injections, HD = high dose (8 mg IVT cohorts), IIV = inter-participant variability, IV = intravenous, K20 = elimination rate of free aflibercept, K40 = elimination rate constant for adjusted bound aflibercept, K62 = rate of absorption from subcutaneous injection dosing compartment, K70 = elimination rate from tissue (platelet) compartment, KM = concentration of free aflibercept at half of maximum binding capacity with VEGF; KMK27 = concentration of free aflibercept at half of maximum binding capacity to platelets, LLOQ = lower limit of quantification, PROP = proportional, QE = inter-compartmental clearance between ocular compartment and central compartment of free aflibercept, QF1 and QF2 = inter-compartmental clearances of free aflibercept, RSE% = percent relative standard error, SC = subcutaneous, TALB = time varying albumin, TWGT = time varying body weight, V2 = central volume of free aflibercept in plasma, V3 and V8 = peripheral volumes of free aflibercept in tissues, V4 = central volume of adjusted bound aflibercept in plasma, VMK24 = maximum binding rate of free aflibercept to VEGF, VMK27 = maximum binding rate of aflibercept to platelets Estimates of fixed-effect parameters are presented in the natural scale; IIV are reported as variances around the log of the parameters or the logit of F6. Residual errors of IV and SC data not presented in the table for LLOQ=0.0156 (*σ* additive=0.00786, *σ*proportional= 0.357) and LLOQ=0.0315 (*σ*additive=0.0206, *σ*proportional=0.167) were fixed in the final model to estimates from run463. C.I.95 and %RSE% for run431 were calculated from bootstrap. *η*-shrinkage: *η*K20= 54.2%, *η*V2,V4= 15.2%, *η*VMK24= 42.2%, *η*K40= 39.1%, *η*QE= 31.6%, *η*K62= 1e-10%, *η*F6= 17.7%. | | | | |

Concentrations of free and bound aflibercept in plasma were measured using validated enzyme-linked immunosorbent assay (ELISA) methods. The assay for bound aflibercept is calibrated using the VEGF:aflibercept standards, and the results are reported for bound aflibercept as weight per volume (*e.g.,* ng/mL or mg/L) of the VEGF:aflibercept complex. Therefore, to account for the difference in molecular weight and normalize the relative concentrations between free and bound aflibercept, the concentration of the bound aflibercept complex is adjusted by multiplying the bound aflibercept concentration by 0.717. This is to account for the presence of VEGF in the bound complex and report the complex in terms of mg/L (i.e., mass/volume) that are corrected for, and consistent with, the molar concentrations (referred to as adjusted bound aflibercept in this module). Herein, concentrations of aflibercept:VEGF complex are limited to the adjusted bound concentrations.

The concentration of bound aflibercept was normalized to determine the amount of aflibercept present in the bound aflibercept complex. The bound aflibercept complex consisted of 71.7% aflibercept and 28.3% human VEGF₁₆₅ based on the molecular weight of each component. Therefore, the concentration of the bound aflibercept complex was multiplied by 0.717 to yield the concentration of adjusted bound aflibercept (Equation 1). Total aflibercept was calculated by summing the plasma concentrations of free and adjusted bound aflibercept (Equation 2).
Equation 1: Adjusted bound aflibercept (mg/L) = Bound aflibercept (mg/L) x 0.717 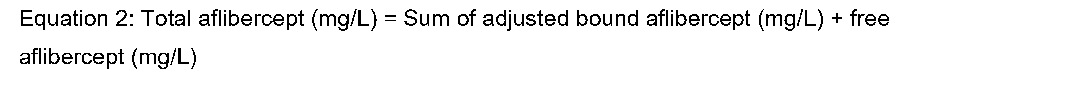 Total aflibercept (mg/L) = Sum of adjusted bound aflibercept (mg/L) + free aflibercept (mg/L) Time-varying body weight was a predictor of the central volumes for free and adjusted bound aflibercept (V2=V4), the peripheral volumes of free aflibercept in tissues (V3, and V8), and elimination rate of free aflibercept (K20) and adjusted bound aflibercept (K40). The effect of time-varying albumin was also a predictor of elimination rate of adjusted bound aflibercept (K40). Age and the effect of HD drug product versus aflibercept groups with doses ≤4 mg presented as the reference drug product were predictors of clearance from the ocular compartment (QE). The clearance of free aflibercept from the ocular compartment slowed with age, with an estimated exponent in the relationship of -1.53, resulting in clearance from the ocular compartment being approximately 25% slower for an 86 year-old (95^{th} percentile of age in the analysis population) participant than a 71 year-old (median age in analysis population) participant.

Following IVT administration, HD drug product was estimated to have 34.3% slower clearance from the ocular compartment compared to the reference IVT aflibercept drug product for doses ≤4 mg. This slower ocular clearance resulted in a longer duration of ocular exposure to free aflibercept in the ocular compartment for the HD drug product. Through PopPK covariate analysis, the 34% slower ocular clearance (QE) and longer duration of free aflibercept ocular exposure for HD drug product is statistically attributed to an "HD aflibercept drug product effect". The exact nature or attributes of the HD drug product responsible for the attenuated ocular clearance cannot be fully explained by increasing the dose alone.

Exposure-Response Analyses. An exposure-response analysis was conducted using the time to dose regimen modification (TTDRM). A KM (Kaplan-Meier) plot of TTDRM stratified by indication showed a statistically significant (p < 0.00001) difference in TTDRM between participants with AMD and participants with DME, per the logrank test. KM plots of TTDRM, stratified by quartiles of ocular clearance (QE) within indication, showed rank ordering of longer TTDRM by lower ocular clearance percentile. A Cox proportional hazard model that included indication, baseline CRT, and ocular clearance as predictors of DRM showed that the rate of DRM due to the HD drug product effect is 20.6% lower than would have been expected if the HD drug product had the same ocular clearance as the 2 mg aflibercept presented as the reference drug product.

The need for DRM is determined by the clinician objective measurements obtained during an office visit, at which time a participant's dosing regimen can be shortened due to suboptimal efficacy. Faster transit of aflibercept from the eye into the systemic circulation leads to earlier depletion of the drug from the ocular space and therefore a more rapid loss of efficacy. While there may be other factors affecting efficacy, such as disease progression, comorbidities, or variability in response, this analysis shows a statistically significant relationship between an independently determined PK parameter (ocular clearance) that describes the transit of aflibercept from the eye and a reduction in efficacy as indicated by an earlier retreatment (DRM) than anticipated based on clinical assessment via BCVA and CRT.

For those participants requiring a DRM, Cox proportional hazard modeling was performed to evaluate factors that may contribute to the need for a reduction in the dosing interval. The results of these analyses estimate a 260% higher rate for DRMs for participants with nAMD compared to participants with DME and DR. After accounting for indication (nAMD or DME and DR), ocular clearance of free aflibercept and baseline CRT were identified as significant covariates contributing to the need for DRM. Within an indication (nAMD or DME and DR), for participants with the same ocular clearance of free aflibercept, a 52.8% higher rate of DRM is predicted for participants at the 75^{th} percentile vs 25^{th} percentile of baseline CRT. Similarly, for participants with the same baseline CRT, a 32.9% higher rate of DRM is predicted for participants at the 75^{th} vs 25^{th} percentile of ocular clearance of free aflibercept. The results of these analyses also estimate that the lower ocular clearance for HD drug product resulted in a 20.6% lower rate of DRM than would have been expected if the HD drug product had the same ocular clearance as 2 mg aflibercept.

Comparison of Pharmacokinetics Across Studies in Participants with Neovascular Age-Related Macular Degeneration or Diabetic Macular Edema. In the clinical development of HD aflibercept for treatment of AMD and DME, a dosage regimen of 8 mg IVT (3 initial monthly doses followed by q12w or q16w IVT dosing) was evaluated and compared to an aflibercept 2 mg IVT dosage regimen (3 or 5 initial monthly doses followed by q8w or q12w IVT dosing) in the clinical studies CANDELA, PULSAR, and PHOTON. This allowed for a direct comparison of the systemic exposures of free and adjusted bound aflibercept across the 3 studies. CANDELA and PULSAR studies included participants with nAMD while PHOTON study included participants with DME and DR.

Following single IVT administration of aflibercept 2 mg or HD aflibercept, the concentration-time profiles of free and adjusted bound aflibercept in plasma in participants who underwent dense sample collection for systemic drug concentrations (dense PK sub-study) after the initial dosing of aflibercept 2 mg or HD aflibercept presented as the HD drug product, respectively, were consistent between the 3 studies in participants with nAMD or DME (Figure 32).

The consistency of the concentration-time profiles for free and adjusted bound aflibercept between the nAMD and DME populations is further supported by Population PK analysis (Figure 33). Population PK estimated *post-hoc* concentration-time profiles and PK parameters for combined nAMD and DME populations following single IVT administration of 2 mg aflibercept or HD aflibercept are provided in Figure 33 and in Table 1-37 and Table 1-38.

**Table 1-37. Summary of Post-hoc Simulated Pharmacokinetic Parameters for Free Aflibercept in Plasma after Single Dose IVT Administration in the Combined nAMD and DME Population Treated Only in the Study Eye and Without Study Eye Dosing Modifications in the Dense PK Sub-studies (DPKS)**

| | | **Aflibercept** | | **HD Aflibercept** | |
|---|---|---|---|---|---|
| | | **2 mg IVT (N = 31)** | | **8 mg IVT (N = 50)** | |
| **PK Parameters** | **Unit** | **Mean (SD)** | **Median** | **Mean (SD)** | **Median** |
| **AUC₀₋₂₈** | **mg x day/L** | 0.282 (0.189) | 0.238 | 2.55 (2.31) | 2 |
| **Cₘₐₓ** | **mg/L** | 0.0394 (0.0391) | 0.0251 | 0.304 (0.267) | 0.222 |
| C_{trough}**,₂₈** | **mg/L** | < LLOQ (0) | < LLOQ | < LLOQ (0.00853) | < LLOQ |
| **tₘₐₓ** | **day** | 2.26 (0.783) | 2.16 | 2.8 (1.08) | 2.89 |

| | | | | | |
|---|---|---|---|---|---|
| AUC = area under the concentration-time curve, Cₘₐₓ = maximum (peak) concentration for a 28-day interval following dosing, C_{trough} = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, PK = pharmacokinetics, SD = Standard deviation, tₘₐₓ = median time to peak concentration; Note: Participants who had fellow eye treatment before day 28 are excluded. | | | | | |

**Table 1-38. Summary of Post-hoc Simulated Pharmacokinetic Parameters for Adjusted Bound Aflibercept in Plasma after Single Dose IVT administration in the Combined nAMD and DME Population Treated Only in the Study Eye and Without Study Eye Dosing Modifications in the Dense PK Sub-studies (DPKS)**

| | | Aflibercept | | **HD Aflibercept** | |
|---|---|---|---|---|---|
| | | **2 mg IVT (N = 31)** | | **8 mg IVT (N = 50)** | |
| **PK Parameters** | **Unit** | **Mean (SD)** | **Median** | **Mean (SD)** | **Median** |
| **AUC₀₋₂₈** | **mg x day/L** | 3.07 (1.31) | 3.05 | 10.8 (6.14) | 9.03 |
| **Cₘₐₓ** | **mg/L** | 0.142 (0.0616) | 0.139 | 0.507 (0.282) | 0.434 |
| C_{trough}**,₂₈** | **mg/L** | 0.105 (0.0393) | 0.0994 | 0.386 (0.21) | 0.338 |
| **tₘₐₓ** | **day** | 14.8 (5.65) | 13.7 | 15.5 (5.22) | 15.8 |

| | | | | | |
|---|---|---|---|---|---|
| AUC = area under the concentration-time curve, Cₘₐₓ = maximum (peak) concentration for a 28-day interval following dosing, C_{trough} = trough concentration, DME = diabetic macular edema, DPKS = dense pharmacokinetic sub-studies, IVT = intravitreally, nAMD = neovascular age-related macular degeneration, PK = pharmacokinetics , SD = standard deviation, tₘₐₓ = median time to peak concentration; Note: Participants who had fellow eye treatment before day 28 are excluded. | | | | | |

The corresponding observed and Population PK estimated *post-hoc* concentration-time profiles and PK parameters for participants with nAMD or DME are provided in Figure 36, Figure 37, Figure 38, Table 1-39 and Table 1-40.

**Table 1-39. Summary of Simulated Pharmacokinetic Parameters for Free Aflibercept in Plasma after Single Dose IVT Administration in Participants with nAMD or DME Treated Only in the Study Eye and Without Study Eye Dosing Modifications in the Dense PK Sub-studies (DPKS)**

| | | **2 mg IVT** | | | **8 mg IVT** | | |
|---|---|---|---|---|---|---|---|
| **PK Parameters** | **Unit** | **N** | **Mean (SD)** | **Median** | **N** | **Mean (SD)** | **Median** |
| **nAMD participants** | | | | | | | |
| **AUC₀₋₂₈** | mg x day/L | 21 | 0.302 (0.223) | 0.258 | 29 | 2.77 (2.77) | 1.95 |
| **Cₘₐₓ** | mg/L | | 0.0419 (0.0439) | 0.0258 | | 0.306 (0.302) | 0.172 |
| **C_{trough,28}** | mg/L | | < LLOQ (0) | < LLOQ | | < LLOQ (0.0094) | < LLOQ |
| **tₘₐₓ** | day | | 2.19 (0.606) | 2.16 | | 2.97 (1.08) | 3.05 |

| **DME participants** | | | | | | | |
|---|---|---|---|---|---|---|---|
| **AUC₀₋₂₈** | mg x day/L | 10 | 0.238 (0.0732) | 0.236 | 21 | 2.25 (1.45) | 2.17 |
| **Cₘₐₓ** | mg/L | | 0.0343 (0.0275) | 0.0212 | | 0.302 (0.216) | 0.265 |
| **C_{trough,28}** | mg/L | | < LLOQ (0) | < LLOQ | | < LLOQ (0.00732) | < LLOQ |
| **tₘₐₓ** | day | | 2.41 (1.09) | 2.23 | | 2.56 (1.06) | 2.36 |

| | | | | | | | |
|---|---|---|---|---|---|---|---|
| AUC₀₋₂₈ = area under the concentration-time curve, Cₘₐₓ = maximum (peak) concentration for a 28-day interval following dosing, C_{trough,28} = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, PK = pharmacokinetic, SD = Standard deviation, tₘₐₓ = median time to peak concentration. Note: Participants who had fellow eye treatment before day 28 are excluded. | | | | | | | |

**Table 1-40. Summary of Simulated Pharmacokinetic Parameters for Adjusted Bound Aflibercept in Plasma after Single Dose IVT administration in Participants with nAMD or DME Treated Only in the Study Eye and Without Study Eye Dosing Modifications in the Dense PK Sub-studies (DPKS)**

| | | **Adjusted Bound Aflibercept** | | | | | |
|---|---|---|---|---|---|---|---|
| | | **2 mg IVT** | | | **8 mg IVT** | | |
| **PK Parameters** | **Unit** | **N** | **Mean (SD)** | **Median** | **N** | **Mean (SD)** | **Median** |
| **nAMD participants** | | | | | | | |
| **AUC₀₋₂₈** | mg x day/L | 21 | 3.35 (1.44) | 3.16 | 29 | 11.8 (7.17) | 11 |
| **Cₘₐₓ** | mg/L | | 0.155 (0.0686) | 0.144 | | 0.558 (0.329) | 0.51 |
| | | | | | | | |
| **C_{trough,28}** | mg/L | | 0.113 (0.0418) | 0.113 | | 0.439 (0.23) | 0.415 |
| **tₘₐₓ** | day | | 14.4 (4.89) | 13.1 | | 16.9 (5.26) | 17.2 |

| **DME participants** | | | | | | | |
|---|---|---|---|---|---|---|---|
| **AUC₀₋₂₈** | mg x day/L | 10 | 2.46 (0.726) | 2.43 | 21 | 9.33 (4.06) | 8.39 |
| **Cₘₐₓ** | mg/L | | 0.115 (0.0317) | 0.117 | | 0.438 (0.187) | 0.4 |
| **C_{trough,28}** | mg/L | | 0.088 (0.0281) | 0.09 | | 0.314 (0.156) | 0.25 |
| **tₘₐₓ** | day | | 15.6 (7.21) | 15.4 | | 13.7 (4.65) | 12.9 |

| | | | | | | | |
|---|---|---|---|---|---|---|---|
| AUC₀₋₂₈ = area under the concentration-time curve, Cₘₐₓ = maximum (peak) concentration for a 28-day interval following dosing, C_{trough,28} = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, SD = standard deviation, tₘₐₓ = median time to peak concentration; Note: Participants who had fellow eye treatment before day 28 are excluded. | | | | | | | |

Following single IVT administration of 2 mg aflibercept or HD aflibercept presented as HD drug product, the concentration-time profiles of free aflibercept are characterized by an initial phase of increasing concentrations, as the drug moved from the ocular space into systemic circulation, followed by a mono-exponential elimination phase. The concentration time profiles of adjusted bound aflibercept in plasma are characterized by a slower attainment of Cₘₐₓ compared to free aflibercept. Following attainment of Cₘₐₓ, a sustained plateau of the concentration-time profiles of adjusted bound aflibercept in plasma was observed until approximately the end of the first dosing interval (Figure 32, Figure 33).

For participants who underwent dense blood sample collection for systemic drug concentrations across the CANDELA, PULSAR, and PHOTON studies, after the initial dosing of 2 mg IVT aflibercept (n = 34), observed concentrations of free aflibercept were detectable in 15 (44.1%) participants by week 1 and in 3 (8.8%) participants by week 2. For participants who underwent dense blood sample collection for systemic drug concentrations after the initial dosing of 8 mg IVT aflibercept (n = 54), observed concentrations of free aflibercept were detectable in 46 (85.2%) participants by week 1 and in 44 (77.8%) participants by week 2.

The observed and Population PK simulated free and adjusted bound aflibercept concentrations in plasma for up to 48 weeks are presented for the combined nAMD and DME population (Figure 34), and the nAMD (Figure 39) and DME (Figure 40) populations. Based on the Population PK analysis, the median time for free aflibercept concentrations to reach LLOQ following HDq12 or HDq16 was 3.5 weeks, which is more than double the median time needed to reach LLOQ (1.5 weeks) following aflibercept 2q8 (Table 1-41).

**Table 1-41. Summary of Model-Predicted Time to LLOQ of Free Aflibercept in Plasma Following IVT for Participants With nAMD and DME, Combined**

| **Regimen** | **Mean (SD) Week** | **Median (90% PI) Week** |
|---|---|---|
| **2q8** | 1.58 (0.712) | 1.5 (0.524, 2.82) |
| **HDq12** | 3.81 (1.61) | 3.51 (1.83, 6.81) |
| **HDq16** | 3.79 (1.58) | 3.50 (1.83, 6.73) |

| | | |
|---|---|---|
| Model-predicted time = time after a single IVT dose of the 2q8, HDq12 or HDq16 regimens. 2q8 = aflibercept 2 mg administered every 8 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, nAMD = neovascular age related macular degeneration, PI = prediction interval, SD = standard deviation | | |

The longer duration of systemic exposure to free aflibercept following HDq12 and HDq16 compared to the 2 mg aflibercept is attributed to not only a higher administered dose and nonlinear systemic target-mediated elimination, but also to a 34% slower ocular clearance of free aflibercept. The 34% slower ocular clearance of free aflibercept for HD aflibercept is attributed to a HD drug product effect which was identified as a statistically significant covariate in the Population PK model.

Ocular Elimination. Based on the Population PK analysis, HD aflibercept, presented as the HD drug product, was estimated to have a 34% slower clearance from the ocular compartment compared to the lower IVT doses of aflibercept (≤ 4 mg doses) that was presented as the standard, or reference drug product. The median time for the amount of free aflibercept to reach the adjusted LLOQ [the adjusted LLOQ imputes the LLOQ of free aflibercept in from the assay in plasma (that is, 0.0156 mg/L) times the assumed volume of the study eye compartment in the PK model (that is, 4 mL)] in the ocular compartment was estimated using Population PK simulation analyses, after a single 2 mg or 8 mg IVT dose. In the combined nAMD and DME population, the median time for the amount of free aflibercept to reach the adjusted LLOQ in the ocular compartment increased from 8.71 weeks after a 2 mg IVT dose to 15 weeks after an 8 mg IVT dose (i.e., the duration of free aflibercept ocular exposure following HD drug product is extended by approximately 6 weeks relative to 2 mg drug product). The slower ocular clearance and longer duration of free aflibercept ocular exposure for HD aflibercept are attributed to an HD aflibercept drug product effect. Assuming no HD aflibercept drug product effect (i.e., that the 8 mg IVT dose has the same ocular clearance as the 2 mg IVT dose), the Population PK simulated median time for the amount of free aflibercept to reach the adjusted LLOQ in the ocular compartment was only 10 weeks for 8 mg aflibercept, which is only 1.3 weeks longer than that for 2 mg aflibercept (Figure 35).

As the PULSAR and PHOTON studies were designed to assess non-inferiority of the HDq12 and HDq16 regimens versus the 2q8 regimen, it was of interest to estimate how long it takes for the amount of free aflibercept in the ocular compartment for the HDq12 and HDq16 regimens to reach the same amount of free aflibercept remaining in the ocular compartment for the 2q8 regimen at the end of an 8-week dosing interval (2q8 target). Using a modified approach, using Population PK simulation analyses in the combined nAMD and DME population, the median time for HDq12 and HDq16 regimens to reach the 2q8 target in the ocular compartment after single IVT administration was 14 weeks, suggesting that the HD aflibercept regimens may provide a 6-week longer duration of efficacy than the 2q8 regimen. In contrast, if there were no HD aflibercept drug product effect, the Population PK simulated median time for the amount of free aflibercept to reach the 2q8 target in the ocular compartment would be only 9.21 weeks for an 8 mg dose, representing an extension of only 1.21 weeks relative to the 2q8 regimen, and is consistent with the prior example.

High-Dose Aflibercept Drug Product. The totality of the composition of the HD drug product used to deliver the 8 mg dose is different from that for the 2 mg aflibercept IVT dose. Based on Population PK analysis, the HD aflibercept drug product is a statistically significant predictor of ocular clearance of free aflibercept that results in a slower ocular clearance for the HD aflibercept versus 2 mg aflibercept when administered by the IVT route. (Table 1-42). The slower ocular clearance and higher molar dose for the HD aflibercept drug product results in a longer duration of ocular exposure to free aflibercept compared to the 2 mg IVT dose. The slower ocular clearance of the HD aflibercept drug product is predicted to provide a 6-week longer duration of efficacy compared to 2q8, as the time to achieve the free aflibercept amount in the ocular compartment for the 2q8 regimen at the end of an 8-week dosing interval occurs 6 weeks later for the HD aflibercept drug product. Consistent with these predictions, the HDq12 and HDq16 regimens demonstrated noninferiority to the 2q8 regimen in the PHOTON (for DME only) and PULSAR studies. Correspondingly, a slower ocular clearance for the HD aflibercept drug product contributes in part to a longer duration of systemic exposure to free aflibercept for HD aflibercept versus the 2 mg IVT dose. The slower ocular clearance for HD aflibercept is attributed to a difference in the HD aflibercept drug product, not just an increase in the IVT dose from 2 mg to 8 mg. These results were further confirmed by a sensitivity analysis conducted in the final model.

**Table 1-42. Comparison of Clearance from the Ocular Compartment (QE) (Mean [95% CI]) of Aflibercept for HD Aflibercept and 2 mg Aflibercept**

| **Dose Group** | **Clearance from the Ocular Compartment (QE) Mean (95% CI) (mL/day)** | **k = QE/0.004 Mean (95% CI (day⁻¹)** |
|---|---|---|
| **2 mg Aflibercept** | 0.624 (0.577 - 0.674) | 0.156 (0.144 - 0.169) |
| **HD 8 mg Aflibercept** | 0.41 (0.367 - 0.458) | 0.102 (0.0916 - 0.115) |

| | | |
|---|---|---|
| QE = inter-compartmental clearance between ocular compartment and central compartment of free aflibercept, 95% CI of parameters are provided. | | |

Pharmacokinetic Conclusions. The concentration time profiles of free and adjusted bound aflibercept in plasma after the initial dose of HD aflibercept by IVT administration were consistent between all studies in participants with nAMD or DME. Population PK analysis confirmed no relevant differences in PK between the nAMD and DME populations, and therefore all subsequent analyses are presented for the combined nAMD and DME population.

Following the initial monthly IVT dose, the observed concentration-time profile of free aflibercept in plasma is characterized by an initial phase of increasing concentrations as the drug is absorbed from the ocular space into the systemic circulation, followed by a mono-exponential elimination phase. The longer duration of systemic exposure to free aflibercept for HD aflibercept is attributed to not only a higher administered dose and non-linear systemic target mediated elimination but also to a 34% slower ocular clearance of free aflibercept, which is statistically attributed to the HD drug product as a covariate in the expanded PopPK model. This slower than expected ocular clearance of free aflibercept when presented as the HD aflibercept drug product is simulated to provide a 6-week longer duration of efficacy compared to 2q8, as the time to achieve the free aflibercept amount in the ocular compartment for the 2q8 regimen at the end of an 8-week dosing interval occurs 6 weeks later for the HD aflibercept drug product. Consistent with these simulations for the 8mg presented as the HD drug product, the HDq12 and HDq16 regimens demonstrated noninferiority (at a longer treatment interval) to the 2q8 regimen presented as the reference drug product in the predefined statistical analysis plan for both the PHOTON (for DME only) and PULSAR phase 3 studies.

Based on expanded population PK analysis, following single IVT doses of 2 mg aflibercept and HD aflibercept, systemic exposures of free aflibercept (AUC₀₋₂₈ and Cₘₐₓ) in the combined nAMD and DME population increase in a greater than dose-proportional manner (approximately 9.0-fold and 7.7-fold). These results demonstrate and are consistent with the known nonlinear PK for free aflibercept. Bioavailability of free aflibercept following IVT administration is estimated to be approximately 72%, and the total volume of distribution of free aflibercept after IV administration is estimated to be approximately 7 L.

Following 3 initial monthly HD aflibercept doses, the population PK simulated mean accumulation ratio of free and adjusted bound aflibercept in plasma based on AUC was 1.16 and 2.28 in the combined DME and nAMD population. After the 3 initial monthly doses of HD aflibercept (presented as the HD drug product), no further accumulation of either free or adjusted bound aflibercept in plasma occurs as the dosing interval is extended from every 4 weeks to every 12 weeks or 16 weeks resulting in a decline in systemic concentrations of both free and adjusted bound aflibercept.

Amongst the covariates evaluated in the Population PK analysis, body weight was the covariate with the greatest impact on systemic exposures to free and adjusted bound aflibercept. For participants in the lowest quintile of body weight (38.1 kg to 64.5 kg), the predicted impact on systemic exposures (Cₘₐₓ and AUCₜₐᵤ) was modest, with 27% to 39% higher exposures to free aflibercept and 25% to 27% higher exposures to adjusted bound aflibercept when compared to the reference body weight range (73.5 to 83.5 kg). The effects of other covariates (age, albumin, disease population, and race, which included evaluation of Japanese race) on systemic exposures (Cₘₐₓ, AUCₜₐᵤ) to free and adjusted bound aflibercept were small (<25% increase in exposure for covariate subgroups relative to the reference group), with several of these other covariate effects correlating with a consistent trend in body weight. All of these covariates were independent of the HD drug product effect on ocular clearance and did not confound the interpretation of the HD drug product effect on the ocular clearance. No dosage adjustments of HD aflibercept are warranted based on the assessed covariates.

Mild to severe renal impairment also had a small impact on free aflibercept systemic exposures, as the increase in free aflibercept Cₘₐₓ and AUCₜₐᵤ in these participants was less than approximately 28% compared to participants with normal renal function. Adjusted bound aflibercept systemic exposures in participants with mild to severe renal impairment ranged from 13% to 39% higher compared to participants with normal renal function. Here too, the perceived impact of renal impairment is best explained by the associated decrease in body weight with increasing renal impairment. Mild hepatic impairment had no effect on systemic exposures to free and adjusted bound aflibercept. No dosage adjustments of aflibercept are warranted for these populations.

Model-Based Exposure-Response Analysis for Proportion of Participants Requiring Dose Regimen Modification Cox proportional hazard modeling was performed to evaluate factors that may contribute to the need for a reduction in the dosing interval. Within any one specific patient population, nAMD, DME (with and without DR), ocular clearance of free aflibercept and baseline CRT were identified as significant predictors of time to DRM. Within an indication (nAMD or DME (with and without DR)), for participants with the same ocular clearance of free aflibercept, a 52.8% higher rate of DRM is modeled for participants at the 75^{th} vs 25^{th} percentile of baseline CRT. Similarly, for participants with the same baseline CRT, a 32.9% higher rate of DRM is modeled for participants at the 75^{th} vs 25^{th} percentile of ocular clearance of free aflibercept, corresponding to those participants who are predicted to have the lowest aflibercept concentration in the eye. These results are shown in Table 1-43. The outcomes of these analyses also estimate that the slower ocular clearance for HD aflibercept, attributable to a HD drug product effect, results in a 20.6% lower rate of DRM than would have been expected if the HD drug product had the same ocular clearance as 2 mg aflibercept presented as the reference drug product.

**Table 1-43. Hazard Ratio Contrasts for Time to DRM Model**

| **Effect** | **Hazard Ratio** |
|---|---|
| Baseline CRT | 1.53 (1.34 - 1.75) |
| Ocular Clearance (QE) | 1.33 (1.18 - 1.49) |
| Indication AMD Participants: DME Participants | 3.6 (2.56-5.06) |

| | |
|---|---|
| AMD = age-related macular degeneration, CRT = central retinal thickness, DME = diabetic macular edema, DRM = dose regimen modification, QE = ocular clearance | |

Dose-Response and Exposure-Response Conclusions. As the IVT dose increased from 2 mg of aflibercept to 8 mg of HD aflibercept, no further increase in PD effect (decrease in CRT) was observed 4 weeks after each initial q4w dose through 12 weeks, in either the nAMD or DME population. Despite 2 mg of aflibercept (as reference drug product) and 8 mg of HD aflibercept (as HD drug product) having similar PD effect during the initial 3 x q4w dosing period, the 8 mg HD drug product provided a longer duration of pharmacological effect in the maintenance phase compared to 2 mg aflibercept. In nAMD participants, the small fluctuations in CRT or CST during a maintenance dosing interval attenuated over time for all dosing regimens, with only minor numerical differences observed between treatment groups. For DME participants, a greater reduction in CRT was observed from weeks 16 to 20 for 2q8 compared to both HD aflibercept regimens (HDq12 and HDq16). This is attributable to a difference in the number of doses administered during this time period, with the 2q8 regimen receiving 2 additional initial q4w doses at weeks 12 and 16 compared to the HD aflibercept regimens which received their last initial q4w dose at week 8. These differences in CRT did not translate into any meaningful difference in mean BCVA response. The fluctuations in CRT response over the course of a maintenance dosing interval attenuated over time for all dosing regimens. For participants with nAMD or DME, the HDq12 and HDq16 regimens provided rapid and durable response in CRT and BCVA over 48 weeks of treatment, with the majority of participants maintaining their randomized HDq12 (79% nAMD; 91% DME) and HDq16 (77% nAMD; 89% DME) treatment regimens, without the need for DRM. Ocular clearance of free aflibercept and baseline CRT were identified as significant covariates contributing to the need for DRM. Higher ocular clearance of free aflibercept and higher baseline CRT (indicative of more severe disease) were associated with an increased rate of DRM. The slower ocular clearance for HD aflibercept, attributable to a HD drug product effect, is estimated to result in a 20.6% lower rate of DRM compared to HD aflibercept if the same ocular clearance was observed as the 2 mg aflibercept when presented as the reference drug product.

Overall Clinical Pharmacology Conclusions. Consistent with the known target-mediated kinetic properties exhibited at low plasma concentrations of aflibercept, free aflibercept exhibited nonlinear systemic PK over the 2 mg to 8 mg IVT dose range. Following the initial IVT dose, the concentration-time profile for free aflibercept in plasma is characterized by an initial absorption phase as drug moves from the ocular space into the systemic circulation. This absorption phase is followed by a mono-exponential elimination phase. The concentration time profile of adjusted bound aflibercept in plasma following the initial IVT dose is characterized by a slower attainment of Cₘₐₓ (tₘₐₓ) compared to free aflibercept, after which the concentrations are sustained or slightly decrease until the end of the dosing interval.

Analyses of observed PK by cross-study comparison and by Population PK analyses suggested similar systemic PK in the nAMD and DME populations. Following IVT administration, Population PK methods estimate the bioavailability of free aflibercept at 72%, a median tₘₐₓ of 2.89 days, and mean Cₘₐₓ of 0.304 mg/L for the 8 mg dose of HD aflibercept. As the aflibercept IVT dose increased from 2 mg to 8 mg and the treatment changes from 2 mg aflibercept (presented as the reference drug product) to 8 mg HD aflibercept (presented as the HD drug product), consistent with the known target-mediated related nonlinear PK of free aflibercept mean AUC₀₋₂₈ and Cₘₐₓ for free aflibercept increased in a greater than dose-proportional manner. After IV administration, free aflibercept has a low total volume of distribution of 7 L, indicating distribution largely in the vascular compartment. Following 3 initial monthly HD aflibercept IVT doses, the mean accumulation ratio of free and adjusted bound aflibercept in plasma based on AUC is 1.16 and 2.28. After the 3 initial monthly doses of HD drug product, no further accumulation of either free or adjusted bound aflibercept in plasma occurred as the dosing interval is extended from every 4 weeks to every 12 weeks or 16 weeks resulting in an expected decline in systemic concentrations of both free and adjusted bound aflibercept.

The longer duration of systemic exposure to free aflibercept for HD aflibercept is attributed to not only a higher administered dose and nonlinear systemic target-mediated elimination, but also to a 34% slower ocular clearance of free aflibercept. This 34% slower ocular clearance of free aflibercept for HD aflibercept is attributed to a HD drug product effect, which was identified as a statistically significant covariate in the Population PK model. Based on the extended PopPK model, the slower ocular clearance of the HD aflibercept drug product provides a 6-week longer duration of efficacy compared to 2q8 when presented as the reference drug product. Resulting from this unexpected and non-obvious slower ocular clearance, was a longer than expected ocular residence time, leading to a greater than expected proportion of patients able to be maintained on the longer dosing intervals of q12 and q16 with HD drug product. Consistent with these predictions, the HDq12 and HDq16 regimens demonstrated non-inferiority to the 2q8 regimen in the PHOTON and PULSAR studies.

Body weight was the covariate with the greatest impact on systemic exposures to free and adjusted bound aflibercept. For participants in the lowest quintile of body weight (38.1 to 64.5 kg), the predicted impact on free aflibercept Cₘₐₓ and AUCₜₐᵤ was modest, with 27% to 39% higher exposures and 25% to 27% higher for adjusted bound aflibercept when compared the reference body weight range (73.5 to 83.5 kg). The effects of other covariates (age, albumin, disease population. and race, which included evaluation of Japanese race) on systemic exposures (Cₘₐₓ, AUCₜₐᵤ) to free and adjusted bound aflibercept were small (<25% increase in exposure for covariate subgroups relative to the reference group). These other covariates did not confound the assessment of the effect of HD drug product on ocular clearance. No dosage adjustments of aflibercept are warranted based on the above findings.

No formal studies were conducted in special populations (e.g., participants with renal or hepatic impairment) because like most therapeutic proteins, the large molecular weight of aflibercept (approximately 115 kDa) is expected to preclude elimination via the kidney, and its metabolism is expected to be limited to proteolytic catabolism to small peptides and individual amino acids. Mild to severe renal impairment had a small impact on free aflibercept systemic exposures, as the increase in free aflibercept Cₘₐₓ and AUCₜₐᵤ in these participants was less than approximately 28% compared to participants with normal renal function. Adjusted bound aflibercept systemic exposures in participants with mild to severe renal impairment ranged from 13% to 39% higher compared to participants with normal renal function. The perceived impact of renal impairment is explained by the associated decrease in body weight with increasing renal impairment. Mild hepatic impairment had no effect on systemic exposures to free and adjusted bound aflibercept. No dosage adjustments of aflibercept are warranted in these populations.

Dose-response analyses of CRT performed in the CANDELA, PULSAR, and PHOTON studies indicated no further increase in PD effect for 2 mg aflibercept and HD aflibercept IVT 4 weeks after each initial q4W dose through 12 weeks. Despite the 2 mg aflibercept and HD aflibercept having similar PD effect during the initial q4w dosing period, the HD aflibercept drug product provided a longer duration (up to 16 weeks) of pharmacological effect in the maintenance phase than the 2 mg dose presented as the reference drug product (up to 8 weeks).

For participants with nAMD or DME, the HDq12 and HDq16 regimens provided rapid and durable response in CRT and BCVA over 48 weeks of treatment, with the majority of participants maintaining their randomized HDq12 (79% nAMD; 91% DME) and HDq16 (77% nAMD; 89% DME) treatment regimens, without the need for DRM.

Ocular clearance of free aflibercept and baseline CRT were identified as significant covariates contributing to the need for DRM. Higher ocular clearance and higher baseline CRT (indicative of more severe disease) were associated with an increased rate of DRM. For HD aflibercept, the slower ocular clearance and longer duration of ocular exposure to free aflibercept, attributable to the HD drug product effect, have been identified in an exposure-response analysis to result in a reduction of DRM of 20.6%.

Immunogenicity of HD aflibercept administered IVT was low across all treatment groups for both nAMD and DME participants. During the 48-week treatment with aflibercept administered IVT, the incidence of ADA in the combined 8 mg HD aflibercept treatment group was 2.7% (25/937 participants with nAMD or DME). None of the TE ADA positive samples were found to be positive in the NAb assay. Based on the lack of impact of ADA on concentrations of aflibercept in plasma, no effect on efficacy is anticipated. Positive responses in the ADA assays were not associated with significant AEs.

Overall, the clinical pharmacology data support the proposed aflibercept dosing regimens of 8 mg every 8 to 16 weeks after 3 initial monthly doses for the treatment of adults with nAMD, DME (with and without DR).

### Baseline Characteristics of Patients Treated With Aflibercept 8 mg Who Did or Did Not Maintain Their Randomized Dosing Intervals Through Week 48

At baseline, best-corrected visual acuity (BCVA), central retinal thickness (CRT), and Diabetic Retinopathy Severity Scale (DRSS) scores were generally balanced across all 3 treatment groups in the overall population. Of patients completing the Week 48 visit, 273/300 (91.0%) in the HDq12 group and 139/156 (89.1%) in the HDq16 group maintained their randomized dosing intervals. In the HDq12 and HDq16 groups, 27/300 (9.0%) and 17/156 (10.9%) patients, respectively, met DRM criteria and had their dosing intervals shortened. Mean (SD) baseline BCVA in eyes with maintained vs shortened dosing intervals was 63.9 (10.1) vs 59.4 (10.0) letters in the HDq12 group and 62.7 (11.2) vs 53.7 (12.8) letters in the HDq16 group. Mean (SD) central retinal thickness (CRT) at baseline (maintained vs shortened dosing intervals) was 444.9 (129.8) vs 511.4 (117.5) µm in the HDq12 group and 447.1 (112.5) vs 534.8 (134.3) µm in the HDq16 group. Baseline DRSS score (maintained vs shortened dosing intervals) was 47 or worse in 33.7% vs 40.7% of patients in the HDq12 group and 26.6% vs 41.2% of patients in the HDq16 group. No clinically meaningful differences were observed based on age, BMI, or HbA1c at baseline.

The vast majority of patients with DME who received aflibercept 8 mg maintained 12- or 16-week dosing. Patients who did not maintain their randomized dosing intervals appeared to have more severe disease at baseline than patients who maintained their randomized dosing intervals, and this trend was more pronounced in the HDq16 group.

### Treatment intervals

For masking purposes, assessments for dose regimen modifications (DRMs) were performed in all participants at all visits (through the interactive web response system [IWRS]) beginning at week 16. Based on these assessments, participants in the HD groups might have had their treatment intervals shortened (year 1 and year 2) or extended (year 2). The minimum interval between injections was 8 weeks which was considered a rescue regimen for participants randomized to HD aflibercept and unable to tolerate a dosing interval greater than every 8 weeks. Participants in the aflibercept 2 mg group remained on fixed q8 dosing throughout the study (i.e., did not have modifications of their treatment intervals regardless of the outcomes of the DRM assessments).

During the first year, beginning at week 16, participants in the HD groups had the dosing interval shortened (at the visits described below) if BOTH of the following criteria were met:
1. > 10 letter loss in BCVA from week 12 in association with persistent or worsening DME; AND
2. > 50 µm increase in CRT from week 12
(It should be noted that the change in CRT for these criteria was assessed at the site).

If a participant in the HDq12 group or the HDq16 group met both criteria at week 16 or week 20, the participant was dosed with 8 mg aflibercept at that visit and continued on a rescue regimen (aflibercept 8 mg, every 8 weeks). If a participant in the HDq16 group who had not met the criteria at week 16 or 20 met both criteria at week 24, the participant was dosed with 8 mg aflibercept at that visit and continued on q12 week dosing.

For participants whose interval was not shortened to q8 dosing at or before week 24, the interval was shortened if the DRM criteria were met at a subsequent dosing visit. Participants in the HDq12 group who met the criteria received the planned dose at that visit and then continued on a rescue regimen (aflibercept 8 mg, every 8 weeks). Participants in the HDq16 group who met these criteria received the planned dose at that visit and were to be continued on an every 12 week regimen if they were on a 16-week interval, or switched to the rescue regimen (aflibercept 8 mg, every 8 weeks) if they were previously shortened to a 12-week interval. Therefore, a participant randomized to HDq16 whose injection interval had been shortened to q12 had their injection interval further shortened to q8 if these criteria were met at any subsequent dosing visit.

From week 52 through the end of study (year 2), all participants in the HD groups will continue to have the interval shortened in 4-week intervals by four weeks if the DRM criteria for shortening are met at dosing visits using the DRM criteria described above for year 1. As in year 1, the minimum dosing interval for participants in all treatment groups is every 8 weeks.

In addition to shortening of the interval, all participants in the HD groups (including participants whose interval was shortened during year 1) may be eligible for interval extension (by 4-week increments), if BOTH the following criteria are met at dosing visits in year 2:
1. <5 letter loss in BCVA from week 12; AND
2. CRT <300 µm on SD-OCT (or <320 µm on Spectralis SD-OCT).
For participants who do not meet the criteria for shortening or extension of the interval, the dosing interval will be maintained.

As in year 1, all participants in all treatment groups (including the 2q8 group) will be evaluated against both DRM criteria at all visits through the IWRS for masking purposes. However, changes to dosing schedule will only be implemented as described above for those participants randomized to HDq12 or HDq16 treatment groups. No changes to the dosing schedule will be made to the 2q8 treatment group at any time.

The optional extension phase will begin at week 96, after all procedures at the EOS visit (week 96) have been completed and will continue through week 156.

Table 1-45 presents the proportion of participants who maintained their assigned dosing intervals through week 48 and week 60, those whose intervals were shortened through week 48 and week 60, and those whose intervals were extended between week 48 and week 60 (exploratory endpoints) among those who completed the respective timepoints. The vast majority of participants in the pooled HD group (≥ 91%) were able to maintain their target interval of either 12 or 16 weeks through week 60 (Table 1-45).

**Table 1-45. Summary of Treatment Exposure in the Study Eye (Safety Analysis Set Completing Week 48 and Week 60, respectively)**

| **Through Week 48** | **2q8 (N=1 57)** | **HD** | | |
|---|---|---|---|---|
| | | **HDq12 (N=300)** | **HDq16 (N=156)** | **All HD (N=456)** |
| Patients maintained with q12 or longer dosing interval, n (%) | - | 273 (91.0%) | 150 (96.2%) | 423 (92.8%) |
| Patients maintained with q16 dosing interval, n (%) | - | - | 139 (89.1%) | - |
| Patients with q12 or longer dosing interval as the last ^{a} intended dosing interval, n (%) | - | 262 (87.3%) | 146 (93.6%) | 408 (89.5%) |
| Patients with q16 dosing interval as the last ^{a} intended dosing interval, n (%) | - | - | 136 (87.2%) | - |
| Patients shortened to q8 dosing interval at week 16, n (%) | - | 3 (1.0%) | 1 (0.6%) | 4 (0.9%) |
| Patients shortened to q8 dosing interval at week 20, n (%) | - | 12 (4.0%) | 3 (1.9%) | 15 (3.3%) |
| Patients with a shortened dosing interval anytime, n (%) | - | 27 (9.0%) | 17 (10.9%) | 44 (9.6%) |
| Patients with a shortened dosing interval to q8 anytime, n (%) | - | 27 (9.0%) | 6 (3.8%) | 33 (7.2%) |
| Patients with a shortened dosing interval to q12 anytime, n (%)b | - | - | 13 (8.3%) | - |

| **Through Week 60** | **2q8 (N=1 55)** | **HDq12 (N=289)** | **HDq16 (N=152)** | **All HD (N=441)** |
|---|---|---|---|---|
| Patients maintained with q12 or longer dosing interval, n (%) | - | 261 (90.3%) | **142** (93.4%) | 403 (91.4%) |
| Patients maintained with q16 or longer dosing interval,- n (%) | - | - | 130 (85.5%) | - |
| Patients with q12 or longer dosing interval as the last ^{c} intended dosing interval, n (%) | - | 248 (85.8%) | 136 (89.5%) | 384 (87.1%) |
| Patients with q16 or longer dosing interval as the last ^{c} intended dosing interval, n (%) | - | 123 (42.6%) | 124 (81.6%) | 247 (56.0%) |
| Patients with q20 dosing interval as the last ^{c} | - | 0 | 52 (34.2%) | 52 (11.8%) |
| intended dosing interval, n (%) | | | | |
| Patients shortened to q8 dosing interval at week 16, n (%) | - | 3 (1.0%) | 1 (0.7%) | 4 (0.9%) |
| Patients shortened to q8 dosing interval at week 20, n (%) | - | 12 (4.2%) | 3 (2.0%) | 15 (3.4%) |
| Patients with a shortened dosing interval anytime, n (%) | - | 28 (9.7%) | 22 (14.5%) | 50 (11.3%) |
| Patients with a shortened dosing interval to q8 anytime, n (%) | - | 28 (9.7%) | 10 (6.6%) | 38 (8.6%) |
| Patients with a shortened dosing interval to q12 anytime,- n (%) b | - | - | 20 (13.2%) | 20 (4.5%) |
| Patients never extended dosing interval, n (%) | 155 | 156 | 93 (61.2%) | 249 |
| | (100 %) | (54.0%) | | (56.5%) |
| Patients extended dosing interval anytime, n (%) | 0 | 133 | 59 (38.8%) | 192 |
| | | (46.0%) | | (43.5%) |

| | | | | |
|---|---|---|---|---|
| Abbreviations: 2q8= Aflibercept 2 mg administered every 8 weeks after 5 initial injections at 4-week intervals; HDq12= High dose aflibercept 8 mg administered every 12 weeks after 3 initial injections at 4-week intervals; HDq16= High dose aflibercept 8 mg administered every 16 weeks after 3 initial injections at 4-week intervals; All HD= Pooled HDq12 and HDq16 groups; n = number; q8= every 8 weeks; q12= every 12 weeks; q16= every 16 weeks. Hyphen indicates categories that do not apply. a. Refers to the patient's assigned interval at week 48. b. Includes participants who were only shortened to q12 as well as participants who were shortened to q12 and further shortened to q8. c. Refers to the patient's assigned interval at week 60. Study drugs given at week 48 or beyond were not included in this table. Study drugs given at week 60 or beyond were not included in this table. | | | | |

### Summary

This is an ongoing Phase 2/3, multi-center, randomized, double-masked study in participants with DME involving the center of the macula that is investigating the efficacy and safety of intravitreal (IVT) HD aflibercept injection (8 mg). The primary objective of this study was to determine if treatment with HD aflibercept at intervals of 12 or 16 weeks (HDq12 or HDq16) provided non inferior BCVA compared to 2 mg aflibercept dosed every 8 weeks (2q8).

A total of 660 participants were randomized into 3 treatment groups, of whom 658 participants received at least 1 dose of study treatment. All treated participants were included in the safety analysis set (SAF). The analysis of efficacy was based on the full analysis set (FAS) (n=658, which was identical to the SAF), and the per protocol set (PPS) (n=649), which included approximately 98% of subjects randomized to each treatment group. The analysis of general PK assessments was based on the data in the pharmacokinetic analysis set (PKAS) (n=648), and the analysis in the dense PK study on the data of the dense pharmacokinetic analysis set (DPKS) (n=35).

The FAS (and SAF) had 401 (60.9%) male and 257 (39.1%) female participants aged from 24 to 90 years (median: 63 years). Most participants were White (71.6%) or Asian (15.3%). The mean (SD) visual acuity score BCVA at baseline was 62.5 (10.86) letters. Participants were stratified by screening CRT category and a majority had a CRT ≥ 400 microns (58.1%); the mean CRT was well balanced across groups and ranged from 449.1 to 457.2 microns. The treatment groups were generally well balanced with respect to demographics. At baseline, the mean BCVA, IOP, CRT, prior DME treatment, and DRSS score were comparable across groups.

The primary endpoint was the change from baseline in BCVA (as measured by ETDRS letter score) at week 48. The primary endpoint was met: the HDq12 and HDq16 groups demonstrated non-inferiority to 2q8 using the margin of 4 letters with least square (LS) mean change from baseline in BCVA of 8.10 letters (HDq12) and 7.23 letters (HDq16), respectively versus 8.67 letters in the 2q8 group. The LSₘₑₐₙ differences compared to 2q8 (95% CI) were 0.57 (-2.26, 1.13) and -1.44 (-3.27, 0.39) for HDq12 and HDq16, respectively. The robustness of these results for the primary endpoint were supported by the sensitivity analyses and the PPS analysis for the primary efficacy endpoint as the supplementary analysis.

The non-inferiority in mean change in BCVA was achieved in the context of participants in the HD groups being treated at extended dosing intervals compared to the 2q8 group. The vast majority of participants were treated only according to their randomized dosing interval, 90% and 85% in the HDq12 and HDq16 groups, respectively, through week 60 without the need for dose regimen modification.

The key secondary efficacy endpoint, the proportion of participants with a ≥ 2 step improvement in DRSS score, was met for HDq12 at week 48 (non-inferiority to 2q8). The non-inferiority margin was pre-specified at 15%, however HDq12 also met a 10% NI margin. In Cochran-Mantel-Haenszel (CMH)-weighted estimates, the adjusted difference (95% CI) was 1.98% (-6.61, 10.57) for HDq12 and 7.52% (-16.88, 1.84) for HDq16, respectively versus 2q8. Non-inferiority was not met for this key secondary endpoint in the HDq16 group, and therefore the hierarchical testing strategy was stopped at this point. The HDq16 group had more participants with moderate to mild (level 43 or better as opposed to level 47 or worse) retinopathy at baseline. Thus, fewer participants in this group would have been expected to achieve ≥ 2-step improvement in DRSS. This was apparent at week 12, a timepoint at which all groups had received the same number of doses; at this visit, the HDq16 group had a numerically lower proportion of participants with ≥ 2-step improvements in DRSS compared to the other treatment groups.

Overall, no relevant differences in the primary and key secondary endpoints were identified on a descriptive level across the various levels of the subgroups prespecified for analysis, which were categorized based on demographic and disease characteristics, including sex, age group, race, ethnicity, baseline BCVA, geographic region, baseline CRT category, and prior DME treatment.

The descriptive analyses of the additional secondary and exploratory endpoints (including proportion of participants without retinal fluid at the foveal center, mean change in CRT, and mean change in leakage on fluorescein angiography) evaluated at week 48 and week 60 suggested similar outcomes for HD aflibercept dosed q12 or q16 compared to 2q8, providing further evidence for the benefit of HD compared to 2q8. Robust reductions from baseline in CRT were observed in both HD groups beginning at week 4 through week 60. Some fluctuation in mean CRT was seen in all treatment groups with attenuation in magnitude over the course of 60 weeks. Despite these fluctuations, similar functional and anatomic outcomes were observed at week 60 across treatment groups.

The safety profile of HD was similar to that of 2 mg aflibercept. The overall rates of ocular and non-ocular TEAEs and SAEs reported up to week 60 were similar across the treatment groups. Most of the reported TEAEs were evaluated as mild and resolved within the observation period with no need to permanently discontinue the study drug. Ocular TEAEs in the study eye that resulted in discontinuation of the study drug affected few participants; 2 (0.6%) participants in the HDq12 group and no participants in the 2q8 and HDq16 groups. Similarly, non-ocular TEAEs resulted in discontinuation of the study drug in few participants; 3 (1.8%) participants in the 2q8 group and 9 (1.8%) participants in the Pooled HD groups.

A total of 18 deaths were reported during this study. None of the deaths were considered related to study drug or study procedure. All cases of death were consistent with concurrent medical conditions and the complications of these conditions associated with an older population.

No dose-relationship in the incidence or the types of TEAEs was apparent between participants in the HD groups and the 2q8 group. The results of the subgroup analyses of the TEAEs were comparable to those in the entire study population and did not suggest clinically relevant differences between the treatment groups in any of the subgroups examined.

The analyses of laboratory data, vital signs, and ECG data (including QT interval) did not show any clinically meaningful changes over time within the HD groups and the 2q8 group or differences between the groups.

There were no clinically meaningful trends in mean or median changes from baseline to pre-dose intraocular pressure (IOP) in the study eye in any treatment group through week 4860, and the proportion of participants meeting the pre-defined IOP criteria was comparable across treatment groups.

After the initial aflibercept dose of 2 mg (2q8) or 8 mg (HDq12+HDq16), the concentration-time profiles of free aflibercept were characterized by an initial phase of increasing concentrations as the drug moved from the ocular space into systemic circulation with a median time to peak concentration (tₘₐₓ) of 0.268 to 0.965 days followed by a mono-exponential elimination phase. The concentration time profiles of adjusted bound aflibercept were characterized by a slower attainment of peak concentration (Cₘₐₓ) compared to free aflibercept with a median tₘₐₓ of 14 days. Following attainment of Cₘₐₓ, a sustained plateau of the concentration-time profile was observed until approximately the end of the dosing interval.

As the intravitreal (IVT) dose of aflibercept increased from 2 mg to 8 mg (a 4-fold increase in dose), the mean Cₘₐₓ and AUCₗₐₛₜ for free aflibercept increased in a greater than dose-proportional manner (approximately 12 to 14-fold). Conversely, mean Cₘₐₓ and AUCₗₐₛₜ for adjusted bound aflibercept increased in a slightly less than dose proportional manner (approximately 3 to 4-fold). These findings are consistent with historical data and the known nonlinear target-mediated kinetics of aflibercept.

Following the third initial monthly IVT dose of aflibercept, based on the ratio of aflibercept concentration at week 12 to week 4 (C_{week12}/C_{week4}), the accumulation of free aflibercept ranged from 1.8 to 2.0 for the 8 mg treatments. The accumulation of free aflibercept could not be determined for the 2 mg treatment since all week 12 aflibercept concentrations were below the limit of quantitation (BLQ). The accumulation of adjusted bound aflibercept ranged from 1.5 to 1.7 for the 2 mg and 8 mg treatments.

The pharmacokinetics of free and adjusted bound aflibercept were similar between Japanese and non-Japanese participants enrolled in the dense PK sub-study.

Immunogenicity was low across all treatment groups. Out of the 541 participants included in the anti-drug antibody analysis set (AAS), the incidence of TE anti-drug antibody (ADA) in the 2q8, HDq12, and HDq16 treatment groups during the 48-week period of treatment with intravitreally administered aflibercept was 0/137 (0%), 3/263 (1.1%), and 2/141 (1.4%), respectively; all of these responses were of low maximum titer. None of the ADA positive samples were found to be positive in the neutralizing antibody (Nab) assay.

All references cited herein are incorporated by reference to the same extent as if each individual publication, database entry (*e*.*g*., Genbank sequences or GeneID entries), patent application, or patent, was specifically and individually indicated to be incorporated by reference. This statement of incorporation by reference is intended by Applicants to relate to each and every individual publication, database entry (*e*.*g*., Genbank sequences or GeneID entries), patent application, or patent, each of which is clearly identified in even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

### Embodiments of the description:

1. A method for treating or preventing diabetic retinopathy and/or diabetic macular edema in a subject in need thereof comprising administering one or more doses of aflibercept at an interval and quantity whereby
   - the clearance of free aflibercept from the ocular compartment is about 0.3, 0.4, 0.41 or 0.37-0.46 mL/day after an intravitreal injection of aflibercept,
   - the time for the amount for free aflibercept to reach the lower limit of quantitation (LLOQ) in the ocular compartment of a subject after said intravitreal injection of aflibercept is about 15 weeks; and/or
   - the time for free aflibercept to reach the lower limit of quantitation (LLOQ) in the plasma of the subject after said intravitreal injection of aflibercept is about 3.8, 3.5 or 3.5-3.8 weeks.
2. A method for slowing the clearance of free aflibercept from the ocular compartment after an intravitreal injection relative to the rate of clearance of aflibercept from the ocular compartment after an intravitreal injection of 2 mg or ≤ 4 mg aflibercept comprising
   intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
3. The method of any one of embodiments 1-2 wherein the clearance of free aflibercept from the ocular compartment is about 34% slower than that from the ocular compartment after an intravitreal injection of 2 or ≤ 4 mg aflibercept.
4. The method of any one of embodiments 1-3 wherein the clearance of free aflibercept from the ocular compartment is about 0.37-0.46 mL/day or 0.41 mL/day after an intravitreal injection of ≥ 8 mg aflibercept.
5. A method for increasing the duration of efficacy and/or the time for the amount of free aflibercept to reach the lower limit of quantitation (LLOQ) in the ocular compartment of a subject after an intravitreal injection of aflibercept, relative to the time to reach LLOQ of the amount of free aflibercept in the ocular compartment of a subject after an intravitreal injection of about 2 mg or ≤4 mg aflibercept, comprising intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
6. The method of embodiment 5 wherein the duration of efficacy and/or the time for the amount of free aflibercept to reach the lower limit of quantitation (LLOQ) in the ocular compartment of a subject after said intravitreal injection of aflibercept is increased by about 5 or 6 weeks, relative to the time to reach LLOQ of the amount of free aflibercept in the ocular compartment of a subject after an intravitreal injection of about 2 or ≤4 mg aflibercept.
7. The method of any one of embodiments 5-6 wherein the time for the amount of free aflibercept to reach the lower limit of quantitation (LLOQ) in the ocular compartment of a subject after said intravitreal injection of aflibercept is increased by more than about 1, 2, 1.2 or 1.3 weeks relative to the time to reach LLOQ of free aflibercept in the ocular compartment of a subject after an intravitreal injection of about 2 or ≤4 mg aflibercept.
8. The method of any one of embodiments 5-7 wherein the time for the amount for free aflibercept to reach the lower limit of quantitation (LLOQ) in the ocular compartment of a subject after said intravitreal injection of ≥ 8 mg aflibercept is about 15 weeks.
9. The method of any one of embodiments 5-8 wherein the time for the amount of free aflibercept to reach the lower limit of quantitation (LLOQ) in the ocular compartment of a subject after said intravitreal injection of ≥ 8 mg aflibercept is greater than about 8, 8.7, 8.71, 9, 9.2, 9.21 or 10 weeks.
10. A method for increasing the time for free aflibercept to reach the lower limit of quantitation (LLOQ) in the plasma of a subject after an intravitreal injection of aflibercept relative to the time to reach LLOQ of free aflibercept in the plasma of a subject after an intravitreal injection of about 2 or ≤4 mg aflibercept, comprising intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
11. The method of embodiment 10 wherein said LLOQ of free aflibercept measured in plasma is about 0.0156 mg/L.
12. The method of any one of embodiments 10-11 wherein the time for free aflibercept to reach the lower limit of quantitation (LLOQ) in the plasma of a subject after said intravitreal injection of aflibercept is increased by about 2 weeks relative to the time to reach LLOQ of free aflibercept in the plasma of a subject after an intravitreal injection of about 2 mg aflibercept.
13. The method of any one of embodiments 10-12 wherein the time for free aflibercept to reach the lower limit of quantitation (LLOQ) in the plasma of a subject after said intravitreal injection of ≥ 8 mg aflibercept is about 3, 3.5, 3.8 or 4 weeks.
14. The method of any one of embodiments 10-13 wherein the time for free aflibercept to reach the lower limit of quantitation (LLOQ) in the plasma of a subject after said intravitreal injection of ≥ 8 mg aflibercept is greater than about 1.5 or 1.6 weeks.
15. The method of any one of embodiments 1-14 wherein ≤ 4mg is about 2 mg or 2-4 mg.
16. The method of any one of embodiments 1-15 wherein the subject suffers from diabetic retinopathy and/or diabetic macular edema.
17. The method of any one of embodiments 1-16 wherein the ≥8 mg aflibercept is in an aqueous pharmaceutical formulation comprising histidine-based buffer.
18. The method of any one of embodiments 1-17 wherein the ≥8 mg aflibercept is in an aqueous pharmaceutical formulation comprising arginine.
19.The method of any one of embodiments 1-18 wherein the ≥8 mg aflibercept is in an aqueous pharmaceutical formulation having a pH of about 5.8.
20.The method of any one of embodiments 1-19 wherein the ≥8 mg aflibercept is in an aqueous pharmaceutical formulation comprising a sugar or polyol.
21.The method of any one of embodiments 1-20 wherein the ≥8 mg aflibercept is in an aqueous pharmaceutical formulation comprising a sucrose.
22.The method of any one of embodiments 1-21 wherein the ≥8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8°C.
23. The method of any one of embodiments 1-22 wherein the ≥8 mg aflibercept is in an aqueous pharmaceutical formulation comprising an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.
24. A method
   - for treating or preventing diabetic retinopathy (DR) and/or diabetic macular edema (DME), in a subject in need thereof,
   - for improving best corrected visual acuity in a subject in need thereof with DR and/or DME; or
   - for promoting retinal drying in a subject with DR and/or DME in need thereof;
   comprising administering to an eye of the subject, one or more doses of about 8 mg or more of VEGF receptor fusion protein once every 12, 13, 14, 15, 16, 17, 18, 19 or 20 or 12-20 or 12-16 or 16-20 weeks.
25. The method of any one of embodiments 1-24 for treating or preventing diabetic retinopathy (DR) and/or diabetic macular edema (DME), in a subject in need thereof, comprising
   administering to an eye of the subject,
   a single initial dose of about 8 mg or more of a VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12-20 weeks after the immediately preceding dose.
26. The method of any one of embodiments 1-24 for treating or preventing diabetic retinopathy (DR) and/or diabetic macular edema (DME), in a subject in need thereof, comprising
   administering to an eye of the subject,
   a single initial dose of about 8 mg or more of a VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12 weeks after the immediately preceding dose.
27. The method of any one of embodiments 1-24 for treating or preventing diabetic retinopathy (DR) and/or diabetic macular edema (DME), in a subject in need thereof, comprising
   administering to an eye of the subject,
   a single initial dose of about 8 mg or more of a VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 16 weeks after the immediately preceding dose.
28. The method of any one of embodiments 1-24 for treating or preventing diabetic retinopathy (DR) and/or diabetic macular edema (DME), in a subject in need thereof, comprising
   administering to an eye of the subject,
   a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein;
   wherein each secondary dose is administered about 2 to 4 weeks after the immediately preceding dose; and wherein each tertiary dose is administered about 20 weeks after the immediately preceding dose.
29. A method for treating or preventing diabetic macular edema, in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days).
30. A method for treating or preventing diabetic retinopathy (DR), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days).
31. A method for treating or preventing diabetic macular edema, in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).
32. A method for treating or preventing diabetic retinopathy (DR), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).
33. A method for treating or preventing diabetic macular edema, in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).
34. A method for treating or preventing diabetic retinopathy (DR), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).
35. A method for treating or preventing diabetic macular edema, in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (2 - 4 months, +/- 7 days).
36. A method for treating or preventing diabetic retinopathy (DR), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (2 - 4 months, +/- 7 days).
37. A method for treating or preventing diabetic macular edema (DME) or diabetic retinopathy (DR) in a subject in need thereof at a dose which is 8 mg aflibercept (equivalent to 70 microliters solution for injection) comprising administering to the subject 1 injection per month (every 4 weeks) for 3 consecutive doses of said 8 mg aflibercept; and then admininstering injections of said 8 mg aflibercept at intervals that may be extended up to every 16 weeks based on the physician's judgement of visual and/or anatomic outcomes.
38. A method for treating or preventing diabetic retinopathy and/or diabetic macular edema, in a subject in need thereof:
   (1) wherein the subject has received an initial 2 mg dose of VEGF receptor fusion protein, then the method comprises, after 1 month, administering to the subject the initial 8 mg dose of VEGF receptor fusion protein and, 1 month thereafter, the 1^{st} 8 mg secondary dose of VEGF receptor fusion protein; and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (2) wherein the subject has received an initial 2 mg dose of VEGF receptor fusion protein, then the method comprises, after 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (3) wherein the subject has received an initial 2 mg dose of VEGF receptor fusion protein, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (4) wherein the subject has received an initial 2 mg dose of VEGF receptor fusion protein, then the method comprises, after 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   (5) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (6) wherein the subject has received an initial 2 mg dose of VEGF receptor fusion protein and a 1^{st} 2 mg secondary dose of VEGF receptor fusion protein after 1 month, then the method comprises, after another 1 month, administering to the subject a first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (7) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (8) wherein the subject has received an initial 2 mg dose of VEGF receptor fusion protein and a 1^{st} 2 mg secondary dose of VEGF receptor fusion protein after 1 month, then the method comprises, after another 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   (9) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (10) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (11) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (12) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
   (13) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the initial 8 mg dose of VEGF receptor fusion protein and 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (14) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the first 8 mg secondary dose of VEGF receptor fusion protein and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (15) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (16) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, then the method comprises, after 2 months, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   (17) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; thereafter, then the method comprises, after 2 months, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (18) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; thereafter, then the method comprises, after 2 months, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (19) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month; thereafter, then the method comprises, after 2 months, administering to the subject the 2^{nd} 8 mg secondary dose of VEGF receptor fusion protein and, 12 or 16 or 20 weeks thereafter, one or more 12 or 16 weekly 8 mg maintenance doses of VEGF receptor fusion protein according to the HDq12 or HDq16 or HDq20 dosing regimen;
   (20) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month, thereafter, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
   (21) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month, and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (22) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (23) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 2^{nd} 8 mg secondary dose of VEGF receptor fusion protein and, 12 or 16 or 20 weeks thereafter, one or more 12 or 16 or 20 weekly 8 mg maintenance doses of VEGF receptor fusion protein according to the HDq12 or HDq16 or HDq20 dosing regimen;
      or
   (24) wherein the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 3^{rd} 2 mg secondary dose of VEGF receptor fusion protein after 1 month; and a 4^{th} 2 mg secondary dose of VEGF receptor fusion protein after 1 month; and one or more 2 mg maintenance doses every 8 weeks thereafter, then the method comprises, 2 months after the last VEGF receptor fusion protein maintenance dose, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
   wherein,
   (i) said HDq12 dosing regimen comprises:
      a single initial dose of about 8 mg or more of VEGF receptor fusion protein, followed by
      one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 12 weeks after the immediately preceding dose;
   (ii) said HDq16 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 16 weeks after the immediately preceding dose;
      and
   (iii) said HDq20 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 20 weeks after the immediately preceding dose.
39. A method for treating or preventing diabetic retinopathy and/or diabetic macular edema, in a subject in need thereof who has been on a dosing regimen for treating or preventing said disorder wherein:
   (a) the subject has received an initial 8 mg dose of VEGF receptor fusion protein then the method comprises, after 1 month, administering to the subject the first 8 mg secondary dose of VEGF receptor fusion protein and 1 month thereafter, administering the 2^{nd} 8 mg secondary dose of VEGF receptor fusion protein; and then, every 12 or 16 or 20 weeks thereafter, administering one or more 8 mg maintenance doses of VEGF receptor fusion protein according to the HDq12 or HDq16 or HDq20 dosing regimen;
      or
   (b) the subject has received an initial 8 mg dose of VEGF receptor fusion protein & 1^{st} 8 mg secondary dose of VEGF receptor fusion protein after 1 month, then the method comprises, after another 1 month, administering to the subject the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
      or
   (c) the subject has received an initial 8 mg dose of VEGF receptor fusion protein & 1^{st} 8 mg secondary dose of VEGF receptor fusion protein after 1 month & the 2^{nd} 8 mg secondary dose of VEGF receptor fusion protein after another month, then the method comprises, after 12 or 16 or 20 weeks administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
      or
   (d) the subject has received an initial 8 mg dose of VEGF receptor fusion protein & a 1^{st} 8 mg secondary dose of VEGF receptor fusion protein after 1 month & the 2^{nd} 8 mg secondary dose of VEGF receptor fusion protein after another month, then every 12 or 16 or 20 weeks thereafter, the subject has received one or more 8 mg maintenance doses of VEGF receptor fusion protein; and, then the method comprises, after 12 or 16 or 20 weeks from the last maintenance dose of VEGF receptor fusion protein, administering to the subject one or more 8 mg maintenance doses of VEGF receptor fusion protein and all further 8 mg maintenance doses of VEGF receptor fusion protein every 12 or 16 or 20 weeks according to the HDq12 or HDq16 or HDq20 dosing regimen;
   wherein,
   (i) said HDq12 dosing regimen comprises:
      a single initial dose of about 8 mg or more of VEGF receptor fusion protein, followed by
      one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 12 weeks after the immediately preceding dose;
   (ii) said HDq16 dosing regimen comprises:
      a single initial dose of about 8 mg or more of VEGF receptor fusion protein, followed by
      one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 16 weeks after the immediately preceding dose;
      and
   (iii) said HDq20 dosing regimen comprises:
      a single initial dose of about 8 mg or more of VEGF receptor fusion protein, followed by
      one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 20 weeks after the immediately preceding dose.
40. A method for treating or preventing an angiogenic eye disorder, in a subject in need thereof who has been on a dosing regimen for treating or preventing the disorder calling for a single initial dose of about 2 mg of VEGF receptor fusion protein, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen,
   comprising administering to an eye of the subject,
   an 8 mg dose of VEGF receptor fusion protein,
   evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with 8 mg VEGF receptor fusion protein; or
   evaluating the subject in about 8 or 10 or 12 weeks after said administering and, if, in the judgment of the treating physician dosing every 12 weeks is appropriate, then administering another 8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.
41. The method of any one of embodiments 1-40 wherein the subject has been on a dosing regimen for treating or preventing diabetic retinopathy and/or diabetic macular edema of a single initial dose of about 2 mg of a VEGF receptor fusion protein, followed by 4 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose.
42. A method of any one of embodiments 1-41 for treating or preventing diabetic retinopathy or diabetic macular edema, in a subject in need thereof, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 weeks after the immediately preceding dose;
   further comprising, after receiving one or more of said tertiary doses about 12 or 16 after the immediately preceding dose, lengthening the tertiary dose interval from
      - 12 weeks to 16 weeks;
      - 12 weeks to 20 weeks; or
      - 16 weeks to 20 weeks,
   after the immediately preceding dose.
43. The method of embodiment 42 wherein, during said treatment, the subject exhibits
   (a) <5 letter loss in BCVA; and/or
   (b) CRT <300 or 320 µm.
44. The method of any one of embodiments 42-43 further comprising evaluating BVCA and/or CRT in the subject and, if the subject exhibits
   (a) <5 letter loss in BCVA; and/or
   (b) CRT <300 or 320 µm.
   lengthening the tertiary dose interval.
45. A method of any one of embodiments 1-45 for treating or preventing diabetic retinopathy and/or diabetic macular edema, in a subject in need thereof, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 or 20 weeks after the immediately preceding dose;
   further comprising, after receiving one or more of said tertiary doses about 12 or 16 or 20 weeks after the immediately preceding dose, shortening the tertiary dose interval from
   - 12 weeks to 8 weeks;
   - 16 weeks to 12 weeks;
   - 16 weeks to 8 weeks,
   - 20 weeks to 8 weeks,
   - 20 weeks to 12 weeks, or
   - 20 weeks to 16 weeks.
46. The method of embodiment 45 wherein, during said treatment, the subject exhibits
   (a) > 10 letter loss in BCVA relative to baseline; and/or
   (b) > 50 µm increase in CRT relative to baseline.
47. The method of any one of embodiments 45-46 further comprising evaluating BVCA and/or CRT in the subject and, if the subject exhibits
   (a) > 10 letter loss in BCVA relative to baseline; and/or
   (b) > 50 µm increase in CRT relative to baseline,
   shortening the tertiary dose interval.
48. The method of any one of embodiments 1-47 wherein if
   (a) greater than 5 letters are lost in BCVA (ETDRS), relative to the BCVA observed at about 12 weeks after treatment initiation;
   (b) a greater than 25 micrometers increase in CRT is observed relative to the CRT observed at about 12 weeks after treatment initiation; and/or
   (c) there is a new onset foveal neovascularization or foveal hemorrhage;

   at week 16 or week 20 after treatment initiation,
   then, the interval between tertiary doses is decreased from 12 weeks or 16 weeks to 8 weeks;
      or
      (a) greater than 5 letters are lost in BCVA (ETDRS), relative to the BCVA observed at about 12 weeks after treatment initiation;
      (b) a greater than 25 micrometers increase in CRT is observed relative to the CRT observed at about 12 weeks after treatment initiation; and/or
      (c) there is a new onset foveal neovascularization or foveal hemorrhage;
   at week 24 after treatment initiation
   then, the interval between tertiary doses is decreased from 16 weeks to 12 weeks.
49. A method for treating or preventing diabetic retinopathy and/or diabetic macular edema, in a subject in need thereof, comprising administering to an eye of the subject 3 doses of about 8 mg VEGF receptor fusion protein in a formulation that comprises about 114.3 mg/ml VEGF receptor fusion protein at an interval of once every 4 weeks; wherein after said 3 doses, administering one or more doses of the VEGF receptor fusion protein at an interval which is lengthened up to 12, 16 or 20 weeks.
50. A method for treating or preventing diabetic retinopathy and/or diabetic macular edema, in a subject in need thereof, comprising administering to an eye of the subject, a single initial dose of about 8 mg or more of VEGF receptor fusion protein, followed by 2 secondary doses of about 8 mg or more of the VEGF receptor fusion protein,
   wherein each secondary dose is administered about 2 to 4 weeks after the immediately preceding dose; and, after said doses,
      a) determining if the subject meets at least one criterion for reducing or lengthening one or more intervals by 2 weeks, 3 weeks, 4 weeks or 2-4 weeks between doses of the VEGF receptor fusion protein; and
      b) if said determination is made, administering further doses of the VEGF receptor fusion protein at said reduced or lengthened intervals between doses
   wherein criteria for lengthening the interval include:
      1. <5 letter loss in BCVA; and/or
      2. CRT <300 or 320 micrometers;
   and, wherein criteria for reducing the interval include:
      1. >10 letter loss in BCVA;
      2. persistent or worsening DME; and/or
      3. >50 micrometers increase in CRT.
51. The method of embodiment 50 wherein criteria for lengthening the interval include both:
   1. <5 letter loss in BCVA from week 12; and
   2. CRT <300 or 320 micrometers as measured by SD-OCT;
   and/or wherein criteria for reducing the interval include both:
   1. >10 letter loss in BCVA from week 12 in association with persistent or worsening DME; and
   2. >50 micrometers increase in CRT from week 12.
52. The method of any one of embodiments 49-51 wherein if said criteria are met, said interval is lengthened to 12, 16 or 20 weeks.
53. A method for treating or preventing diabetic retinopathy and/or diabetic macular edema, in a subject in need thereof that has been pre-treated with one or more 2 mg doses of VEGF receptor fusion protein, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein;
   wherein each secondary dose is administered about 2 to 4 weeks after the immediately preceding dose; and
   wherein each tertiary dose is administered about 12-20 weeks after the immediately preceding dose.
54. A method for treating or preventing an angiogenic eye disorder, in a subject in need thereof, comprising administering to an eye of the subject,
   (1) a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 8 weeks after the immediately preceding dose; or
   (2) one or more doses of 8 mg or more of VEGF receptor fusion protein about every 4 weeks.
55. The method of embodiment 54 wherein the angiogenic eye disorder is diabetic retinopathy and/or diabetic macular edema.
56. The method of any one of embodiments 54-55 wherein one or more secondary doses is 2, 3 or 4 secondary doses and/or about 2-4 weeks is about 4 weeks.
57. The method of any one of embodiments 1-56 wherein a subject having any one or more of ocular or periocular infection;
   active intraocular inflammation; and/or
   hypersensitivity;
   is excluded from administration of VEGF receptor fusion protein to the eye.
58. The method of embodiment 57 further comprising a step of
   evaluating the subject for:
   ocular or periocular infection;
   active intraocular inflammation; and/or
   hypersensitivity;
   and excluding the subject from said administration if any one or more if found in the subject.
59. The method of any one of embodiments 1-58 further comprising monitoring the subject during said treatment or prevention for conjunctival hemorrhage, cataract, vitreous detachment, vitreous floaters, corneal epithelium defect and/or increased intraocular pressure.
60. The method of any one of embodiments 1-59 comprising, prior to each administration, providing
   - one single-dose glass vial having a protective plastic cap and a stopper containing an aqueous formulation comprising 8 mg VEGF receptor fusion protein in about 70 microliters;
   - one 18-gauge x 1½-inch, 5-micron, filter needle that includes a tip and a bevel;
   - one 30-gauge x ½-inch injection needle; and
   - one 1-mL Luer lock syringe having a graduation line marking for 70 microliters of volume;

   packaged together; then
      (1) visually inspecting the aqueous formulation in the vial and, if particulates, cloudiness, or discoloration are visible, then using another vial of aqueous formulation containing the VEGF receptor fusion protein;
      (2) removing the protective plastic cap from the vial; and
      (3) cleaning the top of the vial with an alcohol wipe; then
   using aseptic technique:
      (4) removing the 18-gauge x 1½-inch, 5-micron, filter needle and the 1 mL syringe from their packaging;
      (5) attaching the filter needle to the syringe by twisting it onto the Luer lock syringe tip;
      (6) pushing the filter needle into the center of the vial stopper until the needle is completely inserted into the vial and the tip touches the bottom or a bottom edge of the vial;
      (7) withdrawing all of the VEGF receptor fusion protein vial contents into the syringe, keeping the vial in an upright position, slightly inclined, while ensuring the bevel of the filter needle is submerged into the liquid;
      (8) continuing to tilt the vial during withdrawal keeping the bevel of the filter needle submerged in the formulation;
      (9) drawing the plunger rod sufficiently back when emptying the vial in order to completely empty the filter needle;
      (10) removing the filter needle from the syringe and disposing of the filter needle;
      (11) removing the 30-gauge x ½-inch injection needle from its packaging and attaching the injection needle to the syringe by firmly twisting the injection needle onto the Luer lock syringe tip;
      (12) holding the syringe with the needle pointing up, and checking the syringe for bubbles,
      wherein if there are bubbles, gently tapping the syringe with a finger until the bubbles rise to the top; and
      (13) slowly depressing the plunger so that the plunger tip aligns with the graduation line that marks 70 microliters on the syringe.
61. The method of any one of embodiments 1-60 wherein injection of VEGF receptor fusion protein is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.
62. The method of any one of embodiments 1-61 wherein the subject has been receiving a dosing regimen for treating or preventing diabetic retinopathy and/or diabetic macular edema calling for:
   a single initial dose of about 2 mg of VEGF receptor fusion protein, followed by 4 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose;
   wherein the subject is at any phase (initial dose, secondary dose or tertiary dose) of the 2 mg VEGF receptor fusion protein dosing regimen.
63. The method of any one of embodiments 1-62, wherein
   - one or more secondary doses is 2 secondary doses;
   - 2 to 4 weeks is about 4 weeks;
   - 12-20 weeks is about 12 weeks;
   - 12-20 weeks is about 16 weeks;
   - 12-20 weeks is about 20 weeks;
   - 12-20 weeks is about 12-16 weeks;
   - 8-16 weeks is about 12 weeks;
   - 8-16 weeks is about 16 weeks;
   - 8-16 weeks is about 12-16 weeks;
   - 2 to 4 weeks is about 4 weeks and one or more secondary doses is 2 secondary doses;
   - 12-20 weeks is about 12 weeks and one or more secondary doses is 2 secondary doses;
   - 12-20 weeks is about 16 weeks and one or more secondary doses is 2 secondary doses;
   - 12-20 weeks is about 20 weeks and one or more secondary doses is 2 secondary doses;
   - 12-20 weeks is about 12-16 weeks and one or more secondary doses is 2 secondary doses;
   - 2 to 4 weeks is about 4 weeks and one or more secondary doses is 2 secondary doses and the VEGF receptor fusion protein is aflibercept;
   - 12-20 weeks is about 12 weeks and one or more secondary doses is 2 secondary doses and the VEGF receptor fusion protein is aflibercept;
   - 12-20 weeks is about 16 weeks and one or more secondary doses is 2 secondary doses and the VEGF receptor fusion protein is aflibercept;
   - 12-20 weeks is about 20 weeks and one or more secondary doses is 2 secondary doses and the VEGF receptor fusion protein is aflibercept; and/or
   - 12-20 weeks is about 12-16 weeks and one or more secondary doses is 2 secondary doses and the VEGF receptor fusion protein is aflibercept.
64. The method of any one of embodiments 1-63, wherein the VEGF receptor fusion protein comprises amino acids 27-457 of the amino acid sequence set forth in SEQ ID NO: 2.
65. The method of any one of embodiments 1-64, wherein the VEGF receptor fusion protein is selected from the group consisting of: aflibercept and conbercept.
66. The method of any one of embodiments 1-65, wherein the VEGF receptor fusion protein:
   (i) comprises two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2;
   (ii) comprises two polypeptides that comprise an immunoglobin-like (Ig) domain 2 of VEGFR1, an Ig domain 3 of a VEGFR2, and a multimerizing component;
   (iii) comprises two polypeptides that comprise an immunoglobin-like (Ig) domain 2 of VEGFR1, an Ig domain 3 of VEGFR2, an Ig domain 4 of VEGFR2 and a multimerizing component; or
   (iv) comprises two VEGFR1R2-FcΔC1(a) polypeptides encoded by the nucleic acid sequence of SEQ ID NO: 1.
67. The method of embodiment 66, wherein the VEGF receptor fusion protein comprises two polypeptides that comprise an immunoglobin-like (Ig) domain 2 of VEGFR1, an Ig domain 3 of a VEGFR2, and a multimerizing component.
68. The method of any one of embodiments 1-67, wherein the VEGF receptor fusion protein is in an aqueous pharmaceutical formulation selected from the group consisting of A-KKKK.
69. The method of any one of embodiments 1-68 wherein said VEGF receptor fusion protein is in an aqueous pharmaceutical formulation comprising about 114.3 mg/ml VEGF receptor fusion protein.
70. The method of any one of embodiments 1-69 comprising administering the VEGF receptor fusion protein to both eyes of the subject.
71. The method of any one of embodiments 1-70, wherein the VEGF receptor fusion protein is administered from a pre-filled syringe.
72. The method of embodiment 71, wherein the pre-filled syringe is glass or plastic, and/or sterile
73. The method of any one of embodiments 1-72 wherein the VEGF receptor fusion protein is intravitreally injected with a 30 gauge × ½-inch sterile injection needle.
74. The method of any one of embodiments 1-73 wherein the subject has previously received one or more doses of 2 mg VEGF receptor fusion protein.
75. The method of any one of embodiments 1-74 wherein one or more further doses of VEGF receptor fusion protein are administered.
76. The method of any one of embodiments 1-74 wherein 2 mg VEGF receptor fusion protein is in an aqueous pharmaceutical formulation comprising 40 mg/ml VEGF receptor fusion protein.
77. The method of embodiment 76 wherein 2 mg of VEGF receptor fusion protein is in a pharmaceutical formulation comprising:
   40 mg/ml VEGF receptor fusion protein, 10 mM sodium phosphate, 40 mM NaCl, 0.03% polysorbate 20 and 5% sucrose, with a pH of 6.2.
78. The method of any one of embodiments 1-77 wherein 8 mg of VEGF receptor fusion protein is in an aqueous pharmaceutical formulation that comprises a sugar or polyol.
79. The method of any one of embodiments 1-78 wherein 8 mg VEGF receptor fusion protein in an aqueous pharmaceutical formulation that comprises sucrose.
80. The method of any one of embodiments 1-79 wherein 8 mg of a VEGF receptor fusion protein is in an aqueous pharmaceutical formulation that has a pH of about 5.8.
81. The method of any one of embodiments 1-80 wherein 8 mg of a VEGF receptor fusion protein is in an aqueous pharmaceutical formulation comprising about 103-126 mg/ml VEGF receptor fusion protein, histidine-based buffer and arginine.
82. The method of any one of embodiments 1-81 wherein 8 mg of a VEGF receptor fusion protein is an aqueous pharmaceutical formulation comprising about 114.3 mg/ml VEGF receptor fusion protein, histidine-based buffer and arginine.
83. The method of any one of embodiments 1-82 wherein the ≥8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8° C.
84. The method of any one of embodiments 1-83 wherein the ≥8 mg VEGF receptor fusion protein is in an aqueous pharmaceutical formulation comprising:
   at least about 100 mg/ml of a VEGF receptor fusion protein;
   about 10-100 mM L-arginine;
   sucrose;
   a histidine-based buffer; and
   a surfactant;
   wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.
85. The method of embodiment 1-84 wherein 8 mg of VEGF receptor fusion protein is in an aqueous pharmaceutical formulation comprising
   - >100 mg/ml VEGF receptor fusion protein, histidine-based buffer and L-arginine;
   - 140 mg/ml aflibercept; 20 mM histidine-based buffer; 5 % sucrose; 0.03 % polysorbate 20; 10 mM L-arginine; pH 5.8;
   - 150 + 15 mg/ml aflibercept, 10 mM phosphate-based buffer, 8 + 0.8% (w/v) sucrose, 0.02-0.04% (w/v) polysorbate 20 and 50 mM L-arginine, pH 5.9-6.5;
   - 103-126 mg/ml aflibercept, 10 + 1 mM histidine-based buffer, 5 + 0.5% (w/v) sucrose, 0.02-0.04% (w/v) polysorbate 20, and 50 + 5 mM L-arginine, pH 5.5-6.1;
   - 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
   - 114.3 mg/ml aflibercept, 10 mM histidine-based buffer, 5% (w/v) sucrose, 0.03% (w/v) polysorbate 20 and 50 mM L-arginine, pH 5.8;
   - >100 mg/ml aflibercept, histidine-based buffer and L-arginine;
   - >100 mg/ml aflibercept at about pH 5.8, wherein the formulation forms <3% HMW aggregates after incubation at 5°C for 2 months;
   - About 114.3 mg/mL aflibercept; 10 mM - 50 mM histidine-based buffer, sugar, non-ionic surfactant, L-Arginine, pH 5.8; or
   - About 114.3 mg/mL aflibercept; 10 mM His/His-HCl-based buffer, 5% sucrose, 0.03% polysorbate-20, 50 mM L-Arginine, pH 5.8.
86. The method of any one of embodiments 1-85 wherein the 8 mg of VEGF receptor fusion protein is administered in a volume of about 100 µl or less, about 75 µl or less; about 70 µl or less; or about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl.
87. The method of embodiment 86 wherein said VEGF receptor fusion protein is administered in a volume of about 70 ± 4 or 5 microliters.
88. The method of any one of embodiments 1-87 comprising administering the VEGF receptor fusion protein to both eyes of the subject.
89. The method of any one of embodiments 1-88, wherein the subject achieves and/or maintains one or more of,
   - an improvement in Diabetic Retinopathy Severity Scale (DRSS);
   - an improvement in best corrected visual acuity;
   - a dry retina
   - a gain in best corrected visual acuity;
   - a BCVA of at least 69 letters;
   - a foveal center without fluid;
   - a decrease in central retinal thickness (CRT);
   - no vascular leakage as measured by fluorescein angiography (FA);
   - an improvement from pre-treatment baseline in National Eye Institute Visual Function Questionnaire (NEI-VFQ) total score;
   - a retina without fluid (total fluid, intraretinal fluid [IRF] and/or subretinal fluid [SRF]) at the foveal center and in center subfield;
   - maintenance of a fluid-free retina (total fluid, IRF and/or SRF at foveal center and in the center subfield);
   - a lack of macular edema;
   - a retina free of fluid on spectral domain optical coherence tomography (SD-OCT); and/or
   - Does not deviate from the HDq12 or HDq16 treatment regimen once started.
90. The method of any one of embodiments 1-89, wherein the subject achieves and/or maintains one or more of:
   - Non-inferior BVCA compared to that of aflibercept which is intravitreally dosed at 2 mg approximately every 4 weeks for the first 3, 4 or 5 injections followed by 2 mg approximately once every 8 weeks or once every 2 months;
   - Increase in BCVA (according to ETDRS letter score) of about 7, 8 or 9 letters by week 60 from start of treatment, wherein the baseline BCVA is about 61, 62 or 63;
   - BCVA (according to ETDRS letter score) of at least about 69 letters by week 48 or 60 from start of treatment;
   - Does not lose 5, 10, 15 or 69 letters or more BCVA after week 12, 24, 36, 48, 60, 72, 84, 90 or 96 from start of treatment;
   - Improvement in BCVA (according to ETDRS letter score) by week 12, 24, 36, 48, 60, 72, 84, 90 or 96 from start of treatment;
   - Improvement in BVCA by week 4, week 8, week 12, week 16, week 20, week 24, week 28, week 32, week 36, week 40, week 44, or week 48 from start of treatment;
   - Between weeks 48 and 60, a BCVA score (according to ETDRS letter score) of about 69, 70, 71, 72 or 73;
   - Between weeks 36 and 48, a change in BCVA score (according to ETDRS letter score) from initiation of treatment of about 7, 8 or 9 wherein the BCVA at any point between week 36 to 48 is about 60 or 70;
   - Between weeks 48 and 60, a change in BCVA score (according to ETDRS letter score) from initiation of treatment of about 7, 8 or 9, wherein the BCVA at any point between week 48 to 60 is about 69, 70, 71, 72 or 73;
   - Increase in BCVA as measured by the Early Treatment Diabetic Retinopathy Study (ETDRS) visual acuity chart or Snellen equivalent by week 4, 8, 12, 16, 20, 24, 28, 32, 36, 40, 44 or 48 weeks from start of treatment by ≥4 letters, ≥5 letters, ≥6 letters, ≥7 letters, ≥8 letters, ≥ 9 letters or ≥ 10 letters;
   - Does not lose 5, 10 or 15 letters by week 48 or 60 from start of treatment (according to ETDRS letter score);
   - Gains at least 5, 10 or 15 letter by week 48 or 60 from start of treatment (according to ETDRS letter score);
   - Improvement in BCVA, by 4 weeks after initiation of treatment, of about 4 or 5 letters (ETDRS or Snellen equivalent) when on HDq12 regimen; or of about 4 or 5 letters (ETDRS or Snellen equivalent) when on HDq16 regimen;
   - Improvement in BCVA, by 8 weeks after initiation of treatment, of about 6 letters (ETDRS or Snellen equivalent) when on HDq12 regimen; or of about 5 or 6 letters (ETDRS or Snellen equivalent) when on HDq16 regimen;
   - Improvement in BCVA, by 12 weeks after initiation of treatment, of about 6 or 7 letters (ETDRS or Snellen equivalent) when on HDq12 regimen; or of about 6 letters (ETDRS or Snellen equivalent) when on HDq16 regimen;
   - Improvement in BCVA, by 16 weeks after initiation of treatment, of about 6 or 7 letters (ETDRS or Snellen equivalent) when on HDq12 regimen; or of 7 letters (ETDRS or Snellen equivalent) when on HDq16 regimen;
   - Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
   - Improvement in BCVA, by 24 weeks after initiation of treatment, of about 7 letters (ETDRS or Snellen equivalent) when on HDq12 regimen; or of about 5 or 6 letters (ETDRS or Snellen equivalent) when on HDq16 regimen;
   - Improvement in BCVA, by 28 weeks after initiation of treatment, of about 7 or 8 letters (ETDRS or Snellen equivalent) when on HDq12 regimen; or of about 7 or 8 letters (ETDRS or Snellen equivalent) when on HDq16 regimen;
   - Improvement in BCVA, by 32 weeks after initiation of treatment, of about 7 letters (ETDRS or Snellen equivalent) when on HDq12 regimen; or of about 7 or 8 letters (ETDRS or Snellen equivalent) when on HDq16 regimen;
   - Improvement in BCVA, by 36 weeks after initiation of treatment, of 8 letters (ETDRS or Snellen equivalent) when on HDq12 regimen; or of about 6 or 7 letters (ETDRS or Snellen equivalent) when on HDq16 regimen;
   - Improvement in BCVA, by 40 weeks after initiation of treatment, of about 8 letters (ETDRS or Snellen equivalent) when on HDq12 regimen; or of about 6 or 7 letters (ETDRS or Snellen equivalent) when on HDq16 regimen;
   - Improvement in BCVA, by 44 weeks after initiation of treatment, of about 8 letters (ETDRS or Snellen equivalent) when on HDq12 regimen; or of about 7 or 8 letters (ETDRS or Snellen equivalent) when on HDq16 regimen;
   - Improvement in BCVA, by 48 weeks after initiation of treatment, of about 8 or 9 letters (ETDRS or Snellen equivalent) when on HDq12 regimen; or of about 7 or 8 letters (ETDRS or Snellen equivalent) when on HDq16 regimen;
   - An improvement in BCVA by about week 8 after initiation of treatment which is maintained thereafter during the treatment regimen to at least week 48;
   - Between weeks 36 and 48, an improvement in BCVA score (according to ETDRS letter score) from initiation of treatment of up to 38 letters when on the HDq12 or HDq16 regimen;
   - A BCVA by 4 weeks after initiation of treatment of about 68 letters (ETDRS or Snellen equivalent) when on the HDq12 regimen; or a BCVA of about 66 letters (ETDRS or Snellen equivalent) when on the HDq16 regimen;
   - A BCVA by 8 weeks after initiation of treatment of about 70 letters (ETDRS or Snellen equivalent) when on the HDq12 regimen; or a BCVA of about 67 letters (ETDRS or Snellen equivalent) when on the HDq16 regimen;
   - A BCVA by 12 weeks after initiation of treatment of about 70 letters (ETDRS or Snellen equivalent) when on the HDq12 regimen; or a BCVA of about 68 letters (ETDRS or Snellen equivalent) when on the HDq16 regimen;
   - A BCVA by 16 weeks after initiation of treatment of about 71 letters (ETDRS or Snellen equivalent) when on the HDq12 regimen; or a BCVA of about 69 letters (ETDRS or Snellen equivalent) when on the HDq16 regimen;
   - A BCVA by 20 weeks after initiation of treatment of about 70 letters (ETDRS or Snellen equivalent) when on the HDq12 regimen; or a BCVA of about 68 letters (ETDRS or Snellen equivalent) when on the HDq16 regimen;
   - A BCVA by 24 weeks after initiation of treatment of about 71 letters (ETDRS or Snellen equivalent) when on the HDq12 regimen; or a BCVA of about 67 letters (ETDRS or Snellen equivalent) when on the HDq16 regimen;
   - A BCVA by 28 weeks after initiation of treatment of about 72 letters (ETDRS or Snellen equivalent) when on the HDq12 regimen; or a BCVA of about 70 letters (ETDRS or Snellen equivalent) when on the HDq16 regimen;
   - A BCVA by 32 weeks after initiation of treatment of about 71 letters (ETDRS or Snellen equivalent) when on the HDq12 regimen; or a BCVA of about 70 letters (ETDRS or Snellen equivalent) when on the HDq16 regimen;
   - A BCVA by 36 weeks after initiation of treatment of about 71 letters (ETDRS or Snellen equivalent) when on the HDq12 regimen; or a BCVA of about 68 letters (ETDRS or Snellen equivalent) when on the HDq16 regimen;
   - A BCVA by 40 weeks after initiation of treatment of about 72 letters (ETDRS or Snellen equivalent) when on the HDq12 regimen; or a BCVA of about 69 letters (ETDRS or Snellen equivalent) when on the HDq16 regimen;
   - A BCVA by 44 weeks after initiation of treatment of about 72 letters (ETDRS or Snellen equivalent) when on the HDq12 regimen; or a BCVA of about 70 letters (ETDRS or Snellen equivalent) when on the HDq16 regimen;
   - A BCVA by 48 weeks after initiation of treatment of about 73 letters (ETDRS or Snellen equivalent) when on the HDq12 regimen; or a BCVA of about 70 letters (ETDRS or Snellen equivalent) when on the HDq16 regimen;
   - A BCVA between weeks 36 and 48 of about 71, 72, 73 or 74 (ETDRS or Snellen equivalent) when on the HDq12 regimen; or A BCVA between weeks 36 and 48 of about 69, 70, 71, 72 or 73 (ETDRS or Snellen equivalent) when on the HDq16 regimen;
   - A BCVA improvement, by week 48 following treatment initiation, of about 9 or 10 letters (ETDRS or Snellen equivalent) when baseline BCVA is about ≤73 ETDRS letters when on HDq12 regimen;
   - A BCVA improvement, by week 48 following treatment initiation, of about 5 or 6 letters (ETDRS or Snellen equivalent) when baseline BCVA is about >73 ETDRS letters when on HDq12 regimen;
   - A BCVA improvement, by week 48 following treatment initiation, of about 8 or 9 letters (ETDRS or Snellen equivalent) when baseline BCVA is about ≤73 ETDRS letters when on HDq16 regimen;
   - A BCVA improvement, by week 48 following treatment initiation, of about 4 or 5 letters (ETDRS or Snellen equivalent) when baseline BCVA is about >73 ETDRS letters when on HDq16 regimen;
   - A BCVA improvement, by week 48 following treatment initiation, of about 7 or 8 letters (ETDRS or Snellen equivalent) when baseline CRT is about < about 400 micrometers when on HDq12 regimen;
   - A BCVA improvement, by week 48 following treatment initiation, of about 9 or 10 letters (ETDRS or Snellen equivalent) when baseline CRT is about ≥400 micrometers when on HDq12 regimen;
   - A BCVA improvement, by week 48 following treatment initiation, of about 5 or 6 letters (ETDRS or Snellen equivalent) when baseline CRT is about < about 400 micrometers when on HDq16 regimen;
   - A BCVA improvement, by week 48 following treatment initiation, of about 9 or 10 letters (ETDRS or Snellen equivalent) when baseline CRT is about ≥ about 400 micrometers when on HDq16 regimen;
   - Gain of ≥5, ≥10 or ≥15 letters BCVA (according to ETDRS letter score) by week 12, 24, 36, 48, 60, 72, 84, 90 or 96 from start of treatment;
   - ≥ 2 or ≥3 step improvement in Diabetic Retinopathy Severity Scale (DRSS), by week 12, 24, 36, 48, 60, 72, 84, 90 or 96 from start of treatment;
   - ≥ 2 step improvement in diabetic retinopathy severity scale (DRSS) by week 4, week 8, week 12, 16, 20, 24, 28, 32, 36, 40, 44 or 48 weeks from start of treatment;
   - Retina without fluid (total fluid, intraretinal fluid [IRF] and/or subretinal fluid [SRF]) at the foveal center and in center subfield by week 12, 24, 36, 48, 60, 72, 84, 90 or 96 from start of treatment as measured by optical coherence tomography (OCT);
   - No vascular leakage as measured by fluorescein angiography (FA) by week 12, 24, 36, 48, 60, 72, 84, 90 or 96 from start of treatment;
   - Maintenance of a fluid-free retina (total fluid, IRF and/or SRF at foveal center and in the center subfield) by week 12, 24, 36, 48, 60, 72, 84, 90 or 96 from start of treatment;
   - Reduction in total area of fluorescein leakage within ETDRS grid (mm²) at week 48 or 60 by about 12, 13 or 14 mm² or more as measured by fluorescein angiography;
   - Retina free of fluid on spectral domain optical coherence tomography (SD-OCT) by week 12, 24, 36, 48, 60, 72, 84, 90 or 96 from start of treatment;
   - Retina without fluid (total fluid, intraretinal fluid [IRF] and/or subretinal fluid [SRF]) at the foveal center by week 4, 8, 12, 16, 20, 24, 28, 32, 36, 40, 44 or 48 weeks from start of treatment;
   - Dry retina by week 12, 24, 36, 48, 60, 72, 84, 90 or 96 from start of treatment;
   - Foveal center without fluid by week 12, 24, 36, 48, 60, 72, 84, 90 or 96 from start of treatment as measured by optical coherence tomography (OCT);
   - A change in central retinal thickness, by 4 weeks after initiation of treatment of about - 118 or -118.3 micrometers when on the HDq12 regimen; or of about -124 or -125 or -124.9 or - 125.5 micrometers when on the HDq16 regimen;
   - A change in central retinal thickness, by 8 weeks after initiation of treatment of about - 137 or -137.4 micrometers when on the HDq12 regimen; or of about -139 or -140 or -139.6 or - 140.3 micrometers when on the HDq16 regimen;
   - A change in central retinal thickness, by 12 weeks after initiation of treatment of about - 150 or -150.1 micrometers when on the HDq12 regimen; or of about -152 or -153 or -152.7 or - 153.4 micrometers when on the HDq16 regimen;
   - A change in central retinal thickness, by 16 weeks after initiation of treatment of about - 139 or -139.4 micrometers when on the HDq12 regimen; or of about -145 or -146 or -145.5 or - 146.4 micrometers when on the HDq16 regimen;
   - A change in central retinal thickness, by 20 weeks after initiation of treatment of about - 117 or -117.1 micrometers when on the HDq12 regimen; or of about -112 or -113 or -112.5 or - 113.3 micrometers when on the HDq16 regimen;
   - A change in central retinal thickness, by 24 weeks after initiation of treatment of about - 158 or -158.1 micrometers when on the HDq12 regimen; or of about -103 or -104 or -103.8 or - 104.3 micrometers when on the HDq16 regimen;
   - A change in central retinal thickness, by 28 weeks after initiation of treatment of about - 146 or -147 or -146.7 micrometers when on the HDq12 regimen; or of about -162 or -162.3 micrometers when on the HDq16 regimen;
   - A change in central retinal thickness, by 32 weeks after initiation of treatment of about - 132 micrometers when on the HDq12 regimen; or of about -145 or -146 or -145.8 micrometers when on the HDq16 regimen;
   - A change in central retinal thickness, by 36 weeks after initiation of treatment of about - 168 or -168.1 micrometers when on the HDq12 regimen; or of about -124 or -125 or -124.7 or - 125.2 micrometers when on the HDq16 regimen;
   - A change in central retinal thickness, by 40 weeks after initiation of treatment of about - 163 micrometers when on the HDq12 regimen; or of about -122 or -123 or -122.5 or -123.1 micrometers when on the HDq16 regimen;
   - A change in central retinal thickness, by 44 weeks after initiation of treatment of about - 147 or -148 or -147.4 micrometers when on the HDq12 regimen; or of about -164 or -164.1 or - 164.3 micrometers when on the HDq16 regimen;
   - A change in central retinal thickness, by 48 weeks after initiation of treatment of about - 171 or -172 or -171.7 micrometers when on the HDq12 regimen; or of about -148 or -149 or - 148.3 or -149.4 micrometers when on the HDq16 regimen;
   - A change in central retinal thickness, by 60 weeks after initiation of treatment of about - 181.95 or -176.24 micrometers when on the HDq12 regimen; or of about -166.26 or -167.18 micrometers when on the HDq16 regimen;
   - A change in central retinal thickness of about -118 or -119 or -118.3 micrometers, between initiation of treatment (week 0) and week 4 when on the HDq12 regimen;
   - A change in central retinal thickness of about -19, -20 or -19.1 micrometers, between weeks 4 and 8 when on the HDq12 regimen;
   - A change in central retinal thickness of about -12, -13 or -12.7 micrometers, between weeks 8 and 12 when on the HDq12 regimen;
   - A change in central retinal thickness of about -40, or -41 micrometers, between weeks 20 and 24 when on the HDq12 regimen;
   - A change in central retinal thickness of about -36, -37 or -36.1 micrometers, between weeks 32 and 36 when on the HDq12 regimen;
   - A change in central retinal thickness of about -24, -25 or -24.3 micrometers, between weeks 44 and 48 when on the HDq12 regimen;
   - A change in central retinal thickness of -4, -5 or -4.5 micrometers, between weeks 48 and 60 when on the HDq12 regimen;
   - A change in central retinal thickness of about -124, -125 or -124.9 micrometers, between initiation of treatment (week 0) and week 4 when on the HDq16 regimen;
   - A change in central retinal thickness of about -14, -15 or -14.7 micrometers, between weeks 4 and 8 when on the HDq16 regimen;
   - A change in central retinal thickness of about -13, -14 or -13.1 micrometers, between weeks 8 and 12 when on the HDq16 regimen;
   - A change in central retinal thickness of about -58, -59 or -58.5 micrometers, between weeks 24 and 28 when on the HDq16 regimen;
   - A change in central retinal thickness of about -41, -42 or -41.6 micrometers, between weeks 40 and 44 when on the HDq16 regimen;
   - A reduction in central retinal thickness by week 4, 5, 6, 7 or 8 after initiation of treatment which is maintained within about ±17, ±18 or ±19 micrometers thereafter during the treatment regimen to at least week 48 from initiation of treatment;
   - Decrease in central retinal thickness by about 100, 125, 150, 175 or 200 micrometers by week 12, 24, 36, 48, 60, 72, 84, 90 or 96 from start of treatment;
   - Reduction in central retinal thickness of about 148-182 micrometers by about week 48 or 60 from start of treatment as measured by optical coherence tomography (OCT)) wherein the baseline CRT is about 449, 450, 455 or 460 micrometers;
   - Decrease in central retinal thickness (CRT) by at least about 100, 125, 130, 135, 140, 145, 149, 150, 155, 160, 165, 170, 171, 172, 173, 174 or 175 micrometers by week 4, 8, 12, 16, 20, 24, 28, 32, 36, 40, 44 or 48 from start of treatment;
   - At about 0.1667 days after the first dose, free aflibercept in plasma of about 0.149 (±0.249) mg/l; wherein, at baseline, free aflibercept in was plasma not detectable wherein the subject has not received intravitreal aflibercept treatment for at least 12 weeks;
   - At about 0.3333 days after the first dose, free aflibercept in plasma of about 0.205 (±0.250) mg/l; wherein, at baseline, free aflibercept in plasma not detectable wherein the subject has not received intravitreal aflibercept treatment for at least 12 weeks;
   - At about 1 days after the first dose, free aflibercept in plasma of about 0.266 (±0.211) mg/l wherein, at baseline, free aflibercept in plasma not detectable wherein the subject has not received intravitreal aflibercept treatment for at least 12 weeks;
   - At about 2 days after the first dose, free aflibercept in plasma of about 0.218 (±0.145) mg/l wherein, at baseline, free aflibercept in plasma not detectable wherein the subject has not received intravitreal aflibercept treatment for at least 12 weeks;
   - At about 4 days after the first dose, free aflibercept in plasma of about 0.140 (±0.0741) mg/l wherein, at baseline, free aflibercept in plasma not detectable wherein the subject has not received intravitreal aflibercept treatment for at least 12 weeks;
   - At about 7 days after the first dose, free aflibercept in plasma of about 0.0767 (±0.0436) mg/l wherein, at baseline, free aflibercept in plasma not detectable, wherein the subject has not received intravitreal aflibercept treatment for at least 12 weeks;
   - At about 14 days after the first dose, free aflibercept in plasma of about 0.0309 (±0.0241) mg/l wherein at baseline free aflibercept in plasma not detectable wherein the subject has not received intravitreal aflibercept treatment for at least 12 weeks;
   - At about 21 days after the first dose, free aflibercept in plasma of about 0.0171 (±0.0171) mg/l wherein, at baseline, free aflibercept in plasma not detectable wherein the subject has not received intravitreal aflibercept treatment for at least 12 weeks;
   - At about 28 days after the first dose, free aflibercept in plasma of about 0.00730 (±0.0113) mg/l wherein, at baseline, free aflibercept in plasma not detectable wherein the subject has not received intravitreal aflibercept treatment for at least 12 weeks;
   - At about 0.1667 days after the first dose, adjusted bound aflibercept in plasma of about 0.00698 (±0.0276) mg/l wherein, at baseline, there is about 0.00583 mg/l (±0.0280) adjusted bound aflibercept wherein the subject has not received intravitreal aflibercept treatment for at least 12 weeks;
   - At about 0.3333 days after the first dose, adjusted bound aflibercept in plasma of about 0.00731 (±0.0279) mg/l wherein, at baseline, there is about 0.00583 mg/l (±0.0280) adjusted bound aflibercept wherein the subject has not received intravitreal aflibercept treatment for at least 12 weeks;
   - At about 1 days after the first dose, adjusted bound aflibercept in plasma of about 0.0678 (±0.0486) mg/l wherein, at baseline, there is about 0.00583 mg/l (±0.0280) adjusted bound aflibercept wherein the subject has not received intravitreal aflibercept treatment for at least 12 weeks;
   - At about 2 days after the first dose, adjusted bound aflibercept in plasma of about 0.138 (±0.0618) mg/l wherein at baseline there is about 0.00583 mg/l (±0.0280) adjusted bound aflibercept wherein the subject has not received intravitreal aflibercept treatment for at least 12 weeks;
   - At about 4 days after the first dose, adjusted bound aflibercept in plasma of about 0.259 (±0.126) mg/l wherein at baseline there is about 0.00583 mg/l (±0.0280) adjusted bound aflibercept wherein the subject has not received intravitreal aflibercept treatment for at least 12 weeks;
   - At about 7 days after the first dose, adjusted bound aflibercept in plasma of about 0.346 (±0.151) mg/l wherein at baseline there is about 0.00583 mg/l (±0.0280) adjusted bound aflibercept wherein the subject has not received intravitreal aflibercept treatment for at least 12 weeks;
   - At about 14 days after the first dose, adjusted bound aflibercept in plasma of about 0.374 (±0.110) mg/l wherein at baseline there is about 0.00583 mg/l (±0.0280) adjusted bound aflibercept wherein the subject has not received intravitreal aflibercept treatment for at least 12 weeks;
   - At about 21 days after the first dose, adjusted bound aflibercept in plasma of about 0.343 (±0.128) mg/l wherein at baseline there is about 0.00583 mg/l (±0.0280) adjusted bound aflibercept wherein the subject has not received intravitreal aflibercept treatment for at least 12 weeks;
   - At about 28 days after the first dose, adjusted bound aflibercept in plasma of about 0.269 (±0.149) mg/l wherein at baseline there is about 0.00583 mg/l (±0.0280) adjusted bound aflibercept wherein the subject has not received intravitreal aflibercept treatment for at least 12 weeks;
   - The maximum level of free aflibercept in the plasma is reached about 0.965 days after the first dose;
   - Reaches a maximum level of about 0.310 mg/l (±0.263) free aflibercept in the plasma;
   - Free aflibercept in the plasma of from about 0 to about 1.08 mg/L;
   - Free aflibercept in the plasma maximum (mg/l) per dose (mg) of aflibercept of about 0.388 (±0.0328) mg/l/mg;
   - The maximum level of adjusted bound aflibercept in the plasma is reached about 14 days after the first dose;
   - Reaches a maximum level of about 0.387 mg/l (±0.135) adjusted bound aflibercept in the plasma;
   - Adjusted bound aflibercept in the plasma of from about 0.137 to about 0.774 mg/L;
   - Adjusted bound aflibercept in the plasma maximum (mg/l) per dose (mg) of aflibercept of about 0.483 (±0.0168) mg/l/mg;
   - Does not have anti-drug antibodies against aflibercept after 48 or 60 weeks of treatment;
   - Improvement from pre-treatment baseline in National Eye Institute Visual Function Questionnaire (NEI-VFQ) total score; and/or
   - Lack of macular edema.
91. The method of embodiment 89-90, wherein a dry retina lacks intraretinal fluid and/or subretinal fluid.
92. The method of any one of embodiments 89-91, wherein retinal drying is characterized by no intraretinal fluid (IRF) and no subretinal fluid (SRF) in the eye of the subject, after the subject has received three monthly doses of the VEGF receptor fusion protein.
93. The method of any one of embodiments 89-92, wherein by 4, 8, 12, 16, 20, 24, 28, 32, 36, 40, 44, 48, 52, 56 or 60 weeks from start of treatment is about 48 weeks or 60 weeks from start of treatment.
94. The method of any one of embodiments 1-93 wherein
   - 1 initial dose, 2 secondary doses and 3 tertiary doses of said ≥ 8 mg VEGF receptor fusion protein are administered to the subject in the first year;
   - wherein 1 initial dose, 2 secondary doses and 2 tertiary doses of said ≥ 8 mg VEGF receptor fusion protein are administered to the subject in the first year; or
   - wherein 1 initial dose, 2 secondary doses and 3 tertiary doses of said ≥ 8 mg VEGF receptor fusion protein are administered to the subject in the first year followed by 2 -4 tertiary doses in the second year.
95. The method of any one of embodiments 1-94 wherein the interval between doses are adjusted (increased/maintained/reduced) based on visual and/or anatomic outcomes.
96. The method of any one of embodiments 1-95 further including one or more periods of *pro re nata* (PRN), capped PRN or treat and extend (T&E) dosing.
97. The method of any one of embodiments 1-96 wherein the VEGF receptor fusion protein is aflibercept.
98. A kit comprising
   - a container comprising VEGF receptor fusion protein; and
   - Instruction for use of VEGF receptor fusion protein,

   wherein the container is a vial or a pre-filled syringe,
   wherein the container comprises ≥ 100 mg/mL VEGF receptor fusion protein, or wherein the container comprises ≥ 114.3 mg/mL VEGF receptor fusion protein,
   wherein the instruction comprises instruction for the administration of aflibercept to DME and/or DR patients,
   wherein the instruction comprises instruction that aflibercept 8 mg treatment is initiated with 1 injection per month (every 4 weeks) for 3 consecutive doses,
   wherein the instruction comprises instruction that after the initial 3 consecutive doses the injection interval may be lengthened up to every 16 week or every 20 week, and
   wherein the instruction comprises instruction that the treatment interval may be adjusted based on the physician's judgement of visual and/or anatomic outcomes.

## Claims

1. Aflibercept for use a method of treating diabetic macular edema or diabetic retinopathy in a subject which was pre-treated with 2 mg aflibercept, the method comprising administering to an eye of the subject a single initial dose of about 8 mg aflibercept by intravitreal injection, followed by one or more secondary doses of about 8 mg of aflibercept administered by intravitreal injection, followed by one or more tertiary doses of about 8 mg aflibercept administered by intravitreal injection, wherein each secondary dose is administered about 4 weeks or about every 28 days ± 5 days or about every month after the immediately preceding dose and wherein each tertiary dose is administered about 8, 10, 12, 14, 16, 18 or 20 weeks after the immediately preceding dose.

2. Aflibercept for use according to claim 1, wherein the method comprises administering the one or more tertiary doses according to a treat and extend dosing regimen.

3. Aflibercept for use according to claim 1 or 2, wherein the method comprises administering the one or more tertiary doses every 16 weeks or about every 4 months or about every 112 days ± 5 days.

4. Aflibercept for use according to claim 1 or 2, wherein the method comprises administering the one or more tertiary doses every 12 weeks, about every 3 months, about every 90 days, 84 days, 84 + 5 days or 90 + 5 days.

5. Aflibercept for use of any one of the preceding claims wherein two secondary doses are administered.

6. Aflibercept for use according to any one of the preceding claims, wherein the subject previously received aflibercept according to a 2q8 dosing regimen comprising 5 initial monthly doses followed by one or more maintenance doses every 8 weeks.

7. Aflibercept for use according to any one of the preceding claims wherein the method is for treating diabetic retinopathy in a subject in need thereof.

8. Aflibercept for use according to any one of the preceding claims, wherein the method is for treating diabetic macular edema in a subject in need thereof.

9. Aflibercept for use according to any one of the preceding claims, wherein the 8 mg aflibercept is administered to both eyes of the subject.

10. Aflibercept for use according to any one of the preceding claims, wherein the 8 mg aflibercept is administered from a syringe or pre-filled syringe.

11. Aflibercept for use according to any one of the preceding claims, wherein the 8 mg aflibercept is in an aqueous pharmaceutical formulation comprising 114.3 mg/mL aflibercept.

12. Aflibercept for use according to any one of the preceding claims, wherein the 8 mg aflibercept is administered in a volume of about 70 ± 4 or 5 microliters.

13. Aflibercept for use according to any one of the preceding claims, further comprising monitoring the subject during said treatment for conjunctival hemorrhage, cataract, vitreous detachment, vitreous floaters, corneal epithelium defect and/or increased intraocular pressure.
